# EUROPEAN PATENT APPLICATION

(11) **EP 2 138 589 A1**
(43) Date of publication of application: **30.12.2009**
(21) Application number: 08290628.0
(22) Date of filing: 27.06.2008
(51) Int. Cl.: C12Q 1/68

(54) **Molecular signature of liver tumor grade and use to evaluate prognosis and therapeutic regimen**

(71) Applicant: Institut Pasteur, 75724 Paris Cédex 15 (FR); Centre National de la Recherche Scientifique, 75794 Paris Cedex 16 (FR); Institut National de la Santé et de la Recherche Médicale (INSERM), 75654 Paris Cedex 13 (FR)
(72) Inventor: Buendia, Marie Annick, 94170 Le Perreux sur Marne (FR); Armengol Niell, Carolina, 17300 Blanes (ES); Cairo, Stefano, 75013 Paris (FR); de Reynies, Aurélien, 75005 Paris (FR)
(74) Representative: Desaix, Anne

(57) **Abstract**

The present invention concerns a method to determine the gene expression profile on a sample previously obtained from a patient diagnosed for a liver tumor, comprising assaying the expression of a set of genes in this sample and determining the gene expression profile (signature). In a particular embodiment, said method enables to determine the grade of the liver tumor, such as hepatoblastoma (HB) or a hepatocellular carcinoma (HCC). The invention is also directed to kits comprising a plurality of pairs of primers or a plurality of probes specific for a set of genes, as well as to solid support or composition comprising a set of probes specific for a set of genes. These methods are useful to determine the grade of a liver tumor in a sample obtained from a patient, to determine the risk of developing metastasis and/or to define the therapeutic regimen to apply to a patient.

SEQUENCE LISTING

<110>  INSTITUT PASTEUR
       CENTRE NATIONAL DE LA RECHERCHE SCIENTIFIQUE (C.N.R.S.)
       INSTITUT NATIONAL DE SANTE ET DE LA RECHERCHE MEDICALE
       (INSERM)

<120>  MOLECULAR SIGNATURE OF LIVER TUMOR GRADE AND USE TO EVALUATE
       PROGNOSIS AND THERAPEUTIC REGIMEN

<130>  B07583 - AD/LV/KN

<160>  140

<170>  PatentIn version 3.3

<210>  1
<211>  2014
<212>  DNA
<213>  Homo sapiens


<220>
<221>  CDS
<222>  (31)..(1857)
<223>  AFP mRNA

<400>  1
   ccactgccaa taacaaaata actagcaacc atg aag tgg gtg gaa tca att ttt      54
                                    Met Lys Trp Val Glu Ser Ile Phe
                                    1               5
   tta att ttc cta cta aat ttt act gaa tcc aga aca ctg cat aga aat      102
   Leu Ile Phe Leu Leu Asn Phe Thr Glu Ser Arg Thr Leu His Arg Asn
       10                  15                  20
   gaa tat gga ata gct tcc ata ttg gat tct tac caa tgt act gca gag      150
   Glu Tyr Gly Ile Ala Ser Ile Leu Asp Ser Tyr Gln Cys Thr Ala Glu
   25                  30                  35                  40
   ata agt tta gct gac ctg gct acc ata ttt ttt gcc cag ttt gtt caa      198
   Ile Ser Leu Ala Asp Leu Ala Thr Ile Phe Phe Ala Gln Phe Val Gln
                   45                  50                  55
   gaa gcc act tac aag gaa gta agc aaa atg gtg aaa gat gca ttg act      246
   Glu Ala Thr Tyr Lys Glu Val Ser Lys Met Val Lys Asp Ala Leu Thr
               60                  65                  70
   gca att gag aaa ccc act gga gat gaa cag tct tca ggg tgt tta gaa      294
   Ala Ile Glu Lys Pro Thr Gly Asp Glu Gln Ser Ser Gly Cys Leu Glu
           75                  80                  85
   aac cag cta cct gcc ttt ctg gaa gaa ctt tgc cat gag aaa gaa att      342
   Asn Gln Leu Pro Ala Phe Leu Glu Glu Leu Cys His Glu Lys Glu Ile
       90                  95                  100
   ttg gag aag tac gga cat tca gac tgc tgc agc caa agt gaa gag gga      390
   Leu Glu Lys Tyr Gly His Ser Asp Cys Cys Ser Gln Ser Glu Glu Gly
   105                 110                 115                 120
   aga cat aac tgt ttt ctt gca cac aaa aag ccc act cca gca tcg atc      438
   Arg His Asn Cys Phe Leu Ala His Lys Lys Pro Thr Pro Ala Ser Ile
                   125                 130                 135
   cca ctt ttc caa gtt cca gaa cct gtc aca agc tgt gaa gca tat gaa      486
   Pro Leu Phe Gln Val Pro Glu Pro Val Thr Ser Cys Glu Ala Tyr Glu
               140                 145                 150
   gaa gac agg gag aca ttc atg aac aaa ttc att tat gag ata gca aga      534
   Glu Asp Arg Glu Thr Phe Met Asn Lys Phe Ile Tyr Glu Ile Ala Arg
           155                 160                 165
   agg cat ccc ttc ctg tat gca cct aca att ctt ctt tgg gct gct cgc      582
   Arg His Pro Phe Leu Tyr Ala Pro Thr Ile Leu Leu Trp Ala Ala Arg
       170                 175                 180
   tat gac aaa ata att cca tct tgc tgc aaa gct gaa aat gca gtt gaa      630
   Tyr Asp Lys Ile Ile Pro Ser Cys Cys Lys Ala Glu Asn Ala Val Glu
   185                 190                 195                 200
   tgc ttc caa aca aag gca gca aca gtt aca aaa gaa tta aga gaa agc      678
   Cys Phe Gln Thr Lys Ala Ala Thr Val Thr Lys Glu Leu Arg Glu Ser
                   205                 210                 215
   agc ttg tta aat caa cat gca tgt gca gta atg aaa aat ttt ggg acc      726
   Ser Leu Leu Asn Gln His Ala Cys Ala Val Met Lys Asn Phe Gly Thr
               220                 225                 230
   cga act ttc caa gcc ata act gtt act aaa ctg agt cag aag ttt acc      774
   Arg Thr Phe Gln Ala Ile Thr Val Thr Lys Leu Ser Gln Lys Phe Thr
           235                 240                 245
   aaa gtt aat ttt act gaa atc cag aaa cta gtc ctg gat gtg gcc cat      822
   Lys Val Asn Phe Thr Glu Ile Gln Lys Leu Val Leu Asp Val Ala His
       250                 255                 260
   gta cat gag cac tgt tgc aga gga gat gtg ctg gat tgt ctg cag gat      870
   Val His Glu His Cys Cys Arg Gly Asp Val Leu Asp Cys Leu Gln Asp
   265                 270                 275                 280
   ggg gaa aaa atc atg tcc tac ata tgt tct caa caa gac act ctg tca      918
   Gly Glu Lys Ile Met Ser Tyr Ile Cys Ser Gln Gln Asp Thr Leu Ser
                   285                 290                 295
   aac aaa ata aca gaa tgc tgc aaa ctg acc acg ctg gaa cgt ggt caa      966
   Asn Lys Ile Thr Glu Cys Cys Lys Leu Thr Thr Leu Glu Arg Gly Gln
               300                 305                 310
   tgt ata att cat gca gaa aat gat gaa aaa cct gaa ggt cta tct cca      1014
   Cys Ile Ile His Ala Glu Asn Asp Glu Lys Pro Glu Gly Leu Ser Pro
           315                 320                 325
   aat cta aac agg ttt tta gga gat aga gat ttt aac caa ttt tct tca      1062
   Asn Leu Asn Arg Phe Leu Gly Asp Arg Asp Phe Asn Gln Phe Ser Ser
       330                 335                 340
   ggg gaa aaa aat atc ttc ttg gca agt ttt gtt cat gaa tat tca aga      1110
   Gly Glu Lys Asn Ile Phe Leu Ala Ser Phe Val His Glu Tyr Ser Arg
   345                 350                 355                 360
   aga cat cct cag ctt gct gtc tca gta att cta aga gtt gct aaa gga      1158
   Arg His Pro Gln Leu Ala Val Ser Val Ile Leu Arg Val Ala Lys Gly
                   365                 370                 375
   tac cag gag tta ttg gag aag tgt ttc cag act gaa aac cct ctt gaa      1206
   Tyr Gln Glu Leu Leu Glu Lys Cys Phe Gln Thr Glu Asn Pro Leu Glu
               380                 385                 390
   tgc caa gat aaa gga gaa gaa gaa tta cag aaa tac atc cag gag agc      1254
   Cys Gln Asp Lys Gly Glu Glu Glu Leu Gln Lys Tyr Ile Gln Glu Ser
           395                 400                 405
   caa gca ttg gca aag cga agc tgc ggc ctc ttc cag aaa cta gga gaa      1302
   Gln Ala Leu Ala Lys Arg Ser Cys Gly Leu Phe Gln Lys Leu Gly Glu
       410                 415                 420
   tat tac tta caa aat gcg ttt ctc gtt gct tac aca aag aaa gcc ccc      1350
   Tyr Tyr Leu Gln Asn Ala Phe Leu Val Ala Tyr Thr Lys Lys Ala Pro
   425                 430                 435                 440
   cag ctg acc tcg tcg gag ctg atg gcc atc acc aga aaa atg gca gcc      1398
   Gln Leu Thr Ser Ser Glu Leu Met Ala Ile Thr Arg Lys Met Ala Ala
                   445                 450                 455
   aca gca gcc act tgt tgc caa ctc agt gag gac aaa cta ttg gcc tgt      1446
   Thr Ala Ala Thr Cys Cys Gln Leu Ser Glu Asp Lys Leu Leu Ala Cys
               460                 465                 470
   ggc gag gga gcg gac att att atc gga cac tta tgt atc aga cat gaa      1494
   Gly Glu Gly Ala Asp Ile Ile Ile Gly His Leu Cys Ile Arg His Glu
           475                 480                 485
   atg act cca gta aac cct ggt gtt ggc cag tgc tgc act tct tca tat      1542
   Met Thr Pro Val Asn Pro Gly Val Gly Gln Cys Cys Thr Ser Ser Tyr
       490                 495                 500
   gcc aac agg agg cca tgc ttc agc agc ttg gtg gtg gat gaa aca tat      1590
   Ala Asn Arg Arg Pro Cys Phe Ser Ser Leu Val Val Asp Glu Thr Tyr
   505                 510                 515                 520
   gtc cct cct gca ttc tct gat gac aag ttc att ttc cat aag gat ctg      1638
   Val Pro Pro Ala Phe Ser Asp Asp Lys Phe Ile Phe His Lys Asp Leu
                   525                 530                 535
   tgc caa gct cag ggt gta gcg ctg caa aca atg aag caa gag ttt ctc      1686
   Cys Gln Ala Gln Gly Val Ala Leu Gln Thr Met Lys Gln Glu Phe Leu
               540                 545                 550
   att aac ctt gtg aag caa aag cca caa ata aca gag gaa caa ctt gag      1734
   Ile Asn Leu Val Lys Gln Lys Pro Gln Ile Thr Glu Glu Gln Leu Glu
           555                 560                 565
   gct gtc att gca gat ttc tca ggc ctg ttg gag aaa tgc tgc caa ggc      1782
   Ala Val Ile Ala Asp Phe Ser Gly Leu Leu Glu Lys Cys Cys Gln Gly
       570                 575                 580
   cag gaa cag gaa gtc tgc ttt gct gaa gag gga caa aaa ctg att tca      1830
   Gln Glu Gln Glu Val Cys Phe Ala Glu Glu Gly Gln Lys Leu Ile Ser
   585                 590                 595                 600
   aaa act cgt gct gct ttg gga gtt taa attacttcag gggaagagaa 1877
   Lys Thr Arg Ala Ala Leu Gly Val
   605
   gacaaaacga gtctttcatt cggtgtgaac ttttctcttt aattttaact gatttaacac    1937
   tttttgtgaa ttaatgaaat gataaagact tttatgtgag atttccttat cacagaaata    1997
   aaatatctcc aaatgtt                                                   2014


<210>  2
<211>  608
<212>  PRT
<213>  Homo sapiens

<400>  2

Met Lys Trp Val Glu Ser Ile Phe Leu Ile Phe Leu Leu Asn Phe Thr
1               5                   10                  15


Glu Ser Arg Thr Leu His Arg Asn Glu Tyr Gly Ile Ala Ser Ile Leu
            20                  25                  30


Asp Ser Tyr Gln Cys Thr Ala Glu Ile Ser Leu Ala Asp Leu Ala Thr

35                  40                  45

Ile Phe Phe Ala Gln Phe Val Gln Glu Ala Thr Tyr Lys Glu Val Ser
    50                  55                  60

Lys Met Val Lys Asp Ala Leu Thr Ala Ile Glu Lys Pro Thr Gly Asp
65                  70                  75                  80

Glu Gln Ser Ser Gly Cys Leu Glu Asn Gln Leu Pro Ala Phe Leu Glu
                85                  90                  95

Glu Leu Cys His Glu Lys Glu Ile Leu Glu Lys Tyr Gly His Ser Asp
            100                 105                 110

Cys Cys Ser Gln Ser Glu Glu Gly Arg His Asn Cys Phe Leu Ala His
        115                 120                 125

Lys Lys Pro Thr Pro Ala Ser Ile Pro Leu Phe Gln Val Pro Glu Pro
    130                 135                 140

Val Thr Ser Cys Glu Ala Tyr Glu Glu Asp Arg Glu Thr Phe Met Asn
145                 150                 155                 160

Lys Phe Ile Tyr Glu Ile Ala Arg Arg His Pro Phe Leu Tyr Ala Pro
                165                 170                 175

Thr Ile Leu Leu Trp Ala Ala Arg Tyr Asp Lys Ile Ile Pro Ser Cys
            180                 185                 190

Cys Lys Ala Glu Asn Ala Val Glu Cys Phe Gln Thr Lys Ala Ala Thr
        195                 200                 205

Val Thr Lys Glu Leu Arg Glu Ser Ser Leu Leu Asn Gln His Ala Cys
    210                 215                 220

Ala Val Met Lys Asn Phe Gly Thr Arg Thr Phe Gln Ala Ile Thr Val
225                 230                 235                 240

Thr Lys Leu Ser Gln Lys Phe Thr Lys Val Asn Phe Thr Glu Ile Gln
                245                 250                 255

Lys Leu Val Leu Asp Val Ala His Val His Glu His Cys Cys Arg Gly
            260                 265                 270

Asp Val Leu Asp Cys Leu Gln Asp Gly Glu Lys Ile Met Ser Tyr Ile
        275                 280                 285

Cys Ser Gln Gln Asp Thr Leu Ser Asn Lys Ile Thr Glu Cys Cys Lys
    290                 295                 300

Leu Thr Thr Leu Glu Arg Gly Gln Cys Ile Ile His Ala Glu Asn Asp
305                 310                 315                 320

Glu Lys Pro Glu Gly Leu Ser Pro Asn Leu Asn Arg Phe Leu Gly Asp
                325                 330                 335

Arg Asp Phe Asn Gln Phe Ser Ser Gly Glu Lys Asn Ile Phe Leu Ala
            340                 345                 350

Ser Phe Val His Glu Tyr Ser Arg Arg His Pro Gln Leu Ala Val Ser
        355                 360                 365

Val Ile Leu Arg Val Ala Lys Gly Tyr Gln Glu Leu Leu Glu Lys Cys
    370                 375                 380

Phe Gln Thr Glu Asn Pro Leu Glu Cys Gln Asp Lys Gly Glu Glu Glu
385                 390                 395                 400

Leu Gln Lys Tyr Ile Gln Glu Ser Gln Ala Leu Ala Lys Arg Ser Cys
                405                 410                 415

Gly Leu Phe Gln Lys Leu Gly Glu Tyr Tyr Leu Gln Asn Ala Phe Leu
            420                 425                 430

Val Ala Tyr Thr Lys Lys Ala Pro Gln Leu Thr Ser Ser Glu Leu Met
        435                 440                 445

Ala Ile Thr Arg Lys Met Ala Ala Thr Ala Ala Thr Cys Cys Gln Leu
    450                 455                 460

Ser Glu Asp Lys Leu Leu Ala Cys Gly Glu Gly Ala Asp Ile Ile Ile
465                 470                 475                 480

Gly His Leu Cys Ile Arg His Glu Met Thr Pro Val Asn Pro Gly Val
                485                 490                 495

Gly Gln Cys Cys Thr Ser Ser Tyr Ala Asn Arg Arg Pro Cys Phe Ser
            500                 505                 510

Ser Leu Val Val Asp Glu Thr Tyr Val Pro Pro Ala Phe Ser Asp Asp
        515                 520                 525

Lys Phe Ile Phe His Lys Asp Leu Cys Gln Ala Gln Gly Val Ala Leu
    530                 535                 540

Gln Thr Met Lys Gln Glu Phe Leu Ile Asn Leu Val Lys Gln Lys Pro
545                 550                 555                 560

Gln Ile Thr Glu Glu Gln Leu Glu Ala Val Ile Ala Asp Phe Ser Gly
                565                 570                 575

Leu Leu Glu Lys Cys Cys Gln Gly Gln Glu Gln Glu Val Cys Phe Ala
            580                 585                 590

Glu Glu Gly Gln Lys Leu Ile Ser Lys Thr Arg Ala Ala Leu Gly Val
        595                 600                 605

<210>  3
<211>  2001
<212>  DNA
<213>  Homo sapiens

<220>
<221>  CDS
<222>  (45)..(1598)
<223>  ALDH2 mRNA

<400>  3
ctagctctgc tctcggtccg ctcgctgtcc gctagcccgc tgcg atg ttg cgc gct        56
                                                 Met Leu Arg Ala
                                                 1

gcc gcc cgc ttc ggg ccc cgc ctg ggc cgc cgc ctc ttg tca gcc gcc        104
Ala Ala Arg Phe Gly Pro Arg Leu Gly Arg Arg Leu Leu Ser Ala Ala
5                   10                  15                  20

gcc acc cag gcc gtg cct gcc ccc aac cag cag ccc gag gtc ttc tgc        152
Ala Thr Gln Ala Val Pro Ala Pro Asn Gln Gln Pro Glu Val Phe Cys
                25                  30                  35

aac cag att ttc ata aac aat gaa tgg cac gat gcc gtc agc agg aaa        200
Asn Gln Ile Phe Ile Asn Asn Glu Trp His Asp Ala Val Ser Arg Lys
            40                  45                  50

aca ttc ccc acc gtc aat ccg tcc act gga gag gtc atc tgt cag gta        248
Thr Phe Pro Thr Val Asn Pro Ser Thr Gly Glu Val Ile Cys Gln Val
        55                  60                  65

gct gaa ggg gac aag gaa gat gtg gac aag gca gtg aag gcc gcc cgg        296
Ala Glu Gly Asp Lys Glu Asp Val Asp Lys Ala Val Lys Ala Ala Arg
    70                  75                  80

gcc gcc ttc cag ctg ggc tca cct tgg cgc cgc atg gac gca tca cac        344
Ala Ala Phe Gln Leu Gly Ser Pro Trp Arg Arg Met Asp Ala Ser His
85                  90                  95                  100

agg ggc cgg ctg ctg aac cgc ctg gcc gat ctg atc gag cgg gac cgg        392
Arg Gly Arg Leu Leu Asn Arg Leu Ala Asp Leu Ile Glu Arg Asp Arg
                105                 110                 115

acc tac ctg gcg gcc ttg gag acc ctg gac aat ggc aag ccc tat gtc        440
Thr Tyr Leu Ala Ala Leu Glu Thr Leu Asp Asn Gly Lys Pro Tyr Val
            120                 125                 130

atc tcc tac ctg gtg gat ttg gac atg gtc ctc aaa tgt ctc cgg tat        488
Ile Ser Tyr Leu Val Asp Leu Asp Met Val Leu Lys Cys Leu Arg Tyr
        135                 140                 145

tat gcc ggc tgg gct gat aag tac cac ggg aaa acc atc ccc att gac        536
Tyr Ala Gly Trp Ala Asp Lys Tyr His Gly Lys Thr Ile Pro Ile Asp
    150                 155                 160

gga gac ttc ttc agc tac aca cgc cat gaa cct gtg ggg gtg tgc ggg        584
Gly Asp Phe Phe Ser Tyr Thr Arg His Glu Pro Val Gly Val Cys Gly
165                 170                 175                 180

cag atc att ccg tgg aat ttc ccg ctc ctg atg caa gca tgg aag ctg        632
Gln Ile Ile Pro Trp Asn Phe Pro Leu Leu Met Gln Ala Trp Lys Leu
                185                 190                 195

ggc cca gcc ttg gca act gga aac gtg gtt gtg atg aag gta gct gag     680
Gly Pro Ala Leu Ala Thr Gly Asn Val Val Val Met Lys Val Ala Glu
        200                 205                 210

cag aca ccc ctc acc gcc ctc tat gtg gcc aac ctg atc aag gag gct     728
Gln Thr Pro Leu Thr Ala Leu Tyr Val Ala Asn Leu Ile Lys Glu Ala
        215                 220                 225

ggc ttt ccc cct ggt gtg gtc aac att gtg cct gga ttt ggc ccc acg     776
Gly Phe Pro Pro Gly Val Val Asn Ile Val Pro Gly Phe Gly Pro Thr
    230                 235                 240

gct ggg gcc gcc att gcc tcc cat gag gat gtg gac aaa gtg gca ttc     824
Ala Gly Ala Ala Ile Ala Ser His Glu Asp Val Asp Lys Val Ala Phe
245                 250                 255                 260

aca ggc tcc act gag att ggc cgc gta atc cag gtt gct gct ggg agc     872
Thr Gly Ser Thr Glu Ile Gly Arg Val Ile Gln Val Ala Ala Gly Ser
                265                 270                 275

agc aac ctc aag aga gtg acc ttg gag ctg ggg ggg aag agc ccc aac     920
Ser Asn Leu Lys Arg Val Thr Leu Glu Leu Gly Gly Lys Ser Pro Asn
            280                 285                 290

atc atc atg tca gat gcc gat atg gat tgg gcc gtg gaa cag gcc cac     968
Ile Ile Met Ser Asp Ala Asp Met Asp Trp Ala Val Glu Gln Ala His
        295                 300                 305

ttc gcc ctg ttc ttc aac cag ggc cag tgc tgc tgt gcc ggc tcc cgg     1016
Phe Ala Leu Phe Phe Asn Gln Gly Gln Cys Cys Cys Ala Gly Ser Arg
    310                 315                 320

acc ttc gtg cag gag gac atc tat gat gag ttt gtg gag cgg agc gtt     1064
Thr Phe Val Gln Glu Asp Ile Tyr Asp Glu Phe Val Glu Arg Ser Val
325                 330                 335                 340

gcc cgg gcc aag tct cgg gtg gtc ggg aac ccc ttt gat agc aag acc     1112
Ala Arg Ala Lys Ser Arg Val Val Gly Asn Pro Phe Asp Ser Lys Thr
                345                 350                 355

gag cag ggg ccg cag gtg gat gaa act cag ttt aag aag atc ctc ggc     1160
Glu Gln Gly Pro Gln Val Asp Glu Thr Gln Phe Lys Lys Ile Leu Gly
            360                 365                 370

tac atc aac acg ggg aag caa gag ggg gcg aag ctg ctg tgt ggt ggg     1208
Tyr Ile Asn Thr Gly Lys Gln Glu Gly Ala Lys Leu Leu Cys Gly Gly
        375                 380                 385

ggc att gct gct gac cgt ggt tac ttc atc cag ccc act gtg ttt gga     1256
Gly Ile Ala Ala Asp Arg Gly Tyr Phe Ile Gln Pro Thr Val Phe Gly
    390                 395                 400

gat gtg cag gat ggc atg acc atc gcc aag gag gag atc ttc ggg cca     1304
Asp Val Gln Asp Gly Met Thr Ile Ala Lys Glu Glu Ile Phe Gly Pro
405                 410                 415                 420

gtg atg cag atc ctg aag ttc aag acc ata gag gag gtt gtt ggg aga     1352
Val Met Gln Ile Leu Lys Phe Lys Thr Ile Glu Glu Val Val Gly Arg
                425                 430                 435

gcc aac aat tcc acg tac ggg ctg gcc gca gct gtc ttc aca aag gat     1400
Ala Asn Asn Ser Thr Tyr Gly Leu Ala Ala Ala Val Phe Thr Lys Asp
            440                 445                 450

ttg gac aag gcc aat tac ctg tcc cag gcc ctc cag gcg ggc act gtg     1448
Leu Asp Lys Ala Asn Tyr Leu Ser Gln Ala Leu Gln Ala Gly Thr Val
        455                 460                 465

tgg gtc aac tgc tat gat gtg ttt gga gcc cag tca ccc ttt ggt ggc     1496

Trp Val Asn Cys Tyr Asp Val Phe Gly Ala Gln Ser Pro Phe Gly Gly
    470                 475                 480

tac aag atg tcg ggg agt ggc cgg gag ttg ggc gag tac ggg ctg cag      1544
Tyr Lys Met Ser Gly Ser Gly Arg Glu Leu Gly Glu Tyr Gly Leu Gln
485                 490                 495                 500

gca tac act gaa gtg aaa act gtc aca gtc aaa gtg cct cag aag aac      1592
Ala Tyr Thr Glu Val Lys Thr Val Thr Val Lys Val Pro Gln Lys Asn
                505                 510                 515

tca taa gaatcatgca agcttcctcc ctcagccatt gatggaaagt tcagcaagat      1648
Ser

cagcaacaaa accaagaaaa atgatccttg cgtgctgaat atctgaaaag agaaattttt    1708

cctacaaaat ctcttgggtc aagaaagttc tagaatttga attgataaac atggtgggtt    1768

ggctgagggt aagagtatat gaggaacctt ttaaacgaca acaatactgc tagctttcag    1828

gatgattttt aaaaaataga ttcaaatgtg ttatcctctc tctgaaacgc ttcctataac    1888

tcgagtttat aggggaagaa aaagctattg tttacaatta tatcaccatt aaggcaactg    1948

ctacaccctg ctttgtattc tgggctaaga ttcattaaaa actagctgct ctt           2001

<210>  4
<211>  517
<212>  PRT
<213>  Homo sapiens

<400>  4

Met Leu Arg Ala Ala Ala Arg Phe Gly Pro Arg Leu Gly Arg Arg Leu
1               5                   10                  15

Leu Ser Ala Ala Ala Thr Gln Ala Val Pro Ala Pro Asn Gln Gln Pro
            20                  25                  30

Glu Val Phe Cys Asn Gln Ile Phe Ile Asn Asn Glu Trp His Asp Ala
        35                  40                  45

Val Ser Arg Lys Thr Phe Pro Thr Val Asn Pro Ser Thr Gly Glu Val
    50                  55                  60

Ile Cys Gln Val Ala Glu Gly Asp Lys Glu Asp Val Asp Lys Ala Val
65                  70                  75                  80

Lys Ala Ala Arg Ala Ala Phe Gln Leu Gly Ser Pro Trp Arg Arg Met
                85                  90                  95

Asp Ala Ser His Arg Gly Arg Leu Leu Asn Arg Leu Ala Asp Leu Ile
            100                 105                 110

Glu Arg Asp Arg Thr Tyr Leu Ala Ala Leu Glu Thr Leu Asp Asn Gly
        115                 120                 125

Lys Pro Tyr Val Ile Ser Tyr Leu Val Asp Leu Asp Met Val Leu Lys
    130                 135                 140

Cys Leu Arg Tyr Tyr Ala Gly Trp Ala Asp Lys Tyr His Gly Lys Thr
145                 150                 155                 160

Ile Pro Ile Asp Gly Asp Phe Phe Ser Tyr Thr Arg His Glu Pro Val
                165                 170                 175

Gly Val Cys Gly Gln Ile Ile Pro Trp Asn Phe Pro Leu Leu Met Gln
            180                 185                 190

Ala Trp Lys Leu Gly Pro Ala Leu Ala Thr Gly Asn Val Val Val Met
        195                 200                 205

Lys Val Ala Glu Gln Thr Pro Leu Thr Ala Leu Tyr Val Ala Asn Leu
    210                 215                 220

Ile Lys Glu Ala Gly Phe Pro Pro Gly Val Val Asn Ile Val Pro Gly
225                 230                 235                 240

Phe Gly Pro Thr Ala Gly Ala Ala Ile Ala Ser His Glu Asp Val Asp
                245                 250                 255

Lys Val Ala Phe Thr Gly Ser Thr Glu Ile Gly Arg Val Ile Gln Val
            260                 265                 270

Ala Ala Gly Ser Ser Asn Leu Lys Arg Val Thr Leu Glu Leu Gly Gly
        275                 280                 285

Lys Ser Pro Asn Ile Ile Met Ser Asp Ala Asp Met Asp Trp Ala Val
    290                 295                 300

Glu Gln Ala His Phe Ala Leu Phe Phe Asn Gln Gly Gln Cys Cys Cys
305                 310                 315                 320

Ala Gly Ser Arg Thr Phe Val Gln Glu Asp Ile Tyr Asp Glu Phe Val
                325                 330                 335

Glu Arg Ser Val Ala Arg Ala Lys Ser Arg Val Val Gly Asn Pro Phe
            340                 345                 350

Asp Ser Lys Thr Glu Gln Gly Pro Gln Val Asp Glu Thr Gln Phe Lys
        355                 360                 365

Lys Ile Leu Gly Tyr Ile Asn Thr Gly Lys Gln Glu Gly Ala Lys Leu
    370                 375                 380

Leu Cys Gly Gly Gly Ile Ala Ala Asp Arg Gly Tyr Phe Ile Gln Pro
385                 390                 395                 400

Thr Val Phe Gly Asp Val Gln Asp Gly Met Thr Ile Ala Lys Glu Glu
                405                 410                 415

Ile Phe Gly Pro Val Met Gln Ile Leu Lys Phe Lys Thr Ile Glu Glu
            420                 425                 430

Val Val Gly Arg Ala Asn Asn Ser Thr Tyr Gly Leu Ala Ala Ala Val
        435                 440                 445

Phe Thr Lys Asp Leu Asp Lys Ala Asn Tyr Leu Ser Gln Ala Leu Gln
    450                 455                 460

Ala Gly Thr Val Trp Val Asn Cys Tyr Asp Val Phe Gly Ala Gln Ser
465                 470                 475                 480

Pro Phe Gly Gly Tyr Lys Met Ser Gly Ser Gly Arg Glu Leu Gly Glu
                485                 490                 495

Tyr Gly Leu Gln Ala Tyr Thr Glu Val Lys Thr Val Thr Val Lys Val
            500                 505                 510

Pro Gln Lys Asn Ser
        515

<210>  5
<211>  928
<212>  DNA
<213>  Homo sapiens

<220>
<221>  CDS
<222>  (97)..(768)
<223>  APCS mRNA

<400>  5
gggcatgaat atcagacgct agggggacag ccactgtgtt gtctgctacc ctcatcctgg      60

tcactgcttc tgctataaca gccctaggcc aggaat atg aac aag ccg ctg ctt      114
                                        Met Asn Lys Pro Leu Leu
                                        1               5

tgg atc tct gtc ctc acc agc ctc ctg gaa gcc ttt gct cac aca gac      162
Trp Ile Ser Val Leu Thr Ser Leu Leu Glu Ala Phe Ala His Thr Asp
            10                  15                  20

ctc agt ggg aag gtg ttt gta ttt cct aga gaa tct gtt act gat cat      210
Leu Ser Gly Lys Val Phe Val Phe Pro Arg Glu Ser Val Thr Asp His
        25                  30                  35

gta aac ttg atc aca ccg ctg gag aag cct cta cag aac ttt acc ttg      258
Val Asn Leu Ile Thr Pro Leu Glu Lys Pro Leu Gln Asn Phe Thr Leu
    40                  45                  50

tgt ttt cga gcc tat agt gat ctc tct cgt gcc tac agc ctc ttc tcc      306
Cys Phe Arg Ala Tyr Ser Asp Leu Ser Arg Ala Tyr Ser Leu Phe Ser
55                  60                  65                  70

tac aat acc caa ggc agg gat aat gag cta cta gtt tat aaa gaa aga      354
Tyr Asn Thr Gln Gly Arg Asp Asn Glu Leu Leu Val Tyr Lys Glu Arg
                75                  80                  85

gtt gga gag tat agt cta tac att gga aga cac aaa gtt aca tcc aaa      402
Val Gly Glu Tyr Ser Leu Tyr Ile Gly Arg His Lys Val Thr Ser Lys

                90                  95                  100

gtt atc gaa aag ttc ccg gct cca gtg cac atc tgt gtg agc tgg gag        450
Val Ile Glu Lys Phe Pro Ala Pro Val His Ile Cys Val Ser Trp Glu
        105                 110                 115

tcc tca tca ggt att gct gaa ttt tgg atc aat ggg aca cct ttg gtg        498
Ser Ser Ser Gly Ile Ala Glu Phe Trp Ile Asn Gly Thr Pro Leu Val
    120                 125                 130

aaa aag ggt ctg cga cag ggt tac ttt gta gaa gct cag ccc aag att        546
Lys Lys Gly Leu Arg Gln Gly Tyr Phe Val Glu Ala Gln Pro Lys Ile
135                 140                 145                 150

gtc ctg ggg cag gaa cag gat tcc tat ggg ggc aag ttt gat agg agc        594
Val Leu Gly Gln Glu Gln Asp Ser Tyr Gly Gly Lys Phe Asp Arg Ser
                155                 160                 165

cag tcc ttt gtg gga gag att ggg gat ttg tac atg tgg gac tct gtg        642
Gln Ser Phe Val Gly Glu Ile Gly Asp Leu Tyr Met Trp Asp Ser Val
            170                 175                 180

ctg ccc cca gaa aat atc ctg tct gcc tat cag ggt acc cct ctc cct        690
Leu Pro Pro Glu Asn Ile Leu Ser Ala Tyr Gln Gly Thr Pro Leu Pro
        185                 190                 195

gcc aat atc ctg gac tgg cag gct ctg aac tat gaa atc aga gga tat        738
Ala Asn Ile Leu Asp Trp Gln Ala Leu Asn Tyr Glu Ile Arg Gly Tyr
    200                 205                 210

gtc atc atc aaa ccc ttg gtg tgg gtc tga ggtcttgact caacgagagc        788
Val Ile Ile Lys Pro Leu Val Trp Val
215                 220

acttgaaaat gaaatgactg tctaagagat ctggtcaaag caactggata ctagatctta        848

catctgcagc tctttcttct ttgaatttcc tatctgtatg tctgcctaat taaaaaaata        908

tatattgtat tatgctacct                                                  928

<210>  6
<211>  223
<212>  PRT
<213>  Homo sapiens

<400>  6

Met Asn Lys Pro Leu Leu Trp Ile Ser Val Leu Thr Ser Leu Leu Glu
1               5                   10                  15

Ala Phe Ala His Thr Asp Leu Ser Gly Lys Val Phe Val Phe Pro Arg
            20                  25                  30

Glu Ser Val Thr Asp His Val Asn Leu Ile Thr Pro Leu Glu Lys Pro
        35                  40                  45

Leu Gln Asn Phe Thr Leu Cys Phe Arg Ala Tyr Ser Asp Leu Ser Arg
    50                  55                  60

Ala Tyr Ser Leu Phe Ser Tyr Asn Thr Gln Gly Arg Asp Asn Glu Leu
65                  70                  75                  80

Leu Val Tyr Lys Glu Arg Val Gly Glu Tyr Ser Leu Tyr Ile Gly Arg

85                  90                  95

His Lys Val Thr Ser Lys Val Ile Glu Lys Phe Pro Ala Pro Val His
            100                 105                 110

Ile Cys Val Ser Trp Glu Ser Ser Ser Gly Ile Ala Glu Phe Trp Ile
        115                 120                 125

Asn Gly Thr Pro Leu Val Lys Lys Gly Leu Arg Gln Gly Tyr Phe Val
    130                 135                 140

Glu Ala Gln Pro Lys Ile Val Leu Gly Gln Glu Gln Asp Ser Tyr Gly
145                 150                 155                 160

Gly Lys Phe Asp Arg Ser Gln Ser Phe Val Gly Glu Ile Gly Asp Leu
                165                 170                 175

Tyr Met Trp Asp Ser Val Leu Pro Pro Glu Asn Ile Leu Ser Ala Tyr
            180                 185                 190

Gln Gly Thr Pro Leu Pro Ala Asn Ile Leu Asp Trp Gln Ala Leu Asn
        195                 200                 205

Tyr Glu Ile Arg Gly Tyr Val Ile Ile Lys Pro Leu Val Trp Val
    210                 215                 220

<210>  7
<211>  577
<212>  DNA
<213>  Homo sapiens

<220>
<221>  CDS
<222>  (5)..(388)
<223>  APOC4 mRNA

<400>  7
agaa atg tcc ctc ctc aga aac agg ctc cag gcc ctg cct gcc ctg tgc      49
     Met Ser Leu Leu Arg Asn Arg Leu Gln Ala Leu Pro Ala Leu Cys
     1               5                   10                  15

ctc tgc gtg ctg gtc ctg gcc tgc att ggg gca tgc cag cca gag gcc      97
Leu Cys Val Leu Val Leu Ala Cys Ile Gly Ala Cys Gln Pro Glu Ala
                20                  25                  30

cag gaa gga acc ctg agc ccc cca cca aag cta aag atg agt cgc tgg      145
Gln Glu Gly Thr Leu Ser Pro Pro Pro Lys Leu Lys Met Ser Arg Trp
            35                  40                  45

agc ctg gtg agg ggc agg atg aag gag ctg ctg gag aca gtg gtg aac      193
Ser Leu Val Arg Gly Arg Met Lys Glu Leu Leu Glu Thr Val Val Asn
        50                  55                  60

agg acc aga gac ggg tgg caa tgg ttc tgg agc ccg agc acc ttc cgg      241
Arg Thr Arg Asp Gly Trp Gln Trp Phe Trp Ser Pro Ser Thr Phe Arg
    65                  70                  75

ggc ttc atg cag acc tac tat gac gac cac ctg agg gac ctg ggt ccg      289
Gly Phe Met Gln Thr Tyr Tyr Asp Asp His Leu Arg Asp Leu Gly Pro

80                  85                  90                  95

ctc acc aag gcc tgg ttc ctc gaa tcc aaa gac agc ctc ttg aag aag      337
Leu Thr Lys Ala Trp Phe Leu Glu Ser Lys Asp Ser Leu Leu Lys Lys
                100                 105                 110

acc cac agc ctg tgc ccc agg ctt gtc tgt ggg gac aag gac cag ggt      385
Thr His Ser Leu Cys Pro Arg Leu Val Cys Gly Asp Lys Asp Gln Gly
            115                 120                 125

taa aatgttcata aaagccaggt gtggttgtgg cgggtgcctg tagtcccagc           438

tactcaggag gctgaggtag gatgatggct tgagcccagg agttcgagac cagcctgggc    498

aacacagcga gatctcttgg gggtaaaaca aaaagaaaaa aaaaagttca tacttctcca    558

ataaataaag tctcacctg                                                 577

<210>  8
<211>  127
<212>  PRT
<213>  Homo sapiens

<400>  8

Met Ser Leu Leu Arg Asn Arg Leu Gln Ala Leu Pro Ala Leu Cys Leu
1               5                   10                  15

Cys Val Leu Val Leu Ala Cys Ile Gly Ala Cys Gln Pro Glu Ala Gln
            20                  25                  30

Glu Gly Thr Leu Ser Pro Pro Pro Lys Leu Lys Met Ser Arg Trp Ser
        35                  40                  45

Leu Val Arg Gly Arg Met Lys Glu Leu Leu Glu Thr Val Val Asn Arg
    50                  55                  60

Thr Arg Asp Gly Trp Gln Trp Phe Trp Ser Pro Ser Thr Phe Arg Gly
65                  70                  75                  80

Phe Met Gln Thr Tyr Tyr Asp Asp His Leu Arg Asp Leu Gly Pro Leu
                85                  90                  95

Thr Lys Ala Trp Phe Leu Glu Ser Lys Asp Ser Leu Leu Lys Lys Thr
            100                 105                 110

His Ser Leu Cys Pro Arg Leu Val Cys Gly Asp Lys Asp Gln Gly
        115                 120                 125

<210>  9
<211>  2801
<212>  DNA
<213>  Homo sapiens

<220>
<221>  CDS
<222>  (186)..(1073)
<223>  AQP9 mRNA

<400> 9
ccaccagaag acgattaagc cacagcctct aattggaacg gcatttgtac agtcagagac 60

tcttaccaga catctccagg aatctgtgag ccattgtcaa aacgtccatt ttcatctggc 120

tgtgaaagtg aggaccacaa caggtaggta ttggtagaaa caggagtcct cagagaagcc 180

ccaag atg cag cct gag gga gca gaa aag gga aaa agc ttc aag cag aga 230
Met Gln Pro Glu Gly Ala Glu Lys Gly Lys Ser Phe Lys Gln Arg
1               5                   10                  15

ctg gtc ttg aag agc agc tta gcg aaa gaa acc ctc tct gag ttc ttg 278
Leu Val Leu Lys Ser Ser Leu Ala Lys Glu Thr Leu Ser Glu Phe Leu
                20                  25                  30

ggc acg ttc atc ttg att gtc ctt gga tgt ggc tgt gtt gcc caa gct 326
Gly Thr Phe Ile Leu Ile Val Leu Gly Cys Gly Cys Val Ala Gln Ala
            35                  40                  45

att ctc agt cga gga cgt ttt gga ggg gtc atc act atc aat gtt gga 374
Ile Leu Ser Arg Gly Arg Phe Gly Gly Val Ile Thr Ile Asn Val Gly
        50                  55                  60

ttt tca atg gca gtt gca atg gcc att tat gtg gct ggc ggt gtc tct 422
Phe Ser Met Ala Val Ala Met Ala Ile Tyr Val Ala Gly Gly Val Ser
    65                  70                  75

ggt ggt cac atc aac cca gct gtg tct tta gca atg tgt ctc ttt gga 470
Gly Gly His Ile Asn Pro Ala Val Ser Leu Ala Met Cys Leu Phe Gly
80                  85                  90                  95

cgg atg aaa tgg ttc aaa ttg cca ttt tat gtg gga gcc cag ttc ttg 518
Arg Met Lys Trp Phe Lys Leu Pro Phe Tyr Val Gly Ala Gln Phe Leu
                100                 105                 110

gga gcc ttt gtg ggg gct gca acc gtc ttt ggc att tac tat gat gga 566
Gly Ala Phe Val Gly Ala Ala Thr Val Phe Gly Ile Tyr Tyr Asp Gly
            115                 120                 125

ctt atg tcc ttt gct ggt gga aaa ctg ctg atc gtg gga gaa aat gca 614
Leu Met Ser Phe Ala Gly Gly Lys Leu Leu Ile Val Gly Glu Asn Ala
        130                 135                 140

aca gca cac att ttt gca aca tac cca gct ccg tat cta tct ctg gcg 662
Thr Ala His Ile Phe Ala Thr Tyr Pro Ala Pro Tyr Leu Ser Leu Ala
    145                 150                 155

aac gca ttt gca gat caa gtg gtg gcc acc atg ata ctc ctc ata atc 710
Asn Ala Phe Ala Asp Gln Val Val Ala Thr Met Ile Leu Leu Ile Ile
160                 165                 170                 175

gtc ttt gcc atc ttt gac tcc aga aac ttg gga gcc ccc aga ggc cta 758
Val Phe Ala Ile Phe Asp Ser Arg Asn Leu Gly Ala Pro Arg Gly Leu
                180                 185                 190

gag ccc att gcc atc ggc ctc ctg att att gtc att gct tcc tcc ctg 806
Glu Pro Ile Ala Ile Gly Leu Leu Ile Ile Val Ile Ala Ser Ser Leu
            195                 200                 205

gga ctg aac agt ggc tgt gcc atg aac cca gct cga gac ctg agt ccc 854
Gly Leu Asn Ser Gly Cys Ala Met Asn Pro Ala Arg Asp Leu Ser Pro
        210                 215                 220

aga ctt ttc act gcc ttg gca ggc tgg ggg ttt gaa gtc ttc aga gct 902
Arg Leu Phe Thr Ala Leu Ala Gly Trp Gly Phe Glu Val Phe Arg Ala
    225                 230                 235

gga aac aac ttc tgg tgg att cct gta gtg ggc cct ttg gtt ggt gct 950
Gly Asn Asn Phe Trp Trp Ile Pro Val Val Gly Pro Leu Val Gly Ala

240                 245                 250                 255

gtc att gga ggc ctc atc tat gtt ctt gtc att gaa atc cac cat cca      998
Val Ile Gly Gly Leu Ile Tyr Val Leu Val Ile Glu Ile His His Pro
                260                 265                 270

gag cct gac tca gtc ttt aag aca gaa caa tct gag gac aaa cca gag      1046
Glu Pro Asp Ser Val Phe Lys Thr Glu Gln Ser Glu Asp Lys Pro Glu
            275                 280                 285

aaa tat gaa ctc agt gtc atc atg tag tggcatgctc agctctggat      1093
Lys Tyr Glu Leu Ser Val Ile Met
        290                 295

ttgcagtcag tttgggattc tcttcagaaa gatggcatct aagtgtctgt gttcttgtaa      1153

gcctgaggtg gaatccaccc agttttgtct gctagccata tgggacatct aattggaaaa      1213

gcatctgcat aaaagtttgg aaacaatgac cacttctcta ccattgtccc ccacccccac      1273

cccccagaat aacgctgact gtcccctgaa acagccttct ctcctgccct gtttatttca      1333

tcctcgatgg gaattcttgc taggtaagca ctaataactc ggcatgaaat ggtgtcacca      1393

aaaccctttt cttcagtatc gacaaagatt acattctgag taccaaccaa accctaaatt      1453

gaaagacaaa actatggttt cagtcaacat attcatgaat tagggagcta atgggttaag      1513

cttccagttc ccgctatgct actggatttg tataaatact gatattctcc aaacctagtg      1573

gtgtagggag caagagaatg cagctggaag gcacaagggg aggacattgt ggcattcaga      1633

aactgcagga gacaagatga atttgagaag ccaaatggaa tttttaatgg aaaccattta      1693

tcagattaat ctcttgctct cctgcatttt agaggacacc aattaatttc ctggtcttta      1753

gtatataata acctaaaata ccattgtaac ctcagtcatg aaaaatacat cactctgtct      1813

ttttagctca aatgtatttt cctaattgcc cacttgagaa cagacatttg acaagttata      1873

tcaacgactg tgcttgtcca ttattttaca catgccctag aagccaaaac tgaaagccac      1933

tggatcctgg tctagctgaa tcttcagagt gggaggtctc caaaaagata ttaccttatt      1993

gggcttaaca attcacaagg cactttcaca cccattatct aatttaatcc tcataatgac      2053

tatgtgaggc aaatgccaca ttgcccattt ttcagataaa gaaacaaaat cttagggaag      2113

ataagttgag ttgtccaaga gcacactgaa agttgaatgt tatctaatgc attcctctac      2173

ctttcagaag atcagtagct ggctgacaat ctttgccaaa tcttccttgc tagccagaag      2233

tggaattggc agcttctaga atatgtacac ctctggacaa aatgttcctc aatcttaaga      2293

tacaaagacc ctcattgtct gggtctattc ccacacttac tgagtacaga tgaaggaaag      2353

tggtagcaat ttaatcataa ctttcatttg ctgaaaaaca ttatgagaag gcctcccttc      2413

ctaagccacc tctggtcttg ctaagtcttg atcttgcttc ctgccagcac caaacattac      2473

attcagggga tttcctctgg ctcagtcttt tccccttgaa gttctctaat agatgttact      2533

tttgacaaaa gatcgcctat gagttacaag caccagggga tgctctacat caagggatgc      2593

accttcagtc aaactgtcaa aaagcccaga attcccaaag gcattaggtt tcccaactgc      2653

tttgtgctga tatcagaaca gcagaaatta aatgtgaaat gtttctgatg acttatgttc      2713

tacaatctat ggacatacgg gatttttttt tcttgctttg aagctacctg gatatttcct      2773

atttgaaata aaattgttcg gtcattgt                                          2801

<210>  10
<211>  295
<212>  PRT
<213>  Homo sapiens

<400>  10

Met Gln Pro Glu Gly Ala Glu Lys Gly Lys Ser Phe Lys Gln Arg Leu
1               5                   10                  15

Val Leu Lys Ser Ser Leu Ala Lys Glu Thr Leu Ser Glu Phe Leu Gly
            20                  25                  30

Thr Phe Ile Leu Ile Val Leu Gly Cys Gly Cys Val Ala Gln Ala Ile
        35                  40                  45

Leu Ser Arg Gly Arg Phe Gly Gly Val Ile Thr Ile Asn Val Gly Phe
    50                  55                  60

Ser Met Ala Val Ala Met Ala Ile Tyr Val Ala Gly Gly Val Ser Gly
65                  70                  75                  80

Gly His Ile Asn Pro Ala Val Ser Leu Ala Met Cys Leu Phe Gly Arg
                85                  90                  95

Met Lys Trp Phe Lys Leu Pro Phe Tyr Val Gly Ala Gln Phe Leu Gly
            100                 105                 110

Ala Phe Val Gly Ala Ala Thr Val Phe Gly Ile Tyr Tyr Asp Gly Leu
        115                 120                 125

Met Ser Phe Ala Gly Gly Lys Leu Leu Ile Val Gly Glu Asn Ala Thr
    130                 135                 140

Ala His Ile Phe Ala Thr Tyr Pro Ala Pro Tyr Leu Ser Leu Ala Asn
145                 150                 155                 160

Ala Phe Ala Asp Gln Val Val Ala Thr Met Ile Leu Leu Ile Ile Val
                165                 170                 175

Phe Ala Ile Phe Asp Ser Arg Asn Leu Gly Ala Pro Arg Gly Leu Glu
            180                 185                 190

Pro Ile Ala Ile Gly Leu Leu Ile Ile Val Ile Ala Ser Ser Leu Gly
        195                 200                 205

Leu Asn Ser Gly Cys Ala Met Asn Pro Ala Arg Asp Leu Ser Pro Arg
    210                 215                 220

Leu Phe Thr Ala Leu Ala Gly Trp Gly Phe Glu Val Phe Arg Ala Gly
225                 230                 235                 240

Asn Asn Phe Trp Trp Ile Pro Val Val Gly Pro Leu Val Gly Ala Val
                245                 250                 255

Ile Gly Gly Leu Ile Tyr Val Leu Val Ile Glu Ile His His Pro Glu
            260                 265                 270

Pro Asp Ser Val Phe Lys Thr Glu Gln Ser Glu Asp Lys Pro Glu Lys
        275                 280                 285

Tyr Glu Leu Ser Val Ile Met
    290                 295

<210>  11
<211>  3445
<212>  DNA
<213>  Homo sapiens

<220>
<221>  CDS
<222>  (58)..(3315)
<223>  BUB1 mRNA

<400>  11
cggcggcttc tagtttgcgg ttcaggtttg gccgctgccg gccagcgtcc tctggcc        57

atg gac acc ccg gaa aat gtc ctt cag atg ctt gaa gcc cac atg cag      105
Met Asp Thr Pro Glu Asn Val Leu Gln Met Leu Glu Ala His Met Gln
1               5                   10                  15

agc tac aag ggc aat gac cct ctt ggt gaa tgg gaa aga tac ata cag      153
Ser Tyr Lys Gly Asn Asp Pro Leu Gly Glu Trp Glu Arg Tyr Ile Gln
            20                  25                  30

tgg gta gaa gag aat ttt cct gag aat aaa gaa tac ttg ata act tta      201
Trp Val Glu Glu Asn Phe Pro Glu Asn Lys Glu Tyr Leu Ile Thr Leu
        35                  40                  45

cta gaa cat tta atg aag gaa ttt tta gat aag aag aaa tac cac aat      249
Leu Glu His Leu Met Lys Glu Phe Leu Asp Lys Lys Lys Tyr His Asn
    50                  55                  60

gac cca aga ttc atc agt tat tgt tta aaa ttt gct gag tac aac agt      297
Asp Pro Arg Phe Ile Ser Tyr Cys Leu Lys Phe Ala Glu Tyr Asn Ser
65                  70                  75                  80

gac ctc cat caa ttt ttt gag ttt ctg tac aac cat ggg att gga acc      345
Asp Leu His Gln Phe Phe Glu Phe Leu Tyr Asn His Gly Ile Gly Thr
                85                  90                  95

ctg tca tcc cct ctg tac att gcc tgg gcg ggg cat ctg gaa gcc caa      393
Leu Ser Ser Pro Leu Tyr Ile Ala Trp Ala Gly His Leu Glu Ala Gln
            100                 105                 110

gga gag ctg cag cat gcc agt gct gtc ctt cag aga gga att caa aac      441
Gly Glu Leu Gln His Ala Ser Ala Val Leu Gln Arg Gly Ile Gln Asn
        115                 120                 125

cag gct gaa ccc aga gag ttc ctg caa caa caa tac agg tta ttt cag      489
Gln Ala Glu Pro Arg Glu Phe Leu Gln Gln Gln Tyr Arg Leu Phe Gln
    130                 135                 140

aca cgc ctc act gaa acc cat ttg cca gct caa gct aga acc tca gaa      537

Thr Arg Leu Thr Glu Thr His Leu Pro Ala Gln Ala Arg Thr Ser Glu
145                 150                 155                 160

cct ctg cat aat gtt cag gtt tta aat caa atg ata aca tca aaa tca      585
Pro Leu His Asn Val Gln Val Leu Asn Gln Met Ile Thr Ser Lys Ser
                165                 170                 175

aat cca gga aat aac atg gcc tgc att tct aag aat cag ggt tca gag      633
Asn Pro Gly Asn Asn Met Ala Cys Ile Ser Lys Asn Gln Gly Ser Glu
            180                 185                 190

ctt tct gga gtg ata tct tca gct tgt gat aaa gag tca aat atg gaa      681
Leu Ser Gly Val Ile Ser Ser Ala Cys Asp Lys Glu Ser Asn Met Glu
        195                 200                 205

cga aga gtg atc acg att tct aaa tca gaa tat tct gtg cac tca tct      729
Arg Arg Val Ile Thr Ile Ser Lys Ser Glu Tyr Ser Val His Ser Ser
    210                 215                 220

ttg gca tcc aaa gtt gat gtt gag cag gtt gtt atg tat tgc aag gag      777
Leu Ala Ser Lys Val Asp Val Glu Gln Val Val Met Tyr Cys Lys Glu
225                 230                 235                 240

aag ctt att cgt ggg gaa tca gaa ttt tcc ttt gaa gaa ttg aga gcc      825
Lys Leu Ile Arg Gly Glu Ser Glu Phe Ser Phe Glu Glu Leu Arg Ala
                245                 250                 255

cag aaa tac aat caa cgg aga aag cat gag caa tgg gta aat gaa gac      873
Gln Lys Tyr Asn Gln Arg Arg Lys His Glu Gln Trp Val Asn Glu Asp
            260                 265                 270

aga cat tat atg aaa agg aaa gaa gca aat gct ttt gaa gaa cag cta      921
Arg His Tyr Met Lys Arg Lys Glu Ala Asn Ala Phe Glu Glu Gln Leu
        275                 280                 285

tta aaa cag aaa atg gat gaa ctt cat aag aag ttg cat cag gtg gtg      969
Leu Lys Gln Lys Met Asp Glu Leu His Lys Lys Leu His Gln Val Val
    290                 295                 300

gag aca tcc cat gag gat ctg ccc gct tcc cag gaa agg tcc gag gtt      1017
Glu Thr Ser His Glu Asp Leu Pro Ala Ser Gln Glu Arg Ser Glu Val
305                 310                 315                 320

aat cca gca cgt atg ggg cca agt gta ggc tcc cag cag gaa ctg aga      1065
Asn Pro Ala Arg Met Gly Pro Ser Val Gly Ser Gln Gln Glu Leu Arg
                325                 330                 335

gcg cca tgt ctt cca gta acc tat cag cag aca cca gtg aac atg gaa      1113
Ala Pro Cys Leu Pro Val Thr Tyr Gln Gln Thr Pro Val Asn Met Glu
            340                 345                 350

aag aac cca aga gag gca cct cct gtt gtt cct cct ttg gca aat gct      1161
Lys Asn Pro Arg Glu Ala Pro Pro Val Val Pro Pro Leu Ala Asn Ala
        355                 360                 365

att tct gca gct ttg gtg tcc cca gcc acc agc cag agc att gct cct      1209
Ile Ser Ala Ala Leu Val Ser Pro Ala Thr Ser Gln Ser Ile Ala Pro
    370                 375                 380

cct gtt cct ttg aaa gcc cag aca gta aca gac tcc atg ttt gca gtg      1257
Pro Val Pro Leu Lys Ala Gln Thr Val Thr Asp Ser Met Phe Ala Val
385                 390                 395                 400

gcc agc aaa gat gct gga tgt gtg aat aag agt act cat gaa ttc aag      1305
Ala Ser Lys Asp Ala Gly Cys Val Asn Lys Ser Thr His Glu Phe Lys
                405                 410                 415

cca cag agt gga gca gag atc aaa gaa ggg tgt gaa aca cat aag gtt      1353
Pro Gln Ser Gly Ala Glu Ile Lys Glu Gly Cys Glu Thr His Lys Val

                420                     425                     430

gcc aac aca agt tct ttt cac aca act cca aac aca tca ctg gga atg        1401
Ala Asn Thr Ser Ser Phe His Thr Thr Pro Asn Thr Ser Leu Gly Met
        435                     440                     445

gtt cag gca acg cca tcc aaa gtg cag cca tca ccc acc gtg cac aca        1449
Val Gln Ala Thr Pro Ser Lys Val Gln Pro Ser Pro Thr Val His Thr
    450                     455                     460

aaa gaa gca tta ggt ttc atc atg aat atg ttt cag gct cct aca ctt        1497
Lys Glu Ala Leu Gly Phe Ile Met Asn Met Phe Gln Ala Pro Thr Leu
465                     470                     475                     480

cct gat att tct gat gac aaa gat gaa tgg caa tct cta gat caa aat        1545
Pro Asp Ile Ser Asp Asp Lys Asp Glu Trp Gln Ser Leu Asp Gln Asn
                485                     490                     495

gaa gat gca ttt gaa gcc cag ttt caa aaa aat gta agg tca tct ggg        1593
Glu Asp Ala Phe Glu Ala Gln Phe Gln Lys Asn Val Arg Ser Ser Gly
            500                     505                     510

gct tgg gga gtc aat aag atc atc tct tct ttg tca tct gct ttt cat        1641
Ala Trp Gly Val Asn Lys Ile Ile Ser Ser Leu Ser Ser Ala Phe His
        515                     520                     525

gtg ttt gaa gat gga aac aaa gaa aat tat gga tta cca cag cct aaa        1689
Val Phe Glu Asp Gly Asn Lys Glu Asn Tyr Gly Leu Pro Gln Pro Lys
    530                     535                     540

aat aaa ccc aca gga gcc agg acc ttt gga gaa cgc tct gtc agc aga        1737
Asn Lys Pro Thr Gly Ala Arg Thr Phe Gly Glu Arg Ser Val Ser Arg
545                     550                     555                     560

ctt cct tca aaa cca aag gag gaa gtg cct cat gct gaa gag ttt ttg        1785
Leu Pro Ser Lys Pro Lys Glu Glu Val Pro His Ala Glu Glu Phe Leu
                565                     570                     575

gat gac tca act gta tgg ggt att cgc tgc aac aaa acc ctg gca ccc        1833
Asp Asp Ser Thr Val Trp Gly Ile Arg Cys Asn Lys Thr Leu Ala Pro
            580                     585                     590

agt cct aag agc cca gga gac ttc aca tct gct gca caa ctt gcg tct        1881
Ser Pro Lys Ser Pro Gly Asp Phe Thr Ser Ala Ala Gln Leu Ala Ser
        595                     600                     605

aca cca ttc cac aag ctt cca gtg gag tca gtg cac att tta gaa gat        1929
Thr Pro Phe His Lys Leu Pro Val Glu Ser Val His Ile Leu Glu Asp
    610                     615                     620

aaa gaa aat gtg gta gca aaa cag tgt acc cag gcg act ttg gat tct        1977
Lys Glu Asn Val Val Ala Lys Gln Cys Thr Gln Ala Thr Leu Asp Ser
625                     630                     635                     640

tgt gag gaa aac atg gtg gtg cct tca agg gat gga aaa ttc agt cca        2025
Cys Glu Glu Asn Met Val Val Pro Ser Arg Asp Gly Lys Phe Ser Pro
                645                     650                     655

att caa gag aaa agc cca aaa cag gcc ttg tcg tct cac atg tat tca        2073
Ile Gln Glu Lys Ser Pro Lys Gln Ala Leu Ser Ser His Met Tyr Ser
            660                     665                     670

gca tcc tta ctt cgt ctg agc cag cct gct gca ggt ggg gta ctt acc        2121
Ala Ser Leu Leu Arg Leu Ser Gln Pro Ala Ala Gly Gly Val Leu Thr
        675                     680                     685

tgt gag gca gag ttg ggc gtt gag gct tgc aga ctc aca gac act gac        2169
Cys Glu Ala Glu Leu Gly Val Glu Ala Cys Arg Leu Thr Asp Thr Asp
    690                     695                     700

gct gcc att gca gaa gat cca cca gat gct att gct ggg ctc caa gca 2217
Ala Ala Ile Ala Glu Asp Pro Pro Asp Ala Ile Ala Gly Leu Gln Ala
705                 710                 715                 720

gaa tgg atg cag atg agt tca ctt ggg act gtt gat gct cca aac ttc 2265
Glu Trp Met Gln Met Ser Ser Leu Gly Thr Val Asp Ala Pro Asn Phe
                725                 730                 735

att gtt ggg aac cca tgg gat gat aag ctg att ttc aaa ctt tta tct 2313
Ile Val Gly Asn Pro Trp Asp Asp Lys Leu Ile Phe Lys Leu Leu Ser
            740                 745                 750

ggg ctt tct aaa cca gtg agt tcc tat cca aat act ttt gaa tgg caa 2361
Gly Leu Ser Lys Pro Val Ser Ser Tyr Pro Asn Thr Phe Glu Trp Gln
        755                 760                 765

tgt aaa ctt cca gcc atc aag ccc aag act gaa ttt caa ttg ggt tct 2409
Cys Lys Leu Pro Ala Ile Lys Pro Lys Thr Glu Phe Gln Leu Gly Ser
    770                 775                 780

aag ctg gtc tat gtc cat cac ctt ctt gga gaa gga gcc ttt gcc cag 2457
Lys Leu Val Tyr Val His His Leu Leu Gly Glu Gly Ala Phe Ala Gln
785                 790                 795                 800

gtg tac gaa gct acc cag gga gat ctg aat gat gct aaa aat aaa cag 2505
Val Tyr Glu Ala Thr Gln Gly Asp Leu Asn Asp Ala Lys Asn Lys Gln
                805                 810                 815

aaa ttt gtt tta aag gtc caa aag cct gcc aac ccc tgg gaa ttc tac 2553
Lys Phe Val Leu Lys Val Gln Lys Pro Ala Asn Pro Trp Glu Phe Tyr
            820                 825                 830

att ggg acc cag ttg atg gaa aga cta aag cca tct atg cag cac atg 2601
Ile Gly Thr Gln Leu Met Glu Arg Leu Lys Pro Ser Met Gln His Met
        835                 840                 845

ttt atg aag ttc tat tct gcc cac tta ttc cag aat ggc agt gta tta 2649
Phe Met Lys Phe Tyr Ser Ala His Leu Phe Gln Asn Gly Ser Val Leu
    850                 855                 860

gta gga gag ctc tac agc tat gga aca tta tta aat gcc att aac ctc 2697
Val Gly Glu Leu Tyr Ser Tyr Gly Thr Leu Leu Asn Ala Ile Asn Leu
865                 870                 875                 880

tat aaa aat acc cct gaa aaa gtg atg cct caa ggt ctt gtc atc tct 2745
Tyr Lys Asn Thr Pro Glu Lys Val Met Pro Gln Gly Leu Val Ile Ser
                885                 890                 895

ttt gct atg aga atg ctt tac atg att gag caa gtg cat gac tgt gaa 2793
Phe Ala Met Arg Met Leu Tyr Met Ile Glu Gln Val His Asp Cys Glu
            900                 905                 910

atc att cat gga gac att aaa cca gac aat ttc ata ctt gga aac gga 2841
Ile Ile His Gly Asp Ile Lys Pro Asp Asn Phe Ile Leu Gly Asn Gly
        915                 920                 925

ttt ttg gaa cag gat gat gaa gat gat tta tct gct ggc ttg gca ctg 2889
Phe Leu Glu Gln Asp Asp Glu Asp Asp Leu Ser Ala Gly Leu Ala Leu
    930                 935                 940

att gac ctg ggt cag agt ata gat atg aaa ctt ttt cca aaa gga act 2937
Ile Asp Leu Gly Gln Ser Ile Asp Met Lys Leu Phe Pro Lys Gly Thr
945                 950                 955                 960

ata ttc aca gca aag tgt gaa aca tct ggt ttt cag tgt gtt gag atg 2985
Ile Phe Thr Ala Lys Cys Glu Thr Ser Gly Phe Gln Cys Val Glu Met
                965                 970                 975

ctc agc aac aaa cca tgg aac tac cag atc gat tac ttt ggg gtt gct     3033
Leu Ser Asn Lys Pro Trp Asn Tyr Gln Ile Asp Tyr Phe Gly Val Ala
            980                 985                 990

gca aca gta tat tgc atg ctc ttt  ggc act tac atg aaa  gtg aaa aat     3081
Ala Thr Val Tyr Cys Met Leu Phe  Gly Thr Tyr Met Lys  Val Lys Asn
        995                 1000                 1005

gaa gga  gga gag tgt aag cct  gaa ggt ctt ttt aga  agg ctt cct     3126
Glu Gly  Gly Glu Cys Lys Pro  Glu Gly Leu Phe Arg  Arg Leu Pro
    1010                 1015                 1020

cat ttg  gat atg tgg aat gaa  ttt ttt cat gtt atg  ttg aat att     3171
His Leu  Asp Met Trp Asn Glu  Phe Phe His Val Met  Leu Asn Ile
    1025                 1030                 1035

cca gat  tgt cat cat ctt cca  tct ttg gat ttg tta  agg caa aag     3216
Pro Asp  Cys His His Leu Pro  Ser Leu Asp Leu Leu  Arg Gln Lys
    1040                 1045                 1050

ctg aag  aaa gta ttt caa caa  cac tat act aac aag  att agg gcc     3261
Leu Lys  Lys Val Phe Gln Gln  His Tyr Thr Asn Lys  Ile Arg Ala
    1055                 1060                 1065

cta cgt  aat agg cta att gta  ctg ctc tta gaa tgt  aag cgt tca     3306
Leu Arg  Asn Arg Leu Ile Val  Leu Leu Leu Glu Cys  Lys Arg Ser
    1070                 1075                 1080

cga aaa  taa aatttggata tagacagtcc ttaaaaatca cactgtaaat     3355
Arg Lys
    1085

atgaatctgc tcactttaaa cctgtttttt tttcatttat tgtttatgta aatgtttgtt     3415

aaaaataaat cccatggaat atttccatgt     3445


<210>  12
<211>  1085
<212>  PRT
<213>  Homo sapiens

<400>  12

Met Asp Thr Pro Glu Asn Val Leu Gln Met Leu Glu Ala His Met Gln
1               5                   10                  15


Ser Tyr Lys Gly Asn Asp Pro Leu Gly Glu Trp Glu Arg Tyr Ile Gln
            20                  25                  30


Trp Val Glu Glu Asn Phe Pro Glu Asn Lys Glu Tyr Leu Ile Thr Leu
        35                  40                  45


Leu Glu His Leu Met Lys Glu Phe Leu Asp Lys Lys Lys Tyr His Asn
    50                  55                  60


Asp Pro Arg Phe Ile Ser Tyr Cys Leu Lys Phe Ala Glu Tyr Asn Ser
65                  70                  75                  80


Asp Leu His Gln Phe Phe Glu Phe Leu Tyr Asn His Gly Ile Gly Thr
                85                  90                  95


Leu Ser Ser Pro Leu Tyr Ile Ala Trp Ala Gly His Leu Glu Ala Gln

            100                 105                 110

Gly Glu Leu Gln His Ala Ser Ala Val Leu Gln Arg Gly Ile Gln Asn
        115                 120                 125

Gln Ala Glu Pro Arg Glu Phe Leu Gln Gln Gln Tyr Arg Leu Phe Gln
    130                 135                 140

Thr Arg Leu Thr Glu Thr His Leu Pro Ala Gln Ala Arg Thr Ser Glu
145                 150                 155                 160

Pro Leu His Asn Val Gln Val Leu Asn Gln Met Ile Thr Ser Lys Ser
                165                 170                 175

Asn Pro Gly Asn Asn Met Ala Cys Ile Ser Lys Asn Gln Gly Ser Glu
            180                 185                 190

Leu Ser Gly Val Ile Ser Ser Ala Cys Asp Lys Glu Ser Asn Met Glu
        195                 200                 205

Arg Arg Val Ile Thr Ile Ser Lys Ser Glu Tyr Ser Val His Ser Ser
    210                 215                 220

Leu Ala Ser Lys Val Asp Val Glu Gln Val Val Met Tyr Cys Lys Glu
225                 230                 235                 240

Lys Leu Ile Arg Gly Glu Ser Glu Phe Ser Phe Glu Glu Leu Arg Ala
                245                 250                 255

Gln Lys Tyr Asn Gln Arg Arg Lys His Glu Gln Trp Val Asn Glu Asp
            260                 265                 270

Arg His Tyr Met Lys Arg Lys Glu Ala Asn Ala Phe Glu Glu Gln Leu
        275                 280                 285

Leu Lys Gln Lys Met Asp Glu Leu His Lys Lys Leu His Gln Val Val
    290                 295                 300

Glu Thr Ser His Glu Asp Leu Pro Ala Ser Gln Glu Arg Ser Glu Val
305                 310                 315                 320

Asn Pro Ala Arg Met Gly Pro Ser Val Gly Ser Gln Gln Glu Leu Arg
                325                 330                 335

Ala Pro Cys Leu Pro Val Thr Tyr Gln Gln Thr Pro Val Asn Met Glu
            340                 345                 350

Lys Asn Pro Arg Glu Ala Pro Pro Val Val Pro Pro Leu Ala Asn Ala
        355                 360                 365

Ile Ser Ala Ala Leu Val Ser Pro Ala Thr Ser Gln Ser Ile Ala Pro
    370                 375                 380

Pro Val Pro Leu Lys Ala Gln Thr Val Thr Asp Ser Met Phe Ala Val
385                 390                 395                 400

Ala Ser Lys Asp Ala Gly Cys Val Asn Lys Ser Thr His Glu Phe Lys
                405                 410                 415

Pro Gln Ser Gly Ala Glu Ile Lys Glu Gly Cys Glu Thr His Lys Val
            420                 425                 430

Ala Asn Thr Ser Ser Phe His Thr Thr Pro Asn Thr Ser Leu Gly Met
        435                 440                 445

Val Gln Ala Thr Pro Ser Lys Val Gln Pro Ser Pro Thr Val His Thr
    450                 455                 460

Lys Glu Ala Leu Gly Phe Ile Met Asn Met Phe Gln Ala Pro Thr Leu
465                 470                 475                 480

Pro Asp Ile Ser Asp Asp Lys Asp Glu Trp Gln Ser Leu Asp Gln Asn
                485                 490                 495

Glu Asp Ala Phe Glu Ala Gln Phe Gln Lys Asn Val Arg Ser Ser Gly
            500                 505                 510

Ala Trp Gly Val Asn Lys Ile Ile Ser Ser Leu Ser Ser Ala Phe His
        515                 520                 525

Val Phe Glu Asp Gly Asn Lys Glu Asn Tyr Gly Leu Pro Gln Pro Lys
    530                 535                 540

Asn Lys Pro Thr Gly Ala Arg Thr Phe Gly Glu Arg Ser Val Ser Arg
545                 550                 555                 560

Leu Pro Ser Lys Pro Lys Glu Glu Val Pro His Ala Glu Glu Phe Leu
                565                 570                 575

Asp Asp Ser Thr Val Trp Gly Ile Arg Cys Asn Lys Thr Leu Ala Pro
            580                 585                 590

Ser Pro Lys Ser Pro Gly Asp Phe Thr Ser Ala Ala Gln Leu Ala Ser
        595                 600                 605

Thr Pro Phe His Lys Leu Pro Val Glu Ser Val His Ile Leu Glu Asp
    610                 615                 620

Lys Glu Asn Val Val Ala Lys Gln Cys Thr Gln Ala Thr Leu Asp Ser
625                 630                 635                 640

Cys Glu Glu Asn Met Val Val Pro Ser Arg Asp Gly Lys Phe Ser Pro
                645                 650                 655

Ile Gln Glu Lys Ser Pro Lys Gln Ala Leu Ser Ser His Met Tyr Ser
            660                 665                 670

Ala Ser Leu Leu Arg Leu Ser Gln Pro Ala Ala Gly Gly Val Leu Thr
        675                 680                 685

Cys Glu Ala Glu Leu Gly Val Glu Ala Cys Arg Leu Thr Asp Thr Asp
    690                 695                 700

Ala Ala Ile Ala Glu Asp Pro Pro Asp Ala Ile Ala Gly Leu Gln Ala
705                 710                 715                 720

Glu Trp Met Gln Met Ser Ser Leu Gly Thr Val Asp Ala Pro Asn Phe
                725                 730                 735

Ile Val Gly Asn Pro Trp Asp Asp Lys Leu Ile Phe Lys Leu Leu Ser
            740                 745                 750

Gly Leu Ser Lys Pro Val Ser Ser Tyr Pro Asn Thr Phe Glu Trp Gln
        755                 760                 765

Cys Lys Leu Pro Ala Ile Lys Pro Lys Thr Glu Phe Gln Leu Gly Ser
    770                 775                 780

Lys Leu Val Tyr Val His His Leu Leu Gly Glu Gly Ala Phe Ala Gln
785                 790                 795                 800

Val Tyr Glu Ala Thr Gln Gly Asp Leu Asn Asp Ala Lys Asn Lys Gln
                805                 810                 815

Lys Phe Val Leu Lys Val Gln Lys Pro Ala Asn Pro Trp Glu Phe Tyr
            820                 825                 830

Ile Gly Thr Gln Leu Met Glu Arg Leu Lys Pro Ser Met Gln His Met
        835                 840                 845

Phe Met Lys Phe Tyr Ser Ala His Leu Phe Gln Asn Gly Ser Val Leu
    850                 855                 860

Val Gly Glu Leu Tyr Ser Tyr Gly Thr Leu Leu Asn Ala Ile Asn Leu
865                 870                 875                 880

Tyr Lys Asn Thr Pro Glu Lys Val Met Pro Gln Gly Leu Val Ile Ser
                885                 890                 895

Phe Ala Met Arg Met Leu Tyr Met Ile Glu Gln Val His Asp Cys Glu
            900                 905                 910

Ile Ile His Gly Asp Ile Lys Pro Asp Asn Phe Ile Leu Gly Asn Gly
        915                 920                 925

Phe Leu Glu Gln Asp Asp Glu Asp Asp Leu Ser Ala Gly Leu Ala Leu
    930                 935                 940

Ile Asp Leu Gly Gln Ser Ile Asp Met Lys Leu Phe Pro Lys Gly Thr
945                 950                 955                 960

Ile Phe Thr Ala Lys Cys Glu Thr Ser Gly Phe Gln Cys Val Glu Met
                965                 970                 975

Leu Ser Asn Lys Pro Trp Asn Tyr Gln Ile Asp Tyr Phe Gly Val Ala
            980                 985                 990

Ala Thr Val Tyr Cys Met Leu Phe  Gly Thr Tyr Met Lys  Val Lys Asn
        995                 1000                 1005

Glu Gly  Gly Glu Cys Lys Pro  Glu Gly Leu Phe Arg  Arg Leu Pro
    1010                 1015                 1020

His Leu  Asp Met Trp Asn Glu  Phe Phe His Val Met  Leu Asn Ile
    1025                 1030                 1035

Pro Asp  Cys His His Leu Pro  Ser Leu Asp Leu Leu  Arg Gln Lys
    1040                 1045                 1050

Leu Lys  Lys Val Phe Gln Gln  His Tyr Thr Asn Lys  Ile Arg Ala
    1055                 1060                 1065

Leu Arg  Asn Arg Leu Ile Val  Leu Leu Leu Glu Cys  Lys Arg Ser
    1070                 1075                 1080

Arg Lys
    1085

<210>  13
<211>  2748
<212>  DNA
<213>  Homo sapiens

<220>
<221>  CDS
<222>  (304)..(2370)
<223>  C1S mRNA

<400>  13
ggacagggag gctggccgga ggttcctgca gagggagcgt caaggccctg tgctgctgtc      60
cctgggggcc agaggggttg cccagcatgc ccactggcag gagagaggga actgacccac     120
ttgctcctac cagcttctga aggtgacact gagccccagg tgacgccgca ccaccaaaga     180
aggtgcttgt gtttgtcaga caaatacagc caggcctgcc accccttagg ctccaaagtc     240
cggaggtgca gaaagccagg accaagagac aggcagctca ccagggtgga caaatcgcca     300
gag atg tgg tgc att gtc ctg ttt tca ctt ttg gca tgg gtt tat gct       348
gag cct acc atg tat ggg gag atc ctg tcc cct aac tat cct cag gca 396
tat ccc agt gag gta gag aaa tct tgg gac ata gaa gtt cct gaa ggg 444
tat ggg att cac ctc tac ttc acc cat ctg gac att gag ctg tca gag 492
aac tgt gcg tat gac tca gtg cag ata atc tca gga gac act gaa gaa 540
ggg agg ctc tgt gga cag agg agc agt aac aat ccc cac tct cca att 588
gtg gaa gag ttc caa gtc cca tac aac aaa ctc cag gtg atc ttt aag 636
tca gac ttt tcc aat gaa gag cgt ttt acg ggg ttt gct gca tac tat 684
gtt gcc aca gac ata aat gaa tgc aca gat ttt gta gat gtc cct tgt 732
agc cac ttc tgc aac aat ttc att ggt ggt tac ttc tgc tcc tgc ccc 780
ccg gaa tat ttc ctc cat gat gac atg aag aat tgc gga gtt aat tgc 828
agt ggg gat gta ttc act gca ctg att ggg gag att gca agt ccc aat 876
tat ccc aaa cca tat cca gag aac tca agg tgt gaa tac cag atc cgg 924
ttg gag aaa ggg ttc caa gtg gtg gtg acc ttg cgg aga gaa gat ttt 972
gat gtg gaa gca gct gac tca gcg gga aac tgc ctt gac agt tta gtt 1020
ttt gtt gca gga gat cgg caa ttt ggt cct tac tgt ggt cat gga ttc 1068
cct ggg cct cta aat att gaa acc aag agt aat gct ctt gat atc atc 1116
ttc caa act gat cta aca ggg caa aaa aag ggc tgg aaa ctt cgc tat 1164
cat gga gat cca atg ccc tgc cct aag gaa gac act ccc aat tct gtt     1212
tgg gag cct gcg aag gca aaa tat gtc ttt aga gat gtg gtg cag ata     1260
acc tgt ctg gat ggg ttt gaa gtt gtg gag gga cgt gtt ggt gca aca     1308
tct ttc tat tcg act tgt caa agc aat gga aag tgg agt aat tcc aaa     1356
ctg aaa tgt caa cct gtg gac tgt ggc att cct gaa tcc att gag aat     1404
ggt aaa gtt gaa gac cca gag agc act ttg ttt ggt tct gtc atc cgc     1452
tac act tgt gag gag cca tat tac tac atg gaa aat gga gga ggt ggg     1500
gag tat cac tgt gct ggt aac ggg agc tgg gtg aat gag gtg ctg ggc     1548
ccg gag ctg ccg aaa tgt gtt cca gtc tgt gga gtc ccc aga gaa ccc     1596
ttt gaa gaa aaa cag agg ata att gga gga tcc gat gca gat att aaa     1644
aac ttc ccc tgg caa gtc ttc ttt gac aac cca tgg gct ggt gga gcg     1692
ctc att aat gag tac tgg gtg ctg acg gct gct cat gtt gtg gag gga     1740
aac agg gag cca aca atg tat gtt ggg tcc acc tca gtg cag acc tca     1788
cgg ctg gca aaa tcc aag atg ctc act cct gag cat gtg ttt att cat     1836
ccg gga tgg aag ctg ctg gaa gtc cca gaa gga cga acc aat ttt gat     1884
aat gac att gca ctg gtg cgg ctg aaa gac cca gtg aaa atg gga ccc     1932
acc gtc tct ccc atc tgc cta cca ggc acc tct tcc gac tac aac ctc     1980
atg gat ggg gac ctg gga ctg atc tca ggc tgg ggc cga aca gag aag     2028
aga gat cgt gct gtt cgc ctc aag gcg gca agg tta cct gta gct cct      2076
tta aga aaa tgc aaa gaa gtg aaa gtg gag aaa ccc aca gca gat gca      2124
gag gcc tat gtt ttc act cct aac atg atc tgt gct gga gga gag aag      2172
ggc atg gat agc tgt aaa ggg gac agt ggt ggg gcc ttt gct gta cag      2220
gat ccc aat gac aag acc aaa ttc tac gca gct ggc ctg gtg tcc tgg      2268
ggg ccc cag tgt ggg acc tat ggg ctc tac aca cgg gta aag aac tat      2316
gtt gac tgg ata atg aag act atg cag gaa aat agc acc ccc cgt gag      2364
gac taa tccagataca tcccaccagc ctctccaagg gtggtgacca atgcattacc      2420
ttctgttcct tatgatattc tcattatttc atcatgactg aaagaagaca cgagcgaatg      2480
atttaaatag aacttgattg ttgagacgcc ttgctagagg tagagtttga tcatagaatt      2540
gtgctggtca tacatttgtg gtctgactcc ttggggtcct ttccccggag tacctattgt      2600
agataacact atgggtgggg cactcctttc ttgcactatt ccacagggat accttaattc      2660
tttgtttcct ctttacctgt tcaaaattcc atttacttga tcattctcag tatccactgt      2720
ctatgtacaa taaaggatgt ttataagc                                        2748

<210>  14
<211>  688
<212>  PRT
<213>  Homo sapiens

<400>  14

<210>  15
<211>  1543
<212>  DNA
<213>  Homo sapiens

<220>
<221>  CDS
<222>  (10)..(1491)
<223>  CYP2E1 mRNA

<400>  15
agcggcacc atg tct gcc ctc gga gtc acc gtg gcc ctg ctg gtg tgg gcg      51
          Met Ser Ala Leu Gly Val Thr Val Ala Leu Leu Val Trp Ala
          1               5                   10

gcc ttc ctc ctg ctg gtg tcc atg tgg agg cag gtg cac agc agc tgg      99
Ala Phe Leu Leu Leu Val Ser Met Trp Arg Gln Val His Ser Ser Trp
15                  20                  25                  30

aat ctg ccc cca ggc cct ttc ccg ctt ccc atc atc ggg aac ctc ttc      147
Asn Leu Pro Pro Gly Pro Phe Pro Leu Pro Ile Ile Gly Asn Leu Phe
                35                  40                  45

cag ttg gaa ttg aag aat att ccc aag tcc ttc acc cgg ttg gcc cag      195
Gln Leu Glu Leu Lys Asn Ile Pro Lys Ser Phe Thr Arg Leu Ala Gln
            50                  55                  60

cgc ttc ggg ccg gtg ttc acg ctg tac gtg ggc tcg cag cgc atg gtg      243
Arg Phe Gly Pro Val Phe Thr Leu Tyr Val Gly Ser Gln Arg Met Val
        65                  70                  75

gtg atg cac ggc tac aag gcg gtg aag gaa gcg ctg ctg gac tac aag      291
Val Met His Gly Tyr Lys Ala Val Lys Glu Ala Leu Leu Asp Tyr Lys
    80                  85                  90

gac gag ttc tcg ggc aga ggc gac ctc ccc gcg ttc cat gcg cac agg      339
Asp Glu Phe Ser Gly Arg Gly Asp Leu Pro Ala Phe His Ala His Arg
95                  100                 105                 110

gac agg gga atc att ttt aat aat gga cct acc tgg aag gac atc cgg      387
Asp Arg Gly Ile Ile Phe Asn Asn Gly Pro Thr Trp Lys Asp Ile Arg
                115                 120                 125

cgg ttt tcc ctg acc acc ctc cgg aac tat ggg atg ggg aaa cag ggc      435
Arg Phe Ser Leu Thr Thr Leu Arg Asn Tyr Gly Met Gly Lys Gln Gly
            130                 135                 140

aat gag agc cgg atc cag agg gag gcc cac ttc ctg ctg gaa gca ctc      483

Asn Glu Ser Arg Ile Gln Arg Glu Ala His Phe Leu Leu Glu Ala Leu
        145                 150                 155

agg aag acc caa ggc cag cct ttc gac ccc acc ttc ctc atc ggc tgc      531
Arg Lys Thr Gln Gly Gln Pro Phe Asp Pro Thr Phe Leu Ile Gly Cys
    160                 165                 170

gcg ccc tgc aac gtc ata gcc gac atc ctc ttc cgc aag cat ttt gac      579
Ala Pro Cys Asn Val Ile Ala Asp Ile Leu Phe Arg Lys His Phe Asp
175                 180                 185                 190

tac aat gat gag aag ttt cta agg ctg atg tat ttg ttt aat gag aac      627
Tyr Asn Asp Glu Lys Phe Leu Arg Leu Met Tyr Leu Phe Asn Glu Asn
                195                 200                 205

ttc cac cta ctc agc act ccc tgg ctc cag ctt tac aat aat ttt ccc      675
Phe His Leu Leu Ser Thr Pro Trp Leu Gln Leu Tyr Asn Asn Phe Pro
            210                 215                 220

agc ttt cta cac tac ttg cct gga agc cac aga aaa gtc ata aaa aat      723
Ser Phe Leu His Tyr Leu Pro Gly Ser His Arg Lys Val Ile Lys Asn
        225                 230                 235

gtg gct gaa gta aaa gag tat gtg tct gaa agg gtg aag gag cac cat      771
Val Ala Glu Val Lys Glu Tyr Val Ser Glu Arg Val Lys Glu His His
    240                 245                 250

caa tct ctg gac ccc aac tgt ccc cgg gac ctc acc gac tgc ctg ctc      819
Gln Ser Leu Asp Pro Asn Cys Pro Arg Asp Leu Thr Asp Cys Leu Leu
255                 260                 265                 270

gtg gaa atg gag aag gaa aag cac agt gca gag cgc ttg tac aca atg      867
Val Glu Met Glu Lys Glu Lys His Ser Ala Glu Arg Leu Tyr Thr Met
                275                 280                 285

gac ggt atc acc gtg act gtg gcc gac ctg ttc ttt gcg ggg aca gag      915
Asp Gly Ile Thr Val Thr Val Ala Asp Leu Phe Phe Ala Gly Thr Glu
            290                 295                 300

acc acc agc aca act ctg aga tat ggg ctc ctg att ctc atg aaa tac      963
Thr Thr Ser Thr Thr Leu Arg Tyr Gly Leu Leu Ile Leu Met Lys Tyr
        305                 310                 315

cct gag att gaa gag aag ctc cat gaa gaa att gac agg gtg att ggg     1011
Pro Glu Ile Glu Glu Lys Leu His Glu Glu Ile Asp Arg Val Ile Gly
    320                 325                 330

cca agc cga atc cct gcc atc aag gat agg caa gag atg ccc tac atg     1059
Pro Ser Arg Ile Pro Ala Ile Lys Asp Arg Gln Glu Met Pro Tyr Met
335                 340                 345                 350

gat gct gtg gtg cat gag att cag cgg ttc atc acc ctc gtg ccc tcc     1107
Asp Ala Val Val His Glu Ile Gln Arg Phe Ile Thr Leu Val Pro Ser
                355                 360                 365

aac ctg ccc cat gaa gca acc cga gac acc att ttc aga gga tac ctc     1155
Asn Leu Pro His Glu Ala Thr Arg Asp Thr Ile Phe Arg Gly Tyr Leu
            370                 375                 380

atc ccc aag ggc aca gtc gta gtg cca act ctg gac tct gtt ttg tat     1203
Ile Pro Lys Gly Thr Val Val Val Pro Thr Leu Asp Ser Val Leu Tyr
        385                 390                 395

gac aac caa gaa ttt cct gat cca gaa aag ttt aag cca gaa cac ttc     1251
Asp Asn Gln Glu Phe Pro Asp Pro Glu Lys Phe Lys Pro Glu His Phe
    400                 405                 410

ctg aat gaa aat gga aag ttt aag tac agt gac tat ttc aag cca ttt     1299
Leu Asn Glu Asn Gly Lys Phe Lys Tyr Ser Asp Tyr Phe Lys Pro Phe

415                 420                 425                 430

tcc aca gga aaa cga gtg tgt gct gga gaa ggc ctg gct cgc atg gag      1347
Ser Thr Gly Lys Arg Val Cys Ala Gly Glu Gly Leu Ala Arg Met Glu
                435                 440                 445

ttg ttt ctt ttg ttg tgt gcc att ttg cag cat ttt aat ttg aag cct      1395
Leu Phe Leu Leu Leu Cys Ala Ile Leu Gln His Phe Asn Leu Lys Pro
            450                 455                 460

ctc gtt gac cca aag gat atc gac ctc agc cct ata cat att ggg ttt      1443
Leu Val Asp Pro Lys Asp Ile Asp Leu Ser Pro Ile His Ile Gly Phe
        465                 470                 475

ggc tgt atc cca cca cgt tac aaa ctc tgt gtc att ccc cgc tca tga      1491
Gly Cys Ile Pro Pro Arg Tyr Lys Leu Cys Val Ile Pro Arg Ser
    480                 485                 490

gtgtgtggag gacaccctga accccccgct ttcaaacaag ttttcaaatt gt             1543

<210>  16
<211>  493
<212>  PRT
<213>  Homo sapiens

<400>  16

Met Ser Ala Leu Gly Val Thr Val Ala Leu Leu Val Trp Ala Ala Phe
1               5                   10                  15

Leu Leu Leu Val Ser Met Trp Arg Gln Val His Ser Ser Trp Asn Leu
            20                  25                  30

Pro Pro Gly Pro Phe Pro Leu Pro Ile Ile Gly Asn Leu Phe Gln Leu
        35                  40                  45

Glu Leu Lys Asn Ile Pro Lys Ser Phe Thr Arg Leu Ala Gln Arg Phe
    50                  55                  60

Gly Pro Val Phe Thr Leu Tyr Val Gly Ser Gln Arg Met Val Val Met
65                  70                  75                  80

His Gly Tyr Lys Ala Val Lys Glu Ala Leu Leu Asp Tyr Lys Asp Glu
                85                  90                  95

Phe Ser Gly Arg Gly Asp Leu Pro Ala Phe His Ala His Arg Asp Arg
            100                 105                 110

Gly Ile Ile Phe Asn Asn Gly Pro Thr Trp Lys Asp Ile Arg Arg Phe
        115                 120                 125

Ser Leu Thr Thr Leu Arg Asn Tyr Gly Met Gly Lys Gln Gly Asn Glu
    130                 135                 140

Ser Arg Ile Gln Arg Glu Ala His Phe Leu Leu Glu Ala Leu Arg Lys
145                 150                 155                 160

Thr Gln Gly Gln Pro Phe Asp Pro Thr Phe Leu Ile Gly Cys Ala Pro

                165                 170                 175

Cys Asn Val Ile Ala Asp Ile Leu Phe Arg Lys His Phe Asp Tyr Asn
            180                 185                 190

Asp Glu Lys Phe Leu Arg Leu Met Tyr Leu Phe Asn Glu Asn Phe His
        195                 200                 205

Leu Leu Ser Thr Pro Trp Leu Gln Leu Tyr Asn Asn Phe Pro Ser Phe
    210                 215                 220

Leu His Tyr Leu Pro Gly Ser His Arg Lys Val Ile Lys Asn Val Ala
225                 230                 235                 240

Glu Val Lys Glu Tyr Val Ser Glu Arg Val Lys Glu His His Gln Ser
                245                 250                 255

Leu Asp Pro Asn Cys Pro Arg Asp Leu Thr Asp Cys Leu Leu Val Glu
            260                 265                 270

Met Glu Lys Glu Lys His Ser Ala Glu Arg Leu Tyr Thr Met Asp Gly
        275                 280                 285

Ile Thr Val Thr Val Ala Asp Leu Phe Phe Ala Gly Thr Glu Thr Thr
    290                 295                 300

Ser Thr Thr Leu Arg Tyr Gly Leu Leu Ile Leu Met Lys Tyr Pro Glu
305                 310                 315                 320

Ile Glu Glu Lys Leu His Glu Glu Ile Asp Arg Val Ile Gly Pro Ser
                325                 330                 335

Arg Ile Pro Ala Ile Lys Asp Arg Gln Glu Met Pro Tyr Met Asp Ala
            340                 345                 350

Val Val His Glu Ile Gln Arg Phe Ile Thr Leu Val Pro Ser Asn Leu
        355                 360                 365

Pro His Glu Ala Thr Arg Asp Thr Ile Phe Arg Gly Tyr Leu Ile Pro
    370                 375                 380

Lys Gly Thr Val Val Val Pro Thr Leu Asp Ser Val Leu Tyr Asp Asn
385                 390                 395                 400

Gln Glu Phe Pro Asp Pro Glu Lys Phe Lys Pro Glu His Phe Leu Asn
                405                 410                 415

Glu Asn Gly Lys Phe Lys Tyr Ser Asp Tyr Phe Lys Pro Phe Ser Thr
            420                 425                 430

Gly Lys Arg Val Cys Ala Gly Glu Gly Leu Ala Arg Met Glu Leu Phe
        435                 440                 445

Leu Leu Leu Cys Ala Ile Leu Gln His Phe Asn Leu Lys Pro Leu Val
    450                 455                 460

Asp Pro Lys Asp Ile Asp Leu Ser Pro Ile His Ile Gly Phe Gly Cys
465                 470                 475                 480

Ile Pro Pro Arg Tyr Lys Leu Cys Val Ile Pro Arg Ser
                485                 490

<210>  17
<211>  2871
<212>  DNA
<213>  Homo sapiens

<220>
<221>  CDS
<222>  (110)..(2650)
<223>  DLG7 mRNA

<400>  17
ggaggctcgg gttgtgaggg ttcctgcttc ggagtcggcg gtggtcgtcc agaccgagtg      60

ttctttactt tttgtttggt tgaggtttca cgctagaagg tggctcagg atg tct tca     118
                                                      Met Ser Ser
                                                      1

tca cat ttt gcc agt cga cac agg aag gat ata agt act gaa atg att       166
Ser His Phe Ala Ser Arg His Arg Lys Asp Ile Ser Thr Glu Met Ile
    5                   10                  15

aga act aaa att gct cat agg aaa tca ctg tct cag aaa gaa aat aga       214
Arg Thr Lys Ile Ala His Arg Lys Ser Leu Ser Gln Lys Glu Asn Arg
20                  25                  30                  35

cat aag gaa tac gaa cga aat aga cac ttt ggt ttg aaa gat gta aac       262
His Lys Glu Tyr Glu Arg Asn Arg His Phe Gly Leu Lys Asp Val Asn
                40                  45                  50

att cca acc ttg gaa ggt aga att ctt gtt gaa tta gat gag aca tct       310
Ile Pro Thr Leu Glu Gly Arg Ile Leu Val Glu Leu Asp Glu Thr Ser
            55                  60                  65

caa ggg ctt gtt cca gaa aag acc aat gtt aag cca agg gca atg aaa       358
Gln Gly Leu Val Pro Glu Lys Thr Asn Val Lys Pro Arg Ala Met Lys
        70                  75                  80

act att cta ggt gat caa cga aaa cag atg ctc caa aaa tac aaa gaa       406
Thr Ile Leu Gly Asp Gln Arg Lys Gln Met Leu Gln Lys Tyr Lys Glu
    85                  90                  95

gaa aag caa ctt caa aaa ttg aaa gag cag aga gag aaa gct aaa cga       454
Glu Lys Gln Leu Gln Lys Leu Lys Glu Gln Arg Glu Lys Ala Lys Arg
100                 105                 110                 115

gga ata ttt aaa gtg ggt cgt tat aga cct gat atg cct tgt ttt ctt       502
Gly Ile Phe Lys Val Gly Arg Tyr Arg Pro Asp Met Pro Cys Phe Leu
                120                 125                 130

tta tca aac cag aat gct gtg aaa gct gag cca aaa aag gct att cca       550
Leu Ser Asn Gln Asn Ala Val Lys Ala Glu Pro Lys Lys Ala Ile Pro
            135                 140                 145

tct tct gta cgg att aca agg tca aag gcc aaa gac caa atg gag cag       598

Ser Ser Val Arg Ile Thr Arg Ser Lys Ala Lys Asp Gln Met Glu Gln
        150                 155                 160

act aag att gat aac gag agt gat gtt cga gca atc cga cct ggt cca      646
Thr Lys Ile Asp Asn Glu Ser Asp Val Arg Ala Ile Arg Pro Gly Pro
    165                 170                 175

aga caa act tct gaa aag aaa gtg tca gac aaa gag aaa aaa gtt gtg      694
Arg Gln Thr Ser Glu Lys Lys Val Ser Asp Lys Glu Lys Lys Val Val
180                 185                 190                 195

cag cct gta atg ccc acg tcg ttg aga atg act cga tca gct act caa      742
Gln Pro Val Met Pro Thr Ser Leu Arg Met Thr Arg Ser Ala Thr Gln
                200                 205                 210

gca gca aag cag gtt ccc aga aca gtc tca tct acc aca gca aga aag      790
Ala Ala Lys Gln Val Pro Arg Thr Val Ser Ser Thr Thr Ala Arg Lys
            215                 220                 225

cca gtc aca aga gct gct aat gaa aac gaa cca gaa gga aag gtg cca      838
Pro Val Thr Arg Ala Ala Asn Glu Asn Glu Pro Glu Gly Lys Val Pro
        230                 235                 240

agt aaa gga aga cct gcc aaa aat gta gaa aca aaa ccc gac aag ggt      886
Ser Lys Gly Arg Pro Ala Lys Asn Val Glu Thr Lys Pro Asp Lys Gly
    245                 250                 255

att tct tgt aaa gtc gat agt gaa gaa aat act ttg aat tca caa act      934
Ile Ser Cys Lys Val Asp Ser Glu Glu Asn Thr Leu Asn Ser Gln Thr
260                 265                 270                 275

aat gca aca agt gga atg aat cca gat gga gtc tta tca aaa atg gaa      982
Asn Ala Thr Ser Gly Met Asn Pro Asp Gly Val Leu Ser Lys Met Glu
                280                 285                 290

aac tta cct gag ata aat act gca aaa ata aaa ggg aag aat tcc ttt      1030
Asn Leu Pro Glu Ile Asn Thr Ala Lys Ile Lys Gly Lys Asn Ser Phe
            295                 300                 305

gca cct aag gat ttt atg ttt cag cca ctg gat ggt ctg aag acc tat      1078
Ala Pro Lys Asp Phe Met Phe Gln Pro Leu Asp Gly Leu Lys Thr Tyr
        310                 315                 320

caa gta aca cct atg act ccc aga agt gcc aat gct ttt ttg aca ccc      1126
Gln Val Thr Pro Met Thr Pro Arg Ser Ala Asn Ala Phe Leu Thr Pro
    325                 330                 335

agt tac acc tgg act cct tta aaa aca gaa gtt gat gag tct caa gca      1174
Ser Tyr Thr Trp Thr Pro Leu Lys Thr Glu Val Asp Glu Ser Gln Ala
340                 345                 350                 355

aca aaa gaa att ttg gca caa aaa tgt aaa act tac tct acc aag aca      1222
Thr Lys Glu Ile Leu Ala Gln Lys Cys Lys Thr Tyr Ser Thr Lys Thr
                360                 365                 370

ata cag caa gat tca aat aaa ttg cca tgt cct ttg ggt cct cta act      1270
Ile Gln Gln Asp Ser Asn Lys Leu Pro Cys Pro Leu Gly Pro Leu Thr
            375                 380                 385

gtt tgg cat gaa gaa cat gtt tta aat aaa aat gaa gct act act aaa      1318
Val Trp His Glu Glu His Val Leu Asn Lys Asn Glu Ala Thr Thr Lys
        390                 395                 400

aat tta aat ggc ctt cca ata aaa gaa gtc cca tca ctt gaa aga aat      1366
Asn Leu Asn Gly Leu Pro Ile Lys Glu Val Pro Ser Leu Glu Arg Asn
    405                 410                 415

gaa ggt cga att gct cag ccc cac cat ggt gtg cca tat ttc aga aat      1414
Glu Gly Arg Ile Ala Gln Pro His His Gly Val Pro Tyr Phe Arg Asn

420                 425                 430                 435

atc ctc cag tca gaa act gag aaa tta act tca cat tgc ttc gag tgg      1462
Ile Leu Gln Ser Glu Thr Glu Lys Leu Thr Ser His Cys Phe Glu Trp
                440                 445                 450

gac agg aaa ctt gaa ttg gac att cca gat gat gct aaa gat ctt att      1510
Asp Arg Lys Leu Glu Leu Asp Ile Pro Asp Asp Ala Lys Asp Leu Ile
            455                 460                 465

cgc aca gca gtt ggt caa aca aga ctc ctt atg aag gaa agg ttt aaa      1558
Arg Thr Ala Val Gly Gln Thr Arg Leu Leu Met Lys Glu Arg Phe Lys
        470                 475                 480

cag ttt gaa gga ctg gtt gat gat tgt gaa tat aaa cga ggt ata aag      1606
Gln Phe Glu Gly Leu Val Asp Asp Cys Glu Tyr Lys Arg Gly Ile Lys
    485                 490                 495

gag act acc tgt aca gat ctg gat gga ttt tgg gat atg gtt agt ttt      1654
Glu Thr Thr Cys Thr Asp Leu Asp Gly Phe Trp Asp Met Val Ser Phe
500                 505                 510                 515

cag ata gaa gat gta atc cac aaa ttc aac aat ctg atc aaa ctt gag      1702
Gln Ile Glu Asp Val Ile His Lys Phe Asn Asn Leu Ile Lys Leu Glu
                520                 525                 530

gaa tct ggg tgg caa gtc aat aat aat atg aat cat aat atg aac aaa      1750
Glu Ser Gly Trp Gln Val Asn Asn Asn Met Asn His Asn Met Asn Lys
            535                 540                 545

aat gtc ttt agg aaa aaa gtt gtc tca ggt ata gca agt aaa cca aaa      1798
Asn Val Phe Arg Lys Lys Val Val Ser Gly Ile Ala Ser Lys Pro Lys
        550                 555                 560

cag gat gat gct gga aga att gca gcg aga aat cgc cta gct gcc ata      1846
Gln Asp Asp Ala Gly Arg Ile Ala Ala Arg Asn Arg Leu Ala Ala Ile
    565                 570                 575

aaa aat gca atg aga gag aga att agg cag gaa gaa tgt gct gaa aca      1894
Lys Asn Ala Met Arg Glu Arg Ile Arg Gln Glu Glu Cys Ala Glu Thr
580                 585                 590                 595

gca gtt tct gtg ata cca aag gaa gtt gat aaa ata gtg ttc gat gct      1942
Ala Val Ser Val Ile Pro Lys Glu Val Asp Lys Ile Val Phe Asp Ala
                600                 605                 610

gga ttt ttc aga gtt gaa agt cct gtt aaa tta ttc tca gga ctt tct      1990
Gly Phe Phe Arg Val Glu Ser Pro Val Lys Leu Phe Ser Gly Leu Ser
            615                 620                 625

gtc tct tct gaa ggc cct tct caa aga ctt gga aca cct aag tct gtc      2038
Val Ser Ser Glu Gly Pro Ser Gln Arg Leu Gly Thr Pro Lys Ser Val
        630                 635                 640

aac aaa gct gta tct cag agt aga aat gag atg ggc att cca caa caa      2086
Asn Lys Ala Val Ser Gln Ser Arg Asn Glu Met Gly Ile Pro Gln Gln
    645                 650                 655

act aca tca cca gaa aat gcc ggt cct cag aat acg aaa agt gaa cat      2134
Thr Thr Ser Pro Glu Asn Ala Gly Pro Gln Asn Thr Lys Ser Glu His
660                 665                 670                 675

gtg aag aag act ttg ttt ttg agt att cct gaa agc agg agc agc ata      2182

                680                 685                 690

gaa gat gct cag tgt cct gga tta cca gat tta att gaa gaa aat cat      2230

            695                 700                 705

gtt gta aat aag aca gac ttg aag gtg gat tgt tta tcc agt gag aga     2278

        710                 715                 720

atg agt ttg cct ctt ctt gct ggt gga gta gca gat gat att aat act     2326

    725                 730                 735

aac aaa aaa gaa gga att tca gat gtt gtg gaa gga atg gaa ctg aat     2374

740                 745                 750                 755

tct tca att aca tca cag gat gtt ttg atg agt agc cct gaa aaa aat     2422

                760                 765                 770

aca gct tca caa aat agc atc tta gaa gaa ggg gaa act aaa att tct     2470

            775                 780                 785

cag tca gaa cta ttt gat aat aaa agt ctc act act gaa tgc cac ctt     2518

        790                 795                 800

ctt gat tca cca ggt cta aac tgc agt aat cca ttt act cag ctg gag     2566

    805                 810                 815

agg aga cat caa gaa cat gcc aga cac att tct ttt ggt ggt aac ctg     2614

820                 825                 830                 835

att act ttt tca cct cta caa cca gga gaa ttt tga atttaaaat           2660

                840                 845

aaatccaaac attttccttc atattatcaa tgcttatata ttccttagac tattgaaatt   2720

ttggagaaaa tgtatttgtg ttcacttcta tagcatataa tgttttaata ttctgtgttc   2780

atcaaagtgt attttagata tactctttct caagggaagt ggggatattt tgtacatttt   2840

caacacagaa taaaaaatgt actgtgcctt g                                  2871

<210>  18
<211>  846
<212>  PRT
<213>  Homo sapiens

<400>  18

Met Ser Ser Ser His Phe Ala Ser Arg His Arg Lys Asp Ile Ser Thr
1               5                   10                  15

Glu Met Ile Arg Thr Lys Ile Ala His Arg Lys Ser Leu Ser Gln Lys
            20                  25                  30

Glu Asn Arg His Lys Glu Tyr Glu Arg Asn Arg His Phe Gly Leu Lys
        35                  40                  45

Asp Val Asn Ile Pro Thr Leu Glu Gly Arg Ile Leu Val Glu Leu Asp
    50                  55                  60

Glu Thr Ser Gln Gly Leu Val Pro Glu Lys Thr Asn Val Lys Pro Arg
65                  70                  75                  80

Ala Met Lys Thr Ile Leu Gly Asp Gln Arg Lys Gln Met Leu Gln Lys
                85                  90                  95

Tyr Lys Glu Glu Lys Gln Leu Gln Lys Leu Lys Glu Gln Arg Glu Lys
            100                 105                 110

Ala Lys Arg Gly Ile Phe Lys Val Gly Arg Tyr Arg Pro Asp Met Pro
        115                 120                 125

Cys Phe Leu Leu Ser Asn Gln Asn Ala Val Lys Ala Glu Pro Lys Lys
    130                 135                 140

Ala Ile Pro Ser Ser Val Arg Ile Thr Arg Ser Lys Ala Lys Asp Gln
145                 150                 155                 160

Met Glu Gln Thr Lys Ile Asp Asn Glu Ser Asp Val Arg Ala Ile Arg
                165                 170                 175

Pro Gly Pro Arg Gln Thr Ser Glu Lys Lys Val Ser Asp Lys Glu Lys
            180                 185                 190

Lys Val Val Gln Pro Val Met Pro Thr Ser Leu Arg Met Thr Arg Ser
        195                 200                 205

Ala Thr Gln Ala Ala Lys Gln Val Pro Arg Thr Val Ser Ser Thr Thr
    210                 215                 220

Ala Arg Lys Pro Val Thr Arg Ala Ala Asn Glu Asn Glu Pro Glu Gly
225                 230                 235                 240

Lys Val Pro Ser Lys Gly Arg Pro Ala Lys Asn Val Glu Thr Lys Pro
                245                 250                 255

Asp Lys Gly Ile Ser Cys Lys Val Asp Ser Glu Glu Asn Thr Leu Asn
            260                 265                 270

Ser Gln Thr Asn Ala Thr Ser Gly Met Asn Pro Asp Gly Val Leu Ser
        275                 280                 285

Lys Met Glu Asn Leu Pro Glu Ile Asn Thr Ala Lys Ile Lys Gly Lys
    290                 295                 300

Asn Ser Phe Ala Pro Lys Asp Phe Met Phe Gln Pro Leu Asp Gly Leu
305                 310                 315                 320

Lys Thr Tyr Gln Val Thr Pro Met Thr Pro Arg Ser Ala Asn Ala Phe
                325                 330                 335

Leu Thr Pro Ser Tyr Thr Trp Thr Pro Leu Lys Thr Glu Val Asp Glu

340                 345                 350

Ser Gln Ala Thr Lys Glu Ile Leu Ala Gln Lys Cys Lys Thr Tyr Ser
        355                 360                 365

Thr Lys Thr Ile Gln Gln Asp Ser Asn Lys Leu Pro Cys Pro Leu Gly
    370                 375                 380

Pro Leu Thr Val Trp His Glu Glu His Val Leu Asn Lys Asn Glu Ala
385                 390                 395                 400

Thr Thr Lys Asn Leu Asn Gly Leu Pro Ile Lys Glu Val Pro Ser Leu
                405                 410                 415

Glu Arg Asn Glu Gly Arg Ile Ala Gln Pro His His Gly Val Pro Tyr
            420                 425                 430

Phe Arg Asn Ile Leu Gln Ser Glu Thr Glu Lys Leu Thr Ser His Cys
        435                 440                 445

Phe Glu Trp Asp Arg Lys Leu Glu Leu Asp Ile Pro Asp Asp Ala Lys
    450                 455                 460

Asp Leu Ile Arg Thr Ala Val Gly Gln Thr Arg Leu Leu Met Lys Glu
465                 470                 475                 480

Arg Phe Lys Gln Phe Glu Gly Leu Val Asp Asp Cys Glu Tyr Lys Arg
                485                 490                 495

Gly Ile Lys Glu Thr Thr Cys Thr Asp Leu Asp Gly Phe Trp Asp Met
            500                 505                 510

Val Ser Phe Gln Ile Glu Asp Val Ile His Lys Phe Asn Asn Leu Ile
        515                 520                 525

Lys Leu Glu Glu Ser Gly Trp Gln Val Asn Asn Asn Met Asn His Asn
    530                 535                 540

Met Asn Lys Asn Val Phe Arg Lys Lys Val Val Ser Gly Ile Ala Ser
545                 550                 555                 560

Lys Pro Lys Gln Asp Asp Ala Gly Arg Ile Ala Ala Arg Asn Arg Leu
                565                 570                 575

Ala Ala Ile Lys Asn Ala Met Arg Glu Arg Ile Arg Gln Glu Glu Cys
            580                 585                 590

Ala Glu Thr Ala Val Ser Val Ile Pro Lys Glu Val Asp Lys Ile Val
        595                 600                 605

Phe Asp Ala Gly Phe Phe Arg Val Glu Ser Pro Val Lys Leu Phe Ser
    610                 615                 620

Gly Leu Ser Val Ser Ser Glu Gly Pro Ser Gln Arg Leu Gly Thr Pro
625                 630                 635                 640

Lys Ser Val Asn Lys Ala Val Ser Gln Ser Arg Asn Glu Met Gly Ile
                645                 650                 655

Pro Gln Gln Thr Thr Ser Pro Glu Asn Ala Gly Pro Gln Asn Thr Lys
            660                 665                 670

Ser Glu His Val Lys Lys Thr Leu Phe Leu Ser Ile Pro Glu Ser Arg
        675                 680                 685

Ser Ser Ile Glu Asp Ala Gln Cys Pro Gly Leu Pro Asp Leu Ile Glu
    690                 695                 700

Glu Asn His Val Val Asn Lys Thr Asp Leu Lys Val Asp Cys Leu Ser
705                 710                 715                 720

Ser Glu Arg Met Ser Leu Pro Leu Leu Ala Gly Gly Val Ala Asp Asp
                725                 730                 735

Ile Asn Thr Asn Lys Lys Glu Gly Ile Ser Asp Val Val Glu Gly Met
            740                 745                 750

Glu Leu Asn Ser Ser Ile Thr Ser Gln Asp Val Leu Met Ser Ser Pro
        755                 760                 765

Glu Lys Asn Thr Ala Ser Gln Asn Ser Ile Leu Glu Glu Gly Glu Thr
    770                 775                 780

Lys Ile Ser Gln Ser Glu Leu Phe Asp Asn Lys Ser Leu Thr Thr Glu
785                 790                 795                 800

Cys His Leu Leu Asp Ser Pro Gly Leu Asn Cys Ser Asn Pro Phe Thr
                805                 810                 815

Gln Leu Glu Arg Arg His Gln Glu His Ala Arg His Ile Ser Phe Gly
            820                 825                 830

Gly Asn Leu Ile Thr Phe Ser Pro Leu Gln Pro Gly Glu Phe
        835                 840                 845

<210>  19
<211>  2284
<212>  DNA
<213>  Homo sapiens

<220>
<221>  CDS
<222>  (114)..(1268)
<223>  DUSP9 mRNA

<400> 19
cgcttcccgc cgcccgagct tcggaaactt cccggccgcg acgcagggaa ccggcgcgga 60

gaaccgagca gagcggagcg cccgtggtcc agcgtgtagg gagccgatcg ccc atg 116
Met
1

gag ggt ctg ggc cgc tcg tgc ctg tgg ctg cgt cgg gag ctg tcg ccc 164
Glu Gly Leu Gly Arg Ser Cys Leu Trp Leu Arg Arg Glu Leu Ser Pro
            5                   10                  15

ccg cgg ccg cgg ctc ctg ctc ctg gac tgc cgc agc cgc gag ctg tac 212
Pro Arg Pro Arg Leu Leu Leu Leu Asp Cys Arg Ser Arg Glu Leu Tyr
        20                  25                  30

gag tcg gcg cgc atc ggt ggg gcg ctg agc gtg gcc ctg ccg gcg ctc 260
Glu Ser Ala Arg Ile Gly Gly Ala Leu Ser Val Ala Leu Pro Ala Leu
    35                  40                  45

ctg ctg cgc cgc ctg cgg agg ggc agc ctg tcg gtg cgc gcg ctc ctg 308
Leu Leu Arg Arg Leu Arg Arg Gly Ser Leu Ser Val Arg Ala Leu Leu
50                  55                  60                  65

cct ggg ccg ccg ctg cag ccg ccc ccg cct gcc ccc gtg ctc ctg tac 356
Pro Gly Pro Pro Leu Gln Pro Pro Pro Pro Ala Pro Val Leu Leu Tyr
                70                  75                  80

gac cag ggc ggg ggc cgg cgc cgg cgc ggg gag gcc gag gcc gag gcc 404
Asp Gln Gly Gly Gly Arg Arg Arg Arg Gly Glu Ala Glu Ala Glu Ala
            85                  90                  95

gag gag tgg gag gcc gag tcg gtg ctg ggc acc ctg ctg cag aag ctg 452
Glu Glu Trp Glu Ala Glu Ser Val Leu Gly Thr Leu Leu Gln Lys Leu
        100                 105                 110

cga gag gaa ggc tac ctg gcc tac tac ctc cag gga ggc ttc agc aga 500
Arg Glu Glu Gly Tyr Leu Ala Tyr Tyr Leu Gln Gly Gly Phe Ser Arg
    115                 120                 125

ttc cag gcc gag tgc cct cac ctg tgt gag acc agc ctt gct ggc cgt 548
Phe Gln Ala Glu Cys Pro His Leu Cys Glu Thr Ser Leu Ala Gly Arg
130                 135                 140                 145

gcc ggc tcc agc atg gcg ccg gtg ccc ggt cca gtg ccc gtg gtg ggg 596
Ala Gly Ser Ser Met Ala Pro Val Pro Gly Pro Val Pro Val Val Gly
                150                 155                 160

ttg ggc agc ctg tgc ctg ggc tcc gac tgc tct gat gcg gaa tcc gag 644
Leu Gly Ser Leu Cys Leu Gly Ser Asp Cys Ser Asp Ala Glu Ser Glu
            165                 170                 175

gct gac cgc gac tcc atg agc tgt ggc ctg gat tcg gag ggt gcc aca 692
Ala Asp Arg Asp Ser Met Ser Cys Gly Leu Asp Ser Glu Gly Ala Thr
        180                 185                 190

ccc cca cca gtg ggg ctg cgg gca tcc ttc cct gtc cag atc ctg ccc 740
Pro Pro Pro Val Gly Leu Arg Ala Ser Phe Pro Val Gln Ile Leu Pro
    195                 200                 205

aac ctc tat ctg ggc agt gcc cgg gat tcc gcc aat ttg gag agc ctg 788
Asn Leu Tyr Leu Gly Ser Ala Arg Asp Ser Ala Asn Leu Glu Ser Leu
210                 215                 220                 225

gcc aaa ctg ggc atc cgc tac atc ctc aat gtc acc ccc aac ctc cca 836
Ala Lys Leu Gly Ile Arg Tyr Ile Leu Asn Val Thr Pro Asn Leu Pro
                230                 235                 240

aac ttc ttc gag aag aat ggt gac ttt cac tac aag cag atc ccc atc 884
Asn Phe Phe Glu Lys Asn Gly Asp Phe His Tyr Lys Gln Ile Pro Ile

245                 250                 255

tcc gac cac tgg agc cag aac ctg tcg cgg ttc ttt ccg gag gcc att      932
Ser Asp His Trp Ser Gln Asn Leu Ser Arg Phe Phe Pro Glu Ala Ile
        260                 265                 270

gag ttc att gat gag gcc ttg tcc cag aac tgc ggg gtg ctc gtc cac      980
Glu Phe Ile Asp Glu Ala Leu Ser Gln Asn Cys Gly Val Leu Val His
    275                 280                 285

tgc ttg gcg ggg gtc agc cgt tct gtc acc gtc act gtg gcc tac ctc     1028
Cys Leu Ala Gly Val Ser Arg Ser Val Thr Val Thr Val Ala Tyr Leu
290                 295                 300                 305

atg cag aag ctc cac ctc tct ctc aac gat gcc tat gac ctg gtc aag     1076
Met Gln Lys Leu His Leu Ser Leu Asn Asp Ala Tyr Asp Leu Val Lys
                310                 315                 320

agg aag aag tct aac atc tcc ccc aac ttc aac ttc atg ggg cag ttg     1124
Arg Lys Lys Ser Asn Ile Ser Pro Asn Phe Asn Phe Met Gly Gln Leu
            325                 330                 335

ctg gac ttt gag cgc agc ttg cgg ctg gag gag cgc cac tcg cag gag     1172
Leu Asp Phe Glu Arg Ser Leu Arg Leu Glu Glu Arg His Ser Gln Glu
        340                 345                 350

cag ggc agt ggg ggg cag gca tct gcg gcc tcc aac ccg ccc tcc ttc     1220
Gln Gly Ser Gly Gly Gln Ala Ser Ala Ala Ser Asn Pro Pro Ser Phe
    355                 360                 365

ttc acc acc ccc acc agt gat ggc gcc ttc gag ctg gcc ccc acc tag     1268
Phe Thr Thr Pro Thr Ser Asp Gly Ala Phe Glu Leu Ala Pro Thr
370                 375                 380

ggccccgtgg ccggcaggcc ggcccctgcc ccacccccac ccacgggtgt ccctgcccac     1328

tcgtgtggca agggagggga gggcaggagg gctcggcctg agcagggtgc tggggggaga     1388

gcgcaatacc tcacgcgggc tgccgtccta atcaacgtgc ctatggcggg accacgctcg     1448

gagcctgcct cttctgcgac tgttactttt tctttgcggg atgggggtgg gggttccctc     1508

tccaggtggt tgtccaggcc catgtcccgg ccctgggtgc tcagccagct cggctaggcc     1568

ctgcgcctcc ctgcgcttcc cccttcagga agggtgtgtg ccacctcgtt gcactggatc     1628

ccagtggctg cttgggggag aggcgtttgc catcactggt gttgtcacct ccctgtttct     1688

ccaccaaggg cttgggcctc tcggggctgg ggcctcccag gggatgggga cccagagtgc     1748

agtggccgcc cacatccatg gcctaggagc tactgggcag gttcccggcc acacatctgg     1808

tgggctgttt tgtttttttt ttttcctctt cccccagatg tcttgacggg atcactgggg     1868

ctctttgtga gtgagggtgg ccaaactacc gccggaggag atggggtctc agagcgagag     1928

ctgcggaggg ggaggggaag aagaaggcct cacttttgct gctgcggggc ccacacagcc     1988

gctgctactt tggggggtgg ggaaggggcc aagctgcaga cacacacagt cattcatttc     2048

tgtccacacc cctgtgggtg gcgggtgtgc gtgtgtgtgc ttgtgtgtgc gcacgtgtcg     2108

gcgctcacac acacatgcta gcccactgat gcacccagcc cagggctggc agtctttgca     2168

gcgtggggcc gtctcaccct ggagcctgga gaggatctat gcttgtttgt ttttgtaatc     2228

catatcatag ttgctttctt taattgttcc ttctgaataa acagtttatt taagat     2284

<210>  20
<211>  384
<212>  PRT
<213>  Homo sapiens

<400>  20

Met Glu Gly Leu Gly Arg Ser Cys Leu Trp Leu Arg Arg Glu Leu Ser
1               5                   10                  15

Pro Pro Arg Pro Arg Leu Leu Leu Leu Asp Cys Arg Ser Arg Glu Leu
            20                  25                  30

Tyr Glu Ser Ala Arg Ile Gly Gly Ala Leu Ser Val Ala Leu Pro Ala
        35                  40                  45

Leu Leu Leu Arg Arg Leu Arg Arg Gly Ser Leu Ser Val Arg Ala Leu
    50                  55                  60

Leu Pro Gly Pro Pro Leu Gln Pro Pro Pro Pro Ala Pro Val Leu Leu
65                  70                  75                  80

Tyr Asp Gln Gly Gly Gly Arg Arg Arg Arg Gly Glu Ala Glu Ala Glu
                85                  90                  95

Ala Glu Glu Trp Glu Ala Glu Ser Val Leu Gly Thr Leu Leu Gln Lys
            100                 105                 110

Leu Arg Glu Glu Gly Tyr Leu Ala Tyr Tyr Leu Gln Gly Gly Phe Ser
        115                 120                 125

Arg Phe Gln Ala Glu Cys Pro His Leu Cys Glu Thr Ser Leu Ala Gly
    130                 135                 140

Arg Ala Gly Ser Ser Met Ala Pro Val Pro Gly Pro Val Pro Val Val
145                 150                 155                 160

Gly Leu Gly Ser Leu Cys Leu Gly Ser Asp Cys Ser Asp Ala Glu Ser
                165                 170                 175

Glu Ala Asp Arg Asp Ser Met Ser Cys Gly Leu Asp Ser Glu Gly Ala
            180                 185                 190

Thr Pro Pro Pro Val Gly Leu Arg Ala Ser Phe Pro Val Gln Ile Leu
        195                 200                 205

Pro Asn Leu Tyr Leu Gly Ser Ala Arg Asp Ser Ala Asn Leu Glu Ser
    210                 215                 220

Leu Ala Lys Leu Gly Ile Arg Tyr Ile Leu Asn Val Thr Pro Asn Leu
225                 230                 235                 240

Pro Asn Phe Phe Glu Lys Asn Gly Asp Phe His Tyr Lys Gln Ile Pro
                245                 250                 255

Ile Ser Asp His Trp Ser Gln Asn Leu Ser Arg Phe Phe Pro Glu Ala
            260                 265                 270

Ile Glu Phe Ile Asp Glu Ala Leu Ser Gln Asn Cys Gly Val Leu Val
        275                 280                 285

His Cys Leu Ala Gly Val Ser Arg Ser Val Thr Val Thr Val Ala Tyr
    290                 295                 300

Leu Met Gln Lys Leu His Leu Ser Leu Asn Asp Ala Tyr Asp Leu Val
305                 310                 315                 320

Lys Arg Lys Lys Ser Asn Ile Ser Pro Asn Phe Asn Phe Met Gly Gln
                325                 330                 335

Leu Leu Asp Phe Glu Arg Ser Leu Arg Leu Glu Glu Arg His Ser Gln
            340                 345                 350

Glu Gln Gly Ser Gly Gly Gln Ala Ser Ala Ala Ser Asn Pro Pro Ser
        355                 360                 365

Phe Phe Thr Thr Pro Thr Ser Asp Gly Ala Phe Glu Leu Ala Pro Thr
    370                 375                 380

<210>  21
<211>  1724
<212>  DNA
<213>  Homo sapiens

<220>
<221>  CDS
<222>  (31)..(1071)
<223>  E2F5 mRNA

<400>  21
ggggcccgac caccgcgggg ccgggacgcg atg gcg gcg gca gag ccc gcg agc      54
                                 Met Ala Ala Ala Glu Pro Ala Ser
                                 1               5

tcg ggc cag cag gcg ccg gca ggg cag ggg cag ggc cag cgg ccg ccg      102
Ser Gly Gln Gln Ala Pro Ala Gly Gln Gly Gln Gly Gln Arg Pro Pro
    10                  15                  20

ccg cag cct ccg cag gcg caa gcc ccg cag ccg ccc ccg ccg ccg cag      150
Pro Gln Pro Pro Gln Ala Gln Ala Pro Gln Pro Pro Pro Pro Pro Gln
25                  30                  35                  40

ctc ggg ggc gcc ggg ggc ggc agc agc agg cac gag aag agc ctg ggg      198
Leu Gly Gly Ala Gly Gly Gly Ser Ser Arg His Glu Lys Ser Leu Gly
                45                  50                  55

ctg ctc act acc aag ttc gtg tcg ctg ctg cag gag gcc aag gac ggc      246
Leu Leu Thr Thr Lys Phe Val Ser Leu Leu Gln Glu Ala Lys Asp Gly
            60                  65                  70

gtt ctg gat ctc aaa gcg gct gct gat act ttg gct gtg agg caa aaa      294
Val Leu Asp Leu Lys Ala Ala Ala Asp Thr Leu Ala Val Arg Gln Lys
        75                  80                  85

agg aga att tat gat atc acc aat gtc tta gag gga att gac ttg att 342
Arg Arg Ile Tyr Asp Ile Thr Asn Val Leu Glu Gly Ile Asp Leu Ile
    90                  95                  100

gaa aaa aag tca aaa aac agt atc cag tgg aaa ggt gta ggt gct ggc 390
Glu Lys Lys Ser Lys Asn Ser Ile Gln Trp Lys Gly Val Gly Ala Gly
105                 110                 115                 120

tgt aat act aaa gaa gtc ata gat aga tta aga tat ctt aaa gct gaa 438
Cys Asn Thr Lys Glu Val Ile Asp Arg Leu Arg Tyr Leu Lys Ala Glu
                125                 130                 135

att gaa gat cta gaa ctg aag gaa aga gaa ctt gat cag cag aag ttg 486
Ile Glu Asp Leu Glu Leu Lys Glu Arg Glu Leu Asp Gln Gln Lys Leu
            140                 145                 150

tgg cta cag caa agc atc aaa aat gtg atg gac gat tcc att aat aat 534
Trp Leu Gln Gln Ser Ile Lys Asn Val Met Asp Asp Ser Ile Asn Asn
        155                 160                 165

aga ttt tcc tat gta act cat gaa gac atc tgt aat tgc ttt aat ggt 582
Arg Phe Ser Tyr Val Thr His Glu Asp Ile Cys Asn Cys Phe Asn Gly
    170                 175                 180

gat aca ctt ttg gcc att cag gca cct tct ggt aca caa ctg gag gta 630
Asp Thr Leu Leu Ala Ile Gln Ala Pro Ser Gly Thr Gln Leu Glu Val
185                 190                 195                 200

ccc att cca gaa atg ggt cag aat gga caa aag aaa tac cag atc aat 678
Pro Ile Pro Glu Met Gly Gln Asn Gly Gln Lys Lys Tyr Gln Ile Asn
                205                 210                 215

cta aag agt cat tca gga cct atc cat gtg ctg ctt ata aat aaa gag 726
Leu Lys Ser His Ser Gly Pro Ile His Val Leu Leu Ile Asn Lys Glu
            220                 225                 230

tcg agt tca tct aag ccc gtg gtt ttt cct gtt ccc cca cct gat gac 774
Ser Ser Ser Ser Lys Pro Val Val Phe Pro Val Pro Pro Pro Asp Asp
        235                 240                 245

ctc aca cag cct tcc tcc cag tcc ttg act cca gtg act cca cag aaa 822
Leu Thr Gln Pro Ser Ser Gln Ser Leu Thr Pro Val Thr Pro Gln Lys
    250                 255                 260

tcc agc atg gca act caa aat ctg cct gag caa cat gtc tct gaa aga 870
Ser Ser Met Ala Thr Gln Asn Leu Pro Glu Gln His Val Ser Glu Arg
265                 270                 275                 280

agc cag gct ctg cag cag aca tca gct aca gat ata tct tca gca gga 918
Ser Gln Ala Leu Gln Gln Thr Ser Ala Thr Asp Ile Ser Ser Ala Gly
                285                 290                 295

tct att agt gga gat atc att gat gag tta atg tct tct gac gtg ttt 966
Ser Ile Ser Gly Asp Ile Ile Asp Glu Leu Met Ser Ser Asp Val Phe
            300                 305                 310

cct ctc tta agg ctt tct cct acc ccg gca gat gac tac aac ttt aat 1014
Pro Leu Leu Arg Leu Ser Pro Thr Pro Ala Asp Asp Tyr Asn Phe Asn
        315                 320                 325

tta gat gat aac gaa gga gtt tgt gat ctg ttt gat gtc cag ata cta 1062
Leu Asp Asp Asn Glu Gly Val Cys Asp Leu Phe Asp Val Gln Ile Leu
    330                 335                 340

aat tat tag attccatgga aacttgggac tgttatctac ctctaactgt 1111
Asn Tyr
345

gtaacatttt agacttctta ataacctaaa tatttaaaat aatgaatgta acaccttttt 1171

tagttcactg attctgaagt gttcttccct aatactttct ttacttcaca aaacttcaac 1231

cataaaaaca aagggctctg attgctttag gggataagtg atttaatatc cacaaacgtc 1291

cccactccca aaagtaacta tattctggat ttcaactttt cttctaattg tgaatccttc 1351

tgttttttct tcttaaggag gaaagttaaa ggacactaca ggtcatcaaa aacaagttgg 1411

ccaaggactc attacttgtc ttatattttt actgccacta aactgcctgt atttctgtat 1471

gtccttctat ccaaacagac gttcactgcc acttgtaaag tgaaggatgt aaacgaggat 1531

atataactgt ttcagtgaac agattttgtg aagtgccttc tgttttagca ctttaagttt 1591

atcacatttt gttgacttct gacattccac tttcctaggt tataggaaag atctgtttat 1651

gtagtttgtt tttaaaatgt gccaatgcct gtacattaac aagattttta aaaataaaat 1711

tgtataaaac att 1724

<210> 22
<211> 346
<212> PRT
<213> Homo sapiens

<400> 22

Met Ala Ala Ala Glu Pro Ala Ser Ser Gly Gln Gln Ala Pro Ala Gly
1               5                   10                  15

Gln Gly Gln Gly Gln Arg Pro Pro Pro Gln Pro Pro Gln Ala Gln Ala
            20                  25                  30

Pro Gln Pro Pro Pro Pro Pro Gln Leu Gly Gly Ala Gly Gly Gly Ser
        35                  40                  45

Ser Arg His Glu Lys Ser Leu Gly Leu Leu Thr Thr Lys Phe Val Ser
    50                  55                  60

Leu Leu Gln Glu Ala Lys Asp Gly Val Leu Asp Leu Lys Ala Ala Ala
65                  70                  75                  80

Asp Thr Leu Ala Val Arg Gln Lys Arg Arg Ile Tyr Asp Ile Thr Asn
                85                  90                  95

Val Leu Glu Gly Ile Asp Leu Ile Glu Lys Lys Ser Lys Asn Ser Ile
            100                 105                 110

Gln Trp Lys Gly Val Gly Ala Gly Cys Asn Thr Lys Glu Val Ile Asp
        115                 120                 125

Arg Leu Arg Tyr Leu Lys Ala Glu Ile Glu Asp Leu Glu Leu Lys Glu
    130                 135                 140

Arg Glu Leu Asp Gln Gln Lys Leu Trp Leu Gln Gln Ser Ile Lys Asn
145                 150                 155                 160

Val Met Asp Asp Ser Ile Asn Asn Arg Phe Ser Tyr Val Thr His Glu
                165                 170                 175

Asp Ile Cys Asn Cys Phe Asn Gly Asp Thr Leu Leu Ala Ile Gln Ala
            180                 185                 190

Pro Ser Gly Thr Gln Leu Glu Val Pro Ile Pro Glu Met Gly Gln Asn
        195                 200                 205

Gly Gln Lys Lys Tyr Gln Ile Asn Leu Lys Ser His Ser Gly Pro Ile
    210                 215                 220

His Val Leu Leu Ile Asn Lys Glu Ser Ser Ser Ser Lys Pro Val Val
225                 230                 235                 240

Phe Pro Val Pro Pro Pro Asp Asp Leu Thr Gln Pro Ser Ser Gln Ser
                245                 250                 255

Leu Thr Pro Val Thr Pro Gln Lys Ser Ser Met Ala Thr Gln Asn Leu
            260                 265                 270

Pro Glu Gln His Val Ser Glu Arg Ser Gln Ala Leu Gln Gln Thr Ser
        275                 280                 285

Ala Thr Asp Ile Ser Ser Ala Gly Ser Ile Ser Gly Asp Ile Ile Asp
    290                 295                 300

Glu Leu Met Ser Ser Asp Val Phe Pro Leu Leu Arg Leu Ser Pro Thr
305                 310                 315                 320

Pro Ala Asp Asp Tyr Asn Phe Asn Leu Asp Asp Asn Glu Gly Val Cys
                325                 330                 335

Asp Leu Phe Asp Val Gln Ile Leu Asn Tyr
            340                 345

<210>  23
<211>  4413
<212>  DNA
<213>  Homo sapiens

<220>
<221>  CDS
<222>  (44)..(1960)
<223>  GHR mRNA

<400>  23
ccgcgctctc tgatcagagg cgaagctcgg aggtcctaca ggt atg gat ctc tgg        55
cag ctg ctg ttg acc ttg gca ctg gca gga tca agt gat gct ttt tct        103
gga agt gag gcc aca gca gct atc ctt agc aga gca ccc tgg agt ctg 151
caa agt gtt aat cca ggc cta aag aca aat tct tct aag gag cct aaa 199
ttc acc aag tgc cgt tca cct gag cga gag act ttt tca tgc cac tgg 247
aca gat gag gtt cat cat ggt aca aag aac cta gga ccc ata cag ctg 295
ttc tat acc aga agg aac act caa gaa tgg act caa gaa tgg aaa gaa 343
tgc cct gat tat gtt tct gct ggg gaa aac agc tgt tac ttt aat tca 391
tcg ttt acc tcc atc tgg ata cct tat tgt atc aag cta act agc aat 439
ggt ggt aca gtg gat gaa aag tgt ttc tct gtt gat gaa ata gtg caa 487
cca gat cca ccc att gcc ctc aac tgg act tta ctg aac gtc agt tta 535
act ggg att cat gca gat atc caa gtg aga tgg gaa gca cca cgc aat 583
gca gat att cag aaa gga tgg atg gtt ctg gag tat gaa ctt caa tac 631
aaa gaa gta aat gaa act aaa tgg aaa atg atg gac cct ata ttg aca 679
aca tca gtt cca gtg tac tca ttg aaa gtg gat aag gaa tat gaa gtg 727
cgt gtg aga tcc aaa caa cga aac tct gga aat tat ggc gag ttc agt 775
gag gtg ctc tat gta aca ctt cct cag atg agc caa ttt aca tgt gaa 823
gaa gat ttc tac ttt cca tgg ctc tta att att atc ttt gga ata ttt 871
ggg cta aca gtg atg cta ttt gta ttc tta ttt tct aaa cag caa agg 919
att aaa atg ctg att ctg ccc cca gtt cca gtt cca aag att aaa gga 967
atc gat cca gat ctc ctc aag gaa gga aaa tta gag gag gtg aac aca      1015
atc tta gcc att cat gat agc tat aaa ccc gaa ttc cac agt gat gac      1063
tct tgg gtt gaa ttt att gag cta gat att gat gag cca gat gaa aag      1111
act gag gaa tca gac aca gac aga ctt cta agc agt gac cat gag aaa      1159
tca cat agt aac cta ggg gtg aag gat ggc gac tct gga cgt acc agc      1207
tgt tgt gaa cct gac att ctg gag act gat ttc aat gcc aat gac ata      1255
cat gag ggt acc tca gag gtt gct cag cca cag agg tta aaa ggg gaa      1303
gca gat ctc tta tgc ctt gac cag aag aat caa aat aac tca cct tat      1351
cat gat gct tgc cct gct act cag cag ccc agt gtt atc caa gca gag      1399
aaa aac aaa cca caa cca ctt cct act gaa gga gct gag tca act cac      1447
caa gct gcc cat att cag cta agc aat cca agt tca ctg tca aac atc      1495
gac ttt tat gcc cag gtg agc gac att aca cca gca ggt agt gtg gtc      1543
ctt tcc ccg ggc caa aag aat aag gca ggg atg tcc caa tgt gac atg      1591
cac ccg gaa atg gtc tca ctc tgc caa gaa aac ttc ctt atg gac aat      1639
gcc tac ttc tgt gag gca gat gcc aaa aag tgc atc cct gtg gct cct      1687
cac atc aag gtt gaa tca cac ata cag cca agc tta aac caa gag gac      1735
att tac atc acc aca gaa agc ctt acc act gct gct ggg agg cct ggg      1783
aca gga gaa cat gtt cca ggt tct gag atg cct gtc cca gac tat acc      1831
tcc att cat ata gta cag tcc cca cag ggc ctc ata ctc aat gcg act      1879
gcc ttg ccc ttg cct gac aaa gag ttt ctc tca tca tgt ggc tat gtg      1927
agc aca gac caa ctg aac aaa atc atg cct tag cctttctttg gtttcccaag    1980
agctacgtat ttaatagcaa agaattgact ggggcaataa cgtttaagcc aaaacaatgt    2040
ttaaaccttt tttgggggag tgacaggatg gggtatggat tctaaaatgc cttttcccaa    2100
aatgttgaaa tatgatgtta aaaaaataag aagaatgctt aatcagatag atattcctat    2160
tgtgcaatgt aaatatttta aagaattgtg tcagactgtt tagtagcagt gattgtctta    2220
atattgtggg tgttaatttt tgatactaag cattgaatgg ctatgttttt aatgtatagt    2280
aaatcacgct ttttgaaaaa gcgaaaaaat caggtggctt ttgcggttca ggaaaattga    2340
atgcaaacca tagcacaggc taattttttg ttgtttctta aataagaaac ttttttattt    2400
aaaaaactaa aaactagagg tgagaaattt aaactataag caagaaggca aaaatagttt    2460
ggatatgtaa aacatttatt ttgacataaa gttgataaag attttttaat aatttagact    2520
tcaagcatgg ctattttata ttacactaca cactgtgtac tgcagttggt atgacccctc    2580
taaggagtgt agcaactaca gtctaaagct ggtttaatgt tttggccaat gcacctaaag    2640
aaaaacaaac tcgtttttta caaagccctt ttatacctcc ccagactcct tcaacaattc    2700
taaaatgatt gtagtaatct gcattattgg aatataattg ttttatctga atttttaaac    2760
aagtatttgt taatttagaa aactttaaag cgtttgcaca gatcaactta ccaggcacca    2820
aaagaagtaa aagcaaaaaa gaaaaccttt cttcaccaaa tcttggttga tgccaaaaaa    2880
aaatacatgc taagagaagt agaaatcata gctggttcac actgaccaag atacttaagt    2940
gctgcaattg cacgcggagt gagtttttta gtgcgtgcag atggtgagag ataagatcta    3000
tagcctctgc agcggaatct gttcacaccc aacttggttt tgctacataa ttatccagga    3060
agggaataag gtacaagaag cattttgtaa gttgaagcaa atcgaatgaa attaactggg    3120
taatgaaaca aagagttcaa gaaataagtt tttgtttcac agcctataac cagacacata    3180
ctcatttttc atgataatga acagaacata gacagaagaa acaaggtttt cagtccccac    3240
agataactga aaattattta aaccgctaaa agaaactttc tttctcacta aatcttttat    3300
aggatttatt taaaatagca aaagaagaag tttcatcatt ttttacttcc tctctgagtg    3360
gactggcctc aaagcaagca ttcagaagaa aaagaagcaa cctcagtaat ttagaaatca    3420
ttttgcaatc ccttaatatc ctaaacatca ttcatttttg ttgttgttgt tgttgttgag    3480
acagagtctc gctctgtcgc caggctagag tgcggtggcg cgatcttgac tcactgcaat    3540
ctccacctcc cacaggttca ggcgattccc gtgcctcagc ctcctgagta gctgggacta 3600
caggcacgca ccaccatgcc aggctaattt ttttgtattt tagcagagac ggggtttcac 3660
catgttggcc aggatggtct cgatctcctg acctcgtgat ccacccgact cggcctccca 3720
aagtgctggg attacaggtg taagccaccg tgcccagccc taaacatcat tcttgagagc 3780
attgggatat ctcctgaaaa ggtttatgaa aaagaagaat ctcatctcag tgaagaatac 3840
ttctcatttt ttaaaaaagc ttaaaacttt gaagttagct ttaacttaaa tagtatttcc 3900
catttatcgc agaccttttt taggaagcaa gcttaatggc tgataatttt aaattctctc 3960
tcttgcagga aggactatga aaagctagaa ttgagtgttt aaagttcaac atgttatttg 4020
taatagatgt ttgatagatt ttctgctact ttgctgctat ggttttctcc aagagctaca 4080
taatttagtt tcatataaag tatcatcagt gtagaaccta attcaattca aagctgtgtg 4140
tttggaagac tatcttacta tttcacaaca gcctgacaac atttctatag ccaaaaatag 4200
ctaaatacct caatcagtct cagaatgtca ttttggtact ttggtggcca cataagccat 4260
tattcactag tatgactagt tgtgtctggc agtttatatt taactctctt tatgtctgtg 4320
gattttttcc ttcaaagttt aataaattta ttttcttgga ttcctgatag tgtgcttctg 4380
ttatcaaaca ccaacataaa aatgatctaa acc 4413

<210> 24
<211> 638
<212> PRT
<213> Homo sapiens

<400> 24

<210>  25
<211>  1403
<212>  DNA
<213>  Homo sapiens

<220>
<221>  CDS

<222>  (23)..(1204)
<223>  HPD mRNA

<400>  25
ggaggtttga ctaagatcaa tc atg acg act tac agt gac aaa ggg gca aag      52
                         Met Thr Thr Tyr Ser Asp Lys Gly Ala Lys
                         1               5                   10

cct gag aga ggc cga ttc ctc cac ttc cac tct gtg acc ttc tgg gtt      100
Pro Glu Arg Gly Arg Phe Leu His Phe His Ser Val Thr Phe Trp Val
                15                  20                  25

ggc aac gcc aag cag gcc acg tca ttc tac tgc agc aag atg ggc ttt      148
Gly Asn Ala Lys Gln Ala Thr Ser Phe Tyr Cys Ser Lys Met Gly Phe
            30                  35                  40

gaa cct cta gcc tac agg ggc ctg gag acc ggt tcc cgg gag gtg gtc      196
Glu Pro Leu Ala Tyr Arg Gly Leu Glu Thr Gly Ser Arg Glu Val Val
        45                  50                  55

agc cat gta atc aaa caa ggg aag att gtg ttt gtc ctc tcc tca gcg      244
Ser His Val Ile Lys Gln Gly Lys Ile Val Phe Val Leu Ser Ser Ala
    60                  65                  70

ctc aac ccc tgg aac aaa gag atg ggc gat cac ctg gtg aaa cac ggt      292
Leu Asn Pro Trp Asn Lys Glu Met Gly Asp His Leu Val Lys His Gly
75                  80                  85                  90

gac gga gtg aag gac att gcg ttc gag gtg gaa gat tgt gac tac atc      340
Asp Gly Val Lys Asp Ile Ala Phe Glu Val Glu Asp Cys Asp Tyr Ile
                95                  100                 105

gtg cag aaa gca cgg gaa cgg ggc gcc aaa atc atg cgg gag ccc tgg      388
Val Gln Lys Ala Arg Glu Arg Gly Ala Lys Ile Met Arg Glu Pro Trp
            110                 115                 120

gta gag caa gac aag ttt ggg aag gtg aag ttt gct gtg ctg cag acg      436
Val Glu Gln Asp Lys Phe Gly Lys Val Lys Phe Ala Val Leu Gln Thr
        125                 130                 135

tat ggg gac acc aca cac acc ctg gtg gag aag atg aac tac atc ggc      484
Tyr Gly Asp Thr Thr His Thr Leu Val Glu Lys Met Asn Tyr Ile Gly
    140                 145                 150

caa ttc ttg cct gga tat gag gcc cca gcg ttc atg gac ccc cta ctt      532
Gln Phe Leu Pro Gly Tyr Glu Ala Pro Ala Phe Met Asp Pro Leu Leu
155                 160                 165                 170

cct aaa ctg ccc aaa tgc agt ctg gag atg atc gac cac att gtg gga      580
Pro Lys Leu Pro Lys Cys Ser Leu Glu Met Ile Asp His Ile Val Gly
                175                 180                 185

aac cag cct gat cag gag atg gtg tcc gcc tcc gaa tgg tac ctg aaa      628
Asn Gln Pro Asp Gln Glu Met Val Ser Ala Ser Glu Trp Tyr Leu Lys
            190                 195                 200

aac ctg cag ttc cac cgc ttc tgg tcc gtg gat gac acg cag gtg cac      676
Asn Leu Gln Phe His Arg Phe Trp Ser Val Asp Asp Thr Gln Val His
        205                 210                 215

acg gaa tat agc tct ctg cga tcc att gtg gtg gcc aac tat gaa gag      724
Thr Glu Tyr Ser Ser Leu Arg Ser Ile Val Val Ala Asn Tyr Glu Glu
    220                 225                 230

tcc atc aag atg ccc atc aat gag cca gcg cct ggc aag aag aag tcc      772
Ser Ile Lys Met Pro Ile Asn Glu Pro Ala Pro Gly Lys Lys Lys Ser
235                 240                 245                 250

cag atc cag gaa tat gtg gac tat aac ggg ggc gct ggg gtc cag cac      820

Gln Ile Gln Glu Tyr Val Asp Tyr Asn Gly Gly Ala Gly Val Gln His
                255                 260                 265

atc gct ctc aag acc gaa gac atc atc aca gcg att cgc cac ttg aga      868
Ile Ala Leu Lys Thr Glu Asp Ile Ile Thr Ala Ile Arg His Leu Arg
            270                 275                 280

gag aga ggc ctg gag ttc tta tct gtt ccc tcc acg tac tac aaa caa      916
Glu Arg Gly Leu Glu Phe Leu Ser Val Pro Ser Thr Tyr Tyr Lys Gln
        285                 290                 295

ctg cgg gag aag ctg aag acg gcc aag atc aag gtg aag gag aac att      964
Leu Arg Glu Lys Leu Lys Thr Ala Lys Ile Lys Val Lys Glu Asn Ile
    300                 305                 310

gat gcc ctg gag gag ctg aaa atc ctg gtg gac tac gac gag aaa ggc     1012
Asp Ala Leu Glu Glu Leu Lys Ile Leu Val Asp Tyr Asp Glu Lys Gly
315                 320                 325                 330

tac ctc ctg cag atc ttc acc aaa ccg gtg cag gac cgg ccc acg ctc     1060
Tyr Leu Leu Gln Ile Phe Thr Lys Pro Val Gln Asp Arg Pro Thr Leu
                335                 340                 345

ttc ctg gaa gtc atc cag cgc cac aac cac cag ggt ttt gga gcc ggc     1108
Phe Leu Glu Val Ile Gln Arg His Asn His Gln Gly Phe Gly Ala Gly
            350                 355                 360

aac ttc aac tca ctg ttc aag gct ttc gag gag gag cag aac ctg cgg     1156
Asn Phe Asn Ser Leu Phe Lys Ala Phe Glu Glu Glu Gln Asn Leu Arg
        365                 370                 375

ggt aac ctc acc aac atg gag acc aat ggg gtg gtg ccc ggc atg taa     1204
Gly Asn Leu Thr Asn Met Glu Thr Asn Gly Val Val Pro Gly Met
    380                 385                 390

gccccgccca ccccacggag gccacagcca cacagccacg ccccctgatt ctggaactcg   1264

cccaacttcc ctactggctg ctccccttgg gtcccgccca ccagcggact cggcccccaa   1324

ggctccgccc acactgacca cgcccctcgg cggggccgcc ctctgctcca gccctcccga   1384

ttaaagcgtg ccccggtcc                                                1403

<210>  26
<211>  393
<212>  PRT
<213>  Homo sapiens

<400>  26

Met Thr Thr Tyr Ser Asp Lys Gly Ala Lys Pro Glu Arg Gly Arg Phe
1               5                   10                  15

Leu His Phe His Ser Val Thr Phe Trp Val Gly Asn Ala Lys Gln Ala
            20                  25                  30

Thr Ser Phe Tyr Cys Ser Lys Met Gly Phe Glu Pro Leu Ala Tyr Arg
        35                  40                  45

Gly Leu Glu Thr Gly Ser Arg Glu Val Val Ser His Val Ile Lys Gln
    50                  55                  60

Gly Lys Ile Val Phe Val Leu Ser Ser Ala Leu Asn Pro Trp Asn Lys
65                  70                  75                  80

Glu Met Gly Asp His Leu Val Lys His Gly Asp Gly Val Lys Asp Ile
                85                  90                  95

Ala Phe Glu Val Glu Asp Cys Asp Tyr Ile Val Gln Lys Ala Arg Glu
            100                 105                 110

Arg Gly Ala Lys Ile Met Arg Glu Pro Trp Val Glu Gln Asp Lys Phe
        115                 120                 125

Gly Lys Val Lys Phe Ala Val Leu Gln Thr Tyr Gly Asp Thr Thr His
    130                 135                 140

Thr Leu Val Glu Lys Met Asn Tyr Ile Gly Gln Phe Leu Pro Gly Tyr
145                 150                 155                 160

Glu Ala Pro Ala Phe Met Asp Pro Leu Leu Pro Lys Leu Pro Lys Cys
                165                 170                 175

Ser Leu Glu Met Ile Asp His Ile Val Gly Asn Gln Pro Asp Gln Glu
            180                 185                 190

Met Val Ser Ala Ser Glu Trp Tyr Leu Lys Asn Leu Gln Phe His Arg
        195                 200                 205

Phe Trp Ser Val Asp Asp Thr Gln Val His Thr Glu Tyr Ser Ser Leu
    210                 215                 220

Arg Ser Ile Val Val Ala Asn Tyr Glu Glu Ser Ile Lys Met Pro Ile
225                 230                 235                 240

Asn Glu Pro Ala Pro Gly Lys Lys Lys Ser Gln Ile Gln Glu Tyr Val
                245                 250                 255

Asp Tyr Asn Gly Gly Ala Gly Val Gln His Ile Ala Leu Lys Thr Glu
            260                 265                 270

Asp Ile Ile Thr Ala Ile Arg His Leu Arg Glu Arg Gly Leu Glu Phe
        275                 280                 285

Leu Ser Val Pro Ser Thr Tyr Tyr Lys Gln Leu Arg Glu Lys Leu Lys
    290                 295                 300

Thr Ala Lys Ile Lys Val Lys Glu Asn Ile Asp Ala Leu Glu Glu Leu
305                 310                 315                 320

Lys Ile Leu Val Asp Tyr Asp Glu Lys Gly Tyr Leu Leu Gln Ile Phe
                325                 330                 335

Thr Lys Pro Val Gln Asp Arg Pro Thr Leu Phe Leu Glu Val Ile Gln
            340                 345                 350

Arg His Asn His Gln Gly Phe Gly Ala Gly Asn Phe Asn Ser Leu Phe
        355                 360                 365

Lys Ala Phe Glu Glu Glu Gln Asn Leu Arg Gly Asn Leu Thr Asn Met
    370                 375                 380

Glu Thr Asn Gly Val Val Pro Gly Met
385                 390

<210>  27
<211>  5413
<212>  DNA
<213>  Homo sapiens

<220>
<221>  CDS
<222>  (1145)..(5128)
<223>  IGSF1 mRNA

<400>  27
ctccctgacc ccttgcgctt cccgagtgag gcaatgcctc gccctgcttc ggctcgcgga      60

cggtgcgcgc acccactgac ctgcgcccac tgtctggcac tccctagtga gatgaacccg     120

gtacctcaga tggaaatgca gaaatcaccc gtcttctgcg tcgctcacgc tgggagctgt     180

agatcgcagc tgttcctatt cggccatctt ggctcctcca ttctgctttt aagatacaag     240

tgatgccttc aaatttgagc tcagtttctt atggtcgttt aggagcatga gggtttctgc     300

aataatctac cagtgaaatg atgatcaaga tcttggaaag cactctactg gtcaagcaga     360

atagttcctg tcatctccat taagaatcag cttattggcc gggcgcagtg gctcatgcct     420

gtaatcccag cactttggga ggctgaggcg ggtggatcac gaggtcagga gatcgagacc     480

atcctgtcta acaaggaatc ctttctggca ctcaagagtt ttcaattcaa tcctgatgaa     540

tgaatcatta tttgatacca gcctaagtga agactctgcc tggtgacttt tgcaaggtgg     600

tttctggctg caacatggga agatttggga tcagtgagat ggaagagatt tttctggcta     660

ctagcgttcc taagaaagag ctttagtgtt tatccagcta ttggtatgca gacaactata     720

cccttatgag aaacactagc agagaagaag agttcttgac tgggtctggg ccatgaaact     780

gaccggataa aagtgggaga agaagagatg ttgaaggagg agtttgctgt tttctcagtc     840

tcctgagaaa ttccagatat cttctttcta cacacatcct ggagagggga aagaaagcct     900

ctctctgtca cccaggctgg aatgcactgg tgccatctcc actcactgca gcttcaacct     960

cctgggttca agtgatcctc ctgcctctca gccttccgag tagctgggac tacagtttgc    1020

tgcatctgga ggagctcact ggagaatctc caacatcgga gcgggccttc aactaccatc    1080

ccaccacctg ctgaggagaa aaattcttca agactcagag cacacagcca gcaccagagg    1140

cccc atg acc ctg gac aga cca ggg gag ggg gcc acc atg ctg aag aca     1189
     Met Thr Leu Asp Arg Pro Gly Glu Gly Ala Thr Met Leu Lys Thr
     1               5                   10                  15

ttc act gtt ttg ctc ttt tgc att ctg atg gac cct caa cca gag ttg     1237
Phe Thr Val Leu Leu Phe Cys Ile Leu Met Asp Pro Gln Pro Glu Leu

                    20                        25                        30

tgg ata gag tcc aac tac ccc cag gcc cct tgg gag aac atc acg ctt        1285
Trp Ile Glu Ser Asn Tyr Pro Gln Ala Pro Trp Glu Asn Ile Thr Leu
                35                  40                  45

tgg tgc cga agc ccc tct cgg ata tca agc aag ttc ctg ctg ctg aag        1333
Trp Cys Arg Ser Pro Ser Arg Ile Ser Ser Lys Phe Leu Leu Leu Lys
        50                  55                  60

gat aag aca cag atg acc tgg atc cgc cct tcc cac aag acc ttc caa        1381
Asp Lys Thr Gln Met Thr Trp Ile Arg Pro Ser His Lys Thr Phe Gln
    65                  70                  75

gtt tca ttc ctt ata ggt gcc ctt act gag tcc aat gca ggt ctt tac        1429
Val Ser Phe Leu Ile Gly Ala Leu Thr Glu Ser Asn Ala Gly Leu Tyr
80                  85                  90                  95

cgg tgc tgc tac tgg aag gag aca ggc tgg tca aag ccc agt aaa gtt        1477
Arg Cys Cys Tyr Trp Lys Glu Thr Gly Trp Ser Lys Pro Ser Lys Val
                100                 105                 110

cta gag ttg gag gca cca ggc caa ctg ccc aag ccc atc ttc tgg att        1525
Leu Glu Leu Glu Ala Pro Gly Gln Leu Pro Lys Pro Ile Phe Trp Ile
            115                 120                 125

cag gct gag acc ccc gct ctt cct ggg tgt aat gtt aac atc ctc tgc        1573
Gln Ala Glu Thr Pro Ala Leu Pro Gly Cys Asn Val Asn Ile Leu Cys
        130                 135                 140

cat ggc tgg ctg cag gat ttg gta ttc atg ctg ttt aaa gag gga tat        1621
His Gly Trp Leu Gln Asp Leu Val Phe Met Leu Phe Lys Glu Gly Tyr
    145                 150                 155

gca gag cct gtg gat tac caa gtc cca act ggg aca atg gcc ata ttc        1669
Ala Glu Pro Val Asp Tyr Gln Val Pro Thr Gly Thr Met Ala Ile Phe
160                 165                 170                 175

tcc att gac aac ctg aca cct gag gat gaa ggg gtt tac atc tgc cgc        1717
Ser Ile Asp Asn Leu Thr Pro Glu Asp Glu Gly Val Tyr Ile Cys Arg
                180                 185                 190

act cat atc cag atg ctc ccc acc ctg tgg tca gag ccc agc aac ccc        1765
Thr His Ile Gln Met Leu Pro Thr Leu Trp Ser Glu Pro Ser Asn Pro
            195                 200                 205

ctg aag ctg gtt gta gca gga ctc tac ccc aaa cca act ttg aca gcc        1813
Leu Lys Leu Val Val Ala Gly Leu Tyr Pro Lys Pro Thr Leu Thr Ala
        210                 215                 220

cat cct ggg ccc atc atg gca cct gga gaa agc ctg aat ctc agg tgc        1861
His Pro Gly Pro Ile Met Ala Pro Gly Glu Ser Leu Asn Leu Arg Cys
    225                 230                 235

caa ggg cca atc tat gga atg acc ttt gct cta atg agg gtt gaa gac        1909
Gln Gly Pro Ile Tyr Gly Met Thr Phe Ala Leu Met Arg Val Glu Asp
240                 245                 250                 255

ttg gag aag tcc ttt tac cac aag aag aca ata aaa aat gag gca aat        1957
Leu Glu Lys Ser Phe Tyr His Lys Lys Thr Ile Lys Asn Glu Ala Asn
                260                 265                 270

ttc ttc ttc cag tct ttg aag atc caa gat act gga cat tac ctc tgt        2005
Phe Phe Phe Gln Ser Leu Lys Ile Gln Asp Thr Gly His Tyr Leu Cys
            275                 280                 285

ttt tac tat gac gca tca tat aga ggt tca ctc ctt agt gat gtc ctg        2053
Phe Tyr Tyr Asp Ala Ser Tyr Arg Gly Ser Leu Leu Ser Asp Val Leu
        290                 295                 300

aaa atc tgg gtg act gac act ttc ccc aag acc tgg cta ctt gct cgg 2101
Lys Ile Trp Val Thr Asp Thr Phe Pro Lys Thr Trp Leu Leu Ala Arg
    305                 310                 315

ccc agt gct gtg gtc caa atg ggt cag aat gtg agc cta cgg tgt cga 2149
Pro Ser Ala Val Val Gln Met Gly Gln Asn Val Ser Leu Arg Cys Arg
320                 325                 330                 335

gga cca gtg gat gga gtg ggt ctt gca ctc tat aag aaa gga gaa gac 2197
Gly Pro Val Asp Gly Val Gly Leu Ala Leu Tyr Lys Lys Gly Glu Asp
                340                 345                 350

aaa cca ctt caa ttt ttg gat gcc acc agc atc gat gac aac aca tca 2245
Lys Pro Leu Gln Phe Leu Asp Ala Thr Ser Ile Asp Asp Asn Thr Ser
            355                 360                 365

ttc ttc ctc aac aat gta acc tac agt gat act ggc atc tat agc tgc 2293
Phe Phe Leu Asn Asn Val Thr Tyr Ser Asp Thr Gly Ile Tyr Ser Cys
        370                 375                 380

cac tat ctt ctc acc tgg aag acc tcc att agg atg cca tca cac aac 2341
His Tyr Leu Leu Thr Trp Lys Thr Ser Ile Arg Met Pro Ser His Asn
    385                 390                 395

act gtg gag ctt atg gtt gta gat aag ccc ccc aaa ccc tcc ctg tca 2389
Thr Val Glu Leu Met Val Val Asp Lys Pro Pro Lys Pro Ser Leu Ser
400                 405                 410                 415

gct tgg cca agc act gtg ttc aag cta gga aag gcc atc acc ctt cag 2437
Ala Trp Pro Ser Thr Val Phe Lys Leu Gly Lys Ala Ile Thr Leu Gln
                420                 425                 430

tgc cga gta tct cat cca gta ctg gaa ttt tct ctg gaa tgg gaa gaa 2485
Cys Arg Val Ser His Pro Val Leu Glu Phe Ser Leu Glu Trp Glu Glu
            435                 440                 445

aga gaa aca ttc caa aaa ttc tca gta aac gga gac ttc atc atc agt 2533
Arg Glu Thr Phe Gln Lys Phe Ser Val Asn Gly Asp Phe Ile Ile Ser
        450                 455                 460

aat gtt gac ggg aaa ggc aca ggg acc tac agt tgc agc tat cgc gta 2581
Asn Val Asp Gly Lys Gly Thr Gly Thr Tyr Ser Cys Ser Tyr Arg Val
    465                 470                 475

gag aca cat cct aac atc tgg tca cat cgc agt gag ccc ctg aag ctg 2629
Glu Thr His Pro Asn Ile Trp Ser His Arg Ser Glu Pro Leu Lys Leu
480                 485                 490                 495

atg ggg cca gca ggc tat ctc acc tgg aat tac gtt ctg aat gaa gct 2677
Met Gly Pro Ala Gly Tyr Leu Thr Trp Asn Tyr Val Leu Asn Glu Ala
                500                 505                 510

atc agg ttg tct cta atc atg cag ctt gtt gcc ttg ctg ttg gta gtg 2725
Ile Arg Leu Ser Leu Ile Met Gln Leu Val Ala Leu Leu Leu Val Val
            515                 520                 525

ctg tgg ata agg tgg aag tgt cgg aga ctc aga atc aga gaa gcc tgg 2773
Leu Trp Ile Arg Trp Lys Cys Arg Arg Leu Arg Ile Arg Glu Ala Trp
        530                 535                 540

ttg ctg gga aca gct caa ggg gtc acc atg ctc ttc ata gtc acg gcc 2821
Leu Leu Gly Thr Ala Gln Gly Val Thr Met Leu Phe Ile Val Thr Ala
    545                 550                 555

ctt ctc tgc tgt gga ctg tgc aat ggg gta ttg ata gaa gag act gaa 2869
Leu Leu Cys Cys Gly Leu Cys Asn Gly Val Leu Ile Glu Glu Thr Glu
560                 565                 570                 575

ata gtc atg cca acc cct aag cct gag ctg tgg gca gag acc aac ttt 2917
Ile Val Met Pro Thr Pro Lys Pro Glu Leu Trp Ala Glu Thr Asn Phe
                580                 585                 590

cct ctg gcc ccg tgg aag aac tta acc ctc tgg tgc aga agc cct tct 2965
Pro Leu Ala Pro Trp Lys Asn Leu Thr Leu Trp Cys Arg Ser Pro Ser
            595                 600                 605

ggc tca act aag gag ttt gtg ttg ctg aag gat ggg acc ggg tgg atc 3013
Gly Ser Thr Lys Glu Phe Val Leu Leu Lys Asp Gly Thr Gly Trp Ile
        610                 615                 620

gcc act cgc ccg gcc tca gag cag gtc cgg gct gcc ttc ccc ctt ggc 3061
Ala Thr Arg Pro Ala Ser Glu Gln Val Arg Ala Ala Phe Pro Leu Gly
    625                 630                 635

gcc ctg acc cag agc cac acc ggg agc tac cac tgc cat tca tgg gag 3109
Ala Leu Thr Gln Ser His Thr Gly Ser Tyr His Cys His Ser Trp Glu
640                 645                 650                 655

gag atg gct gta tcg gag ccc agt gag gca ctt gag ctg gtg ggg aca 3157
Glu Met Ala Val Ser Glu Pro Ser Glu Ala Leu Glu Leu Val Gly Thr
                660                 665                 670

gac atc ctc ccc aaa cct gtc att tct gct tcc ccc aca atc cgg ggc 3205
Asp Ile Leu Pro Lys Pro Val Ile Ser Ala Ser Pro Thr Ile Arg Gly
            675                 680                 685

cag gaa cta caa ctc cgg tgc aaa gga tgg ctg gca ggc atg ggg ttt 3253
Gln Glu Leu Gln Leu Arg Cys Lys Gly Trp Leu Ala Gly Met Gly Phe
        690                 695                 700

gct ctg tat aag gag gga gag caa gaa cct gtc cag caa ctt ggt gct 3301
Ala Leu Tyr Lys Glu Gly Glu Gln Glu Pro Val Gln Gln Leu Gly Ala
    705                 710                 715

gtt gga aga gaa gcc ttc ttt aca atc cag aga atg gag gat aaa gac 3349
Val Gly Arg Glu Ala Phe Phe Thr Ile Gln Arg Met Glu Asp Lys Asp
720                 725                 730                 735

gaa ggc aat tac agc tgc cgc act cac act gaa aaa cgc ccc ttc aag 3397
Glu Gly Asn Tyr Ser Cys Arg Thr His Thr Glu Lys Arg Pro Phe Lys
                740                 745                 750

tgg tct gag ccc agt gag ccg ctg gag ctt gtc ata aaa gaa atg tac 3445
Trp Ser Glu Pro Ser Glu Pro Leu Glu Leu Val Ile Lys Glu Met Tyr
            755                 760                 765

cct aag ccc ttc ttc aag aca tgg gcc agc cct gtg gtc acc cct ggt 3493
Pro Lys Pro Phe Phe Lys Thr Trp Ala Ser Pro Val Val Thr Pro Gly
        770                 775                 780

gcc cga gtg act ttc aat tgc tcc acc ccc cac cag cat atg agc ttt 3541
Ala Arg Val Thr Phe Asn Cys Ser Thr Pro His Gln His Met Ser Phe
    785                 790                 795

att ctt tac aaa gat gga agt gaa ata gca tcc agt gac agg tcc tgg 3589
Ile Leu Tyr Lys Asp Gly Ser Glu Ile Ala Ser Ser Asp Arg Ser Trp
800                 805                 810                 815

gca agt ccg ggg gcc agt gca gct cac ttt cta atc att tcg gtg ggc 3637
Ala Ser Pro Gly Ala Ser Ala Ala His Phe Leu Ile Ile Ser Val Gly
                820                 825                 830

att ggt gat gga ggg aat tac agc tgc cga tat tat gac ttt tct atc 3685
Ile Gly Asp Gly Gly Asn Tyr Ser Cys Arg Tyr Tyr Asp Phe Ser Ile
            835                 840                 845

tgg tct gag ccc agc gac cct gtg gag ctc gtg gtg aca gaa ttc tac 3733

Trp Ser Glu Pro Ser Asp Pro Val Glu Leu Val Val Thr Glu Phe Tyr
        850                 855                 860

ccc aaa ccc act ctc ctg gca cag cca ggt cct gtg gtg ttt cct ggg      3781
Pro Lys Pro Thr Leu Leu Ala Gln Pro Gly Pro Val Val Phe Pro Gly
    865                 870                 875

aag agt gtg atc ctg cgc tgc caa ggg act ttc cag ggc atg agg ttc      3829
Lys Ser Val Ile Leu Arg Cys Gln Gly Thr Phe Gln Gly Met Arg Phe
880                 885                 890                 895

gcc ctc ttg cag gag gga gcc cat gtt ccc tta cag ttt cgg agt gtc      3877
Ala Leu Leu Gln Glu Gly Ala His Val Pro Leu Gln Phe Arg Ser Val
                900                 905                 910

tca ggg aac tca gct gac ttc ctt ctc cac act gtt gga gca gag gac      3925
Ser Gly Asn Ser Ala Asp Phe Leu Leu His Thr Val Gly Ala Glu Asp
            915                 920                 925

tct ggg aac tat agc tgt atc tac tat gag aca acc atg tca aac agg      3973
Ser Gly Asn Tyr Ser Cys Ile Tyr Tyr Glu Thr Thr Met Ser Asn Arg
        930                 935                 940

ggg tca tat ctc agt atg ccc ctt atg atc tgg gtg act gac aca ttc      4021
Gly Ser Tyr Leu Ser Met Pro Leu Met Ile Trp Val Thr Asp Thr Phe
    945                 950                 955

cct aag cca tgg ttg ttt gct gag ccc agt tct gtg gtt ccc atg ggg      4069
Pro Lys Pro Trp Leu Phe Ala Glu Pro Ser Ser Val Val Pro Met Gly
960                 965                 970                 975

cag aat gtt act ctc tgg tgc cga ggg ccg gtc cat gga gta gga tac      4117
Gln Asn Val Thr Leu Trp Cys Arg Gly Pro Val His Gly Val Gly Tyr
                980                 985                 990

att ctg cac aaa gaa gga gaa gcc act  tca atg cag ctc tgg  gga tcc      4165
Ile Leu His Lys Glu Gly Glu Ala Thr  Ser Met Gln Leu Trp  Gly Ser
            995                 1000                1005

acc agt aat  gac ggg gca ttc ccc  atc acc aat ata tct  ggt act      4210
Thr Ser Asn  Asp Gly Ala Phe Pro  Ile Thr Asn Ile Ser  Gly Thr
        1010                1015                1020

agc atg ggg  cgt tac agc tgc tgc  tac cac cct gac tgg  acc agt      4255
Ser Met Gly  Arg Tyr Ser Cys Cys  Tyr His Pro Asp Trp  Thr Ser
        1025                1030                1035

tct atc aag  ata caa cct agc aac  acc ctg gaa ctc cta  gtc aca      4300
Ser Ile Lys  Ile Gln Pro Ser Asn  Thr Leu Glu Leu Leu  Val Thr
        1040                1045                1050

ggc tta ctc  ccc aaa ccc agc cta  tta gcc cag cct ggt  ccc atg      4345
Gly Leu Leu  Pro Lys Pro Ser Leu  Leu Ala Gln Pro Gly  Pro Met
        1055                1060                1065

gtg gcc cct  ggc gaa aat atg act  ctt cag tgt caa ggg  gaa ctg      4390
Val Ala Pro  Gly Glu Asn Met Thr  Leu Gln Cys Gln Gly  Glu Leu
        1070                1075                1080

cca gac tca  aca ttt gtc ctg ttg  aag gag ggg gct cag  gag cct      4435
Pro Asp Ser  Thr Phe Val Leu Leu  Lys Glu Gly Ala Gln  Glu Pro
        1085                1090                1095

tta gag caa  cag agg cca agt ggg  tac agg gct gac ttc  tgg atg      4480
Leu Glu Gln  Gln Arg Pro Ser Gly  Tyr Arg Ala Asp Phe  Trp Met
        1100                1105                1110

cca gca gtg  aga ggt gaa gac tct  ggg atc tat agc tgt  gtt tat      4525
Pro Ala Val  Arg Gly Glu Asp Ser  Gly Ile Tyr Ser Cys  Val Tyr

1115                 1120                1125

tat ttg gac  tct act ccc ttt gca  gct tca aat cac agt  gac tcc      4570
Tyr Leu Asp  Ser Thr Pro Phe Ala  Ala Ser Asn His Ser  Asp Ser
        1130                 1135                 1140

ctg gag atc  tgg gtg act gat aag  ccc cct aaa ccc tct  ctg tca      4615
Leu Glu Ile  Trp Val Thr Asp Lys  Pro Pro Lys Pro Ser  Leu Ser
        1145                 1150                 1155

gcc tgg ccc  agc acc atg ttc aag  tta ggg aag gac atc  acc ctt      4660
Ala Trp Pro  Ser Thr Met Phe Lys  Leu Gly Lys Asp Ile  Thr Leu
        1160                 1165                 1170

cag tgc cga  gga ccc ctg cca ggt  gtt gaa ttt gtc cta  gaa cat      4705
Gln Cys Arg  Gly Pro Leu Pro Gly  Val Glu Phe Val Leu  Glu His
        1175                 1180                 1185

gat gga gaa  gaa gca cct cag cag  ttt tca gag gat gga  gac ttt      4750
Asp Gly Glu  Glu Ala Pro Gln Gln  Phe Ser Glu Asp Gly  Asp Phe
        1190                 1195                 1200

gtc atc aac  aac gta gaa gga aaa  ggc att gga aac tac  agc tgc      4795
Val Ile Asn  Asn Val Glu Gly Lys  Gly Ile Gly Asn Tyr  Ser Cys
        1205                 1210                 1215

agc tac cgc  ctc cag gcc tac cct  gat atc tgg tca gag  cct agt      4840
Ser Tyr Arg  Leu Gln Ala Tyr Pro  Asp Ile Trp Ser Glu  Pro Ser
        1220                 1225                 1230

gat ccc ctg  gag ctg gtg ggg gca  gca ggg cct gtt gct  cag gag      4885
Asp Pro Leu  Glu Leu Val Gly Ala  Ala Gly Pro Val Ala  Gln Glu
        1235                 1240                 1245

tgc act gta  ggg aac att gtc cga  agt agc cta atc gtg  gtg gtt      4930
Cys Thr Val  Gly Asn Ile Val Arg  Ser Ser Leu Ile Val  Val Val
        1250                 1255                 1260

gtt gta gcc  ttg ggg gta gtg cta  gcc ata gag tgg aag  aag tgg      4975
Val Val Ala  Leu Gly Val Val Leu  Ala Ile Glu Trp Lys  Lys Trp
        1265                 1270                 1275

cct cga ctg  cga acc aga ggc tca  gag aca gac gga aga  gac cag      5020
Pro Arg Leu  Arg Thr Arg Gly Ser  Glu Thr Asp Gly Arg  Asp Gln
        1280                 1285                 1290

acc att gcc  ctt gaa gag tgt aac  caa gaa gga gaa cca  ggc acc      5065
Thr Ile Ala  Leu Glu Glu Cys Asn  Gln Glu Gly Glu Pro  Gly Thr
        1295                 1300                 1305

cct gcc aat  tct cct tca tca acc  tct cag aga atc tct  gtg gaa      5110
Pro Ala Asn  Ser Pro Ser Ser Thr  Ser Gln Arg Ile Ser  Val Glu
        1310                 1315                 1320

ctg ccc gtt  cca ata taa taatctcctc ctttacaaga gctttcctct           5158
Leu Pro Val  Pro Ile
        1325

cctctctctt gctctcagag acctataaat ccaaccagtt accctgcaag tcagccccat   5218

ctgctgttcc ttggtctcta atcacctgag ctgggtaaag gggattctgg gagttgagag   5278

ctctgccagg gtgagatgtt tcctgaagag aggttcccca cccctgtaac tcctcactgt   5338

actgatttac tggcgcatga aattctatta aaaatgcatt cttctgaata aaaagagtat   5398

tcactattta acttc                                                    5413

<210>  28
<211>  1327
<212>  PRT
<213>  Homo sapiens

<400>  28 .

Met Thr Leu Asp Arg Pro Gly Glu Gly Ala Thr Met Leu Lys Thr Phe
1               5                   10                  15

Thr Val Leu Leu Phe Cys Ile Leu Met Asp Pro Gln Pro Glu Leu Trp
            20                  25                  30

Ile Glu Ser Asn Tyr Pro Gln Ala Pro Trp Glu Asn Ile Thr Leu Trp
        35                  40                  45

Cys Arg Ser Pro Ser Arg Ile Ser Ser Lys Phe Leu Leu Leu Lys Asp
    50                  55                  60

Lys Thr Gln Met Thr Trp Ile Arg Pro Ser His Lys Thr Phe Gln Val
65                  70                  75                  80

Ser Phe Leu Ile Gly Ala Leu Thr Glu Ser Asn Ala Gly Leu Tyr Arg
                85                  90                  95

Cys Cys Tyr Trp Lys Glu Thr Gly Trp Ser Lys Pro Ser Lys Val Leu
            100                 105                 110

Glu Leu Glu Ala Pro Gly Gln Leu Pro Lys Pro Ile Phe Trp Ile Gln
        115                 120                 125

Ala Glu Thr Pro Ala Leu Pro Gly Cys Asn Val Asn Ile Leu Cys His
    130                 135                 140

Gly Trp Leu Gln Asp Leu Val Phe Met Leu Phe Lys Glu Gly Tyr Ala
145                 150                 155                 160

Glu Pro Val Asp Tyr Gln Val Pro Thr Gly Thr Met Ala Ile Phe Ser
                165                 170                 175

Ile Asp Asn Leu Thr Pro Glu Asp Glu Gly Val Tyr Ile Cys Arg Thr
            180                 185                 190

His Ile Gln Met Leu Pro Thr Leu Trp Ser Glu Pro Ser Asn Pro Leu
        195                 200                 205

Lys Leu Val Val Ala Gly Leu Tyr Pro Lys Pro Thr Leu Thr Ala His
    210                 215                 220

Pro Gly Pro Ile Met Ala Pro Gly Glu Ser Leu Asn Leu Arg Cys Gln
225                 230                 235                 240

Gly Pro Ile Tyr Gly Met Thr Phe Ala Leu Met Arg Val Glu Asp Leu
                245                 250                 255

Glu Lys Ser Phe Tyr His Lys Lys Thr Ile Lys Asn Glu Ala Asn Phe
            260                 265                 270

Phe Phe Gln Ser Leu Lys Ile Gln Asp Thr Gly His Tyr Leu Cys Phe
        275                 280                 285

Tyr Tyr Asp Ala Ser Tyr Arg Gly Ser Leu Leu Ser Asp Val Leu Lys
    290                 295                 300

Ile Trp Val Thr Asp Thr Phe Pro Lys Thr Trp Leu Leu Ala Arg Pro
305                 310                 315                 320

Ser Ala Val Val Gln Met Gly Gln Asn Val Ser Leu Arg Cys Arg Gly
                325                 330                 335

Pro Val Asp Gly Val Gly Leu Ala Leu Tyr Lys Lys Gly Glu Asp Lys
            340                 345                 350

Pro Leu Gln Phe Leu Asp Ala Thr Ser Ile Asp Asp Asn Thr Ser Phe
        355                 360                 365

Phe Leu Asn Asn Val Thr Tyr Ser Asp Thr Gly Ile Tyr Ser Cys His
    370                 375                 380

Tyr Leu Leu Thr Trp Lys Thr Ser Ile Arg Met Pro Ser His Asn Thr
385                 390                 395                 400

Val Glu Leu Met Val Val Asp Lys Pro Pro Lys Pro Ser Leu Ser Ala
                405                 410                 415

Trp Pro Ser Thr Val Phe Lys Leu Gly Lys Ala Ile Thr Leu Gln Cys
            420                 425                 430

Arg Val Ser His Pro Val Leu Glu Phe Ser Leu Glu Trp Glu Glu Arg
        435                 440                 445

Glu Thr Phe Gln Lys Phe Ser Val Asn Gly Asp Phe Ile Ile Ser Asn
    450                 455                 460

Val Asp Gly Lys Gly Thr Gly Thr Tyr Ser Cys Ser Tyr Arg Val Glu
465                 470                 475                 480

Thr His Pro Asn Ile Trp Ser His Arg Ser Glu Pro Leu Lys Leu Met
                485                 490                 495

Gly Pro Ala Gly Tyr Leu Thr Trp Asn Tyr Val Leu Asn Glu Ala Ile
            500                 505                 510

Arg Leu Ser Leu Ile Met Gln Leu Val Ala Leu Leu Leu Val Val Leu
        515                 520                 525

Trp Ile Arg Trp Lys Cys Arg Arg Leu Arg Ile Arg Glu Ala Trp Leu
    530                 535                 540

Leu Gly Thr Ala Gln Gly Val Thr Met Leu Phe Ile Val Thr Ala Leu
545                 550                 555                 560

Leu Cys Cys Gly Leu Cys Asn Gly Val Leu Ile Glu Glu Thr Glu Ile
                565                 570                 575

Val Met Pro Thr Pro Lys Pro Glu Leu Trp Ala Glu Thr Asn Phe Pro
            580                 585                 590

Leu Ala Pro Trp Lys Asn Leu Thr Leu Trp Cys Arg Ser Pro Ser Gly
        595                 600                 605

Ser Thr Lys Glu Phe Val Leu Leu Lys Asp Gly Thr Gly Trp Ile Ala
    610                 615                 620

Thr Arg Pro Ala Ser Glu Gln Val Arg Ala Ala Phe Pro Leu Gly Ala
625                 630                 635                 640

Leu Thr Gln Ser His Thr Gly Ser Tyr His Cys His Ser Trp Glu Glu
                645                 650                 655

Met Ala Val Ser Glu Pro Ser Glu Ala Leu Glu Leu Val Gly Thr Asp
            660                 665                 670

Ile Leu Pro Lys Pro Val Ile Ser Ala Ser Pro Thr Ile Arg Gly Gln
        675                 680                 685

Glu Leu Gln Leu Arg Cys Lys Gly Trp Leu Ala Gly Met Gly Phe Ala
    690                 695                 700

Leu Tyr Lys Glu Gly Glu Gln Glu Pro Val Gln Gln Leu Gly Ala Val
705                 710                 715                 720

Gly Arg Glu Ala Phe Phe Thr Ile Gln Arg Met Glu Asp Lys Asp Glu
                725                 730                 735

Gly Asn Tyr Ser Cys Arg Thr His Thr Glu Lys Arg Pro Phe Lys Trp
            740                 745                 750

Ser Glu Pro Ser Glu Pro Leu Glu Leu Val Ile Lys Glu Met Tyr Pro
        755                 760                 765

Lys Pro Phe Phe Lys Thr Trp Ala Ser Pro Val Val Thr Pro Gly Ala
    770                 775                 780

Arg Val Thr Phe Asn Cys Ser Thr Pro His Gln His Met Ser Phe Ile
785                 790                 795                 800

Leu Tyr Lys Asp Gly Ser Glu Ile Ala Ser Ser Asp Arg Ser Trp Ala
                805                     810                     815

Ser Pro Gly Ala Ser Ala Ala His Phe Leu Ile Ile Ser Val Gly Ile
            820                     825                     830

Gly Asp Gly Gly Asn Tyr Ser Cys Arg Tyr Tyr Asp Phe Ser Ile Trp
        835                     840                     845

Ser Glu Pro Ser Asp Pro Val Glu Leu Val Val Thr Glu Phe Tyr Pro
    850                     855                     860

Lys Pro Thr Leu Leu Ala Gln Pro Gly Pro Val Val Phe Pro Gly Lys
865                     870                     875                     880

Ser Val Ile Leu Arg Cys Gln Gly Thr Phe Gln Gly Met Arg Phe Ala
                885                     890                     895

Leu Leu Gln Glu Gly Ala His Val Pro Leu Gln Phe Arg Ser Val Ser
            900                     905                     910

Gly Asn Ser Ala Asp Phe Leu Leu His Thr Val Gly Ala Glu Asp Ser
        915                     920                     925

Gly Asn Tyr Ser Cys Ile Tyr Tyr Glu Thr Thr Met Ser Asn Arg Gly
    930                     935                     940

Ser Tyr Leu Ser Met Pro Leu Met Ile Trp Val Thr Asp Thr Phe Pro
945                     950                     955                     960

Lys Pro Trp Leu Phe Ala Glu Pro Ser Ser Val Val Pro Met Gly Gln
                965                     970                     975

Asn Val Thr Leu Trp Cys Arg Gly Pro Val His Gly Val Gly Tyr Ile
            980                     985                     990

Leu His Lys Glu Gly Glu Ala Thr  Ser Met Gln Leu Trp  Gly Ser Thr
        995                     1000                    1005

Ser Asn  Asp Gly Ala Phe Pro  Ile Thr Asn Ile Ser  Gly Thr Ser
    1010                    1015                    1020

Met Gly  Arg Tyr Ser Cys Cys  Tyr His Pro Asp Trp  Thr Ser Ser
    1025                    1030                    1035

Ile Lys  Ile Gln Pro Ser Asn  Thr Leu Glu Leu Leu  Val Thr Gly
    1040                    1045                    1050

Leu Leu  Pro Lys Pro Ser Leu  Leu Ala Gln Pro Gly  Pro Met Val
    1055                    1060                    1065

Ala Pro  Gly Glu Asn Met Thr  Leu Gln Cys Gln Gly  Glu Leu Pro

1070                1075                1080

Asp Ser Thr Phe Val Leu Leu Lys Glu Gly Ala Gln Glu Pro Leu
    1085                1090                1095

Glu Gln Gln Arg Pro Ser Gly Tyr Arg Ala Asp Phe Trp Met Pro
    1100                1105                1110

Ala Val Arg Gly Glu Asp Ser Gly Ile Tyr Ser Cys Val Tyr Tyr
    1115                1120                1125

Leu Asp Ser Thr Pro Phe Ala Ala Ser Asn His Ser Asp Ser Leu
    1130                1135                1140

Glu Ile Trp Val Thr Asp Lys Pro Pro Lys Pro Ser Leu Ser Ala
    1145                1150                1155

Trp Pro Ser Thr Met Phe Lys Leu Gly Lys Asp Ile Thr Leu Gln
    1160                1165                1170

Cys Arg Gly Pro Leu Pro Gly Val Glu Phe Val Leu Glu His Asp
    1175                1180                1185

Gly Glu Glu Ala Pro Gln Gln Phe Ser Glu Asp Gly Asp Phe Val
    1190                1195                1200

Ile Asn Asn Val Glu Gly Lys Gly Ile Gly Asn Tyr Ser Cys Ser
    1205                1210                1215

Tyr Arg Leu Gln Ala Tyr Pro Asp Ile Trp Ser Glu Pro Ser Asp
    1220                1225                1230

Pro Leu Glu Leu Val Gly Ala Ala Gly Pro Val Ala Gln Glu Cys
    1235                1240                1245

Thr Val Gly Asn Ile Val Arg Ser Ser Leu Ile Val Val Val Val
    1250                1255                1260

Val Ala Leu Gly Val Val Leu Ala Ile Glu Trp Lys Lys Trp Pro
    1265                1270                1275

Arg Leu Arg Thr Arg Gly Ser Glu Thr Asp Gly Arg Asp Gln Thr
    1280                1285                1290

Ile Ala Leu Glu Glu Cys Asn Gln Glu Gly Glu Pro Gly Thr Pro
    1295                1300                1305

Ala Asn Ser Pro Ser Ser Thr Ser Gln Arg Ile Ser Val Glu Leu
    1310                1315                1320

Pro Val Pro Ile
    1325

<210>  29
<211>  2557
<212>  DNA
<213>  Homo sapiens


<220>
<221>  CDS
<222>  (14)..(1471)
<223>  NLE1 mRNA

<400>  29
gtggggacgc agg atg gcg gca gca gtg ccg gac gag gcg gtg gcg cgc        49
               Met Ala Ala Ala Val Pro Asp Glu Ala Val Ala Arg
               1               5                   10

gat gtg cag cgg ttg cta gtg cag ttc cag gat gag ggc ggg cag ctg        97
Asp Val Gln Arg Leu Leu Val Gln Phe Gln Asp Glu Gly Gly Gln Leu
        15                  20                  25

ctg ggt tcc ccg ttc gac gtg ccc gtg gac atc acc ccg gac agg ctg       145
Leu Gly Ser Pro Phe Asp Val Pro Val Asp Ile Thr Pro Asp Arg Leu
    30                  35                  40

cag ctc gtg tgc aac gcg cta ctg gcc cag gag gat ccc ctg cca ctg       193
Gln Leu Val Cys Asn Ala Leu Leu Ala Gln Glu Asp Pro Leu Pro Leu
45                  50                  55                  60

gct ttc ttt gtc cac gat gct gag atc gtc tcc tca ctg ggg aag acg       241
Ala Phe Phe Val His Asp Ala Glu Ile Val Ser Ser Leu Gly Lys Thr
                65                  70                  75

ttg gag tcc cag gca gtg gag aca gag aag gtc cta gac atc atc tac       289
Leu Glu Ser Gln Ala Val Glu Thr Glu Lys Val Leu Asp Ile Ile Tyr
            80                  85                  90

cag cca cag gct atc ttc aga gtc cgg gct gtg act cgc tgc acc agc       337
Gln Pro Gln Ala Ile Phe Arg Val Arg Ala Val Thr Arg Cys Thr Ser
        95                  100                 105

tcc ttg gag ggt cac agt gag gca gtc att tct gtg gcc ttc agc cct       385
Ser Leu Glu Gly His Ser Glu Ala Val Ile Ser Val Ala Phe Ser Pro
    110                 115                 120

acg gga aag tac ctg gcc agt ggc tct gga gac acc acc gtg cgc ttc       433
Thr Gly Lys Tyr Leu Ala Ser Gly Ser Gly Asp Thr Thr Val Arg Phe
125                 130                 135                 140

tgg gat ctc agc aca gag aca cca cat ttc aca tgc aag gga cac aga       481
Trp Asp Leu Ser Thr Glu Thr Pro His Phe Thr Cys Lys Gly His Arg
                145                 150                 155

cac tgg gtc ctt agt ata tcc tgg tct cca gat ggc agg aag ctg gcc       529
His Trp Val Leu Ser Ile Ser Trp Ser Pro Asp Gly Arg Lys Leu Ala
            160                 165                 170

tca ggc tgc aag aat ggc cag att ctc ctc tgg gac cca agc aca ggg       577
Ser Gly Cys Lys Asn Gly Gln Ile Leu Leu Trp Asp Pro Ser Thr Gly
        175                 180                 185

aag cag gtg ggc agg acc ctc gct ggc cac agc aag tgg atc aca ggc       625
Lys Gln Val Gly Arg Thr Leu Ala Gly His Ser Lys Trp Ile Thr Gly
    190                 195                 200

ctg agc tgg gag ccc ctc cat gcg aac cct gag tgc cgc tat gtg gcc       673
Leu Ser Trp Glu Pro Leu His Ala Asn Pro Glu Cys Arg Tyr Val Ala
205                 210                 215                 220

agc agc tcc aag gat ggc agt gtg cgg atc tgg gac aca act gca ggc 721
Ser Ser Ser Lys Asp Gly Ser Val Arg Ile Trp Asp Thr Thr Ala Gly
                225                 230                 235

cgc tgt gag cgc atc ctc acc ggg cac acc cag tcg gtc acc tgt ctc 769
Arg Cys Glu Arg Ile Leu Thr Gly His Thr Gln Ser Val Thr Cys Leu
            240                 245                 250

cgg tgg gga ggg gac ggg ctt ctc tac tct gcc tcc cag gac cgc acc 817
Arg Trp Gly Gly Asp Gly Leu Leu Tyr Ser Ala Ser Gln Asp Arg Thr
        255                 260                 265

atc aaa gtc tgg aga gct cat gac ggt gtg ctg tgc cgg act ctg caa 865
Ile Lys Val Trp Arg Ala His Asp Gly Val Leu Cys Arg Thr Leu Gln
    270                 275                 280

ggc cac ggc cac tgg gtg aac acc atg gcc ctc agc act gac tat gcc 913
Gly His Gly His Trp Val Asn Thr Met Ala Leu Ser Thr Asp Tyr Ala
285                 290                 295                 300

ctg cgc act ggg gcc ttt gaa cct gct gag gcc tca gtt aat ccc caa 961
Leu Arg Thr Gly Ala Phe Glu Pro Ala Glu Ala Ser Val Asn Pro Gln
                305                 310                 315

gac ctc caa gga tcc ttg cag gag ttg aag gag agg gct ctg agc cga 1009
Asp Leu Gln Gly Ser Leu Gln Glu Leu Lys Glu Arg Ala Leu Ser Arg
            320                 325                 330

tac aac ctc gtg cgg ggc cag ggt cca gag agg ctg gtg tct ggc tcc 1057
Tyr Asn Leu Val Arg Gly Gln Gly Pro Glu Arg Leu Val Ser Gly Ser
        335                 340                 345

gac gac ttc acc tta ttc ctg tgg tcc cca gca gag gac aaa aag cct 1105
Asp Asp Phe Thr Leu Phe Leu Trp Ser Pro Ala Glu Asp Lys Lys Pro
    350                 355                 360

ctc act cgg atg aca gga cac caa gct ctc atc aac cag gtg ctc ttc 1153
Leu Thr Arg Met Thr Gly His Gln Ala Leu Ile Asn Gln Val Leu Phe
365                 370                 375                 380

tct cct gac tcc cgc atc gtg gct agt gcc tcc ttt gac aag tcc atc 1201
Ser Pro Asp Ser Arg Ile Val Ala Ser Ala Ser Phe Asp Lys Ser Ile
                385                 390                 395

aag ctg tgg gat ggc agg acg ggc aag tac ctg gct tcc cta cgc ggc 1249
Lys Leu Trp Asp Gly Arg Thr Gly Lys Tyr Leu Ala Ser Leu Arg Gly
            400                 405                 410

cac gtg gct gcc gtg tac cag att gcg tgg tca gct gac agt cgg ctc 1297
His Val Ala Ala Val Tyr Gln Ile Ala Trp Ser Ala Asp Ser Arg Leu
        415                 420                 425

ctg gtc agc ggc agc agt gac agc aca ctg aag gtg tgg gat gtg aag 1345
Leu Val Ser Gly Ser Ser Asp Ser Thr Leu Lys Val Trp Asp Val Lys
    430                 435                 440

gcc cag aag ctg gcc atg gac ctg ccc ggc cac gcg gat gag gta tat 1393
Ala Gln Lys Leu Ala Met Asp Leu Pro Gly His Ala Asp Glu Val Tyr
445                 450                 455                 460

gct gtt gac tgg agt cca gat ggc cag aga gtg gca agt ggt ggg aag 1441
Ala Val Asp Trp Ser Pro Asp Gly Gln Arg Val Ala Ser Gly Gly Lys
                465                 470                 475

gac aaa tgc ctc cgg ata tgg agg aga tga gacggcccga agttctctct 1491
Asp Lys Cys Leu Arg Ile Trp Arg Arg
            480                 485

gacccccacc tcgactcggc ctctgccagc tgccttccct gccagagaac aaaggctgag 1551

atggcagtgc acacaccctc cccaccagtg gggacctgag aatgcgtgtg gcctgctgtc 1611

ctcgatagac cggaatgggg ttttcccaca gatccccgcc tgtggcacac cccagagcca 1671

gaaatcgaag gtcacaggaa gttgtcactg aacttggccc gtgtctgcta ctctgtacct 1731

tgctggtaca gacaggggtg gtgggcagcc aggctctatg agtgggcccc tagtgtcagc 1791

tctgtacagg gtcagatccc aggttctatg accaaataag taacttaagt tttgtgtgtt 1851

gggttctaat tccttgtcct agaatcccca tgactcaatc aaggactgtg ctaaatgaga 1911

ttgtccagcc cccgcccttg cactggacta cgccaaaacc acactgacca ggcacttgcc 1971

ttccctctct tcccccgtgt tggtaagaga gaggccagtt gtgatagtgg ccaaggagaa 2031

tctagggctg tattgttgtc cactgcagta ggcaccggcc acatgtgact gctggcatga 2091

aatagaagtg cagttcctcc atcgcactgg gtaaggcctc cagtattgga cagcacacag 2151

aaaggttttc atcatcaaga gagttctgct ggtcagccct gctccagggg atgcctctgc 2211

cttcgcatag cacactgctt gaggccctgc caggcaccaa gcactgccct gggcccatgg 2271

gatagagcgg ggaaggtgat ggctcttcca gaggattccc tcagatgggg aggcagcagt 2331

atgagctctg agcagaagtg ggtattgttg atacagagga agttctttgc cacgagaact 2391

ttcaagcagt gaaaggaatt cccatcagga ctcagacccc aggccgagat cttgccctga 2451

atgtaccctg cctctgcttt ctcctgcatc ccatgctaag cagggtcatg gtctgaacta 2511

ctcagattgg atttccaaac catccttgta taaactgctc agaact 2557

<210> 30
<211> 485
<212> PRT
<213> Homo sapiens

<400> 30

Met Ala Ala Ala Val Pro Asp Glu Ala Val Ala Arg Asp Val Gln Arg
1               5                   10                  15

Leu Leu Val Gln Phe Gln Asp Glu Gly Gly Gln Leu Leu Gly Ser Pro
            20                  25                  30

Phe Asp Val Pro Val Asp Ile Thr Pro Asp Arg Leu Gln Leu Val Cys
        35                  40                  45

Asn Ala Leu Leu Ala Gln Glu Asp Pro Leu Pro Leu Ala Phe Phe Val
    50                  55                  60

His Asp Ala Glu Ile Val Ser Ser Leu Gly Lys Thr Leu Glu Ser Gln
65                  70                  75                  80

Ala Val Glu Thr Glu Lys Val Leu Asp Ile Ile Tyr Gln Pro Gln Ala
                85                  90                  95

Ile Phe Arg Val Arg Ala Val Thr Arg Cys Thr Ser Ser Leu Glu Gly

            100                 105                 110

His Ser Glu Ala Val Ile Ser Val Ala Phe Ser Pro Thr Gly Lys Tyr
        115                 120                 125

Leu Ala Ser Gly Ser Gly Asp Thr Thr Val Arg Phe Trp Asp Leu Ser
    130                 135                 140

Thr Glu Thr Pro His Phe Thr Cys Lys Gly His Arg His Trp Val Leu
145                 150                 155                 160

Ser Ile Ser Trp Ser Pro Asp Gly Arg Lys Leu Ala Ser Gly Cys Lys
                165                 170                 175

Asn Gly Gln Ile Leu Leu Trp Asp Pro Ser Thr Gly Lys Gln Val Gly
            180                 185                 190

Arg Thr Leu Ala Gly His Ser Lys Trp Ile Thr Gly Leu Ser Trp Glu
        195                 200                 205

Pro Leu His Ala Asn Pro Glu Cys Arg Tyr Val Ala Ser Ser Ser Lys
    210                 215                 220

Asp Gly Ser Val Arg Ile Trp Asp Thr Thr Ala Gly Arg Cys Glu Arg
225                 230                 235                 240

Ile Leu Thr Gly His Thr Gln Ser Val Thr Cys Leu Arg Trp Gly Gly
                245                 250                 255

Asp Gly Leu Leu Tyr Ser Ala Ser Gln Asp Arg Thr Ile Lys Val Trp
            260                 265                 270

Arg Ala His Asp Gly Val Leu Cys Arg Thr Leu Gln Gly His Gly His
        275                 280                 285

Trp Val Asn Thr Met Ala Leu Ser Thr Asp Tyr Ala Leu Arg Thr Gly
    290                 295                 300

Ala Phe Glu Pro Ala Glu Ala Ser Val Asn Pro Gln Asp Leu Gln Gly
305                 310                 315                 320

Ser Leu Gln Glu Leu Lys Glu Arg Ala Leu Ser Arg Tyr Asn Leu Val
                325                 330                 335

Arg Gly Gln Gly Pro Glu Arg Leu Val Ser Gly Ser Asp Asp Phe Thr
            340                 345                 350

Leu Phe Leu Trp Ser Pro Ala Glu Asp Lys Lys Pro Leu Thr Arg Met
        355                 360                 365

Thr Gly His Gln Ala Leu Ile Asn Gln Val Leu Phe Ser Pro Asp Ser
    370                 375                 380

Arg Ile Val Ala Ser Ala Ser Phe Asp Lys Ser Ile Lys Leu Trp Asp
385                 390                 395                 400

Gly Arg Thr Gly Lys Tyr Leu Ala Ser Leu Arg Gly His Val Ala Ala
                405                 410                 415

Val Tyr Gln Ile Ala Trp Ser Ala Asp Ser Arg Leu Leu Val Ser Gly
            420                 425                 430

Ser Ser Asp Ser Thr Leu Lys Val Trp Asp Val Lys Ala Gln Lys Leu
        435                 440                 445

Ala Met Asp Leu Pro Gly His Ala Asp Glu Val Tyr Ala Val Asp Trp
    450                 455                 460

Ser Pro Asp Gly Gln Arg Val Ala Ser Gly Gly Lys Asp Lys Cys Leu
465                 470                 475                 480

Arg Ile Trp Arg Arg
                485

<210>  31
<211>  704
<212>  DNA
<213>  Homo sapiens

<220>
<221>  CDS
<222>  (23)..(676)
<223>  RPL10A mRNA

<400>  31
ggttagcgcg gcgtgagaag cc atg agc agc aaa gtc tct cgc gac acc ctg       52
                         Met Ser Ser Lys Val Ser Arg Asp Thr Leu
                         1               5                   10

tac gag gcg gtg cgg gaa gtc ctg cac ggg aac cag cgc aag cgc cgc       100
Tyr Glu Ala Val Arg Glu Val Leu His Gly Asn Gln Arg Lys Arg Arg
                15                  20                  25

aag ttc ctg gag acg gtg gag ttg cag atc agc ttg aag aac tat gat       148
Lys Phe Leu Glu Thr Val Glu Leu Gln Ile Ser Leu Lys Asn Tyr Asp
            30                  35                  40

ccc cag aag gac aag cgc ttc tcg ggc acc gtc agg ctt aag tcc act       196
Pro Gln Lys Asp Lys Arg Phe Ser Gly Thr Val Arg Leu Lys Ser Thr
        45                  50                  55

ccc cgc cct aag ttc tct gtg tgt gtc ctg ggg gac cag cag cac tgt       244
Pro Arg Pro Lys Phe Ser Val Cys Val Leu Gly Asp Gln Gln His Cys
    60                  65                  70

gac gag gct aag gcc gtg gat atc ccc cac atg gac atc gag gcg ctg       292
Asp Glu Ala Lys Ala Val Asp Ile Pro His Met Asp Ile Glu Ala Leu
75                  80                  85                  90

aaa aaa ctc aac aag aat aaa aaa ctg gtc aag aag ctg gcc aag aag       340
Lys Lys Leu Asn Lys Asn Lys Lys Leu Val Lys Lys Leu Ala Lys Lys
                95                  100                 105

tat gat gcg ttt ttg gcc tca gag tct ctg atc aag cag att cca cga 388
Tyr Asp Ala Phe Leu Ala Ser Glu Ser Leu Ile Lys Gln Ile Pro Arg
            110                 115                 120

atc ctc ggc cca ggt tta aat aag gca gga aag ttc cct tcc ctg ctc 436
Ile Leu Gly Pro Gly Leu Asn Lys Ala Gly Lys Phe Pro Ser Leu Leu
        125                 130                 135

aca cac aac gaa aac atg gtg gcc aaa gtg gat gag gtg aag tcc aca 484
Thr His Asn Glu Asn Met Val Ala Lys Val Asp Glu Val Lys Ser Thr
    140                 145                 150

atc aag ttc caa atg aag aag gtg tta tgt ctg gct gta gct gtt ggt 532
Ile Lys Phe Gln Met Lys Lys Val Leu Cys Leu Ala Val Ala Val Gly
155                 160                 165                 170

cac gtg aag atg aca gac gat gag ctt gtg tat aac att cac ctg gct 580
His Val Lys Met Thr Asp Asp Glu Leu Val Tyr Asn Ile His Leu Ala
                175                 180                 185

gtc aac ttc ttg gtg tca ttg ctc aag aaa aac tgg cag aat gtc cgg 628
Val Asn Phe Leu Val Ser Leu Leu Lys Lys Asn Trp Gln Asn Val Arg
            190                 195                 200

gcc tta tat atc aag agc acc atg ggc aag ccc cag cgc cta tat taa 676
Ala Leu Tyr Ile Lys Ser Thr Met Gly Lys Pro Gln Arg Leu Tyr
        205                 210                 215

ggcacatttg aataaattct attaccag 704

<210> 32
<211> 217
<212> PRT
<213> Homo sapiens

<400> 32

Met Ser Ser Lys Val Ser Arg Asp Thr Leu Tyr Glu Ala Val Arg Glu
1               5                   10                  15

Val Leu His Gly Asn Gln Arg Lys Arg Arg Lys Phe Leu Glu Thr Val
            20                  25                  30

Glu Leu Gln Ile Ser Leu Lys Asn Tyr Asp Pro Gln Lys Asp Lys Arg
        35                  40                  45

Phe Ser Gly Thr Val Arg Leu Lys Ser Thr Pro Arg Pro Lys Phe Ser
    50                  55                  60

Val Cys Val Leu Gly Asp Gln Gln His Cys Asp Glu Ala Lys Ala Val
65                  70                  75                  80

Asp Ile Pro His Met Asp Ile Glu Ala Leu Lys Lys Leu Asn Lys Asn
                85                  90                  95

Lys Lys Leu Val Lys Lys Leu Ala Lys Lys Tyr Asp Ala Phe Leu Ala
            100                 105                 110

Ser Glu Ser Leu Ile Lys Gln Ile Pro Arg Ile Leu Gly Pro Gly Leu
        115                 120                 125

Asn Lys Ala Gly Lys Phe Pro Ser Leu Leu Thr His Asn Glu Asn Met
    130                 135                 140

Val Ala Lys Val Asp Glu Val Lys Ser Thr Ile Lys Phe Gln Met Lys
145                 150                 155                 160

Lys Val Leu Cys Leu Ala Val Ala Val Gly His Val Lys Met Thr Asp
                165                 170                 175

Asp Glu Leu Val Tyr Asn Ile His Leu Ala Val Asn Phe Leu Val Ser
            180                 185                 190

Leu Leu Lys Lys Asn Trp Gln Asn Val Arg Ala Leu Tyr Ile Lys Ser
        195                 200                 205

Thr Met Gly Lys Pro Gln Arg Leu Tyr
    210                 215

<210>  33
<211>  1919
<212>  DNA
<213>  Homo sapiens

<220>
<221>  CDS
<222>  (75)..(1202)
<223>  ACTG1 mRNA

<400>  33
gtctcagtcg ccgctgccag ctctcgcact ctgttcttcc gccgctccgc cgtcgcgttt      60
ctctgccggt cgca atg gaa gaa gag atc gcc gcg ctg gtc att gac aat      110
ggc tcc ggc atg tgc aaa gct ggt ttt gct ggg gac gac gct ccc cga      158
gcc gtg ttt cct tcc atc gtc ggg cgc ccc aga cac cag ggc gtc atg      206
gtg ggc atg ggc cag aag gac tcc tac gtg ggc gac gag gcc cag agc      254
aag cgt ggc atc ctg acc ctg aag tac ccc att gag cat ggc atc gtc      302
acc aac tgg gac gac atg gag aag atc tgg cac cac acc ttc tac aac      350
gag ctg cgc gtg gcc ccg gag gag cac cca gtg ctg ctg acc gag gcc      398
ccc ctg aac ccc aag gcc aac aga gag aag atg act cag att atg ttt      446
gag acc ttc aac acc ccg gcc atg tac gtg gcc atc cag gcc gtg ctg      494
tcc ctc tac gcc tct ggg cgc acc act ggc att gtc atg gac tct gga      542
gac ggg gtc acc cac acg gtg ccc atc tac gag ggc tac gcc ctc ccc      590
cac gcc atc ctg cgt ctg gac ctg gct ggc cgg gac ctg acc gac tac      638
ctc atg aag atc ctc act gag cga ggc tac agc ttc acc acc acg gcc      686
gag cgg gaa atc gtg cgc gac atc aag gag aag ctg tgc tac gtc gcc      734
ctg gac ttc gag cag gag atg gcc acc gcc gca tcc tcc tct tct ctg      782
gag aag agc tac gag ctg ccc gat ggc cag gtc atc acc att ggc aat      830
gag cgg ttc cgg tgt ccg gag gcg ctg ttc cag cct tcc ttc ctg ggt      878
atg gaa tct tgc ggc atc cac gag acc acc ttc aac tcc atc atg aag      926
tgt gac gtg gac atc cgc aaa gac ctg tac gcc aac acg gtg ctg tcg      974
ggc ggc acc acc atg tac ccg ggc att gcc gac agg atg cag aag gag     1022
atc acc gcc ctg gcg ccc agc acc atg aag atc aag atc atc gca ccc     1070
cca gag cgc aag tac tcg gtg tgg atc ggt ggc tcc atc ctg gcc tca     1118
ctg tcc acc ttc cag cag atg tgg att agc aag cag gag tac gac gag     1166
tcg ggc ccc tcc atc gtc cac cgc aaa tgc ttc taa acggactcag          1212
cagatgcgta gcatttgctg catgggttaa ttgagaatag aaatttgccc ctggcaaatg   1272
cacacacctc atgctagcct cacgaaactg gaataagcct tcgaaaagaa attgtccttg 1332
aagcttgtat ctgatatcag cactggattg tagaacttgt tgctgatttt gaccttgtat 1392
tgaagttaac tgttcccctt ggtatttgtt taataccctg tacatatctt tgagttcaac 1452
ctttagtacg tgtggcttgg tcacttcgtg gctaaggtaa gaacgtgctt gtggaagaca 1512
agtctgtggc ttggtgagtc tgtgtggcca gcagcctctg atctgtgcag ggtattaacg 1572
tgtcagggct gagtgttctg ggatttctct agaggctggc aagaaccagt tgttttgtct 1632
tgcgggtctg tcagggttgg aaagtccaag ccgtaggacc cagtttcctt tcttagctga 1692
tgtctttggc cagaacaccg tgggctgtta cttgctttga gttggaagcg gtttgcattt 1752
acgcctgtaa atgtattcat tcttaattta tgtaaggttt tttttgtacg caattctcga 1812
ttctttgaag agatgacaac aaattttggt tttctactgt tatgtgagaa cattaggccc 1872
cagcaacacg tcattgtgta aggaaaaata aaagtgctgc cgtaacc 1919

<210> 34
<211> 375
<212> PRT
<213> Homo sapiens

<400> 34

<210>  35
<211>  3528
<212>  DNA
<213>  Homo sapiens

<220>
<221>  CDS
<222>  (64)..(1452)
<223>  EEF1A1 mRNA

<400>  35
   ctttttcgca acgggtttgc cgccagaaca caggtgtcgt gaaaactacc cctaaaagcc 60
   aaa atg gga aag gaa aag act cat atc aac att gtc gtc att gga cac 108
       Met Gly Lys Glu Lys Thr His Ile Asn Ile Val Val Ile Gly His
       1               5                   10                  15
   gta gat tcg ggc aag tcc acc act act ggc cat ctg atc tat aaa tgc 156
   Val Asp Ser Gly Lys Ser Thr Thr Thr Gly His Leu Ile Tyr Lys Cys
                   20                  25                  30
   ggt ggc atc gac aaa aga acc att gaa aaa ttt gag aag gag gct gct 204
   Gly Gly Ile Asp Lys Arg Thr Ile Glu Lys Phe Glu Lys Glu Ala Ala
               35                  40                  45
   gag atg gga aag ggc tcc ttc aag tat gcc tgg gtc ttg gat aaa ctg 252
   Glu Met Gly Lys Gly Ser Phe Lys Tyr Ala Trp Val Leu Asp Lys Leu
           50                  55                  60
   aaa gct gag cgt gaa cgt ggt atc acc att gat atc tcc ttg tgg aaa 300
   Lys Ala Glu Arg Glu Arg Gly Ile Thr Ile Asp Ile Ser Leu Trp Lys
       65                  70                  75
   ttt gag acc agc aag tac tat gtg act atc att gat gcc cca gga cac 348
   Phe Glu Thr Ser Lys Tyr Tyr Val Thr Ile Ile Asp Ala Pro Gly His
   80                  85                  90                  95
   aga gac ttt atc aaa aac atg att aca ggg aca tct cag gct gac tgt 396
   Arg Asp Phe Ile Lys Asn Met Ile Thr Gly Thr Ser Gln Ala Asp Cys
                   100                 105                 110
   gct gtc ctg att gtt gct gct ggt gtt ggt gaa ttt gaa gct ggt atc 444
   Ala Val Leu Ile Val Ala Ala Gly Val Gly Glu Phe Glu Ala Gly Ile
               115                 120                 125
   tcc aag aat ggg cag acc cga gag cat gcc ctt ctg gct tac aca ctg 492
   Ser Lys Asn Gly Gln Thr Arg Glu His Ala Leu Leu Ala Tyr Thr Leu
           130                 135                 140
   ggt gtg aaa caa cta att gtc ggt gtt aac aaa atg gat tcc act gag 540
   Gly Val Lys Gln Leu Ile Val Gly Val Asn Lys Met Asp Ser Thr Glu
       145                 150                 155
   cca ccc tac agc cag aag aga tat gag gaa att gtt aag gaa gtc agc 588
   Pro Pro Tyr Ser Gln Lys Arg Tyr Glu Glu Ile Val Lys Glu Val Ser
   160                 165                 170                 175
   act tac att aag aaa att ggc tac aac ccc gac aca gta gca ttt gtg 636
   Thr Tyr Ile Lys Lys Ile Gly Tyr Asn Pro Asp Thr Val Ala Phe Val
                   180                 185                 190
   cca att tct ggt tgg aat ggt gac aac atg ctg gag cca agt gct aac 684
   Pro Ile Ser Gly Trp Asn Gly Asp Asn Met Leu Glu Pro Ser Ala Asn
               195                 200                 205
   atg cct tgg ttc aag gga tgg aaa gtc acc cgt aag gat ggc aat gcc 732
   Met Pro Trp Phe Lys Gly Trp Lys Val Thr Arg Lys Asp Gly Asn Ala
           210                 215                 220
   agt gga acc acg ctg ctt gag gct ctg gac tgc atc cta cca cca act 780
   Ser Gly Thr Thr Leu Leu Glu Ala Leu Asp Cys Ile Leu Pro Pro Thr
       225                 230                 235
   cgt cca act gac aag ccc ttg cgc ctg cct ctc cag gat gtc tac aaa 828
   Arg Pro Thr Asp Lys Pro Leu Arg Leu Pro Leu Gln Asp Val Tyr Lys
   240                 245                 250                 255
   att ggt ggt att ggt act gtt cct gtt ggc cga gtg gag act ggt gtt 876
   Ile Gly Gly Ile Gly Thr Val Pro Val Gly Arg Val Glu Thr Gly Val
                   260                 265                 270
   ctc aaa ccc ggt atg gtg gtc acc ttt gct cca gtc aac gtt aca acg 924
   Leu Lys Pro Gly Met Val Val Thr Phe Ala Pro Val Asn Val Thr Thr
   275 280 285
   gaa gta aaa tct gtc gaa atg cac cat gaa gct ttg agt gaa gct ctt 972
   Glu Val Lys Ser Val Glu Met His His Glu Ala Leu Ser Glu Ala Leu
   290 295 300
   cct ggg gac aat gtg ggc ttc aat gtc aag aat gtg tct gtc aag gat 1020
   Pro Gly Asp Asn Val Gly Phe Asn Val Lys Asn Val Ser Val Lys Asp
   305 310 315
   gtt cgt cgt ggc aac gtt gct ggt gac agc aaa aat gac cca cca atg 1068
   Val Arg Arg Gly Asn Val Ala Gly Asp Ser Lys Asn Asp Pro Pro Met
   320 325 330 335
   gaa gca gct ggc ttc act gct cag gtg att atc ctg aac cat cca ggc 1116
   Glu Ala Ala Gly Phe Thr Ala Gln Val Ile Ile Leu Asn His Pro Gly
   340 345 350
   caa ata agc gcc ggc tat gcc cct gta ttg gat tgc cac acg gct cac 1164
   Gln Ile Ser Ala Gly Tyr Ala Pro Val Leu Asp Cys His Thr Ala His
   355 360 365
   att gca tgc aag ttt gct gag ctg aag gaa aag att gat cgc cgt tct 1212
   Ile Ala Cys Lys Phe Ala Glu Leu Lys Glu Lys Ile Asp Arg Arg Ser
   370 375 380
   ggt aaa aag ctg gaa gat ggc cct aaa ttc ttg aag tct ggt gat gct 1260
   Gly Lys Lys Leu Glu Asp Gly Pro Lys Phe Leu Lys Ser Gly Asp Ala
   385 390 395
   gcc att gtt gat atg gtt cct ggc aag ccc atg tgt gtt gag agc ttc 1308
   Ala Ile Val Asp Met Val Pro Gly Lys Pro Met Cys Val Glu Ser Phe
   400 405 410 415
   tca gac tat cca cct ttg ggt cgc ttt gct gtt cgt gat atg aga cag 1356
   Ser Asp Tyr Pro Pro Leu Gly Arg Phe Ala Val Arg Asp Met Arg Gln
   420 425 430
   aca gtt gcg gtg ggt gtc atc aaa gca gtg gac aag aag gct gct gga 1404
   Thr Val Ala Val Gly Val Ile Lys Ala Val Asp Lys Lys Ala Ala Gly
   435 440 445
   gct ggc aag gtc acc aag tct gcc cag aaa gct cag aag gct aaa tga 1452
   Ala Gly Lys Val Thr Lys Ser Ala Gln Lys Ala Gln Lys Ala Lys
   450 455 460
   atattatccc taatacctgc caccccactc ttaatcagtg gtggaagaac ggtctcagaa 1512
   ctgtttgttt caattggcca tttaagttta gtagtaaaag actggttaat gataacaatg 1572
   catcgtaaaa ccttcagaag gaaaggagaa tgttttgtgg accactttgg ttttcttttt 1632
   tgcgtgtggc agttttaagt tattagtttt taaaatcagt actttttaat ggaaacaact 1692
   tgaccaaaaa tttgtcacag aattttgaga cccattaaaa aagttaaatg agaaacctgt 1752
   gtgttccttt ggtcaacacc gagacattta ggtgaaagac atctaattct ggttttacga 1812
   atctggaaac ttcttgaaaa tgtaattctt gagttaacac ttctgggtgg agaatagggt 1872
   tgttttcccc ccacataatt ggaaggggaa ggaatatcat ttaaagctat gggagggttg 1932
   ctttgattac aacactggag agaaatgcag catgttgctg attgcctgtc actaaaacag 1992
   gccaaaaact gagtccttgt gttgcataga aagcttcatg ttgctaaacc aatgttaagt 2052
   gaatctttgg aaacaaaatg tttccaaatt actgggatgt gcatgttgaa acgtgggtta 2112
   aaatgactgg gcagtgaaag ttgactattt gccatgacat aagaaataag tgtagtggct 2172
   agtgtacacc ctatgagtgg aagggtccat tttgaagtca gtggagtaag ctttatgcca 2232
   gtttgatggt ttcacaagtt ctattgagtg ctattcagaa taggaacaag gttctaatag 2292
   aaaaagatgg caatttgaag tagctataaa attagactaa tctacattgc ttttctcctg 2352
   cagagtctaa taccttttat gctttgataa ttagcagttt gtctacttgg tcactaggaa 2412
   tgaaactaca tggtaatagg cttaacaggt gtaatagccc acttactcct gaatctttaa 2472
   gcatttgtgc atttgaaaaa tgcttttcgc gatcttcctg ctgggattac aggcatgagc 2532
   cactgtgcct gacctcccat atgtaaaagt gtctaaaggt ttttttttgg ttataaaagg 2592
   aaaatttttg cttaagtttg aaggataggt aaaattaaag gacatgcttt ctgtttgtgt 2652
   gatggttttt aaaaattttt tttaagatgg agttcttgtt gcccaggcta gaatgcaatg 2712
   gcaaaatctc actgcaatct cctcctcctg ggttcaagca attctcctac ttcagcctcc 2772
   caagtagctg ggattacagg catgtgctaa tttggtgttt ttaatagaga tgaggttttt 2832
   ccatgttggt caggctggtc tcaaactcct gaccttaggt gatcgcctcg gcctcctaaa 2892
   gtgctggaat tacaggcatg agccaccatg cctggccagg acatgtgttc ttaaggacat 2952
   gctaagcagg agttaaagca gcccaagaga taaggcctct taaagtgact ggcaatgtgt 3012
   attgctcaag attcaaaggt acttgaattg gccatagaca agtctgtaat gaagtgttat 3072
   cgttttccct catctgagtc tgaattagat aaaatgcctt cccatcagcc agtgctctga 3132
   ggtatcaagt ctaaattgaa ctagagattt ttgtccttag tttctttgct atctaatgtt 3192
   tacacaagta aatagtctaa gatttgctgg atgacagaaa aaacaggtaa ggcctttaat 3252
   agatggccaa tagatgccct gataatgaaa gttgacacct gtaagattta ccagtagaga 3312
   attcttgaca tgcaaggaag caagatttaa ctgaaaaatt gttcccactg gaagcaggaa 3372
   tgagtcagtt tacttgcata tactgagatt gagattaact tcctgtgaaa cccagtgtct 3432
   tagacaactg tggcttgagc accacctgct ggtattcatt acaaacttgc tcactacaat 3492
   aaatgaattt taagctttaa aaaaaaaaaa aaaaaa 3528

<210> 36
<211> 462
<212> PRT
<213> Homo sapiens

<400> 36

Met Gly Lys Glu Lys Thr His Ile Asn Ile Val Val Ile Gly His Val
1               5                   10                  15

Asp Ser Gly Lys Ser Thr Thr Thr Gly His Leu Ile Tyr Lys Cys Gly
            20                  25                  30

Gly Ile Asp Lys Arg Thr Ile Glu Lys Phe Glu Lys Glu Ala Ala Glu
        35                  40                  45

Met Gly Lys Gly Ser Phe Lys Tyr Ala Trp Val Leu Asp Lys Leu Lys
    50                  55                  60

Ala Glu Arg Glu Arg Gly Ile Thr Ile Asp Ile Ser Leu Trp Lys Phe
65                  70                  75                  80

Glu Thr Ser Lys Tyr Tyr Val Thr Ile Ile Asp Ala Pro Gly His Arg
                85                  90                  95

Asp Phe Ile Lys Asn Met Ile Thr Gly Thr Ser Gln Ala Asp Cys Ala
            100                 105                 110

Val Leu Ile Val Ala Ala Gly Val Gly Glu Phe Glu Ala Gly Ile Ser
        115                 120                 125

Lys Asn Gly Gln Thr Arg Glu His Ala Leu Leu Ala Tyr Thr Leu Gly
    130                 135                 140

Val Lys Gln Leu Ile Val Gly Val Asn Lys Met Asp Ser Thr Glu Pro
145                 150                 155                 160

Pro Tyr Ser Gln Lys Arg Tyr Glu Glu Ile Val Lys Glu Val Ser Thr
                165                 170                 175

Tyr Ile Lys Lys Ile Gly Tyr Asn Pro Asp Thr Val Ala Phe Val Pro
            180                 185                 190

Ile Ser Gly Trp Asn Gly Asp Asn Met Leu Glu Pro Ser Ala Asn Met
        195                 200                 205

Pro Trp Phe Lys Gly Trp Lys Val Thr Arg Lys Asp Gly Asn Ala Ser
    210                 215                 220

Gly Thr Thr Leu Leu Glu Ala Leu Asp Cys Ile Leu Pro Pro Thr Arg
225                 230                 235                 240

Pro Thr Asp Lys Pro Leu Arg Leu Pro Leu Gln Asp Val Tyr Lys Ile
                245                 250                 255

Gly Gly Ile Gly Thr Val Pro Val Gly Arg Val Glu Thr Gly Val Leu
            260                 265                 270

Lys Pro Gly Met Val Val Thr Phe Ala Pro Val Asn Val Thr Thr Glu
        275                 280                 285

Val Lys Ser Val Glu Met His His Glu Ala Leu Ser Glu Ala Leu Pro
    290                 295                 300

Gly Asp Asn Val Gly Phe Asn Val Lys Asn Val Ser Val Lys Asp Val
305                 310                 315                 320

Arg Arg Gly Asn Val Ala Gly Asp Ser Lys Asn Asp Pro Pro Met Glu
                325                 330                 335

Ala Ala Gly Phe Thr Ala Gln Val Ile Ile Leu Asn His Pro Gly Gln
            340                 345                 350

Ile Ser Ala Gly Tyr Ala Pro Val Leu Asp Cys His Thr Ala His Ile
        355                 360                 365

Ala Cys Lys Phe Ala Glu Leu Lys Glu Lys Ile Asp Arg Arg Ser Gly
    370                 375                 380

Lys Lys Leu Glu Asp Gly Pro Lys Phe Leu Lys Ser Gly Asp Ala Ala
385                 390                 395                 400

Ile Val Asp Met Val Pro Gly Lys Pro Met Cys Val Glu Ser Phe Ser
                405                 410                 415

Asp Tyr Pro Pro Leu Gly Arg Phe Ala Val Arg Asp Met Arg Gln Thr
            420                 425                 430

Val Ala Val Gly Val Ile Lys Ala Val Asp Lys Lys Ala Ala Gly Ala
        435                 440                 445

Gly Lys Val Thr Lys Ser Ala Gln Lys Ala Gln Lys Ala Lys
    450                 455                 460

<210> 37
<211> 3606
<212> DNA
<213> Homo sapiens

<220>
<221> CDS
<222> (98)..(2251)
<223> PNN mRNA

<400> 37
attggctgag cccggctgtc agtcctttcg cgcctcggcg gcgcggcata gcccggctcg 60

gcctgtaaag cagtctcaag cctgccgcag ggagaag atg gcg gtc gcc gtg aga 115
Met Ala Val Ala Val Arg
1               5

act ttg cag gaa cag ctg gaa aag gcc aaa gag agt ctt aag aac gtg 163
Thr Leu Gln Glu Gln Leu Glu Lys Ala Lys Glu Ser Leu Lys Asn Val
            10                  15                  20

gat gag aac att cgc aag ctc acc ggg cgg gat ccg aat gac gtg agg 211
Asp Glu Asn Ile Arg Lys Leu Thr Gly Arg Asp Pro Asn Asp Val Arg
        25                  30                  35

ccc atc caa gcc aga ttg ctg gcc ctt tct ggt cct ggt gga ggt aga 259
Pro Ile Gln Ala Arg Leu Leu Ala Leu Ser Gly Pro Gly Gly Gly Arg
    40                  45                  50

gga cgt ggt agt tta tta ctg agg cgt gga ttc tca gat agt gga gga 307
Gly Arg Gly Ser Leu Leu Leu Arg Arg Gly Phe Ser Asp Ser Gly Gly
55                  60                  65                  70

gga ccc cca gcc aaa cag aga gac ctt gaa ggg gca gtc agt agg ctg 355
Gly Pro Pro Ala Lys Gln Arg Asp Leu Glu Gly Ala Val Ser Arg Leu
                75                  80                  85

ggc ggg gag cgt cgg acc aga aga gaa tca cgc cag gaa agc gac ccg 403
Gly Gly Glu Arg Arg Thr Arg Arg Glu Ser Arg Gln Glu Ser Asp Pro
            90                  95                  100

gag gat gat gat gtt aaa aag cca gca ttg cag tct tca gtt gta gct 451
Glu Asp Asp Asp Val Lys Lys Pro Ala Leu Gln Ser Ser Val Val Ala
        105                 110                 115

acc tcc aaa gag cgc aca cgt aga gac ctt atc cag gat caa aat atg 499
Thr Ser Lys Glu Arg Thr Arg Arg Asp Leu Ile Gln Asp Gln Asn Met
    120                 125                 130

gat gaa aag gga aag caa agg aac cgg cga ata ttt ggc ttg ttg atg 547
Asp Glu Lys Gly Lys Gln Arg Asn Arg Arg Ile Phe Gly Leu Leu Met
135                 140                 145                 150

ggt acc ctt caa aaa ttt aaa caa gaa tcc act gtt gct act gaa agg 595
Gly Thr Leu Gln Lys Phe Lys Gln Glu Ser Thr Val Ala Thr Glu Arg
                155                 160                 165

caa aag cgg cgc cag gaa att gaa caa aaa ctt gaa gtt cag gca gaa 643
Gln Lys Arg Arg Gln Glu Ile Glu Gln Lys Leu Glu Val Gln Ala Glu
            170                 175                 180

gaa gag aga aag cag gtt gaa aat gaa agg aga gaa ctg ttt gaa gag 691
Glu Glu Arg Lys Gln Val Glu Asn Glu Arg Arg Glu Leu Phe Glu Glu
        185                 190                 195

agg cgt gct aaa cag aca gaa ctg cgg ctt ttg gaa cag aaa gtt gag 739
Arg Arg Ala Lys Gln Thr Glu Leu Arg Leu Leu Glu Gln Lys Val Glu
    200                 205                 210

ctt gcg cag ctg caa gaa gaa tgg aat gaa cat aat gcc aaa ata att 787
Leu Ala Gln Leu Gln Glu Glu Trp Asn Glu His Asn Ala Lys Ile Ile
215                 220                 225                 230

aaa tat ata aga act aag aca aag ccc cat ttg ttt tat att cct gga 835
Lys Tyr Ile Arg Thr Lys Thr Lys Pro His Leu Phe Tyr Ile Pro Gly
                235                 240                 245

aga atg tgt cca gct acc caa aaa cta ata gaa gag tca cag aga aaa 883
Arg Met Cys Pro Ala Thr Gln Lys Leu Ile Glu Glu Ser Gln Arg Lys
            250                 255                 260

atg aac gct tta ttt gaa ggt aga cgc atc gaa ttt gca gaa caa ata 931
Met Asn Ala Leu Phe Glu Gly Arg Arg Ile Glu Phe Ala Glu Gln Ile
        265                 270                 275

aat aaa atg gag gct agg cct aga aga caa tca atg aag gaa aaa gag 979
Asn Lys Met Glu Ala Arg Pro Arg Arg Gln Ser Met Lys Glu Lys Glu
    280                 285                 290

cat cag gtg gtg cgt aat gaa gaa cag aag gcg gaa caa gaa gag ggt 1027
His Gln Val Val Arg Asn Glu Glu Gln Lys Ala Glu Gln Glu Glu Gly
295                 300                 305                 310

aag gtg gct cag cga gag gaa gag ttg gag gag aca ggt aat cag cac 1075
Lys Val Ala Gln Arg Glu Glu Glu Leu Glu Glu Thr Gly Asn Gln His
                315                 320                 325

aat gat gta gaa ata gag gaa gca gga gag gaa gag gaa aag gaa ata 1123
Asn Asp Val Glu Ile Glu Glu Ala Gly Glu Glu Glu Glu Lys Glu Ile
            330                 335                 340

gcg att gtt cat agt gat gca gag aaa gaa cag gag gag gaa gaa caa 1171
Ala Ile Val His Ser Asp Ala Glu Lys Glu Gln Glu Glu Glu Glu Gln
        345                 350                 355

aaa cag gaa atg gag gtt aag atg gag gag gaa act gag gta agg gaa 1219
Lys Gln Glu Met Glu Val Lys Met Glu Glu Glu Thr Glu Val Arg Glu
    360                 365                 370

agt gag aag cag cag gat agt cag cct gaa gaa gtt atg gat gtg cta 1267
Ser Glu Lys Gln Gln Asp Ser Gln Pro Glu Glu Val Met Asp Val Leu
375                 380                 385                 390

gag atg gtt gag aat gtc aaa cat gta att gct gac cag gag gta atg 1315
Glu Met Val Glu Asn Val Lys His Val Ile Ala Asp Gln Glu Val Met
                395                 400                 405

gaa act aat cga gtt gaa agt gta gaa cct tca gaa aat gaa gct agc 1363
Glu Thr Asn Arg Val Glu Ser Val Glu Pro Ser Glu Asn Glu Ala Ser
            410                 415                 420

aaa gaa ttg gaa cca gaa atg gaa ttt gaa att gag cca gat aaa gaa 1411
Lys Glu Leu Glu Pro Glu Met Glu Phe Glu Ile Glu Pro Asp Lys Glu
        425                 430                 435

tgt aaa tcc ctt tct cct ggg aaa gag aat gtc agt gct tta gac atg 1459
Cys Lys Ser Leu Ser Pro Gly Lys Glu Asn Val Ser Ala Leu Asp Met
    440                 445                 450

gaa aag gag tct gag gaa aaa gaa gaa aaa gaa tct gag ccc caa cct 1507
Glu Lys Glu Ser Glu Glu Lys Glu Glu Lys Glu Ser Glu Pro Gln Pro
455                 460                 465                 470

gag cct gtg gct caa cct cag cct cag tct cag ccc cag ctt cag ctt 1555
Glu Pro Val Ala Gln Pro Gln Pro Gln Ser Gln Pro Gln Leu Gln Leu
                475                 480                 485

caa tcc cag tcc caa cca gta ctc cag tcc cag cct ccc tct cag cct 1603
Gln Ser Gln Ser Gln Pro Val Leu Gln Ser Gln Pro Pro Ser Gln Pro
            490                 495                 500

gag gat ttg tca tta gct gtt tta cag cca aca ccc caa gtt act cag 1651
Glu Asp Leu Ser Leu Ala Val Leu Gln Pro Thr Pro Gln Val Thr Gln
        505                 510                 515

gag caa ggg cat tta cta cct gag agg aag gat ttt cct gta gag tct 1699
Glu Gln Gly His Leu Leu Pro Glu Arg Lys Asp Phe Pro Val Glu Ser
    520                 525                 530

gta aaa ctc act gag gta cca gta gag cca gtc ttg aca gta cat cca 1747
Val Lys Leu Thr Glu Val Pro Val Glu Pro Val Leu Thr Val His Pro
535                 540                 545                 550

gag agc aag agc aaa acc aaa act agg agc aga agt aga ggt cga gct 1795
Glu Ser Lys Ser Lys Thr Lys Thr Arg Ser Arg Ser Arg Gly Arg Ala
                555                 560                 565

aga aat aaa aca agc aag agt aga agt cga agc agt agc agt agc agt 1843
Arg Asn Lys Thr Ser Lys Ser Arg Ser Arg Ser Ser Ser Ser Ser Ser
            570                 575                 580

tct agt agc agt tca acc agt agc agc agt gga agt agt tcc agc agt 1891
Ser Ser Ser Ser Ser Thr Ser Ser Ser Ser Gly Ser Ser Ser Ser Ser
        585                 590                 595

gga agt agt agc agt cgc agt agt tcc agt agc agc tcc agt aca agt 1939
Gly Ser Ser Ser Ser Arg Ser Ser Ser Ser Ser Ser Ser Ser Thr Ser
    600                 605                 610

ggc agc agc agc aga gat agt agc agt agc act agt agt agt agt gag 1987

Gly Ser Ser Ser Arg Asp Ser Ser Ser Ser Thr Ser Ser Ser Ser Glu
615                 620                 625                 630

agt aga agt cgg agt agg ggc cgg gga cat aat aga gat aga aag cac     2035
Ser Arg Ser Arg Ser Arg Gly Arg Gly His Asn Arg Asp Arg Lys His
                635                 640                 645

aga agg agc gtg gat cgg aag aga agg gat act tca gga cta gaa aga     2083
Arg Arg Ser Val Asp Arg Lys Arg Arg Asp Thr Ser Gly Leu Glu Arg
            650                 655                 660

agt cac aaa tct tca aaa ggt ggt agt agt aga gat aca aaa gga tca     2131
Ser His Lys Ser Ser Lys Gly Gly Ser Ser Arg Asp Thr Lys Gly Ser
        665                 670                 675

aag gat aag aat tcc cgg tcc gac aga aag agg tct ata tca gag agt     2179
Lys Asp Lys Asn Ser Arg Ser Asp Arg Lys Arg Ser Ile Ser Glu Ser
    680                 685                 690

agt cga tca ggc aaa aga tct tca aga agt gaa aga gac cga aaa tca     2227
Ser Arg Ser Gly Lys Arg Ser Ser Arg Ser Glu Arg Asp Arg Lys Ser
695                 700                 705                 710

gac agg aaa gac aaa agg cgt taa tggaagaagc caggctttct tagccattct     2281
Asp Arg Lys Asp Lys Arg Arg
                715

ttgcagcaga agatttcttg ataaaaaagg attacctttc cttgtaaaga ggatgctgcc     2341

ttaagaattg catgttgtaa aaaatctttt tggaaaatac agactgtttg tttaccagac     2401

attcttgtac tttttgcata attttgtaag agttatttat caaaattatg tgaggttcca     2461

aaatatgtaa aaatgataat aataaaaaaa gattaacatc ccttgtcatc ttttttaaat     2521

atcctatact cttcagtaag aatctgtata ttttaatagg caaatcttta agtctgttcc     2581

cttctaattc tgtatcatac attgcttttg tagaaataaa tgtgtgttta tttcattatt     2641

tttgggatgt cctcgttgac acttgtataa taaatatcct ctttatcatt ttcagctttt     2701

aacactagat actgcacgtg attagaatgt ttttgaaggt ttcctcgttt ttatttgcct     2761

tggacagttt ttagttgtca gagtcagagc tttgcagctt tgagggggaa cagttctctt     2821

taaaatcatt tgctattttc tattctccct tgttatttta atctaagcat tttcccccgt     2881

ttctcatatt ttaaccatat gttcggtaga taactaaaca gtatgatctg gttggcattt     2941

tcttcctgat gatgggagcg tcattctttt gtcttcatgg ttacttgtgt gatataacat     3001

acatctgtta aagaaaatca cttctttcta ggggagggag gtagaaaagt atctttcaaa     3061

cttggttttt gagtttgtgt cttgtcttaa ctttgtgttg gctctaactt aaacatgctg     3121

atatgtgttt tcaagaattt tgtttaagga agtattgtat ggaagtccac aaaatgaagg     3181

aagttcatct aggttttaat atgtaagcaa gataacacac aagtgtacca agtgattatt     3241

aactttgttg ttttacaaat ttgtatgaac ttggagtatc tgttggccat tactatacat     3301

gtgcaaataa atgtggctta gacttgtgtg actgcttaag actagtactt gtattaactt     3361

tctgatgctt tatacaagag agcacttaaa ttgcatcctt ctttgagttt aacactgtag     3421

ctttagcctt gactttgaat attcattttg ccttcccttg acaagtaaat ggttacagtg     3481

aaaatgtgaa gaagtatttc cagttagttt ttgtgtgtat ataactcact tgtgtaagtg     3541

gatgtgttaa aagatctctt tttaaaaatc cttttattgg agtaattatt aaaacagtaa 3601

atgca 3606

<210> 38
<211> 717
<212> PRT
<213> Homo sapiens

<400> 38

Met Ala Val Ala Val Arg Thr Leu Gln Glu Gln Leu Glu Lys Ala Lys
1               5                   10                  15

Glu Ser Leu Lys Asn Val Asp Glu Asn Ile Arg Lys Leu Thr Gly Arg
            20                  25                  30

Asp Pro Asn Asp Val Arg Pro Ile Gln Ala Arg Leu Leu Ala Leu Ser
        35                  40                  45

Gly Pro Gly Gly Gly Arg Gly Arg Gly Ser Leu Leu Leu Arg Arg Gly
    50                  55                  60

Phe Ser Asp Ser Gly Gly Gly Pro Pro Ala Lys Gln Arg Asp Leu Glu
65                  70                  75                  80

Gly Ala Val Ser Arg Leu Gly Gly Glu Arg Arg Thr Arg Arg Glu Ser
                85                  90                  95

Arg Gln Glu Ser Asp Pro Glu Asp Asp Asp Val Lys Lys Pro Ala Leu
            100                 105                 110

Gln Ser Ser Val Val Ala Thr Ser Lys Glu Arg Thr Arg Arg Asp Leu
        115                 120                 125

Ile Gln Asp Gln Asn Met Asp Glu Lys Gly Lys Gln Arg Asn Arg Arg
    130                 135                 140

Ile Phe Gly Leu Leu Met Gly Thr Leu Gln Lys Phe Lys Gln Glu Ser
145                 150                 155                 160

Thr Val Ala Thr Glu Arg Gln Lys Arg Arg Gln Glu Ile Glu Gln Lys
                165                 170                 175

Leu Glu Val Gln Ala Glu Glu Glu Arg Lys Gln Val Glu Asn Glu Arg
            180                 185                 190

Arg Glu Leu Phe Glu Glu Arg Arg Ala Lys Gln Thr Glu Leu Arg Leu
        195                 200                 205

Leu Glu Gln Lys Val Glu Leu Ala Gln Leu Gln Glu Glu Trp Asn Glu
    210                 215                 220

His Asn Ala Lys Ile Ile Lys Tyr Ile Arg Thr Lys Thr Lys Pro His

225                 230                 235                 240

Leu Phe Tyr Ile Pro Gly Arg Met Cys Pro Ala Thr Gln Lys Leu Ile
                245                 250                 255

Glu Glu Ser Gln Arg Lys Met Asn Ala Leu Phe Glu Gly Arg Arg Ile
            260                 265                 270

Glu Phe Ala Glu Gln Ile Asn Lys Met Glu Ala Arg Pro Arg Arg Gln
        275                 280                 285

Ser Met Lys Glu Lys Glu His Gln Val Val Arg Asn Glu Glu Gln Lys
    290                 295                 300

Ala Glu Gln Glu Glu Gly Lys Val Ala Gln Arg Glu Glu Glu Leu Glu
305                 310                 315                 320

Glu Thr Gly Asn Gln His Asn Asp Val Glu Ile Glu Glu Ala Gly Glu
                325                 330                 335

Glu Glu Glu Lys Glu Ile Ala Ile Val His Ser Asp Ala Glu Lys Glu
            340                 345                 350

Gln Glu Glu Glu Glu Gln Lys Gln Glu Met Glu Val Lys Met Glu Glu
        355                 360                 365

Glu Thr Glu Val Arg Glu Ser Glu Lys Gln Gln Asp Ser Gln Pro Glu
    370                 375                 380

Glu Val Met Asp Val Leu Glu Met Val Glu Asn Val Lys His Val Ile
385                 390                 395                 400

Ala Asp Gln Glu Val Met Glu Thr Asn Arg Val Glu Ser Val Glu Pro
                405                 410                 415

Ser Glu Asn Glu Ala Ser Lys Glu Leu Glu Pro Glu Met Glu Phe Glu
            420                 425                 430

Ile Glu Pro Asp Lys Glu Cys Lys Ser Leu Ser Pro Gly Lys Glu Asn
        435                 440                 445

Val Ser Ala Leu Asp Met Glu Lys Glu Ser Glu Glu Lys Glu Glu Lys
    450                 455                 460

Glu Ser Glu Pro Gln Pro Glu Pro Val Ala Gln Pro Gln Pro Gln Ser
465                 470                 475                 480

Gln Pro Gln Leu Gln Leu Gln Ser Gln Ser Gln Pro Val Leu Gln Ser
                485                 490                 495

Gln Pro Pro Ser Gln Pro Glu Asp Leu Ser Leu Ala Val Leu Gln Pro
            500                 505                 510

Thr Pro Gln Val Thr Gln Glu Gln Gly His Leu Leu Pro Glu Arg Lys
        515                 520                 525

Asp Phe Pro Val Glu Ser Val Lys Leu Thr Glu Val Pro Val Glu Pro
    530                 535                 540

Val Leu Thr Val His Pro Glu Ser Lys Ser Lys Thr Lys Thr Arg Ser
545                 550                 555                 560

Arg Ser Arg Gly Arg Ala Arg Asn Lys Thr Ser Lys Ser Arg Ser Arg
                565                 570                 575

Ser Ser Ser Ser Ser Ser Ser Ser Ser Ser Ser Thr Ser Ser Ser Ser
            580                 585                 590

Gly Ser Ser Ser Ser Ser Gly Ser Ser Ser Ser Arg Ser Ser Ser Ser
        595                 600                 605

Ser Ser Ser Ser Thr Ser Gly Ser Ser Ser Arg Asp Ser Ser Ser Ser
    610                 615                 620

Thr Ser Ser Ser Ser Glu Ser Arg Ser Arg Ser Arg Gly Arg Gly His
625                 630                 635                 640

Asn Arg Asp Arg Lys His Arg Arg Ser Val Asp Arg Lys Arg Arg Asp
                645                 650                 655

Thr Ser Gly Leu Glu Arg Ser His Lys Ser Ser Lys Gly Gly Ser Ser
            660                 665                 670

Arg Asp Thr Lys Gly Ser Lys Asp Lys Asn Ser Arg Ser Asp Arg Lys
        675                 680                 685

Arg Ser Ile Ser Glu Ser Ser Arg Ser Gly Lys Arg Ser Ser Arg Ser
    690                 695                 700

Glu Arg Asp Arg Lys Ser Asp Arg Lys Asp Lys Arg Arg
705                 710                 715

<210>  39
<211>  2509
<212>  DNA
<213>  Homo sapiens

<220>
<221>  CDS
<222>  (68)..(1924)
<223>  RHOT2 mRNA

<400>  39
gagcgaaagg cttgaggacc aggtcggggc cgggttccgg gtcggggagc ggctccgggc      60

ggcagct atg agg cgg gac gtg cgc atc ctg tta ctg ggc gag gcc cag      109

Met Arg Arg Asp Val Arg Ile Leu Leu Leu Gly Glu Ala Gln
1               5                   10

gtg ggg aag acg tcg ctg atc ctg tcc ctg gtg ggc gag gag ttc ccc     157
Val Gly Lys Thr Ser Leu Ile Leu Ser Leu Val Gly Glu Glu Phe Pro
15                  20                  25                  30

gag gag gtc cct ccc cgc gcg gag gag atc acg atc ccc gcg gac gtc     205
Glu Glu Val Pro Pro Arg Ala Glu Glu Ile Thr Ile Pro Ala Asp Val
                35                  40                  45

acc ccg gag aag gtg ccc acc cac atc gtg gac tac tca gaa gcc gag     253
Thr Pro Glu Lys Val Pro Thr His Ile Val Asp Tyr Ser Glu Ala Glu
            50                  55                  60

cag acg gac gag gag ctg cgg gag gag atc cac aag gca aac gtg gtg     301
Gln Thr Asp Glu Glu Leu Arg Glu Glu Ile His Lys Ala Asn Val Val
        65                  70                  75

tgt gtg gtg tat gac gtc tct gag gag gcc acc att gag aag att cga     349
Cys Val Val Tyr Asp Val Ser Glu Glu Ala Thr Ile Glu Lys Ile Arg
    80                  85                  90

act aag tgg atc cca ctg gtg aat ggg ggg acc acg cag ggg ccc agg     397
Thr Lys Trp Ile Pro Leu Val Asn Gly Gly Thr Thr Gln Gly Pro Arg
95                  100                 105                 110

gtg ccc atc atc cta gtg ggc aac aag tca gac ctg cgg tcg ggg agc     445
Val Pro Ile Ile Leu Val Gly Asn Lys Ser Asp Leu Arg Ser Gly Ser
                115                 120                 125

tcc atg gag gcc gtg ctc ccc atc atg agc cag ttt ccc gag att gag     493
Ser Met Glu Ala Val Leu Pro Ile Met Ser Gln Phe Pro Glu Ile Glu
            130                 135                 140

acc tgc gtg gag tgt tcg gcc aag aac ctg agg aac atc tca gag ctg     541
Thr Cys Val Glu Cys Ser Ala Lys Asn Leu Arg Asn Ile Ser Glu Leu
        145                 150                 155

ttc tac tac gcc cag aag gcc gtc ctg cat ccc aca gcc ccc ctc tat     589
Phe Tyr Tyr Ala Gln Lys Ala Val Leu His Pro Thr Ala Pro Leu Tyr
    160                 165                 170

gac cct gag gcc aag cag ttg agg ccc gcg tgc gcc cag gcg ctg acg     637
Asp Pro Glu Ala Lys Gln Leu Arg Pro Ala Cys Ala Gln Ala Leu Thr
175                 180                 185                 190

cgc atc ttc agg ctc tca gat cag gac ctg gac cag gcg ctc agt gac     685
Arg Ile Phe Arg Leu Ser Asp Gln Asp Leu Asp Gln Ala Leu Ser Asp
                195                 200                 205

gaa gag ctc aac gct ttc cag aaa tcc tgc ttt ggg cac ccc ctg gcc     733
Glu Glu Leu Asn Ala Phe Gln Lys Ser Cys Phe Gly His Pro Leu Ala
            210                 215                 220

ccg cag gcc ctg gag gac gtg aag acg gtg gtg tgc agg aac gtg gcg     781
Pro Gln Ala Leu Glu Asp Val Lys Thr Val Val Cys Arg Asn Val Ala
        225                 230                 235

ggc ggc gtg cgg gag gac cgg ctg acc ctg gat ggt ttc ctc ttc ctg     829
Gly Gly Val Arg Glu Asp Arg Leu Thr Leu Asp Gly Phe Leu Phe Leu
    240                 245                 250

aac acg ctc ttc atc cag cgc ggc cgg cac gag acc acc tgg acc atc     877
Asn Thr Leu Phe Ile Gln Arg Gly Arg His Glu Thr Thr Trp Thr Ile
255                 260                 265                 270

ctg cgg cgc ttc ggc tac agc gat gcc ctg gag ctg act gcg gac tat     925
Leu Arg Arg Phe Gly Tyr Ser Asp Ala Leu Glu Leu Thr Ala Asp Tyr

                275                 280                 285

ctc tcc cct ctg atc cac gtg ccc ccc ggc tgc agc acg gag ctc aac 973
Leu Ser Pro Leu Ile His Val Pro Pro Gly Cys Ser Thr Glu Leu Asn
            290                 295                 300

cac ctt ggc tac cag ttt gtg cag aga gtg ttt gag aag cac gac cag 1021
His Leu Gly Tyr Gln Phe Val Gln Arg Val Phe Glu Lys His Asp Gln
        305                 310                 315

gac cgc gac ggc gcc ctc tcg ccc gtg gag ctg caa agc ctt ttc agt 1069
Asp Arg Asp Gly Ala Leu Ser Pro Val Glu Leu Gln Ser Leu Phe Ser
    320                 325                 330

gtg ttc cca gca gcg ccc tgg ggc ccc gag ctc cca cgc aca gtc cgc 1117
Val Phe Pro Ala Ala Pro Trp Gly Pro Glu Leu Pro Arg Thr Val Arg
335                 340                 345                 350

aca gag gcc ggc cgg ttg ccc ctg cac gga tac ctc tgc cag tgg acc 1165
Thr Glu Ala Gly Arg Leu Pro Leu His Gly Tyr Leu Cys Gln Trp Thr
                355                 360                 365

ctg gtg acc tac ctg gac gtc cgg agc tgc ctt gga cac cta ggc tac 1213
Leu Val Thr Tyr Leu Asp Val Arg Ser Cys Leu Gly His Leu Gly Tyr
            370                 375                 380

ctg ggc tac ccc acc ctc tgt gag cag gac cag gcc cat gcc atc aca 1261
Leu Gly Tyr Pro Thr Leu Cys Glu Gln Asp Gln Ala His Ala Ile Thr
        385                 390                 395

gtc act cgt gag aag agg ctg gac cag gag aag gga cag acg cag cgg 1309
Val Thr Arg Glu Lys Arg Leu Asp Gln Glu Lys Gly Gln Thr Gln Arg
    400                 405                 410

agc gtc ctc ctg tgc aag gtg gta ggg gcc cgt gga gtg ggc aag tct 1357
Ser Val Leu Leu Cys Lys Val Val Gly Ala Arg Gly Val Gly Lys Ser
415                 420                 425                 430

gcc ttc ctg cag gct ttt ctc ggc cgc ggc ctg ggg cac cag gac acg 1405
Ala Phe Leu Gln Ala Phe Leu Gly Arg Gly Leu Gly His Gln Asp Thr
                435                 440                 445

agg gag cag cct ccc ggc tac gcc atc gac acg gtg cag gtc aat gga 1453
Arg Glu Gln Pro Pro Gly Tyr Ala Ile Asp Thr Val Gln Val Asn Gly
            450                 455                 460

cag gag aag tac ttg atc ctc tgt gag gtg ggc aca gat ggt ctg ctg 1501
Gln Glu Lys Tyr Leu Ile Leu Cys Glu Val Gly Thr Asp Gly Leu Leu
        465                 470                 475

gcc aca tcg ctg gac gcc acc tgt gac gtt gcc tgc ttg atg ttt gat 1549
Ala Thr Ser Leu Asp Ala Thr Cys Asp Val Ala Cys Leu Met Phe Asp
    480                 485                 490

ggc agt gac cca aag tcc ttt gca cat tgt gcc agc gtc tac aag cac 1597
Gly Ser Asp Pro Lys Ser Phe Ala His Cys Ala Ser Val Tyr Lys His
495                 500                 505                 510

cat tac atg gac ggg cag acc ccc tgc ctc ttt gtc tcc tcc aag gcc 1645
His Tyr Met Asp Gly Gln Thr Pro Cys Leu Phe Val Ser Ser Lys Ala
                515                 520                 525

gac ctg ccc gaa ggt gtc gcg gtg tct ggc cca tca ccg gcc gag ttt 1693
Asp Leu Pro Glu Gly Val Ala Val Ser Gly Pro Ser Pro Ala Glu Phe
            530                 535                 540

tgc cgc aag cac cgg cta ccc gct ccc gtg ccg ttc tcc tgt gct ggc 1741
Cys Arg Lys His Arg Leu Pro Ala Pro Val Pro Phe Ser Cys Ala Gly
        545                 550                 555

cca gcc gag ccc agc acc acc atc ttc acc cag ctc gcc acc atg gcc     1789
Pro Ala Glu Pro Ser Thr Thr Ile Phe Thr Gln Leu Ala Thr Met Ala
    560                 565                 570

gcc ttc cca cat ttg gtc cac gca gag ctg cat ccc tct tcc ttc tgg     1837
Ala Phe Pro His Leu Val His Ala Glu Leu His Pro Ser Ser Phe Trp
575                 580                 585                 590

ctc cgg ggg ctg ctg ggg gtt gtc ggg gcc gcc gtg gcc gca gtc ctc     1885
Leu Arg Gly Leu Leu Gly Val Val Gly Ala Ala Val Ala Ala Val Leu
                595                 600                 605

agc ttc tca ctc tac agg gtc ctg gtg aag agc cag tga ggcccctggt     1934
Ser Phe Ser Leu Tyr Arg Val Leu Val Lys Ser Gln
            610                 615

acccaagccc cctcccctga cctgggtgtg cctcgctgct ggggctctgc aggggcagca     1994

cagctggggt gcaggccagg ctgccactcc gggaacgcct ttgcgccggg actttttgtt     2054

tctgaaggca gtcgatctgc agcggggcct tatgctgcca tgcactgccc tggctcctgc     2114

cggaccccca gggtgggccg tggcaggtgg ctgagcagga gctcccaagt gccggccacc     2174

gctgtcaggg attgcccacc cctgggcatc atgtgtgtgg ggccggggag cacaggtgtg     2234

ggagctggtg accccagacc cagaattctc agggctctac ccccctttcc tggtcctagg     2294

tggccagtgg gtatgaggag ggctggaagg cagagctttg ggccaaaagc aggcgttggg     2354

gggtcccccc tcaagtttgg agccgtttcc gtggttgtag cagaggaccg gaggttgggt     2414

tcctgattaa acttcactgt gtgttttcta tctcggatcc cagtctctga agacaacttg     2474

ctttgattca acctaaaaaa aaaaaaaaaa aaaaa                                2509

<210>  40
<211>  618
<212>  PRT
<213>  Homo sapiens

<400>  40

Met Arg Arg Asp Val Arg Ile Leu Leu Leu Gly Glu Ala Gln Val Gly
1               5                   10                  15

Lys Thr Ser Leu Ile Leu Ser Leu Val Gly Glu Glu Phe Pro Glu Glu
            20                  25                  30

Val Pro Pro Arg Ala Glu Glu Ile Thr Ile Pro Ala Asp Val Thr Pro
        35                  40                  45

Glu Lys Val Pro Thr His Ile Val Asp Tyr Ser Glu Ala Glu Gln Thr
    50                  55                  60

Asp Glu Glu Leu Arg Glu Glu Ile His Lys Ala Asn Val Val Cys Val
65                  70                  75                  80

Val Tyr Asp Val Ser Glu Glu Ala Thr Ile Glu Lys Ile Arg Thr Lys
                85                  90                  95

Trp Ile Pro Leu Val Asn Gly Gly Thr Thr Gln Gly Pro Arg Val Pro
            100                 105                 110

Ile Ile Leu Val Gly Asn Lys Ser Asp Leu Arg Ser Gly Ser Ser Met
        115                 120                 125

Glu Ala Val Leu Pro Ile Met Ser Gln Phe Pro Glu Ile Glu Thr Cys
    130                 135                 140

Val Glu Cys Ser Ala Lys Asn Leu Arg Asn Ile Ser Glu Leu Phe Tyr
145                 150                 155                 160

Tyr Ala Gln Lys Ala Val Leu His Pro Thr Ala Pro Leu Tyr Asp Pro
                165                 170                 175

Glu Ala Lys Gln Leu Arg Pro Ala Cys Ala Gln Ala Leu Thr Arg Ile
            180                 185                 190

Phe Arg Leu Ser Asp Gln Asp Leu Asp Gln Ala Leu Ser Asp Glu Glu
        195                 200                 205

Leu Asn Ala Phe Gln Lys Ser Cys Phe Gly His Pro Leu Ala Pro Gln
    210                 215                 220

Ala Leu Glu Asp Val Lys Thr Val Val Cys Arg Asn Val Ala Gly Gly
225                 230                 235                 240

Val Arg Glu Asp Arg Leu Thr Leu Asp Gly Phe Leu Phe Leu Asn Thr
                245                 250                 255

Leu Phe Ile Gln Arg Gly Arg His Glu Thr Thr Trp Thr Ile Leu Arg
            260                 265                 270

Arg Phe Gly Tyr Ser Asp Ala Leu Glu Leu Thr Ala Asp Tyr Leu Ser
        275                 280                 285

Pro Leu Ile His Val Pro Pro Gly Cys Ser Thr Glu Leu Asn His Leu
    290                 295                 300

Gly Tyr Gln Phe Val Gln Arg Val Phe Glu Lys His Asp Gln Asp Arg
305                 310                 315                 320

Asp Gly Ala Leu Ser Pro Val Glu Leu Gln Ser Leu Phe Ser Val Phe
                325                 330                 335

Pro Ala Ala Pro Trp Gly Pro Glu Leu Pro Arg Thr Val Arg Thr Glu
            340                 345                 350

Ala Gly Arg Leu Pro Leu His Gly Tyr Leu Cys Gln Trp Thr Leu Val
        355                 360                 365

Thr Tyr Leu Asp Val Arg Ser Cys Leu Gly His Leu Gly Tyr Leu Gly

370                 375                 380

Tyr Pro Thr Leu Cys Glu Gln Asp Gln Ala His Ala Ile Thr Val Thr
385                 390                 395                 400

Arg Glu Lys Arg Leu Asp Gln Glu Lys Gly Gln Thr Gln Arg Ser Val
                405                 410                 415

Leu Leu Cys Lys Val Val Gly Ala Arg Gly Val Gly Lys Ser Ala Phe
            420                 425                 430

Leu Gln Ala Phe Leu Gly Arg Gly Leu Gly His Gln Asp Thr Arg Glu
        435                 440                 445

Gln Pro Pro Gly Tyr Ala Ile Asp Thr Val Gln Val Asn Gly Gln Glu
    450                 455                 460

Lys Tyr Leu Ile Leu Cys Glu Val Gly Thr Asp Gly Leu Leu Ala Thr
465                 470                 475                 480

Ser Leu Asp Ala Thr Cys Asp Val Ala Cys Leu Met Phe Asp Gly Ser
                485                 490                 495

Asp Pro Lys Ser Phe Ala His Cys Ala Ser Val Tyr Lys His His Tyr
            500                 505                 510

Met Asp Gly Gln Thr Pro Cys Leu Phe Val Ser Ser Lys Ala Asp Leu
        515                 520                 525

Pro Glu Gly Val Ala Val Ser Gly Pro Ser Pro Ala Glu Phe Cys Arg
    530                 535                 540

Lys His Arg Leu Pro Ala Pro Val Pro Phe Ser Cys Ala Gly Pro Ala
545                 550                 555                 560

Glu Pro Ser Thr Thr Ile Phe Thr Gln Leu Ala Thr Met Ala Ala Phe
                565                 570                 575

Pro His Leu Val His Ala Glu Leu His Pro Ser Ser Phe Trp Leu Arg
            580                 585                 590

Gly Leu Leu Gly Val Val Gly Ala Ala Val Ala Ala Val Leu Ser Phe
        595                 600                 605

Ser Leu Tyr Arg Val Leu Val Lys Ser Gln
    610                 615

<210>  41
<211>  20
<212>  DNA
<213>  Artificial

<220>

<223>  AFP Forward primer (5'-3')

<400>  41
aactattggc ctgtggcgag                                                    20


<210>  42
<211>  19
<212>  DNA
<213>  Artificial

<220>
<223>  AFP Reverse primer (5'-3')

<400>  42
tcatccacca ccaagctgc                                                     19


<210>  43
<211>  20
<212>  DNA
<213>  Artificial

<220>
<223>  ALDH2 Forward primer (5'-3')

<400>  43
gtttggagcc cagtcaccct                                                    20


<210>  44
<211>  21
<212>  DNA
<213>  Artificial

<220>
<223>  ALDH2 Reverse primer (5'-3')

<400>  44
gggaggaagc ttgcatgatt c                                                  21


<210>  45
<211>  21
<212>  DNA
<213>  Artificial

<220>
<223>  APCS Forward primer (5'-3')

<400>  45
ggccaggaat atgaacaagc c                                                  21


<210>  46
<211>  20
<212>  DNA
<213>  Artificial

<220>
<223>  APCS Reverse primer (5'-3')

<400>  46
cttctccagc ggtgtgatca                                                    20


<210>  47
<211>  20
<212>  DNA

<213>  Artificial

<220>
<223>  APOC4 Forward primer (5'-3')

<400>  47
ggagctgctg gagacagtgg                                                    20


<210>  48
<211>  20
<212>  DNA
<213>  Artificial

<220>
<223>  APOC4 Reverse primer (5'-3')

<400>  48
tttggattcg aggaaccagg                                                    20


<210>  49
<211>  19
<212>  DNA
<213>  Artificial

<220>
<223>  AQP9 Forward primer (5'-3')

<400>  49
gcttcctccc tgggactga                                                     19


<210>  50
<211>  20
<212>  DNA
<213>  Artificial

<220>
<223>  AQP9 Reverse primer (5'-3')

<400>  50
caaccaaagg gcccactaca                                                    20


<210>  51
<211>  21
<212>  DNA
<213>  Artificial

<220>
<223>  BUB1 Forward primer (5'-3')

<400>  51
acccctgaaa aagtgatgcc t                                                  21


<210>  52
<211>  21
<212>  DNA
<213>  Artificial

<220>
<223>  BUB1 Reverse primer (5'-3')

<400>  52
tcatcctgtt ccaaaaatcc g                                                  21

<210> 53
<211> 21
<212> DNA
<213> Artificial

<220>
<223> C1S Forward primer (5'-3')

<400> 53
ttgtttggtt ctgtcatccg c 21

<210> 54
<211> 19
<212> DNA
<213> Artificial

<220>
<223> C1S Reverse primer (5'-3')

<400> 54
tggaacacat ttcggcagc 19

<210> 55
<211> 23
<212> DNA
<213> Artificial

<220>
<223> CYP2E1 Forward primer (5'-3')

<400> 55
caaccaagaa tttcctgatc cag 23

<210> 56
<211> 21
<212> DNA
<213> Artificial

<220>
<223> CYP2E1 Reverse primer (5'-3')

<400> 56
aagaaacaac tccatgcgag c 21

<210> 57
<211> 22
<212> DNA
<213> Artificial

<220>
<223> DLG7 Forward primer (5'-3')

<400> 57
gcaggaagaa tgtgctgaaa ca 22

<210> 58
<211> 21
<212> DNA
<213> Artificial

<220>
<223> DLG7 Reverse primer (5'-3')

<400> 58

tccaagtctt tgagaagggc c                                                21

<210>  59
<211>  20
<212>  DNA
<213>  Artificial

<220>
<223>  DUSP9 Forward primer (5'-3')

<400>  59
cggaggccat tgagttcatt                                                   20

<210>  60
<211>  21
<212>  DNA
<213>  Artificial

<220>
<223>  DUSP9 Reverse primer (5'-3')

<400>  60
accaggtcat aggcatcgtt g                                                 21

<210>  61
<211>  20
<212>  DNA
<213>  Artificial

<220>
<223>  E2F5 Forward primer (5'-3')

<400>  61
ccattcaggc accttctggt                                                   20

<210>  62
<211>  22
<212>  DNA
<213>  Artificial

<220>
<223>  E2F5 Reverse primer (5'-3')

<400>  62
acgggcttag atgaactcga ct                                                22

<210>  63
<211>  19
<212>  DNA
<213>  Artificial

<220>
<223>  GHR Forward primer (5'-3')

<400>  63
cttggcactg gcaggatca                                                    19

<210>  64
<211>  19
<212>  DNA
<213>  Artificial

<220>

<223>  GHR Reverse primer (5'-3')

<400>  64
aggtgaacgg cacttggtg                                                    19


<210>  65
<211>  20
<212>  DNA
<213>  Artificial

<220>
<223>  HPD Forward primer (5'-3')

<400>  65
atcttcacca aaccggtgca                                                   20


<210>  66
<211>  21
<212>  DNA
<213>  Artificial

<220>
<223>  HPD Reverse primer (5'-3')

<400>  66
ccatgttggt gaggttaccc c                                                 21


<210>  67
<211>  21
<212>  DNA
<213>  Artificial

<220>
<223>  IGSF1 Forward primer (5'-3')

<400>  67
cactcacact gaaaaacgcc c                                                 21


<210>  68
<211>  21
<212>  DNA
<213>  Artificial

<220>
<223>  IGSF1 Reverse primer (5'-3')

<400>  68
gggtggagca attgaaagtc a                                                 21


<210>  69
<211>  20
<212>  DNA
<213>  Artificial

<220>
<223>  NLE1 Forward primer (5'-3')

<400>  69
atgtgaaggc ccagaagctg                                                   20


<210>  70
<211>  19
<212>  DNA

<213>  Artificial

<220>
<223>  NLE1 Reverse primer (5'-3')

<400>  70
gagaacttcg ggccgtctc                                                    19


<210>  71
<211>  20
<212>  DNA
<213>  Artificial

<220>
<223>  RPL10A Forward primer (5'-3')

<400>  71
tatcccccac atggacatcg                                                   20


<210>  72
<211>  21
<212>  DNA
<213>  Artificial

<220>
<223>  RPL10A Reverse primer (5'-3')

<400>  72
tgccttattt aaacctgggc c                                                 21


<210>  73
<211>  20
<212>  DNA
<213>  Artificial

<220>
<223>  ACTG1 Forward primer (5'-3')

<400>  73
gatggccagg tcatcaccat                                                   20


<210>  74
<211>  20
<212>  DNA
<213>  Artificial

<220>
<223>  ACTG1 Reverse primer (5'-3')

<400>  74
acaggtcttt gcggatgtcc                                                   20


<210>  75
<211>  20
<212>  DNA
<213>  Artificial

<220>
<223>  EFF1A1 Forward primer (5'-3')

<400>  75
tcacccgtaa ggatggcaat                                                   20

<210>  76
<211>  20
<212>  DNA
<213>  Artificial

<220>
<223>  EFF1A1 Reverse primer (5'-3')

<400>  76
cggccaacag gaacagtacc                                                    20


<210>  77
<211>  20
<212>  DNA
<213>  Artificial

<220>
<223>  PNN Forward primer (5'-3')

<400>  77
cctttctggt cctggtggag                                                    20


<210>  78
<211>  21
<212>  DNA
<213>  Artificial

<220>
<223>  PNN Reverse primer (5'-3')

<400>  78
tgattctctt ctggtccgac g                                                  21


<210>  79
<211>  21
<212>  DNA
<213>  Artificial

<220>
<223>  RHOT2 Forward primer (5'-3')

<400>  79
ctgcggacta tctctcccct c                                                  21


<210>  80
<211>  20
<212>  DNA
<213>  Artificial

<220>
<223>  RHOT2 Reverse primer (5'-3')

<400>  80
aaaaggcttt gcagctccac                                                    20


<210>  81
<211>  20
<212>  DNA
<213>  Artificial

<220>
<223>  AFP Forward primer

<400>  81

gccagtgctg cacttcttca                                                    20

<210>  82
<211>  21
<212>  DNA
<213>  Artificial

<220>
<223>  AFP Reverse primer

<400>  82
tgtttcatcc accaccaagc t                                                  21

<210>  83
<211>  24
<212>  DNA
<213>  Artificial

<220>
<223>  AFP probe

<400>  83
atgccaacag gaggccatgc ttca                                               24

<210>  84
<211>  19
<212>  DNA
<213>  Artificial

<220>
<223>  ALDH2 Forward primer

<400>  84
tgcaggatgg catgaccat                                                     19

<210>  85
<211>  24
<212>  DNA
<213>  Artificial

<220>
<223>  ALDH2 Reverse primer

<400>  85
tcttgaactt caggatctgc atca                                               24

<210>  86
<211>  23
<212>  DNA
<213>  Artificial

<220>
<223>  ALDH2 probe

<400>  86
ccaaggagga gatcttcggg cca                                                23

<210>  87
<211>  22
<212>  DNA
<213>  Artificial

<220>

<223>  APCS Forward primer

<400>  87
agctgggagt cctcatcagg ta                                              22


<210>  88
<211>  20
<212>  DNA
<213>  Artificial

<220>
<223>  APCS Reverse primer

<400>  88
cgcagaccct ttttcaccaa                                                 20


<210>  89
<211>  27
<212>  DNA
<213>  Artificial

<220>
<223>  APCS probe

<400>  89
tgctgaattt tggatcaatg ggacacc                                         27


<210>  90
<211>  20
<212>  DNA
<213>  Artificial

<220>
<223>  APOC4 Forward primer

<400>  90
tgaaggagct gctggagaca                                                 20


<210>  91
<211>  18
<212>  DNA
<213>  Artificial

<220>
<223>  APOC4 Reverse primer

<400>  91
cgggctccag aaccattg                                                   18


<210>  92
<211>  25
<212>  DNA
<213>  Artificial

<220>
<223>  APOC4 probe

<400>  92
tggtgaacag gaccagagac gggtg                                           25


<210>  93
<211>  19
<212>  DNA

<213>  Artificial

<220>
<223>  AQP9 Forward primer

<400>  93
gccatcggcc tcctgatta                                                    19


<210>  94
<211>  20
<212>  DNA
<213>  Artificial

<220>
<223>  AQP9 Reverse primer

<400>  94
gttcatggca cagccactgt                                                   20


<210>  95
<211>  25
<212>  DNA
<213>  Artificial

<220>
<223>  AQP9 probe

<400>  95
tgtcattgct tcctccctgg gactg                                             25


<210>  96
<211>  25
<212>  DNA
<213>  Artificial

<220>
<223>  BUB1 Forward primer

<400>  96
acatctggtt ttcagtgtgt tgaga                                             25


<210>  97
<211>  21
<212>  DNA
<213>  Artificial

<220>
<223>  BUB1 Reverse primer

<400>  97
gttgcagcaa ccccaaagta a                                                 21


<210>  98
<211>  30
<212>  DNA
<213>  Artificial

<220>
<223>  BUB1 probe

<400>  98
tcagcaacaa accatggaac taccagatcg                                        30

<210>  99
<211>  23
<212>  DNA
<213>  Artificial

<220>
<223>  C1S Forward primer

<400>  99
tcccaatgac aagaccaaat tct                                              23


<210>  100
<211>  21
<212>  DNA
<213>  Artificial

<220>
<223>  C1S Reverse primer

<400>  100
agagcccata ggtcccacac t                                                21


<210>  101
<211>  21
<212>  DNA
<213>  Artificial

<220>
<223>  C1S probe

<400>  101
cgcagctggc ctggtgtcct g                                                21


<210>  102
<211>  22
<212>  DNA
<213>  Artificial

<220>
<223>  CYP2E1 Forward primer

<400>  102
catgagattc agcggttcat ca                                               22


<210>  103
<211>  19
<212>  DNA
<213>  Artificial

<220>
<223>  CYP2E1 Reverse primer

<400>  103
ggtgtctcgg gttgcttca                                                   19


<210>  104
<211>  21
<212>  DNA
<213>  Artificial

<220>
<223>  CYP2E1 probe

<400>  104

cctcgtgccc tccaacctgc c                                                21

<210>  105
<211>  23
<212>  DNA
<213>  Artificial

<220>
<223>  DLG7 Forward primer

<400>  105
gctggagagg agacatcaag aac                                              23

<210>  106
<211>  27
<212>  DNA
<213>  Artificial

<220>
<223>  DLG7 Reverse primer

<400>  106
cctggttgta gaggtgaaaa agtaatc                                          27

<210>  107
<211>  29
<212>  DNA
<213>  Artificial

<220>
<223>  DLG7 probe

<400>  107
tgccagacac atttcttttg gtggtaacc                                       29

<210>  108
<211>  21
<212>  DNA
<213>  Artificial

<220>
<223>  DUSP9 Forward primer

<400>  108
ggcctacctc atgcagaagc t                                                21

<210>  109
<211>  26
<212>  DNA
<213>  Artificial

<220>
<223>  DUSP9 Reverse primer

<400>  109
gggagatgtt agacttcttc ctcttg                                           26

<210>  110
<211>  30
<212>  DNA
<213>  Artificial

<220>

<223>  DUSP9 probe

<400>  110
cacctctctc tcaacgatgc ctatgacctg                                        30


<210>  111
<211>  19
<212>  DNA
<213>  Artificial

<220>
<223>  E2F5 Forward primer

<400>  111
cctgttcccc cacctgatg                                                    19


<210>  112
<211>  23
<212>  DNA
<213>  Artificial

<220>
<223>  E2F5 Reverse primer

<400>  112
tttctgtgga gtcactggag tca                                               23


<210>  113
<211>  25
<212>  DNA
<213>  Artificial

<220>
<223>  E2F5 probe

<400>  113
cctcacacag ccttcctccc agtcc                                             25


<210>  114
<211>  20
<212>  DNA
<213>  Artificial

<220>
<223>  GHR Forward primer

<400>  114
cccaggtgag cgacattaca                                                   20


<210>  115
<211>  22
<212>  DNA
<213>  Artificial

<220>
<223>  GHR Reverse primer

<400>  115
catccctgcc ttattctttt gg                                                22


<210>  116
<211>  26
<212>  DNA

<213>  Artificial

<220>
<223>  GHR probe

<400>  116
cagcaggtag tgtggtcctt tccccg                                          26


<210>  117
<211>  18
<212>  DNA
<213>  Artificial

<220>
<223>  HPD Forward primer

<400>  117
cccacgctct tcctggaa                                                   18


<210>  118
<211>  17
<212>  DNA
<213>  Artificial

<220>
<223>  HPD Reverse primer

<400>  118
ttgccggctc caaaacc                                                    17


<210>  119
<211>  22
<212>  DNA
<213>  Artificial

<220>
<223>  HPD probe

<400>  119
tcatccagcg ccacaaccac ca                                              22


<210>  120
<211>  22
<212>  DNA
<213>  Artificial

<220>
<223>  IGSF1 Forward primer

<400>  120
gaccattgcc cttgaagagt gt                                              22


<210>  121
<211>  24
<212>  DNA
<213>  Artificial

<220>
<223>  IGSF1 Reverse primer

<400>  121
gagaggttga tgaaggagaa ttgg                                            24

<210>  122
<211>  25
<212>  DNA
<213>  Artificial

<220>
<223>  IGSF1 probe

<400>  122
accaagaagg agaaccaggc acccc                                          25


<210>  123
<211>  21
<212>  DNA
<213>  Artificial

<220>
<223>  NLE1 Forward primer

<400>  123
tgcctccttt gacaagtcca t                                              21


<210>  124
<211>  19
<212>  DNA
<213>  Artificial

<220>
<223>  NLE1 Reverse primer

<400>  124
cgcgtaggga agccaggta                                                 19


<210>  125
<211>  19
<212>  DNA
<213>  Artificial

<220>
<223>  NLE1 probe

<400>  125
tgggatggca ggacgggca                                                 19


<210>  126
<211>  21
<212>  DNA
<213>  Artificial

<220>
<223>  RPL10A Forward primer

<400>  126
tcggcccagg tttaaataag g                                              21


<210>  127
<211>  20
<212>  DNA
<213>  Artificial

<220>
<223>  RPL10A Reverse primer

<400>  127

ccactttggc caccatgttt                                                    20


<210>  128
<211>  27
<212>  DNA
<213>  Artificial

<220>
<223>  RPL10A probe

<400>  128
agttcccttc cctgctcaca cacaacg                                             27


<210>  129
<211>  15
<212>  DNA
<213>  Artificial

<220>
<223>  ACTG1 Forward primer

<400>  129
ggcgcccagc accat                                                          15


<210>  130
<211>  21
<212>  DNA
<213>  Artificial

<220>
<223>  ACTG1 Reverse primer

<400>  130
ccgatccaca ccgagtactt g                                                   21


<210>  131
<211>  25
<212>  DNA
<213>  Artificial

<220>
<223>  ACTG1 probe

<400>  131
atcaagatca tcgcaccccc agagc                                               25


<210>  132
<211>  19
<212>  DNA
<213>  Artificial

<220>
<223>  EFF1A1 Forward primer

<400>  132
gcggtgggtg tcatcaaag                                                      19


<210>  133
<211>  20
<212>  DNA
<213>  Artificial

<220>

<223>  EFF1A1 Reverse primer

<400>  133
tgggcagact tggtgacctt                                                    20


<210>  134
<211>  26
<212>  DNA
<213>  Artificial

<220>
<223>  EFF1A1 probe

<400>  134
agtggacaag aaggctgctg gagctg                                             26


<210>  135
<211>  19
<212>  DNA
<213>  Artificial

<220>
<223>  PNN Forward primer

<400>  135
gaattcccgg tccgacaga                                                     19


<210>  136
<211>  24
<212>  DNA
<213>  Artificial

<220>
<223>  PNN Reverse primer

<400>  136
tttcggtctc tttcacttct tgaa                                               24


<210>  137
<211>  38
<212>  DNA
<213>  Artificial

<220>
<223>  PNN probe

<400>  137
agaggtctat atcagagagt agtcgatcag gcaaaaga                                38


<210>  138
<211>  20
<212>  DNA
<213>  Artificial

<220>
<223>  RHOT2 Forward Primer

<400>  138
cccagcacca ccatcttcac                                                    20


<210>  139
<211>  20
<212>  DNA

<213>  Artificial

<220>
<223>  RHOT2 Reverse primer

<400>  139
ccagaaggaa gagggatgca                                                    20


<210>  140
<211>  19
<212>  DNA
<213>  Artificial

<220>
<223>  RHOT2  probe

<400>  140
cagctcgcca ccatggccg                                                     19

## Description

The present invention relates to a method to *in vitro* determine the grade of a liver tumor in a sample previously obtained from a patient, using a molecular signature based on the expression of a set of genes comprising at least 2, especially has or consist of 2 to 16 genes, preferably a set of 16 genes. In a particular embodiment, the method focuses on hepatoblastoma (HB) or hepatocellular carcinoma (HCC), in adults or in children. The invention is also directed to sets of primers, sets of probes, compositions, kits or arrays, comprising primers or probes specific for a set of genes comprising at least 2 genes, especially has or consists of 2 to 16 genes, preferably exactly 16 genes. Said sets, kits and arrays are tools suitable to determine the grade of a liver tumor in a patient.

The liver is a common site of metastases from a variety of organs such as lung, breast, colon and rectum. However, liver is also a site of different kinds of cancerous tumors that start in the liver (primary liver cancers). The most frequent is the Hepatocellular Carcinoma (HCC) (about 3 out of 4 primary liver cancers are this type) and is mainly diagnosed in adults. In the United States approximately 10,000 new patients are diagnosed with hepatocellular carcinoma each year. Less frequent liver tumours are cholangiocarcinoma (CC) in adults and hepatoblastoma (HB) in children.

The prognosis and treatment options associated with these different kinds of cancers is difficult to predict, and is dependent in particular on the stage of the cancer (such as the size of the tumor, whether it affects part or all of the liver, has spread to other places in the body or its aggressiveness). Therefore, it is important for clinicians and physicians to establish a classification of primary liver cancers (HCC or HB) to propose the most appropriate treatment and adopt the most appropriate surgery strategy. Some factors are currently used (degree of local invasion, histological types of cancer with specific grading, tumour markers and general status of the patient) but have been found to not be accurate and sufficient enough to ensure a correct classification.

As far as the HB is concerned, the PRETEXT (pre-treatment extent of disease) system designed by the International Childhood Liver Tumor Strategy Group (SIOPEL) is a non invasive technique commonly used by clinicians, to assess the extent of liver cancer, to determine the time of surgery and to adapt the treatment protocol. This system is based on the division of the liver in four parts and the determination of the number of liver sections that are free of tumor (Aronson et al. 2005; Journal of Clinical Oncology; 23(6): 1245-1252). A revised staging system taking into account other criteria, such as caudate lobe involvement, extrahepatic abdominal disease, tumor focality, tumor rupture or intraperitoneal haemorrhage, distant metastases, lymph node metastases, portal vein involvement and involvement of the IVC (inferior vena cava) and/or hepatic veins, has been recently proposed (Roebuck; 2007; Pediatr Radiol; 37: 123-132). However, the PRETEXT system, even if reproducible and providing good prognostic value, is based on imaging and clinical symptoms, making this system dependent upon the technicians and clinicians. There is thus a need for a system, complementary to the PRETEXT system, based on genetic and molecular features of the liver tumors.

The present invention concerns a method or process of profiling gene expression for a set of genes, in a sample previously obtained from a patient diagnosed for a liver tumor. In a particular embodiment said method is designed to determine the grade of a liver tumor in a patient.

By "*liver tumor*" or "*hepatic tumor*", it is meant a tumor originating from the liver of a patient, which is a malignant tumor (comprising cancerous cells), as opposed to a benign tumor (non cancerous) which is explicitly excluded. Malignant liver tumors encompass two main kinds of tumors: hepatoblastoma (HB) or hepatocellular carcinoma (HCC). These two tumor types can be assayed for the presently reported molecular signature. However, the present method may also be used to assay malignant liver tumors which are classified as unspecified (non-HB, non-HCC).

The present method may be used to determine the grade of a liver tumor or several liver tumors of the same patient, depending on the extent of the liver cancer. For convenience, the expression "*a liver tumor*" will be used throughout the specification to possibly apply to *"one or several liver tumor(s)*". The term "*neoplasm*" may also be used as a synonymous of "*tumor*".

In a particular embodiment, the tumor whose grade has to be determined is located in the liver. The presence of the tumor(s) in the liver may be diagnosed by ultrasound scan, x-rays, blood test, CT scans (computerised tomography) and/or MRI scans (magnetic resonance imaging).

In a particular embodiment, the tumor, although originating from the liver, has extended to other tissues or has given rise to metastasis.

In a particular embodiment, the patient is a child *i*.*e*., a human host who is under 20 years of age according to the present application. Therefore, in a particular embodiment, the liver tumor is a paediatric HB or a paediatric HCC. In another embodiment, the liver tumor is an adult HCC.

A grade is defined as a subclass of the liver tumor, corresponding to prognostic factors, such as tumor status, liver function and general health status. The present method of the invention allows or at least contributes to differentiating liver tumors having a good prognosis from tumors with a bad prognosis, in terms of evolution of the patient's disease. A good prognosis tumor is defined as a tumor with good survival probability for the patient (more than 80% survival at two years for HB and more than50% survival at two years for HCC), low probability of metastases and good response to treatment for the patient. In contrast, a bad prognosis tumor is defined as a tumor with an advanced stage, such as one having vascular invasion or/and extrahepatic metastasis, and associated with a low survival probability for the patient (less than 50% survival et two years).

The method of the invention is carried out on a sample isolated from the patient who has previously been diagnosed for the tumor(s) and who, optionally, may have been treated by surgery. In a preferred embodiment, the sample is the liver tumor or of one of the liver tumors identified by diagnosis imaging and obtained by surgery or a biopsy of this tumor. The tumor located in the liver tumor is called the primary tumor.

In another embodiment, the sample is not the liver tumor, but is representative of this tumor. By "*representative*", it is meant that the sample is regarded as having the same features as the primary tumors, when considering the gene expression profile assayed in the present invention. Therefore, the sample may also consist of metastatic cells (secondary tumors spread into different part(s) of the body) or of a biological fluid containing cancerous cells (such as blood).

One advantage of the method of the present invention is that, despite the possible heterogeneity of some liver tumors (comprising epithelial tumor cells at different stages of liver differentiation within the same tumor), the assay has proved to be reproducible and efficient on liver tumor biopsies obtained from any part of the whole tumor. Therefore, there is no requirement for the isolation of cells presenting particular features except from the fact that they are obtained from a liver tumor or are representative thereof, to carry out the gene expression profile assay.

In a particular embodiment, the tumor originates from a patient having a Caucasian origin, in particular European, North American, Australian, New-Zealander or Afrikaners.

In a first step, the method or process of the invention comprises assaying the expression level of a set of genes in a sample, in order to get an expression profile thereof.

By "*expression of a set of genes*" (or *"gene expression*"), it is meant assaying, in particular detecting, the product or several products resulting from the expression of a gene, this product being in the form of a nucleic acid, especially RNA, mRNA, cDNA, polypeptide, protein or any other formats. In a particular embodiment, the assay of the gene expression profile comprises detecting a set of nucleotide targets, each nucleotide target corresponding to the expression product of a gene encompassed in the set.

The expression "*nucleotide target*" means a nucleic acid molecule whose expression must be measured, preferably quantitatively measured. By "*expression measured*", it is meant that the expression product(s), in particular the transcription product(s) of a gene, are measured. By "*quantitative*" it is meant that the method is used to determine the quantity or the number of copies of the expression products, in particular the transcription products or nucleotide targets, originally present in the sample. This must be opposed to the qualitative measurement, whose aim is to determine the presence or absence of said expression product(s) only.

A nucleotide target is in particular a RNA, and most particularly a total RNA. In a preferred embodiment, the nucleotide target is mRNA or transcripts. According to the methods used to measure the gene expression level, the mRNA initially present in the sample may be used to obtain cDNA or cRNA, which is then detected and possibly measured.

In an embodiment, the expression of the gene is assayed directly on the sample, in particular in the tumor. In an alternative embodiment, the expression products or the nucleotide targets are prepared from the sample, in particular are isolated or even purified. When the nucleotide targets are mRNA, a further step comprising or consisting in the retro-transcription of said mRNA into cDNA (complementary DNA) may also be performed prior to the step of detecting expression. Optionally, the cDNA may also be transcribed *in vitro* to provide cRNA.

During the step of preparation, and before assaying the expression, the expression product(s) or the nucleotide target(s) may be labelled, with isotopic (such as radioactive) or non isotopic (such as fluorescent, coloured, luminescent, affinity, enzymatic, magnetic, thermal or electrical) markers or labels.

It is noteworthy that steps carried out for assaying the gene expression must not alter the qualitative or the quantitative expression (number of copies) of the expression product(s) or of the nucleotide target(s), or must not interfere with the subsequent step comprising assaying the qualitative or the quantitative expression of said expression product(s) or nucleotide target(s).

The step of profiling gene expression comprises determining the expression of a set of genes. Such a set is defined as a group of genes that must be assayed for one test, and especially performed at the same time, on the same patient's sample. A set comprises at least 2 and has especially from 2 to 16 genes, said 2 to 16 genes being chosen from the 16 following genes: alpha-fetoprotein (AFP), aldehyde dehydrogenase 2 (ALDH2), amyloid P component serum (APCS), apolipoprotein C-IV (APOC4), aquaporin 9 (AQP9), budding uninhibited by benzimidazoles 1 (BUB1), complement componant 1 (C1S), cytochrome p450 2E1 (CYP2E1), discs large homolog 7 (DLG7), dual specificity phosphatase 9 (DUSP9), E2F5 transcription factor (E2F5), growth hormone receptor (GHR), 4-hydroxyphenylpyruvase dioxygenase (HPD), immunoglogulin superfamily member 1 (IGSF1), Notchless homolog 1 (NLE1) and the ribosomal protein L10a (RPL10A) genes.

A complete description of these 16 genes is given in Table 1. This table lists, from left to right, the symbol of the gene, the complete name of the gene, the number of the SEQ ID provided in the sequence listing, the Accession Number from the NCBI database on June 2008, the human chromosomal location and the reported function (when known).

A set of genes comprises at least 2 out the 16 genes of Table 1, and particularly at least or exactly 2, 3, 4, 5, 6, 7, 8, 9, 10, 11, 12, 13, 14 or 15 out of the 16 genes of Table 1. In a particular embodiment, the set comprises or consists of the 16 genes of Table 1 *i*.*e*., the set of genes comprises or consists of AFP, ALDH2, APCS, APOC4, AQP9, BUB1, C1 S, CYP2E1, DLG7, DUSP9, E2F5, GHR, HPD, IGSF1, NLE1 and RPL10A genes.

In other particular embodiments, the set of genes comprises or consists of one of the following sets: (a) the E2F5 and HPD genes, (b) the APCS, BUB1, E2F5, GHR and HPD genes, (c) the ALDH2, APCS, APOC4, BUB1, C1S, CYP2E1, E2F5, GHR and HPD genes, (d) the ALDH2, APCS, APOC4, AQP9, BUB1, C1S, DUSP9, E2F5 and RPL10A genes, or (e) the ALDH2, APCS, APOC4, AQP9, C1S, CYP2E1, E2F5, GHR, IGSF1 and RPL10A genes.

As indicated by the expression "*comprises from 2 to 16 genes of Table 1*", the set may, besides the specific genes of Table 1, contain additional genes not listed in Table 1. This means that the set must comprises from 2 to 16 genes of Table 1, *i*.*e*. 2 to 16 genes of Table 1 (in particular 2, 3, 4, 5, 6, 7, 8, 9, 10, 11, 12, 13, 14, 15, or 16 genes), and optionally comprises one or more additional genes.

Additional genes may be selected for the difference of expression observed between the various grades of liver cancer, in particular between a tumor of good prognosis and a tumor of poor prognosis.

**Table 1**

| **symbol** | **Gene name** | **mRNA SEQ ID** | **Accession No** | **Location** | **Function** | **Protein SEQ ID** |
|---|---|---|---|---|---|---|
| **AFP** | alpha-fetoprotein | 1 | NM_001134 | 4q11-q13 | plasma protein synthesized by the fetal liver | 2 |
| **ALDH2** | aldehyde dehydrogenase 2 family (mitochondrial) | 3 | NM_000690 | 12q24.2 | liver enzyme involved in alcohol metabolism | 4 |
| **APCS** | amyloid P component, serum | 5 | NM_001639 | 1q21-q23 | secreted glycoprotein | 6 |
| **APOC4** | apolipoprotein C-IV | 7 | NM_001646 | 19q13.2 | secreted liver protein | 8 |
| **AQP9** | aquaporin 9 | 9 | NM_020980 | 15q22.1-22.2 | water-selective membrane channel | 10 |
| **BUB1** | BUB1 budding uninhibited by benzimidazoles 1 homolog (yeast) | 11 | AF043294 | 2q14 | kinase involved in spindle checkpoint | 12 |
| **C1S** | complement component 1, s subcomponent | 13 | M18767 | 12p13 | component of the cleavage and polyadenylation specificity factor complex | 14 |
| **CYP2E1** | cytochrome P450, family 2, subfamily E, polypeptide 1 | 15 | AF182276 | 10q24.3-qter | cytochrome P450 family member involved in drug metabolism | 16 |
| **DLG7** | discs, large homolog7 (Drosophila) (DLGAP5) | 17 | NM_014750 | 14q22.3 | cell cycle regulator involved in kinetocore formation | 18 |
| **DUSP9** | dual specificity phosphatase 9 | 19 | NM_001395 | Xq28 | phosphatase involved in regulation of MAP Kinases | 20 |
| **E2F5** | E2F transcription factor 5, p130-binding | 21 | U15642 | 8q21.2 | transcription factor involved in cell cycle regulation | 22 |
| **GHR** | Growth hormone receptor | 23 | NM_000163 | 5p13-p12 | transmembrane receptor for growth hormone | 24 |
| **HPD** | 4-hydroxyphenylpyruvate dioxygenase | 25 | NM_002150 | 12q24-qter | enzyme involved in amino-acid degradation | 26 |
| **IGSF1** | immunoglobulin superfamily, member 1 | 27 | NM_001555 | Xq25 | cell recognition and regulation of cell behavior | 28 |
| **NLE1** | notchless homolog 1 (Drosophila) | 29 | NM_018096 | 17q12 | unknown | 30 |
| **RPL10A** | ribosomal protein L10a | 31 | NM_007104 | 6p21.3-p21.2 | ribosomal protein of 60S subunit | 32 |

. The invention also relates to a set of genes comprising or consisting of the 16 genes of Table 1 (i.e., AFP, ALDH2, APCS, APOC4, AQP9, BUB1, C1S, CYP2E1, DLG7, DUSP9, E2F5, GHR, HPD, IGSF1, NLE1 and RPL10A genes), in which 1, 2, 3, 4 or 5 genes out of the 16 genes are substituted by a gene presenting the same features in terms of difference of expression between a tumor of a good prognosis and a tumor of poor prognosis.

In a particular embodiment, the number of genes of the set does not exceed 100, particularly 50, 30, 20, more particularly 16 and even more particularly is maximum 5, 6, 7, 8, 9 or 10.

When considering adding or substituting a gene or several genes to the disclosed set, the person skilled in the art will consider one or several of the following features:
(a) the added gene(s) and/or the substituted gene(s) of Table 1 must present the same features in terms of difference of expression between a tumor of a good prognosis and a tumor of poor prognosis as the genes of Table 1 when taken as a whole. Thus, the expression of the added gene or of the substituted gene in a tumor of a good prognosis is either overexpressed or underexpressed of a factor of at least 2, preferably of at least 5, and more preferably of at least 10, as compared to its expression in a tumor of poor prognosis.
(b) besides presenting the feature in a), the added gene and/or the substituted gene may also provide, in combination with the other genes of the set, discriminant results with respect to the grade of the liver tumors; this discrimination is reflected by the homogeneity of expression profile of this gene in the tumors of a good prognosis on the one hand, and the tumors of poor prognosis in the other hand; and
(c) finally, besides features of a) and/or b), the added gene and/or the substituted gene is optionally chosen among genes that are involved in liver differentiation, in particular having a specific expression in fetal liver, or genes that are involved in proliferation, for example in mitosis or associated with ribosomes.

Examples of genes which can be added or may replace genes of the set may be identified in following Table 2.

**Table2: list of genes according to p value. The Accession Numbers of the genes of Table 2, as found in NCBI database in June 2008, are the following: IPO4 (BC136759), CPSF1 (NM_013291), MCM4 (NM_005914.2; NM_182746.1; two accession numbers for the same gene correspond to 2 different isoforms of the gene), EIF3S3 (NM_003756.2), NCL (NM_005381.2), CDC25C (NM_001790.3), CENPA (NM_001809.3; NM_001042426.1), KIF14 (BC113742), IPW (U12897), KNTC2 (AK313184), TMEM48 (NM_018087), BOP1 (NM_015201), EIF3S9 (NM_003751; NM_001037283), PH-4 (NM_177939), SMC4L1 (NM_005496; NM_001002800), TTK (AK315696), LAMA3 (NM_198129), C10orf72 (NM_001031746; NM_144984), TPX2 (NM_012112), MSH2 (NM_000251), DKC1 (NM_001363), STK6 (AY892410), CCT6A (NM_001762; NM_001009186), SULT1C1 (AK313193), ILF3 (NM_012218; NM_004516), IMPDH2 (NM_000884), HIC2 (NM_015094), AFM (NM_001133), MCM7 (NM_005916; NM_182776), CNAP1(AK128354), CBARA1 (AK225695), PLA2G4C (NM_003706), CPSF1 (NM_013291), SNRPN (BC000611), RPL5 (AK314720), C1R (NM_001733), C16orf34 (CH471112), PHB (AK312649), BZW2 (BC017794), ALAS1(AK312566), FLJ20364 (NM_017785), RANBP1 (NM_002882), SKB1 (AF015913), ABHD6 (NM_020676), CCNB1 (NM_031966), NOL5A (NM_006392), RPL8 (NM_000973; NM_033301), BLNK (NM_013314; NM_001114094), BYSL (NM_004053), UBE1L(AY889910), CHD7 (NM_017780), DKFZp762E1312 (mM_018410), NUP210(NM_024923), PLK1(NM_005030), ENPEP(NM_001977), HCAP-G(NM_022346), UGT2B4 (NM_021139), C20orf27 (NM_001039140) and C6orf149 (NM_020408).**

| **Gene symbol** | **mean rC1** | **mean rC2** | **ratio rC2/rC1** | **Parametric p-value** | **FDR** | **Description** |
|---|---|---|---|---|---|---|
| IPO4 | 123,7 | 248,3 | 2,0 | 2.00E-07 | 0,00036 | importin 4 |
| CPSF1 | 467,8 | 1010,7 | 2,2 | 2,00E-07 | 0,00036 | cleavage and polyadenylation specific factor 1, 160kDa specific |
| MCM4 | 25,8 | 90,7 | 3,5 | 1,10E-06 | 0,00115 | MCM4 minichromosome maintenance deficient 4 (S. cerevisiae) |
| EIF3S3 | 1319 | 2601,2 | 2,0 | 1,20E-06 | 0,00119 | eukaryotic translation initiation factor 3, subunit 3 gamma, 40kDa |
| NCL | 1319 | 2655,6 | 2,0 | 1,30E-06 | 0,00122 | nucleolin |
| CDC25C | 35,7 | 99,3 | 2,8 | 1,40E-06 | 0,00124 | cell division cycle 25C |
| CENPA | 28,2 | 78,4 | 2,8 | 1,50E-06 | 0,00124 | centromere protein A, 17kDa |
| KIF14 | 24,7 | 54,2 | 2,2 | 1,50E-06 | 0,00124 | kinesin family member 14 |
| IPW | 145,7 | 397,6 | 2,7 | 1,90E-06 | 0,0015 | imprinted in Prader-Willi syndrome |
| KNTC2 | 26,8 | 65,1 | 2,4 | 2,20E-06 | 0,00157 | kinetochore associated 2 |
| TMEM48 | 26,4 | 71,7 | 2,7 | 2,30E-06 | 0,00157 | transmembrane protein 48 |
| BOP1 | 87,2 | 270,9 | 3,1 | 2,30E-06 | 0,00157 | block of proliferation 1 |
| EIF3S9 | 170 | 372,4 | 2,2 | 2,30E-06 | 0,00157 | eukaryotic translation initiation factor 3, subunit 9 eta, 116kDa |
| PH-4 | 340,9 | 168,2 | 0,5 | 2,40E-06 | 0,00158 | hypoxia-inducible factor prolyl 4-hydroxylase |
| SMC4L1 | 151,5 | 359,3 | 2,4 | 2,50E-06 | 0,0016 | SMC4 structural maintenance of chromosomes 4-like 1 (yeast) |
| TTK | 23,7 | 74,2 | 3,1 | 2,60E-06 | 0,00161 | TTK protein kinase |
| LAMA3 | 696 | 136,3 | 0,2 | 2,80E-06 | 0,00168 | laminin, alpha 3 |
| C10orf72 | 192,6 | 67,7 | 0,4 | 2,90E-06 | 0,00169 | Chromosome 10 open reading frame 72 |
| TPX2 | 73,4 | 401,5 | 5,5 | 3,10E-06 | 0,00171 | TPX2, microtubule-associated, homolog (Xenopus laevis) |
| MSH2 | 75,5 | 212.1 | 2.8 | 3.20E-06 | 0,00171 | mutS homolog 2, colon cancer, nonpolyposis type 1 (E. coli) |
| DKC1 | 358,1 | 833,5 | 2,3 | 3,20E-06 | 0,00171 | dyskeratosis congenita 1, dyskerin |
| STK6 | 86,4 | 395,3 | 4,6 | 3,30E-06 | 0,00172 | serine/threonine kinase 6 |
| CCT6A | 200,5 | 526,6 | 2,6 | 3,50E-06 | 0,00173 | chaperonin containing TCP1, subunit 6A (zeta 1) |
| SULT1 C1 | 67,5 | 314,8 | 4,7 | 3,50E-06 | 0,00173 | sulfotransferase family, cytosolic, 1C, member 1 |
| ILF3 | 142,3 | 294,5 | 2,1 | 3,70E-06 | 0,00174 | interleukin enhancer binding factor 3, 90kDa |
| IMPDH2 | 916,9 | 2385,6 | 2,6 | 3,70E-O6 | 0,00174 | IMP (inosine monophosphate) dehydrogenase 2 |
| HIC2 | 63,4 | 208,8 | 3,3 | 3,90E-06 | 0,00179 | hypermethylated in cancer 2 |
| AFM | 1310,3 | 237,4 | 0,2 | 4,10E-06 | 0,00184 | afamin |
| MCM7 | 187,3 | 465,3 | 2,5 | 4,30E-06 | 0,00189 | MCM7 minichromosome maintenance deficient 7 (S. cerevisiae) |
| CNAP1 | 70,2 | 177,5 | 2,5 | 4,40E-06 | 0,00189 | chromosome condensation-related SMC-associated protein 1 |
| CBARA1 | 958 | 475 | 0,5 | 4,60E-06 | 0,00194 | calcium binding atopy-related autoantigen 1 |
| PLA2G4C | 123.3 | 51,2 | 0,4 | 4,90E-06 | 0,00194 | phospholipase A2, group IVC (cytosolic, calcium-independent) |
| CPSF1 | 301,9 | 616 | 2,0 | 5,00E-06 | 0.00194 | cleavage and polyadenylation specific factor 1, 160kDa |
| SNRPN | 30,9 | 100,6 | 3,3 | 5,00E-06 | 0,00194 | Small nuclear ribonucleoprotein polypeptide N |
| RPL5 | 2754,8 | 4961 | 1,8 | 5,20E-06 | 0,00194 | ribosomal protein L5 |
| C1 R | 1446,5 | 366,4 | 0,3 | 5,30E-06 | 0,00194 | complement component 1, r subcomponent |
| C16₀rf34 | 630,4 | 1109,6 | 1,8 | 5,30E-06 | 0,00194 | chromosome 16 open reading frame 34 |
| PHB | 309,3 | 915,1 | 3,0 | 5,30E-06 | 0,00194 | prohibitin |
| BZW2 | 387,4 | 946,4 | 2,4 | 5,40E-06 | 0,00194 | basic leucine zipper and W2 domains 2 |
| ALAS1 | 1075,8 | 466,5 | 0,4 | 5,50E-06 | 0,00194 | aminolevulinate, delta-, synthase 1 |
| FLJ20364 | 48,6 | 112,4 | 2,3 | 5,70E-06 | 0,00198 | hypothetical protein FLJ20364 |
| RANBP1 | 593,7 | 1168,1 | 2,0 | 5,90E-06 | 0,00201 | RAN binding protein 1 |
| SKB1 | 354,7 | 687,4 | 1,9 | 6,20E-06 | 0,00208 | SKB1 homolog (S. pombe) |
| ABHD6 | 402,2 | 196,9 | 0,5 | 6,50E-06 | 0,00213 | abhydrolase domain containing 6 |
| CCNB1 | 60,4 | 330 | 5,5 | 6,60E-06 | 0,00213 | cyclin B1 |
| NOLSA | 246,9 | 716,2 | 2,9 | 7,00E-06 | 0,00213 | nucleolar protein 5A (56kDa with KKE/D repeat) |
| RPL8 | 3805,7 | 7390,5 | 1,9 | 7,OOE-06 | 0,00213 | ribosomal protein L8 |
| BLNK | 211,1 | 39,8 | 0,2 | 7,10E-O6 | 0,00213 | B-celllinker |
| BYSL | | 269,7 | 1,6 | 7,10E-06 | 0,00213 | bystin-like |
| UBE1L | 247,6 | 142,3 | 0,6 | 7,20E-06 | 0,00213 3 | ubiquitin-activating enzyme E1-like |
| CHD7 | 118,6 | 312 | 2,6 | 7,40E-06 | 0,00215 | chromodomain helicase DNA binding protein 7 |
| DKFZp762E1 312 | 70,2 | 219,4 | 3,1 | 7,60E-06 | 0,00218 | hypothetical protein DKFZp762E1312 (HJURP) |
| NUP210 | 178,4 | 284,9 | 1,6 | 7,70E-06 | 0,00218 | nucleoporin 210kDa |
| PLK1 | 72,8 | 185,2 | 2,5 | 7,90E-06 | 0,0022 | polo-like kinase 1 (Drosophila) |
| ENPEP | 116,2 | 29,4 | 0,3 | 8,00E-06 | 0,0022 | glutamyl aminopeptidase (aminopeptidase A) |
| HCAP-G | 17,7 | 57,8 | 3,3 | 8,40E-06 | 0,00228 | chromosome condensation protein G |
| UGT2B4 | 1117,8 | 246,7 | 0,2 | 9,20E-06 | 0,00245 | UDP glucuronosyltransferase 2 family, polypeptide B4 |
| C20orf27 | 129,7 | 245,3 | 1,9 | 9,30E-06 | 0,00245 | chromosome 20 open reading frame 27 |
| C6orf149 | 178,7 | 491,1 | 2,7 | 9,40E-06 | 0,00245 | chromosome 6 open reading frame 149 (LYRM4) |

In a particular embodiment of the invention, the set of genes of the invention is designed to determine the grade of hepatoblastoma, in particular paediatric hepatoblastoma. In another embodiment, the set of genes is designed to determine the grade of hepatocellular carcinoma, in particular paediatric HCC or adult HCC.

The expression of the genes of the set may be assayed by any conventional methods, in particular any conventional methods known to measure the quantitative expression of RNA, preferably mRNA.

The expression may be measured after carrying out an amplification process, such as by PCR, quantitative PCR (qPCR) or real-time PCR. Kits designed for measuring expression after an amplification step are disclosed below.

The expression may be measured using hybridization method, especially with a step of hybridizing on a solid support, especially an array, a macroarray or a microarray or in other conditions especially in solution. Arrays and kits of the invention, designed for measuring expression by hybridization method are disclosed below.

The expression of a gene may be assayed in two manners:
- to determine absolute gene expression that corresponds to the number of copies of the product of expression of a gene, in particular the number of copies of a nucleotide target, in the sample; and
- to determine the relative expression that corresponds to the number of copies of the product of expression of a gene, in particular the number of copies of a nucleotide target, in the sample over the number of copies of the expression product or the number of copies of a nucleotide target of a different gene (calculation also known as normalisation). This different gene is not one of the genes contained in the set to be assayed. This different gene is assayed on the same sample and at the same time as the genes of the set to be assayed, and is called an invariant gene or a normalizer. The invariant gene is generally selected for the fact that its expression is steady whatever the sample to be tested. The expression "*steady whatever the sample*" means that the expression of an invariant gene does not vary significantly between a normal liver cell and the corresponding tumor cell in a same patient and/or between different liver tumor samples in a same patient. In the present specification, a gene is defined as invariant when its absolute expression does not vary in function of the grade of the liver tumors, in particular does not vary in function of the grade of the HB or HCC tumor, and/or does not vary between liver tumor and normal liver cells.

In the present invention, the expression which is assayed is preferably the relative expression of each gene, calculated with reference to at least one (preferably 1, 2, 3 or 4) invariant gene(s). Invariant genes, suitable to perform the invention, are genes whose expression is constant whatever the grade of the liver tumors, such as for example ACTG1, EFF1A1, PNN and RHOT2 genes, whose features are summarized in Table 3. In a particular embodiment, the relative expression is calculated with respect to at least the RHOT2 gene or with respect to the RHOT2 gene. The calculation of the absolute expression or of the relative expression of each gene of the set and of each invariant gene being assayed with the same method from the same sample, preferably at the same time, enables to determine for each sample a gene expression profile.

**Table 3: Features of invariant genes. ACTG1, EEF1A1, PNN and RHOT2 proteins are defined in SEQ ID NOs: 34, 36, 38 and 40 respectively.**

| **symbol** | **Gene name** | **SEQ ID*** | **Accession No** | **Location** | **Function** |
|---|---|---|---|---|---|
| **ACTG1** | actin, gamma 1 | 33 | NM_001614 | 17q25 | cytoplasmic actin cytoskeleton in nonmuscle cells |
| **EEF1A1** | eukaryotic translation elongation factor 1 alpha 1 | 35 | NM_001402 | 6q14.1 | enzymatic delivery of aminoacyl tRNAs to the ribosome |
| **PNN** | pinin, desmosome associated protein | 37 | NM_002687 | 14q21.1 | transcriptional corepressor, RNA splicing regulator |
| **RHOT2** | ras homolog gene family, member T2 | 39 | NM_1 38769 | 16p13.3 | Signaling by Rho mitochondrial protein |

An additional step of the method or process comprises the determination of the grade of said liver tumor, referring to the gene expression profile that has been assayed. In a particular embodiment of the invention, the method is designed to determine the grade of hepatoblastoma, in particular paediatric hepatoblastoma. In another embodiment, the method is designed to determine the grade of hepatocellular carcinoma, in particular paediatric HCC or adult HCC.

In this step of the method, a gene expression profile or a signature (preferably obtained after normalization), which is thus specific for each sample, is compared to the gene expression profile of a reference sample or to the gene expression profiles of each sample of a collection of reference samples (individually tested) whose grade is known, so as to determine the grade of said liver tumor. This comparison step is carried out with at least one prediction algorithm. In a particular embodiment, the comparison step is carried out with 1, 2, 3, 4, 5 or 6 prediction algorithms chosen in the following prediction algorithms: Compound Covariate Predictor (CCP), Linear Discriminator Analysis (LDA), One Nearest Neighbor (1 NN), Three Nearest Neighbor (3NN), Nearest Centroid (NC) and Support Vector Machine (SVM). These six algorithms are part of the "Biometric Research Branch (BRB) Tools" developed by the National Cancer Institut (NCI) and are available on http://linus.nci.nih.gov/BRB-ArrayTools.html. Equivalent algorithms may be used instead of or in addition to the above ones. Each algorithm classifies tumors within either of the two groups, defined as tumors with good prognosis (such as C1) or tumors with bad prognosis (such as C2); each group comprises the respective reference samples used for comparison, and one of these two groups also comprises the tumor to be classified.

Therefore, when 6 algorithms are used, the grade of a tumor sample may be assigned with certainty to the class of good prognosis or to the class of bad prognosis, when 5 or 6 of the above algorithms classified the tumor sample in the same group. In contrast, when less than 5 of the above algorithms classifie a tumor sample in the same group, it provides an indication of the grade rather than a definite classification.

Reference samples which can be used for comparison with the gene expression profile of a tumor to be tested are one or several sample(s) representative for tumor with poor prognosis (such as C2), one or several sample(s) representative of tumor with good prognosis (such as C1), one or several sample(s) of a normal adult liver and/or one or several sample(s) of a fetal liver.

Table 4 lists the level of expression of each gene of Table 1 depending upon the status of the reference sample *i*.*e*., robust tumor with poor prognostic and robust tumor with good prognostic. An example of method to identify such robust tumors is provided in the examples, and in particular in paragraphs [140] to [143].

**Table 4: Level of expression of the genes of Table 1, with respect to the status of the robust tumors**

| **Nucleotide target** | **Expression status in robust tumor** | |
|---|---|---|
| | **with poor prognosis** | **with good prognosis** |
| **AFP** | overexpressed | underexpressed |
| **ALDH2** | underexpressed | overexpressed |
| **APCS** | underexpressed | overexpressed |
| **APOC4** | underexpressed | overexpressed |
| **AQP9** | underexpressed | overexpressed |
| **BUB1** | overexpressed | underexpressed |
| **C1S** | underexpressed | overexpressed |
| **CYP2E1** | underexpressed | overexpressed |
| **DLG7** | overexpressed | underexpressed |
| **DUSP9** | overexpressed | underexpressed |
| **E2F5** | overexpressed | underexpressed |
| **GHR** | underexpressed | overexpressed |
| **HPD** | underexpressed | overexpressed |
| **IGSF1** | overexpressed | underexpressed |
| **NLE1** | overexpressed | underexpressed |
| **RPL10A** | overexpressed | underexpressed |

Reference samples usually correspond to so-called "*robust tumor*" for which all the marker genes providing the signature are expressed (either under expressed or overexpressed) as expected *i*.*e*., in accordance with the results disclosed in Table 5, when tested in similar conditions, as disclosed in the examples hereafter.

A robust tumor having an overexpression of one or several gene(s) selected among ALDH2, APCS, APOC4, AQP9, C1S, CYP2E1, GHR and HPD genes, and/or an underexpression of one or several gene(s) selected among AFP, BUB1, DLG7, DUSP9, E2F5, IGSF1, NLE1 and RPL10A genes, is an indicator of a robust liver tumor, in particular of a hepatoblastoma, with a good prognosis. A robust tumor having an overexpression of one or several gene(s) selected among AFP, BUB1, DLG7, DUSP9, E2F5, IGSF1, NLE1 and RPL10A genes, and/or an underexpression of one or several gene(s) among ALDH2, APCS, APOC4, AQP9, C1 S, CYP2E1, GHR and HPD genes, is an indicator of a robust liver tumor, in particular of a hepatoblastoma, with a poor prognosis. In the present application, a gene is said "*underexpressed*" when its expression is lower than the expression of the same gene in the other tumor grade, and a gene is said "*overexpressed*" when its expression is higher than the expression of the same gene in the other tumor grade.

In a particular embodiment, Table 5 provides the gene expression profiles of the 16 genes of Table 1 in 13 samples of hepatoblastoma (HB) including 8 samples that have been previously identified as rC1 subtype and 5 samples that have been previously identified as rC2 subtype. This Table can therefore be used for comparison, to determine the gene expression profile of a HB tumor to be classified, with the robust tumors disclosed (constituting reference samples), for a set of genes as defined in the present application. Said comparison involves using the classification algorithms which are disclosed herein, for both the selected reference samples and the assayed sample.

**Table 5: Normalized qPCR data of 16 genes in 13 HB samples including 8 samples of the rC1 subtype and 5 samples of the rC2 subtype (in grey). The qpCR values have been obtained by measuring the expression of the 16 genes in 8 samples of the rC1 subtype and 5 samples of the rC2 subtype by the SYBR green method using the primers as disclosed in Table 6 below and in the conditions reported in the examples, and normalized by the ROTH2 gene (primers in Table 7).**

| **HB** | **grade** | **AFP** | **ALDH2** | **APCS** | **APOC4** | **AQP9** | **BUB1** | **C1S** | **CYP2E1** | **DLG7** | **DUSP9** | **E2F5** | **GHR** | **HPD** | **IGSF1** | **NLE** | **RPL10A** |
|---|---|---|---|---|---|---|---|---|---|---|---|---|---|---|---|---|---|
| HB5 | rC1 | 0.22 | 1.32 | 1.04 | 1.23 | 1.4 | -0.11 | 1.46 | 0.99 | -0.22 | 0.76 | 0.37 | 1.29 | 1.72 | 0.04 | -0.12 | 0.78 |
| HB49 | rC1 | -0.82 | 1.2 | 0.6 | 1.72 | 0.78 | -1.09 | 0.77 | 0.89 | -1.17 | -1.15 | -1.21 | 0.89 | 1.31 | -1.27 | -2.06 | -0.8 |
| HB59 | rC1 | -0.57 | 0.71 | 0.9 | 1.09 | 0.8 | 0.48 | 0.32 | 0.62 | 0.31 | -0.7 | -1.28 | 0.44 | 0.67 | -1.26 | -0.73 | 0.03 |
| HB69 | rC1 | -1.3 | 0.42 | 0.66 | 0.79 | 0.48 | -1.35 | 0.23 | 1.1 | -1.1 | -1.62 | -0.96 | 0.9 | 0.53 | -1.88 | -1.33 | -0.26 |
| HB72 | rC2 | 1.19 | -2.01 | -1.47 | -1.05 | -1.01 | 1.22 | -1.13 | -1.15 | 1.01 | 0.8 | 0.84 | -0.71 | -1.38 | 0.64 | 0.45 | 0.53 |
| HB73 | rC2 | 0.88 | 0.24 | -0.49 | -0.92 | -1.23 | 1.09 | -0.43 | -0.27 | 1.17 | 0.96 | 0.89 | -1.28 | -0.19 | 0.77 | 0.99 | 0.72 |
| HB74e | rC1 | 0.52 | 0.18 | -0.58 | -0.75 | -0.28 | -0.06 | -0.95 | -0.79 | 0.46 | 0.62 | 0.75 | -0.65 | -0.86 | 0.63 | 0.93 | 0.5 |
| HB74F | rC2 | -1.11 | 1.21 | 0.29 | 1.37 | 0.81 | -1.36 | 0.29 | 0.92 | -1.35 | -0.78 | -1.89 | 0.68 | 0.07 | -1.74 | -0.88 | -0.05 |
| HB75 | rC1 | -0.38 | 0.45 | -0.3 | 0.62 | 0.96 | -0.74 | 0.05 | 0.91 | -0.59 | 0.15 | 0.4 | 0.32 | 0.79 | -0.3 | -0.35 | -0.5 |
| HB80 | rC2 | 1.63 | -0.4 | -0.26 | 0.06 | -0.99 | 1.31 | -1.26 | -0.34 | 1.37 | 0.95 | 0.81 | -0.64 | -0.87 | 1.61 | 1.23 | 1.11 |
| HB81 | rC1 | -0.56 | 0.66 | 1.1 | 0.88 | 0.84 | -0.66 | 1.16 | 1.32 | -0.69 | -0.14 | -0.1 | 1.11 | 1.29 | 0.06 | 0.06 | 0.44 |
| HB83 | rC1 | -0.73 | 0.5 | 0.78 | 1.04 | 1.2 | -0.24 | 1.11 | -0.21 | -0.84 | -0.64 | -1.41 | 0.63 | 0.61 | -0.22 | -1.09 | -1.92 |
| HB86 | rC2 | 1.08 | -0.09 | -1.29 | -0.43 | -0.3 | 1.21 | -1.12 | -0.25 | 1.13 | 1.26 | 0.88 | -0.52 | -0.98 | 1.08 | 0.46 | 1.56 |

The method of the present invention is also suitable to classify new tumor samples, and to use them as new reference samples. Therefore, the gene expression values of these new reference samples may be used in combination or in place of some of the values reported in Table 5.

From the 16 genes expressed in liver cells listed in Table 1, a set comprising from 2 to 16 genes (or more generally a set as defined herein) may be used to assay the grade of tumor cells in a tumor originating from the liver. The results obtained, after determining the expression of each of the genes of the set, are then treated for classification according to the steps disclosed herein. The invention relates to each and any combination of genes disclosed in Table 1, to provide a set comprising from 2 to 16 of these genes, in particular a set comprising or consisting of 2, 3, 4, 5, 6, 7, 8, 9, 10, 11, 12, 13, 14, 15 or 16 of these genes. In the designed set, one or many genes of Table 1 may be modified by substitution or by addition of one or several genes as explained above, which also enable to determine the grade of the liver tumor, when assayed in combination with the other genes.

In a preferred embodiment, the liver tumor is a paediatric HB, and the method or process of the invention enables to distinguish a first class, called C1, qualifying as a good prognosis tumor and a second class, called C2, qualifying as a poor prognosis tumor. The C1 grade is predominantly composed of fetal histotype cells (*i*.*e*., well differentiated and non proliferative cells). In contrast, the C2 grade presents cells other than the fetal histotype such as embryonic, atypic (crowded fetal), small cell undifferiantiated (SCUD) and/or macrotrabecular cells.

The present invention also relates to a kit suitable to determine the grade of a liver tumor from the sample obtained from a patient. This kit is appropriate to carry out the method or process described in the present application.

In a particular embodiment, the kit comprises a plurality of pairs of primers specific for a set of genes to be assayed, said set comprising from 2 to 16 genes, said 2 to 16 genes being chosen in the group consisting of AFP, ALDH2, APCS, APOC4, AQP9, BUB1, C1S, CYP2E1, DLG7, DUSP9, E2F5, GHR, HPD, IGSF1, NLE1 and RPL10A genes.

By "*plurality*", it is mean that the kit comprises at least as many pairs of primers as genes to enable assaying each selected gene, and in particular the nucleotide target of this gene. Accordingly, each gene and in particular its nucleotide target is specifically targeted by a least one of these pairs of primers. In a particular embodiment, the kit comprises the same number of pairs of primers as the number of genes to assay and each primer pair specifically targets one of the genes, and in particular the nucleotide targets of one of these genes, and does not hybridize with the other genes of the set.

The kits of the invention are defined to amplify the nucleotide targets of the sets of genes as described in the present invention. Therefore, the kit of the invention comprises from 2 to 16 pairs of primers which, when taken as a whole, are specific for said from 2 to 16 genes out of the 16 genes of Table 1. In particular, the kit comprises or consists of 2, 3, 4, 5, 6, 7, 8, 9, 10, 11, 12, 13, 14 or 15 pairs of primers specific for 2, 3, 4, 5, 6, 7, 8, 9, 10, 11, 12, 13, 14 or 15 out of the 16 genes of Table 1. In a particular embodiment, the kit comprises or consists of 16 pairs of primers specific for the 16 genes of Table 1 *i.e.,* a primer pair specific for each of the following genes: AFP, ALDH2, APCS, APOC4, AQP9, BUB1, C1S, CYP2E1, DLG7, DUSP9, E2F5, GHR, HPD, IGSF1, NLE1 and RPL10A genes.

When the set of genes has been modified by the addition or substitution of at least one gene as described above, the kit is adapted to contain a pair of primers specific for each added or substituted gene(s). As indicated by the term "*comprises*", the kit may, besides the pairs of primers specific for the genes of Table 1, contain additional pair(s) of primers.

In a particular embodiment, the kit comprises at least one pair of primers (preferably one) for at least one invariant gene (preferably one or two) to be assayed for the determination of the expression profile of the genes, by comparison with the expression profile of the invariant gene.

The number of pairs of primers of the kit usually does not exceed 100, particularly 50, 30, 20, more particularly 16, and even more particularly is maximum 5, 6, 7, 8, 9 or 10.

In the kits of the invention, it is understood that, for each gene, at least one pair of primers and preferably exactly one pair, enabling to amplify the nucleotide targets of this gene, is present. When the kits provide several pairs of primers for the same gene, the gene expression level is measured by amplification with only one pair of primers. It is excluded that amplification may be performed using simultaneously several pairs of primers for the same gene.

As defined herein, a pair of primers consists of a forward polynucleotide and a backward polynucleotide, having the capacity to match its nucleotide target and to amplify, when appropriate conditions and reagents are brought, a nucleotide sequence framed by their complementary sequence, in the sequence of their nucleotide target.

The pairs of primers present in the kits of the invention are specific for a gene *i.e.,* each pair of primers amplifies the nucleotide targets of one and only one gene among the set. Therefore, it is excluded that a pair of primers specific for a gene amplifies, in a exponential or even in a linear way, the nucleotide targets of another gene and/or other nucleic acids contained in sample. In this way, the sequence of a primer (whose pair is specific for a gene) is selected to be not found in a sequence found in another gene, is not complementary to a sequence found in this another gene and/or is not able to hybridize in amplification conditions as defined in the present application with the sequence of the nucleotide targets of this another gene.

In a particular embodiment, the forward and/or backward primer(s) may be labelled, either by isotopic (such as radioactive) or non isotopic (such as fluorescent, biotin, flurorochrome) methods. The label of the primer(s) leads to the labelling of the amplicon (product of amplification), since the primers are incorporated in the final product.

The design of a pair of primers is well known in the art and in particular may be carried out by reference to Sambrook et al. (Molecular Cloning, A laboratory Manual, Third Edition; chapter 8 and in particular pages 8.13 to 8.16). Various softwares are available to design pairs of primers, such as Oligo™ or Primer3.

Therefore, each primer of the pair (forward and backward) has, independently from each other, the following features:
- their size is from 10 and 50 bp, preferably 15 to 30 bp; and
- they have the capacity to hybridize with the sequence of the nucleotide targets of a gene.

In a particular embodiment, when the pairs of primers are used in a simultaneous amplification reaction carried out on the sample, the various primers have the capacity to hybridize with their respective nucleotide targets at the same temperature and in the same conditions.

Conventional conditions for PCR amplification are well known in the art and in particular in Sambrook et al. An example of common conditions for amplification by PCR is dNTP (200 mM), MgCl₂ (0.5 - 3 mM) and primers (100-200 nM).

In a particular embodiment, the sequence of the primer is 100% identical to one of the strands of the sequence of the nucleotide target to which it must hybridize with, *i*.*e*. is 100% complementary to the sequence of the nucleotide target to which it must hybridize. In another embodiment, the identity or complementarity is not 100%, but the similarity is at least 80%, at least 85%, at least 90% or at least 95% with its complementary sequence in the nucleotide target. In a particular embodiment, the primer differs from its counterpart in the sequence of the sequence of the nucleotide target by 1, 2, 3, 4 or 5 mutation(s) (deletion, insertion and/or substitution), preferably by 1, 2, 3, 4 or 5 nucleotide substitutions. In a particular embodiment, the mutations are not located in the last 5 nucleotides of the 3' end of the primer.

In a particular embodiment, the primer, which is not 100% identical or complementary, keeps the capacity to hybridize with the sequence of the nucleotide target, similarly to the primer that is 100% identical or 100% complementary with the sequence of the nucleotide target (in the hybridization conditions defined herein). In order to be specific, at least one of the primers (having at least 80% similarity as defined above) of the pair specific for a gene can not hybridize with the sequence found in the nucleotide targets of another gene of the set and of another gene of the sample.

In a particular embodiment, the pairs of primers used for amplifying a particular set of genes are designed, besides some or all of the features explained herein, in order that the amplification products (or amplicons) of each gene have approximately the same size. By "*approximately*" is meant that the difference of size between the longest amplicon and the shortest amplicon of the set is less than 30% (of the size of the longest amplicon), preferably less than 20%, more preferably less than 10%. As particular embodiments, the size of the amplicon is between 100 and 300 bp, such as about 100, 150, 200, 250 or 300 bp.

The nucleotide sequences of the 16 genes of Table 1 are provided in the Figures, and may be used to design specific pairs of primers for amplification, in view of the explanations above.

Examples of primers that may be used to measure the expression of the genes of Table 1, in particular to amplify the nucleotide targets of the genes of Table 1, are the primers having the sequence provided in Table 6 or variant primers having at least 80% similarity (or more as defined above) with the sequences defined in Table 6.

**Table 6: Sequence of forward and backward primers of the 16 genes defined in Table 1. These primers may be used in any real-time PCR, in particular the SYBR green technique, except for the Taqman® protocol.**

| **Target** | **Product size (bp)** | **Forward primer (5'-3')** | **Reverse primer (5'-3')** |
|---|---|---|---|
| AFP | 151 | AACTATTGGCCTGTGGCGAG | TCATCCACCACCAAGCTGC |
| ALDH2 | 151 | GTTTGGAGCCCAGTCACCCT | GGGAGGAAGCTTGCATGATTC |
| APCS | 151 | GGCCAGGAATATGAACAAGCC | CTTCTCCAGCGGTGTGATCA |
| APOC4 | 151 | GGAGCTGCTGGAGACAGTGG | TTTGGATTCGAGGAACCAGG |
| AQP9 | 151 | GCTTCCTCCCTGGGACTGA | CAACCAAAGGGCCCACTACA |
| BUB1 | 152 | ACCCCTGAAAAAGTGATGCCT | TCATCCTGTTCCAAAAATCCG |
| C1S | 141 | TTGTTTGGTTCTGTCATCCGC | TGGAACACATTTCGGCAGC |
| CYP2E1 | 151 | CAACCAAGAATTTCCTGATCCAG | AAGAAACAACTCCATGCGAGC |
| DLG7 | 151 | GCAGGAAGAATGTGCTGAAACA | TCCAAGTCTTTGAGAAGGGCC |
| DUSP9 | 151 | CGGAGGCCATTGAGTTCATT | ACCAGGTCATAGGCATCGTTG |
| E2F5 | 151 | CCATTCAGGCACCTTCTGGT | ACGGGCTTAGATGAACTCGACT |
| GHR | 151 | CTTGGCACTGGCAGGATCA | AGGTGAACGGCACTTGGTG |
| HPD | 151 | ATCTTCACCAAACCGGTGCA | CCATGTTGGTGAGGTTACCCC |
| IGSF1 | 152 | CACTCACACTGAAAAACGCCC | GGGTGGAGCAATTGAAAGTCA |
| NLE1 | 151 | ATGTGAAGGCCCAGAAGCTG | GAGAACTTCGGGCCGTCTC |
| RPL10A | 151 | TATCCCCCACATGGACATCG | TGCCTTATTTAAACCTGGGCC |

The kit of the invention may further comprise one or many pairs of primers specific for one or many invariant genes, in particular specific for ACTG1, EFF1A1, PNN and/or RHOT2 genes. The pair of primers specific for invariant gene(s) may be designed and selected as explained above for the pair of primers specific for the genes of the set of the invention. In a particular embodiment, the pairs of primers of the invariant genes are designed in order that their amplification product (or amplicon) has approximately the same size as the amplicon of the genes of the set to be assayed (the term approximately being defined as above, with respect to the longest amplicon of the set of genes). Examples of primers that may be used to amplify the particular invariant genes are primers having the sequence provided in Table 7 or primers having at least 80% similarity (or more as defined above) with the sequences defined in Table 7.

**Table 7: Sequence of forward and backward primers specific for the invariant genes defined in Table 3. These primers may be used in real-time PCR, in particular the SYBR green technique, except for the Taqman® protocol.**

| **Target** | **Product size (bp)** | **Forward primer (5'-3')** | **Reverse primer (5'-3')** |
|---|---|---|---|
| ACTG1 | 151 | GATGGCCAGGTCATCACCAT | ACAGGTCTTTGCGGATGTCC |
| EFF1A1 | 151 | TCACCCGTAAGGATGGCAAT | CGGCCAACAGGAACAGTACC |
| PNN | 151 | CCTTTCTGGTCCTGGTGGAG | TGATTCTCTTCTGGTCCGACG |
| RHOT2 | 151 | CTGCGGACTATCTCTCCCCTC | AAAAGGCTTTGCAGCTCCAC |

The kits of the invention may also further comprise, in association with or independently of the pairs of primers specific for the invariant gene(s), reagents necessary for the amplification of the nucleotide targets of the sets of the invention and if any, of the nucleotide targets of the invariant genes.

The kits of the invention may also comprise probes as disclosed herein in the context of sets of probes, compositions and arrays. In particular, the kits also comprise the four dNTPs (nucleotides), amplification buffer, a polymerase (in particular a DNA polymerase, and more particularly a thermostable DNA polymerase) and/or salts necessary for the activity of the polymerase (such as Mg²⁺).

Finally, the kits may also comprise one or several control sample(s) *i*.*e*., at least one sample(s) representative of tumor with bad (*i*.*e*., poor) prognosis (in particular a HB C2 grade), at least one sample(s) representative of tumor with good prognosis (in particular a HB C1 grade), at least one sample of a normal adult liver and/or at least one sample of a fetal liver.

The kits may also comprise instructions to carry out the amplification step or the various steps of the method of the invention.

The invention is also directed to a set of probes suitable to determine the grade of a liver tumor from the sample obtained from a patient. This set of probes is appropriate to carry out the method or process described in the present invention. It may also be part of the kit.

This set of probes comprises a plurality of probes in particular from 2 to 16 probes, these 2 to 16 probes being specific for genes chosen in the group consisting of AFP, ALDH2, APCS, APOC4, AQP9, BUB1, C1S, CYP2E1, DLG7, DUSP9, E2F5, GHR, HPD, IGSF1, NLE1 and RPL10A genes.

By "*plurality*", it is mean that the set of probes comprises at least as many probes as genes to assay. In a particular embodiment, the array comprises the same number of probes as the number of genes to assay.

The probes of the sets of the invention are selected for their capacity to hybridize to the nucleotide targets of the sets of genes as described in the present invention. Therefore, the set of probes of the invention comprise from 2 to 16 probes specific for 2 to 16 genes out of the 16 genes of Table 1. In particular, the sets of probes comprise or consist of 2, 3, 4, 5, 6, 7, 8, 9, 10, 11, 12, 13, 14 or 15 probes specific of 2, 3, 4, 5, 6, 7, 8, 9, 10, 11, 12, 13, 14 or 15 out of the 16 genes of Table 1. In a particular embodiment, the sets of probes comprise or consist of 16 probes specific for the 16 genes of Table 1 *i*.*e*., a probe specific of each of the following genes: AFP, ALDH2, APCS, APOC4, AQP9, BUB1, C1S, CYP2E1, DLG7, DUSP9, E2F5, GHR, HPD, IGSF1, NLE1 and RPL10A genes.

The specificity of the probes is defined according to the same parameters as those applying to define specific primers.

When the set of genes has been modified by the addition or substitution of at least one gene as described above, the set of probes is adapted to contain a probe specific for the added or substituted gene(s). As indicated by the term "*comprises*", the set of probes may, besides the probes specific for the genes of Table 1, contain additional probe(s).

The number of probes of the set does usually not exceed 100, particularly 50, 30, 20, more particularly 16, and even more particularly is maximum 5, 6, 7, 8, 9 or 10.

In the set of probes of the invention, it is understood that for each gene corresponds at least one probe to which the nucleotide target of this gene hybridize to. The set of probes may comprise several probes for the same gene, either probes having the same sequence or probes having different sequences.

As defined herein, a probe is a polynucleotide, especially DNA, having the capacity to hybridize to the nucleotide target of a gene. Hybridization is usually carried out at a temperature ranging from 40 to 60°C in hybridization buffer (see example of buffers below). These probes may be oligonucleotides, PCR products or cDNA vectors or purified inserts. The size of each probe is independently to each other from 15 and 1000 bp, preferably 100 to 500 bp or 15 to 500 bp, more preferably 50 to 200bp or 15 to 100bp. The design of probes is well known in the art and in particular may be carried out by reference to Sambrook et al. (Molecular Cloning, A laboratory Manual, Third Edition; chapters 9 and 10 and in particular pages 10.1 to 10.10).

The probes may be optionally labelled, either by isotopic (radioactive) or non isotopic (biotin, flurorochrome) methods. Methods to label probes are disclosed in Sambrook et al. (Molecular Cloning, A laboratory Manual, Third Edition; chapter 8 and in particular page 9.3.). In a particular embodiment, the probes are modified to confer them different physicochemical properties (such as by methylation, ethylation). In another particular embodiment, the probes may be modified to add a functional group (such as a thiol group), and optionally immobilized on bead (preferably glass beads).

In a particular embodiment, the sequence of the probe is 100% identical to a part of one strand of the sequence of the nucleotide target to which it must hybridize, *i*.*e*. is 100% complementary to a part of the sequence of the nucleotide target to which it must hybridize. In another embodiment, the identity or complementarity is not 100% and the similarity is at least 80%, at least 85%, at least 90% or at least 95% with a part of the sequence of the nucleotide target. In a particular embodiment, the probe differs from a part of one strand of the sequence of the nucleotide target by 1 to 10 mutation(s) (deletion, insertion and/or substitution), preferably by 1 to 10 nucleotide substitutions. By "*a part of*", it is meant consecutive nucleotides of the nucleotide target, which correspond to the sequence of the probe.

In a particular embodiment, the probe, which is not 100% identical or complementary, keeps the capacity to hybridize, in particular to specifically hybridize, to the sequence of the nucleotide target, similarly to the probe which is 100% identical or 100% complementary with the sequence of the nucleotide target (in the hybridization conditions defined herein).

In a particular embodiment, the size of the probes used to assay a set of genes is approximately the same for all the probes. By "*approximately*" is meant that the difference of size between the longest probe and the shortest probe of the set is less than 30% (of the size of the longest probe), preferably less than 20%, more preferably less than 10%.

The set of probes of the invention may further comprise at least one (preferably one) probe specific for at least one invariant gene (preferably one or two), in particular specific for ACTG1, EFF1A1, PNN and/or RHOT2 genes. The probes specific for invariant gene(s) may be designed and selected as explained above for the probes specific for genes of the sets of the invention. In a particular embodiment, the probes specific of the invariant genes have approximately the same size as the probes specific of the genes of the set of be assayed (the term approximately being defined as above, with respect to the longest probes of the set of genes).

The invention is also directed to an array suitable to determine the grade of a liver tumor from the sample obtained from a patient. This array is appropriate to carry out the method or process described in the present application.

An array is defined as a solid support on which probes as defined above, are spotted or ommobilized. The solid support may be porous or non-porous, and is usually glass slides, silica, nitrocellulose, acrylamide or nylon membranes or filters.

The arrays of the invention comprise a plurality of probes specific for a set of genes to be assayed. In particular, the array comprises, spotted on it, a set of probes as defined above.

The invention also relates to a composition comprising a set of probes as defined above in solution.

In a first embodiment, the probes (as defined above in the set of probes) may be modified to confer them different physicochemical properties (such as methylation, ethylation). The nucleotide targets (as defined herein and prepared from the sample) are linked to particles, preferably magnetic particles, for example covered with ITO (indium tin oxide) or polymide. The solution of probes is then put in contact with the target nucleotides linked to the particles. The probe/target complexes are then detected, for example by mass spectrometry.

Alternatively, probes may be modified to add a functional group (such as a thiol group) and immobilized on beads (preferably glass beads). These probes immobilized on beads are put in contact with a sample comprising the nucleotide targets, and the probe/target complexes are detected, for example by capillary reaction.

The invention is also directed to kits comprising the sets of probes, the compositions or the arrays of the invention and preferably the primer pairs disclosed herein. These kits may also further comprise reagents necessary for the hybridization of the nucleotide targets of the sets of genes and/or of the invariant genes, to the probes (as such, in the compositions or on the arrays) and the washing of the array to remove unbound nucleotides targets.

In a particular embodiment, the kits also comprise reagents necessary for the hybridization, such as prehybridization buffer (for example containing 5×SSC, 0.1% SDS and 1% bovine serum albumin), hybridization buffer (for example containing 50% formamide, 10×SSC, and 0.2% SDS), low-stringency wash buffer (for example containing 1×SSC and 0.2% SDS) and/or high-stringency wash buffer (for example containing 0.1×SSC and 0.2%SDS).

The kits may also comprise one or several control sample(s) *i.e.*, at least one sample(s) representative for tumor with poor prognosis, at least one sample(s) representative of tumor with good prognosis, at least one sample of a normal adult liver and/or at least one sample of a fetal liver. Alternatively, it may comprise the representation of a gene expression profile of such tumors.

Finally, the invention provides a kit as described above further comprising instructions to carry out the method or process of the invention.

The arrays and/or kits (either comprising pairs of primers or probes or arrays or compositions of the invention or all the components) according to the invention may be used in various aspects, in particular to determine the grade of a liver tumor from a patient, especially by the method disclosed in the present application.

The arrays and/or kits according to the invention are also useful to determine, depending upon the grade of the liver tumor, the risk for a patient to develop metastasis. Indeed, the classification of a liver tumor in the class with poor prognosis is highly associated with the risk of developing metastasis.

In another embodiment, the arrays and/or kits according to the invention are also useful to define, depending upon the grade of the liver tumor, the therapeutic regimen to apply to the patient.

The invention also relates to a support comprising the data identifying the gene expression profile obtained when carrying out the method of the invention.

BRIEF DESCRIPTION OF THE DRAWINGS

The colour version of the drawings as filed is available upon request to the European Patent Office.

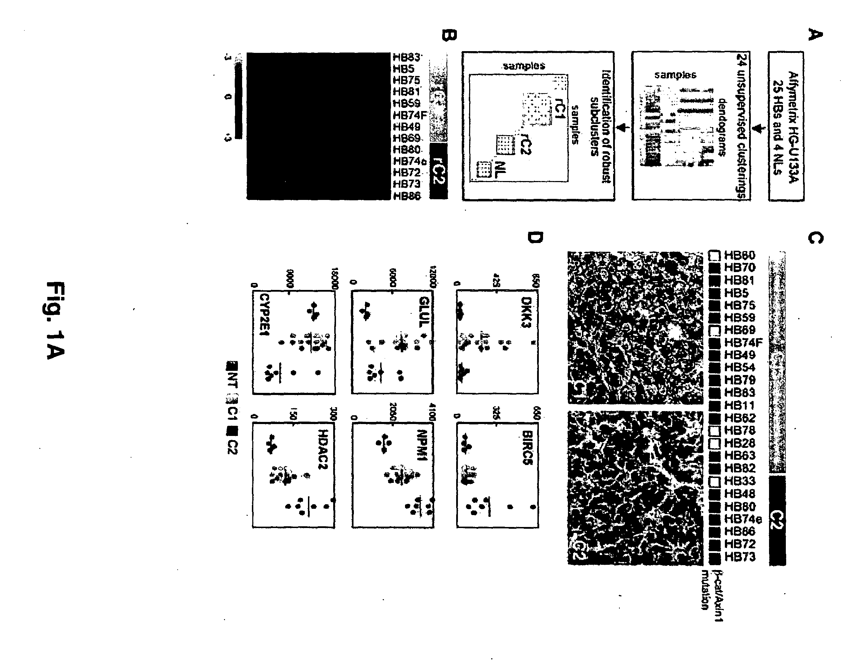

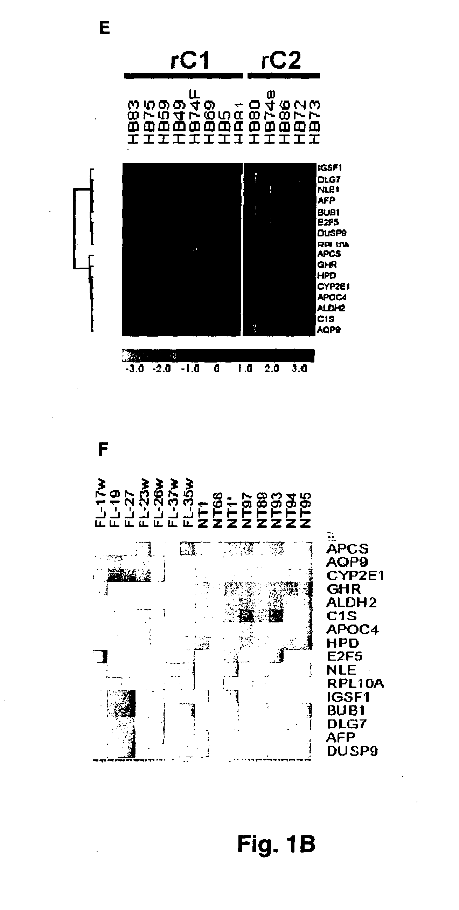

**Figure 1****. Identification of two HB subclasses by expression profiling.**
**(A)** Schematic overview of the approach used to identify robust clusters of samples, including two tumor clusters (rC1 and rC2) and one non-tumor cluster (NL) **(B)** Expression profiles of 982 probe sets (824 genes) that discriminate rC1 and rC2 samples (p < 0.001, two-sample *t* test). Data are plotted as a heatmap where red and green correspond to high and low expression in log₂-transformed scale. **(C)** Molecular classification of 25 HB samples and status of *CTNNB1* gene and β-catenin protein. C1 and C2 classification was based on rC1 and rC2 gene signature by using six different statistical predictive methods (CCP, LDA, 1 NN, 3NN, NC and SVM) and the leave-one-out cross-validation. Black and gray squares indicate mutations of the *CTNNB1* and *AXIN1* genes. Immunohistochemical analysis of β-catenin in representative C1 and C2 cases is shown. **(D)** Expression of representative Wnt-related and β-catenin target genes (p < 0.005, two-sample *t* test) in HB subclasses and non-tumor livers (NL). **(E)** Classification of hepatoblastoma by expression profile of a 16-gene signature. **(F)** Classification of normal human livers of children with HB (from 3 months to 6 years of age) (NT) or fetal livers at 17 to 35 weeks of gestation (FL) by expression profile of a 16-gene signature.

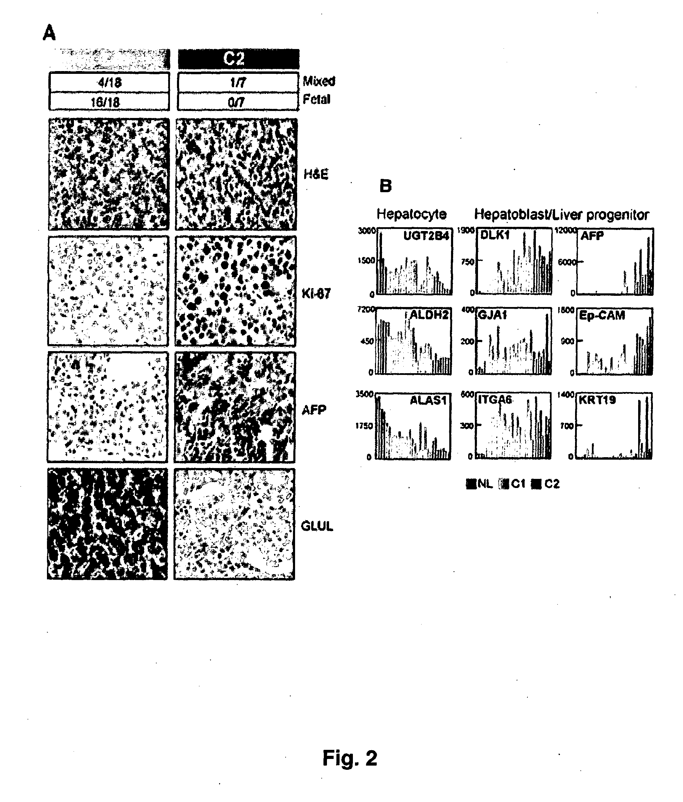

**Figure 2****: Molecular HB subclasses are related to liver development stages. (A)** Distinctive histologic and immunostaining patterns of HB subclasses C1 and C2. From top to bottom: numbers indicate the ratio of mixed epithelial-mesenchymal tumors and of tumors with predominant fetal histotype in C1 and C2 subtypes; hematoxylin and eosin (H&E) and immunostaining of Ki-67, AFP and GLUL in representative samples. Magnification, x400. **(B)** Expression of selected markers of mature hepatocytes and hepatoblast/liver progenitors in HB subclasses and non-tumor livers.

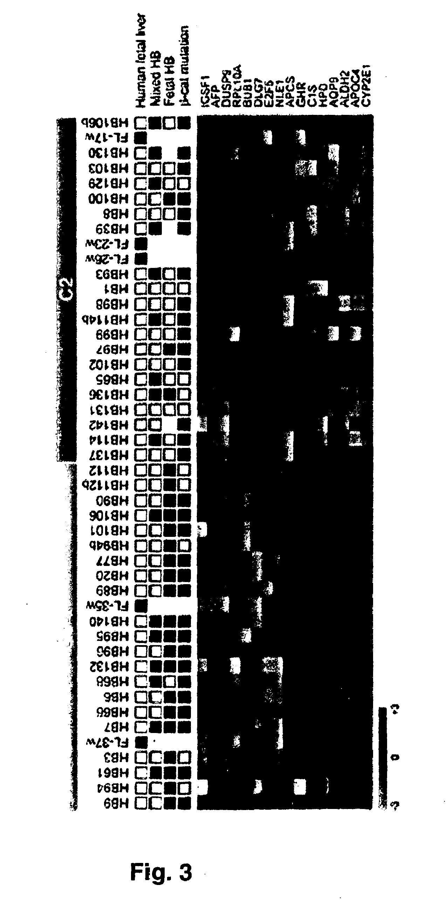

**Figure 3****: Validation of the 16-gene signature by qPCR in an independent set of 41 HBs.** Expression profiles of the 16 genes forming the HB classifier are shown as a heatmap that indicates high (red) and low (green) expression according to log₂-transformed scale. HB tumors, HB biopsies (b) and human fetal livers (FL) at different weeks (w) of gestation were assigned to class 1 or 2 by using the 16-gene expression profile, six different statistical predictive methods (CCP, LDA, 1 NN, 3NN, NC and SVM) and leave-one-out cross-validation. Black boxes in the rows indicate from top to bottom: human fetal liver, mixed epithelial-mesenchymal histology, predominant fetal histotype, and β-catenin mutation.

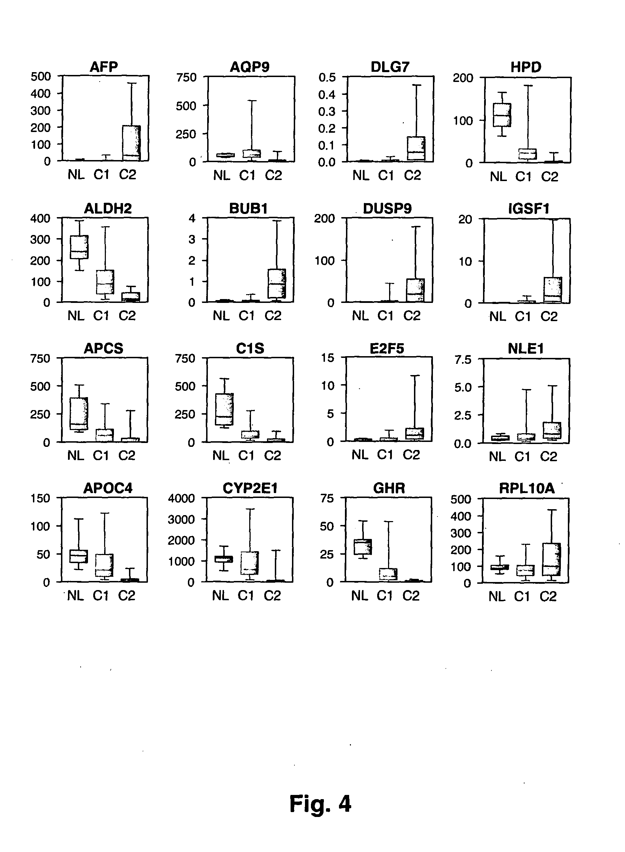

**Figure 4****: Gene expression of the 16 genes of the prognostic liver cancer signature assessed by qPCR is presented as box-plot.** The boxes represent the 25-75 percentile range, the lines the 10-90 percentile range, and the horizontal bars the median values.

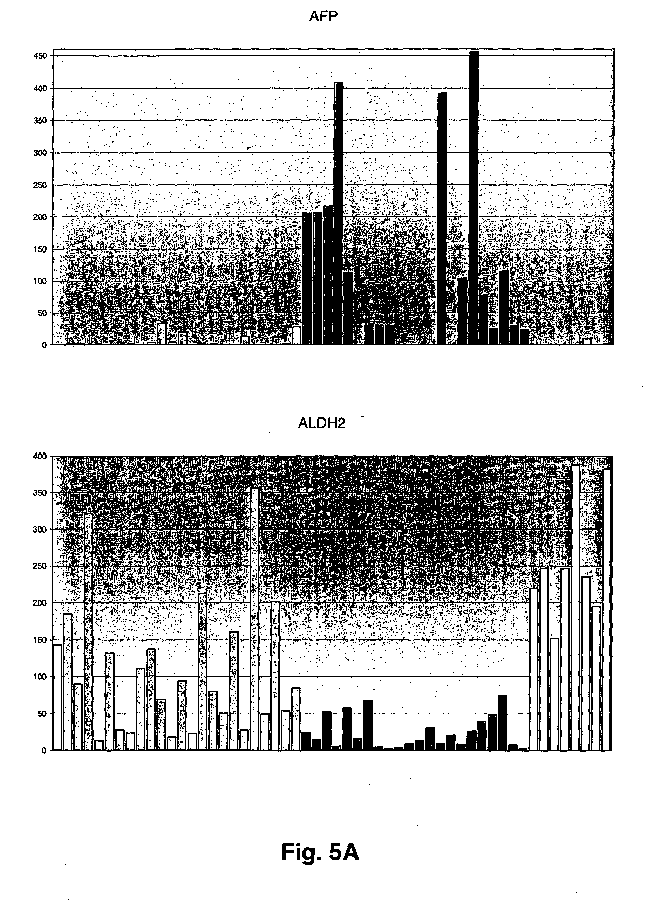

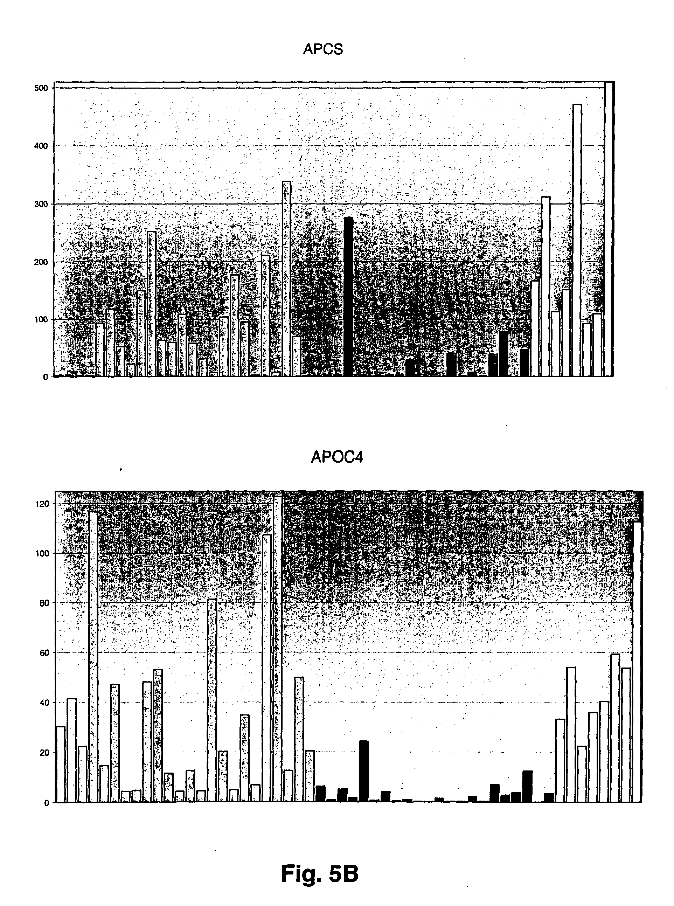

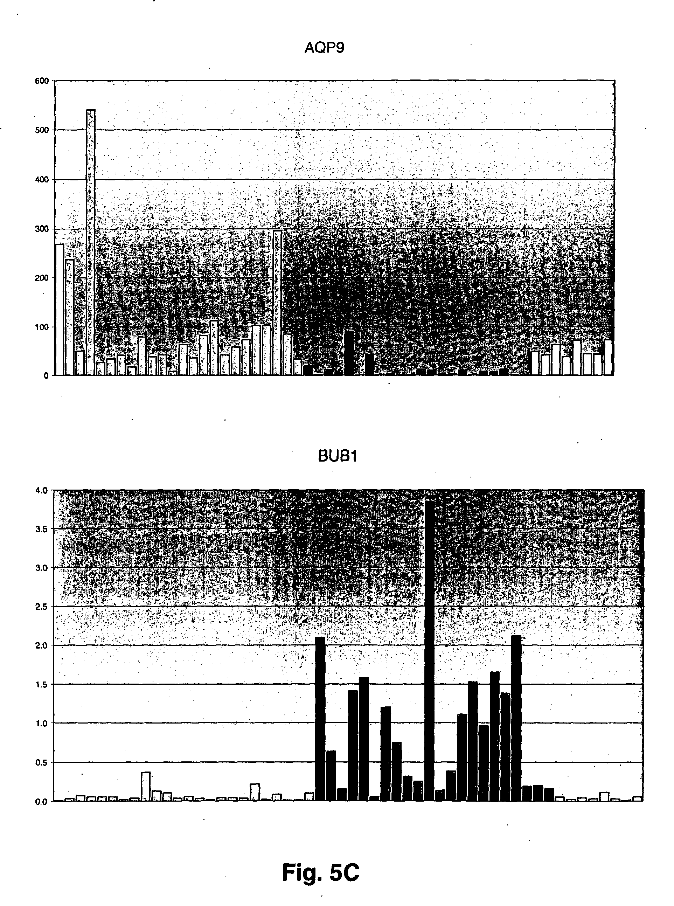

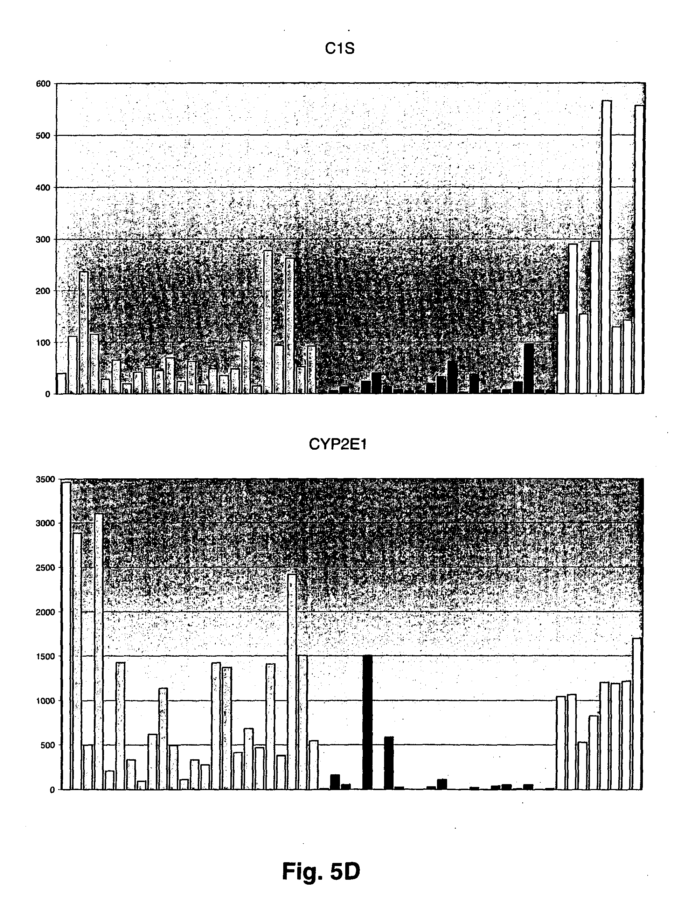

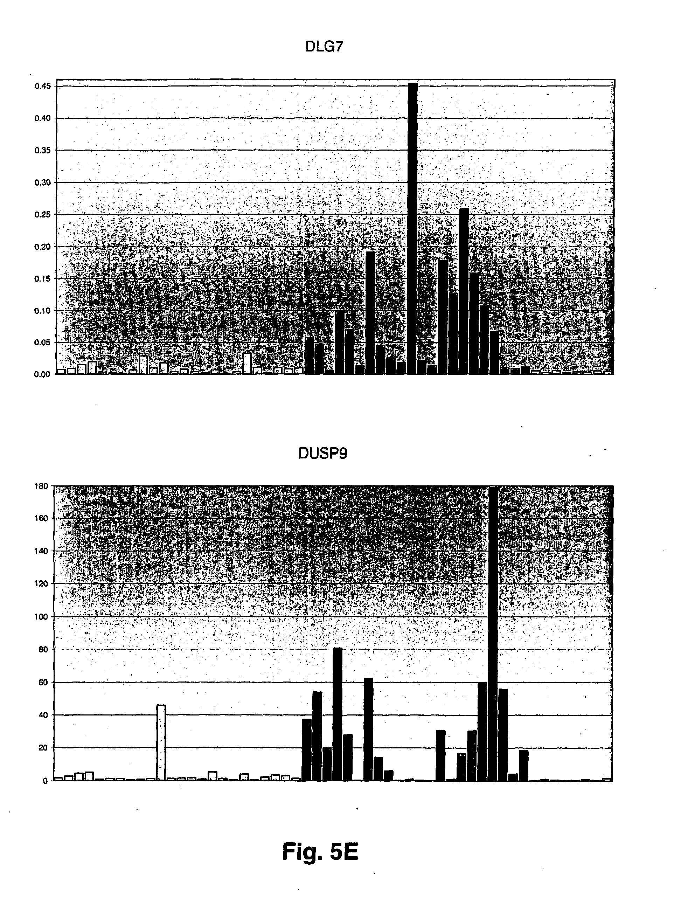

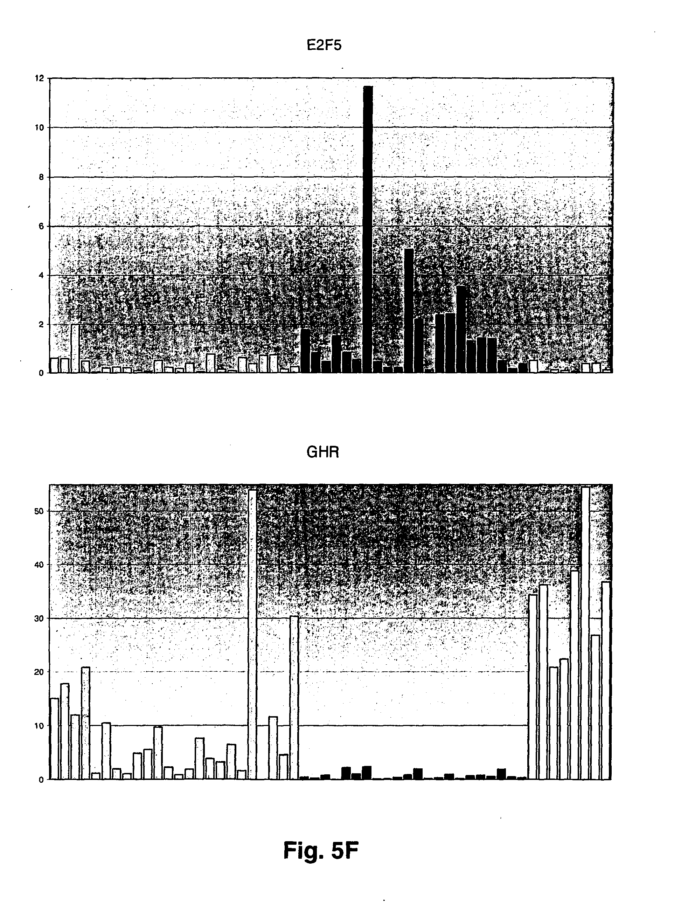

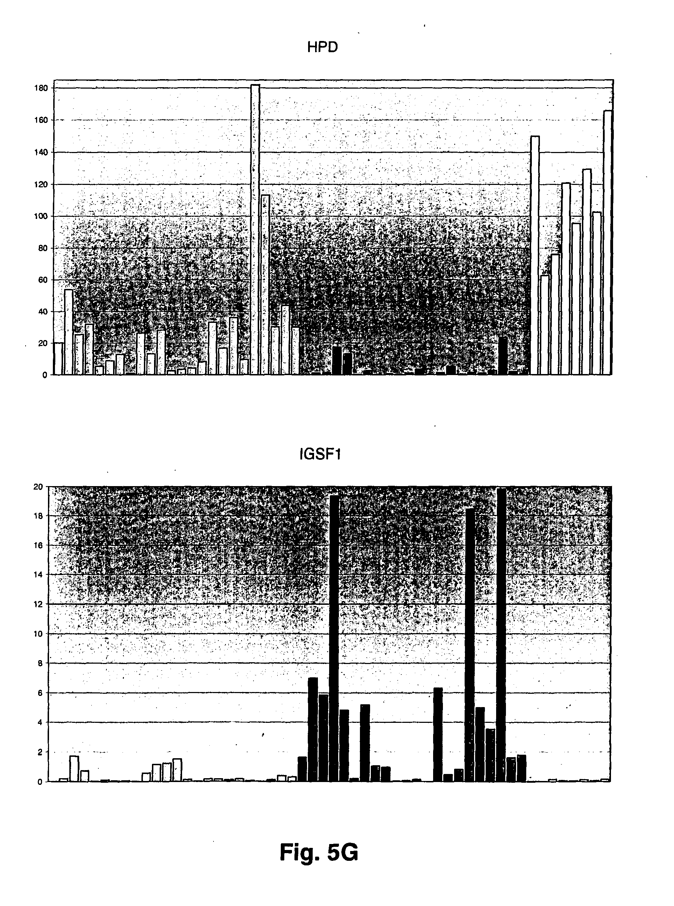

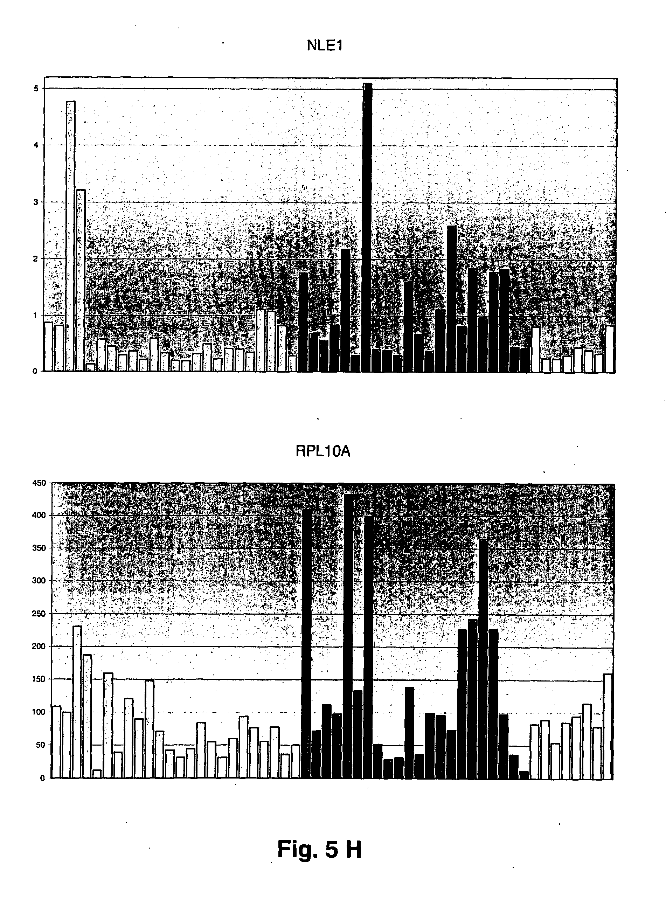

**Figure 5****: Expression level of the 16 liver prognostic signature genes shown case by case in 46 hepatoblastomas and 8 normal livers.** C1 tumors (green), C2 tumors (red) and normal liver (white).

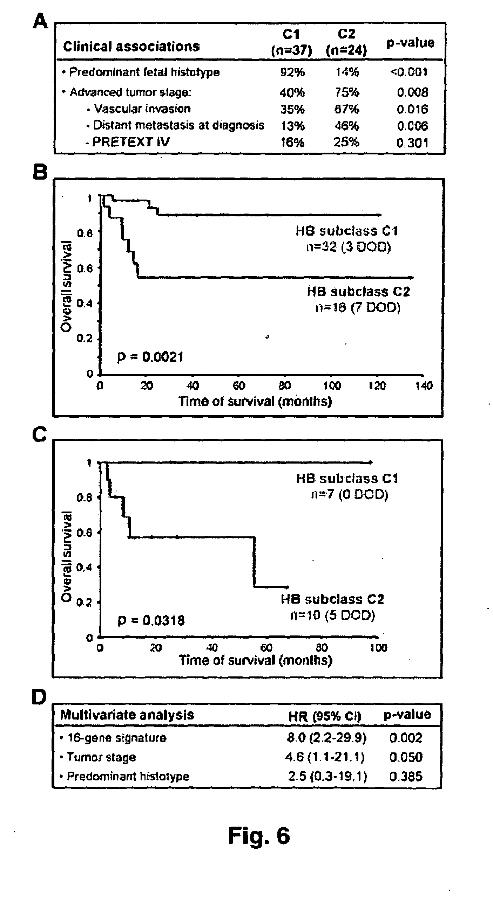

**Figure 6****. Correlation between molecular HB subtypes and clinical outcome in 61 patients.** (**A**) Association of clinical and pathological data with HB classification in the complete set of 61 patients. Only significant correlations (Chi-square test) are shown. PRETEXT IV stage indicates tumorous involvement of all liver sections. **(B)** Kaplan-Meier plots of overall survival for 48 patients that received preoperative chemotherapy. Profiling via the 16-gene expression signature was used to define C1 and C2 subclasses in tumors resected after chemotherapy, and differences between survival curves were assessed with the log-rank test. **(C)** Overall survival of 17 HB patients for which pretreatment biopsies or primary surgery specimens were available. The signature was applied exclusively to tumor samples without prior therapy. **(D)** Multivariate analysis including 3 variables associated to patient's survival. The predominant histotype is defined as either fetal or other (including embryonal, crowed-fetal, macrotrabecular or SCUD types). Tumor stage is defined by PRETEXT stage (Perilongo et al., 2000) and/or distant metastasis at diagnosis and/or vascular invasion. HR, Hazard Ratio; CI, Confidence Interval.

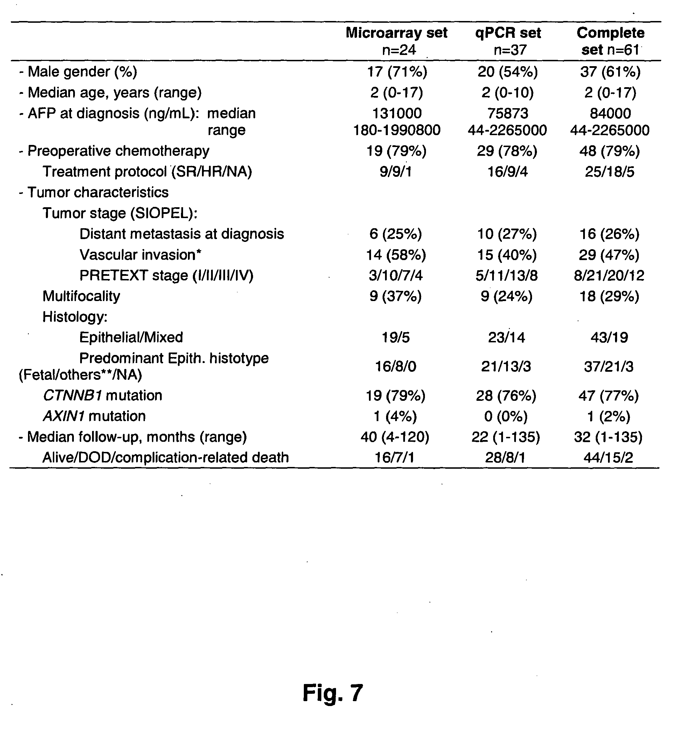

**Figure 7****: Clinical, pathological and genetic characteristics of 61 HB cases.** SR: standard risk; HR: high risk according to SIOPEL criteria; NA: not available; PRETEXT: pre-treatment extent of disease according to SIOPEL; DOD: dead of disease; *: Vascular invasion was defined by radiological analysis; **: The predominant epithelial histotype variable categorized as "others" included embryonal, crowded fetal, macrotrabecular, and undifferentiated histotypes.

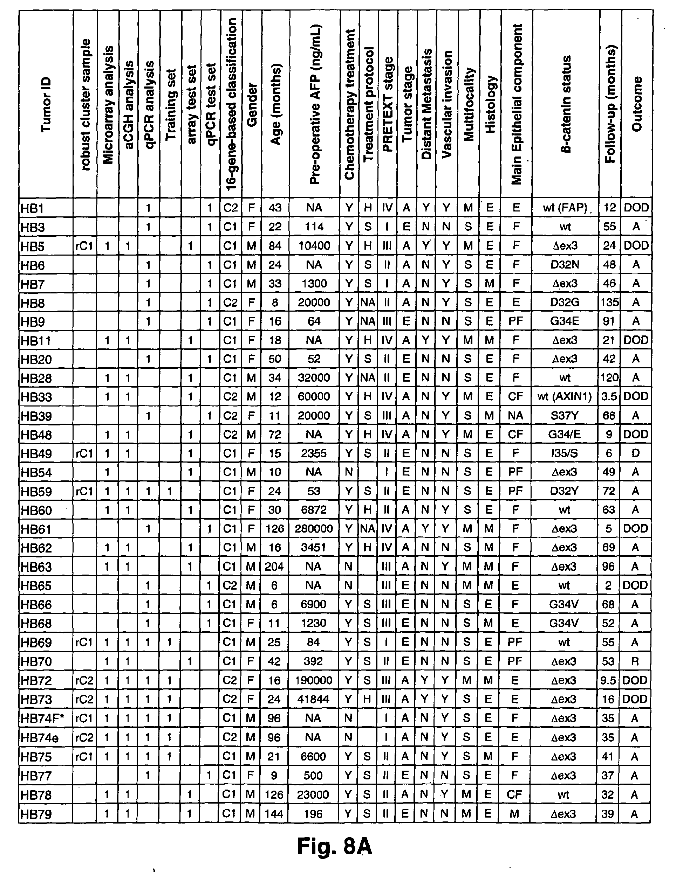

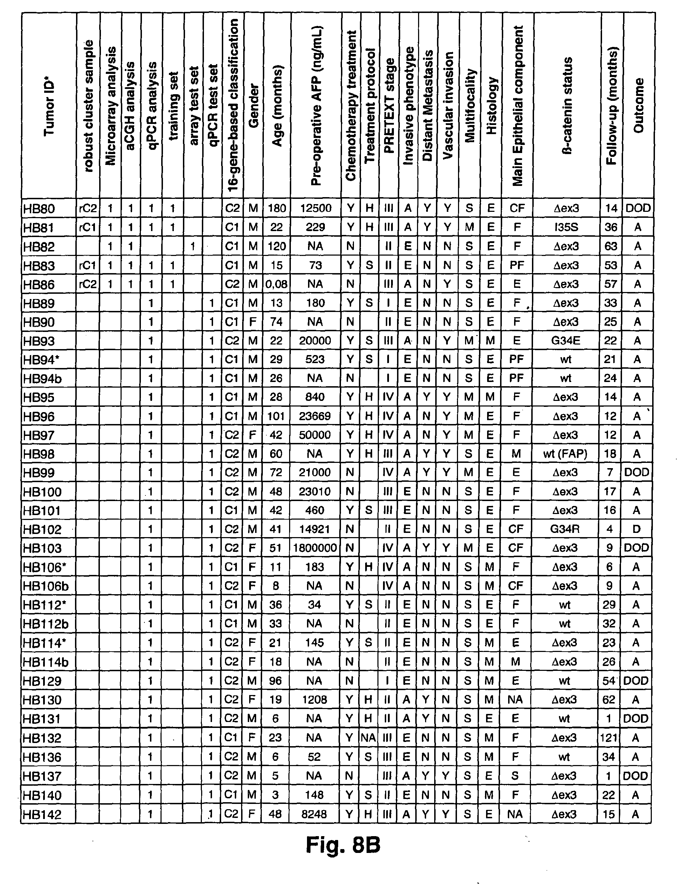

**Figure 8****: Clinical, pathological and genetic characteristics of 66 HB samples;** Tumor ID number indicates patient number. When more than one sample from the same patient was analyzed, the representative sample used for statistical analysis of clinical correlations is marked by an asterisk; b: biopsy. HB74F: fetal component of HB74; HB74e: embryonal component of HB74. Gender: M, male; F, female; Y, yes; N, no; NA, not available. Multifocality: S, solitary nodules; M, multiple nodules. Histology: E, epithelial; M, mixed; CF, crowded fetal; F, fetal; E, embryonal; M, macrotrabecular; PF, pure fetal; S, SCUD. PRETEXT β-catenin status : wt, wild-type; Δex3, in-frame deletion of part or all exon 3 sequence; FAP, familial polyposis kindred; AXIN1, Axin 1 nonsense mutation (R533stop, CGA to TGA).stage: I to IV according to SIOPEL (Aronson et al., 2005). Treatment protocol: S, standard risk; H, high risk according to SIOPEL. Outcome: A, alive free of disease; DOD, dead of disease; D, death unrelated to cancer; R, alive with recurrence of disease.

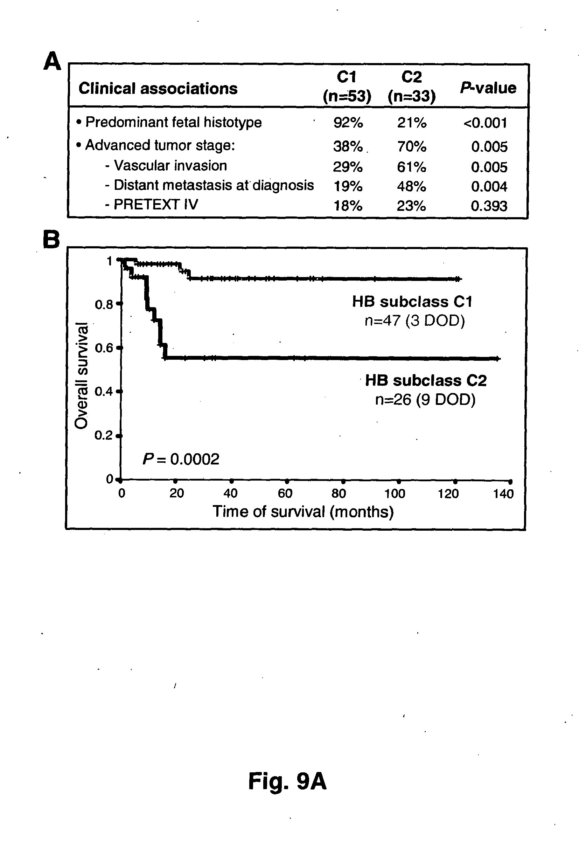

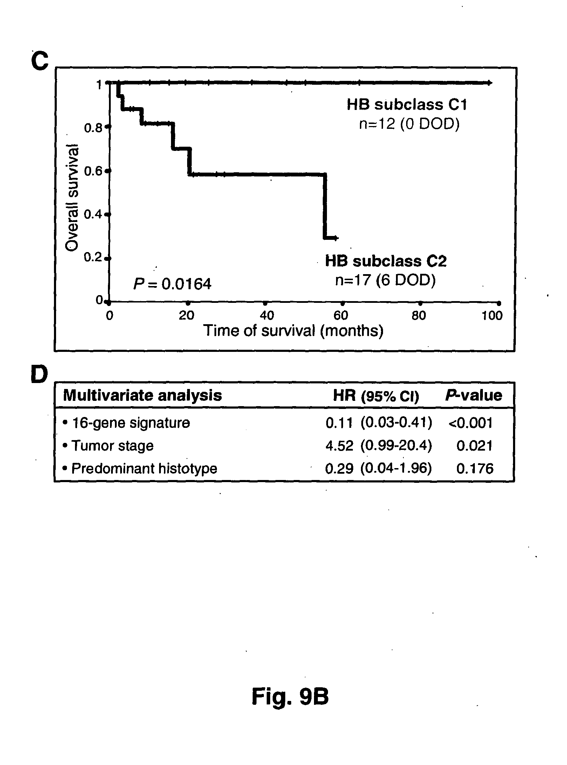

**Figure 9****: Correlation between molecular HB subtypes and clinical outcome in 86 patients. (A)** Association of clinical and pathological data with HB classification in the complete set of 86 patients. Only significant correlations (Chi-square test) are shown. PRETEXT IV stage indicates tumorous involvement of all liver sections. **(B)** Kaplan-Meier plots of overall survival for 73 patients that received preoperative chemotherapy. Profiling via the 16-gene expression signature was used to define C1 and C2 subclasses in tumors resected after chemotherapy, and differences between survival curves were assessed with the log-rank test. **(C)** Overall survival of 29 HB patients for which pretreatment biopsies or primary surgery specimens were available. The signature was applied exclusively to tumor samples without prior therapy. **(D)** Multivariate analysis including 3 variables associated to patient's survival. The predominant histotype is defined as either fetal or other (including embryonal, crowed-fetal, macrotrabecular or SCUD types). Tumor stage is defined by PRETEXT stage (Perilongo et al., 2000) and/or distant metastasis at diagnosis and/or vascular invasion. HR, Hazard Ratio; CI, Confidence Interval

### EXAMPLES

### EXPERIMENTAL PROCEDURES

**A. Patients and tissue samples.**

Sixty-six tumor specimens and biopsies from 61 patients with hepatoblastoma were collected from different hospitals in France (52 cases), Italy (6 cases), United Kingdom (1 case), Switzerland (1 case) and Slovakia (1 case). Forty-eight patients received chemotherapy treatment prior to surgery, most being enrolled in clinical trials of the International Childhood Liver Tumour Strategy Group (SIOPEL) (Perilongo et al., 2000). Samples from fresh tumors avoiding fibrotic and necrotic areas and from adjacent non tumor livers were snap frozen at the time of surgery and stored at -80°C. Figure 7 describes patient characteristics and clinicopathological parameters.

Patients were children with median age of 2 years, and male: female ratio of 1.5. The median follow-up was 32 months; during this period, 15 patients died from disease. The histology of all tumor specimens was centrally reviewed by expert pathologist according to previously described criteria (Finegold et al., 2007; Zimmermann, 2005). Twenty-five tumors were analyzed on oligonucleotide microarrays and 24 of them, for which DNA was available, were subjected to aCGH analysis, while a second set of 41 tumors was analyzed by qPCR (Figure 8). No difference was observed in significant clinical and pathological data as well as in the percentage of cases carrying β-catenin mutation between the two sets. This study has been approved by the Ethics Committee of Institut Pasteur, and informed consent of the families was obtained at each Medical Center, in accordance with European Guidelines for biomedical research and with national laws in each country.

**B. Oligonucleotide microarrays and gene expression data analysis**

Twenty-five HB samples and 4 non-tumor samples including a pool of livers from 3 males and a second from 3 females were analyzed using Affymetrix HG-U133A oligonucleotide arrays. Total RNA was prepared using FastPrep® system (Qbiogene, Strasbourg, France) and RNeasy mini Kit (Qiagen, Courtaboeuf, France). RNA quality was checked with the Agilent 2100 Bioanalyzer (Agilent Technologies, Palo Alto, CA). Microarray experiments were performed according to the manufacturer's instructions. Affymetrix microarray data were normalized using RMA method (Irizarry et al., 2003). Class discovery was done as described elsewhere (Lamant et al., 2007). Pathway and Gene Ontology enrichment analyses were performed using GSEA method (Subramanian et al., 2005) and hypergeometric tests. For supervised tests and class prediction, we used Biometric Research Branch (BRB) ArrayTools v3.2.2 software, developed by R. Simon and A. Peng. Permutations of the measurements are then used to estimate the FDR (the percentage of genes identified by chance). Additionally, mouse fetal livers at E18.5 and postnatal livers at 8 days of birth were profiled on Affymetrix MG-U74A, B v2 arrays. Data were processed and analyzed as aforementioned.

Except when indicated, transcriptome analysis was carried out using either an assortment of R system software packages (http://www.R-project.org, v2.3.0) including those of Bioconductor v1.8 (Gentleman et al., 2004) or original R code.

**B.1. Normalization**

Raw data from Affymetrix HG-U133A 2.0 GeneChip™ microarrays were normalized in batch using robust multi-array average method (R package *affy*, v1.10.0) (Irizarry et al., 2003). Probe sets corresponding to control genes or having a "_x_" annotation were masked yielding a total of 19,787 probe sets available for further analyses.

### B.2. Class discovery

### Step 1

### Variance test

The variance of each probe set across samples was tested and compared to the median variance of all the probe sets, using the model: ((n-1)×Var(_{probe set})/ Var_{med}), where n refers to the number of samples. By using the same filtering tool of BRB ArrayTools software, the *P*-value for each probe set was obtained by comparison of this model to a percentile of Chi-square distribution with (n-1) degrees of freedom.

### Robust coefficient of variation (rCV)

The rCV was calculated for each probe set as follows. After ordering the intensity values of n samples from min to max, we eliminated the min and max values and we calculated the coefficient of variation (CV) for the remaining values.

### Unsupervised probe sets selection

Unsupervised selection of probe set lists was based on the two following criteria:
(i) variance test at *P* < 0.01,
(ii) rCV less than 10 and superior to a given rCV percentile. We used eight rCV percentile thresholds (60%; 70%; 80%; 90%; 95%; 97.5%; 99%; 99.5%), which yielded 8 probe set lists.

### Step2: Generation of a series of 24 dendrograms

Hierarchical clustering was performed by using the 8 rCV-ranked probe sets lists, 3 different linkage methods (average, complete and Ward's), and 1-Pearson correlation as a distance metric (package cluster v1.9.3). This analysis generated 24 dendrograms.

### Step 3:

### Stability assessment

The intrinsic stability of each of the 24 dendrograms was assessed by comparing each dendrogram to the dendrograms obtained after data "perturbation" or "resampling" (100 iterations). Perturbation stands for the addition of random gaussian noise (µ = 0, σ = 1.5 × median variance calculated from the data set) to the data matrix, and resampling for the random substitution of 5% of the samples by virtual sample's profiles, generated randomly. The comparison between dendrograms across all iterations yielded a mean 'similarity score' (see below). The overall stability was assessed by calculating a mean similarity score, using all pairs of the 24 dendrograms.

### Similarity score

To compare two dendrograms, we compared the two partitions in k clusters (k = 2 to 8) obtained from these two dendrograms. To compare a pair of partitions, we used a similarity measure, which corresponds to the symmetric difference distance (Robinson and Foulds, 1981).

### Step 4: Identification of robust clusters

We identified groups in which any pair of samples was co-classified in at least 22 of the 24 partitions, and considered only groups made of 4 samples or more. Then, for any pair of these groups, we calculated the mean number of co-classification of any sample in the first group with any sample in the second group. We aggregated the groups for which this score was at least 18 (over the 24 partitions).

**B.3. Supervised tests**

We compared gene expression between two classes of samples by using the Student's t test with random variance model option (BRB ArrayTools software, version 3.4.0a, developed by Dr. Richard Simon and Amy Peng Lam, http://linus.nci.nih.gov/BRB-ArrayTools.html). False Discovery Rates were assessed by using 1000 random permutations of labels (Monte Carlo approach).

**B.4. Classification**

To classify samples according to gene expression profile, we used the Class prediction tool of BRB ArrayTools software using all 6 following algorithms: Compound Covariate Predictor (CCP), Linear Discriminant Analysis (LDA), 1-Nearest Neighbor (1NN), 3-Nearest Neighbors (3NN), Nearest Centroid (NC) and Support Vector Machines (SVM). Each sample was classified according to the majority of the 6 algorithms. Samples classified as C2 by at least 3 algorithms were classified accordingly.

**B.5. Gene ontology and pathway analysis**

We used a hypergeometric test to measure the association between a gene (probe set) list and a gene ontology term (GO term), as in GO stats R package (R. Gentleman). To this end, we mapped the gene list and the GO terms to non-redundant Entrez Gene identifiers by using the annotation file HG-U133_Plus_2.annot.csv (hftp://www.affymetrix.com, 12/14/2006). GO terms and their relationships (parent/child) were downloaded from hftp://www.geneontology.org (version 12/31/2006). The list of proteins associated to GO terms (table gene_association.goa_human) and mapping the Entrez Gene ids (table human.xrefs) were downloaded from ftp://ftp.ebi.ac.uk/pub/databases/GO/goa.

KEGG pathway annotation was done by Onto-tools software (http://vortex.cs.wayne.edu/ontoexpress/servlet/UserInfo). We designated a significance threshold of each hypergeometric test at *P* < 0.001, and the condition that a GO term or pathway be represented by at least 3 Entrez Gene identifiers.

**B.6. Gene Set Enrichment Analysis (gsea)**

GSEA (Subramanian et al., 2005) was used to evaluate the correlation of a specific gene list with two different sample groups (phenotypes). Briefly, this method calculates an enrichment score after ranking all genes in the dataset based on their correlation with a chosen phenotype and identifying the rank positions of all the members of a defined gene set. We used the signal2noise ratio as a statistic to compare specific and random phenotypes in order to evaluate statistical differences.

**C. Array-based Comparative Genomic Hybridization (aCGH)**

Genomic DNA from 24 HBs and 3 non-tumor liver samples was analyzed using aCGH chips designed by the CIT-CGH consortium. This array contains 3400 sequence-verified PAC/BAC clones spaced at approximately 1 Mb intervals, spotted in triplicate on Ultra Gaps slides (Corning Inc, Corning, NY).

The aCGH chip was designed by CIT-CGH consortium (Olivier Delattre laboratory, Curie Institute, Paris; Charles Theillet laboratory, CRLC Val d'Aurelle, Montpellier; Stanislas du Manoir laboratory, IGBMC, Strasbourg and the company IntegraGenTM). DNAs were labeled by the random priming method (Bioprime DNA labelling system; Invitrogen, Cergy-Pontoise, France) with cyanine-5 (Perkin-Elmer, Wellesley, MA). Using the same procedure, we labeled control DNAs with cyanine-3. After ethanol-precipitation with 210 µg of Human Cot-1 DNA (Invitrogen), resuspension in hybridization buffer (50% formamide), denaturation at 95°C for 10 minutes and prehybridization at 37°C for 90 minutes, probes were cohybridized on aCGH. The aCGH slides were previously preblocked with a buffer containing 2.6 mg succinic anhydride/118ml N-methyl-2-pyrrolidinone/32ml sodium tetraborate decahydrate, pH 8.0 (Sigma-Aldrich, Lyon, France). After washing, arrays were scanned using a 4000B scan (Axon, Union City, CA). Image analysis was performed with Genepix 5.1 software (Axon) and ratios of Cy5/Cy3 signals were determined. The aCGH data were normalized using lowess per block method (Dudoit et al., 2002). Comparison between groups was done using chi-square test or Fisher's exact test, as appropriate.

Status assignment (Gain/Loss) was performed using R package GLAD v1.6.0. Computation of recurrent minimal genomic alterations was done using slight modification of a previously described method (Rouveirol et al., 2006). For comparison between groups, we used the Fischer exact test. Complete aCGH data will be published elsewhere.

**D. Mouse microarray analysis**

Murine Genome Affymetrix U74v2 A and B arrays were used to investigate liver expression at embryonic day 18.5 (E18.5) and at 8 days after birth (PN8). Each time point consisted of a pool of livers from 3-5 animals analyzed in triplicate. Microarray experiments were performed according to the manufacturer's instructions.

Publicly available Affymetrix Mouse Genome (MG) 430 2.0 array liver expression data at embryonic time points E11.5, E12.5, E13.5, E14.5, and E16.5 days of gestation (Otu et al., 2007), were downloaded from the Gene Expression Omnibus (GEO) database (http://www.ncbi.nlm.nih.gov/geo/query/acc.cgi?acc=GSE6998).

MG-U74v2, MG-430 2.0 and HG-133A 2.0 array intra- and cross-species probeset comparison was achieved by using the Affymetrix NetAffx analysis center and by choosing "Good Match" degree of specificity. Unification of sample replicates, multiple array data standardization and Heatmap visualization was done by using dCHIP v1.6 software. Comparison of fetal liver stages by supervised analysis was performed using BRB ArrayTools software as previously described, by classing E11.5 and E12.5 as "Early" and E14.5 and E16.5 as "Late" fetal liver stage. Supervised signature was applied to HB array data, and intensity cut-off = 60 was chosen in order to remove probesets that did not reach such intensity level in at least one sample.

**E. Quantitative PCR analysis (qPCR)**

For qPCR analysis, we used RNA from 52 tumor samples (including 11 samples analyzed on microarrays, see Figure 8), and from 8 non-tumor livers and 5 human fetal livers (RNAs purchased from BioChain Institute, Hayward, CA).

RNA was extracted by using either Trizol, RNeasy kit (QIAGEN) or miRvana kit (Ambion), then quantified and quality-checked by Agilent technology. For each cDNA preparation, 1µg of RNA was diluted at the final concentration of 100 ng/µl, and reverse transcribed with the Superscript RT kit (Invitrogen, Carlsbad, CA) following the manufacturer's protocol. Random primers (Promega, Charbonnières-les-Bains, France) were added at the final concentration of 30 ng/µl and the final volume was 20 µl.

The cDNA was diluted 1:25, and 5 µl were used for each qPCR reaction. We added 5 µl of 2XSybr Green Master mix (Applied Biosystems) and 0.3 µl of each specific primer (final concentration 300nM). Each reaction was performed in triplicate. qPCR reactions were run on the Applied Biosystems 7900HT Fast Real-Time PCR System with a 384-well thermo-block, in the following conditions: 2 min at 50°C to activate Uracil-N-glycosylase (UNG)-mediated erase of aspecific reaction; 10 min at 95°C to activate the polymerase and inactivate the UNG; 40 cycles (15 sec at 95°C denaturation step and 1 min at 60°C annealing and extension); and final dissociation step to verify amplicon specificity.

The lists of primers used for qPCR are provided in Table 6 and Table 7 above.

**F. Immunohistochemistry (IHC)**

IHC was carried out as reported previously (Wei et al., 2000). For antigen retrieval at 95°C, we used 1 mM EDTA (pH 8) for β-catenin and Ki-67 IHC, and 10 mM citrate buffer (pH 6) for AFP and GLUL IHC. We used monoclonal antibodies against β-catenin and GLUL (Cat. Nos. 610154 and 610517; BD Biosciences, Le Pont de Claix, France) and Ki-67 (M7240, Dako, Trappes, France) and polyclonal antibody against AFP (N1501, Dako). Reactions were visualized using the ChemMate Dako Envision Detection kit (Dako) and diaminobenzidine. Subcellular distribution and quantitative evaluation of immunostaining in the different histotypes were assessed by examining at least ten random high-power fields.

**G. Clinical data analysis**

We used the Chi-square test for comparisons between groups. Survival curves were calculated according to the Kaplan-Meier method, using the log-rank test to assess differences between curves. Variables independently related to survival were determined by stepwise forward Cox regression analysis. Follow-up was closed at February 2007 or at time of death. Statistical analysis was done with SPSS software v10.0 (SPSS Inc., Chicago, IL).

**H. Examples of other pairs of primers and probes for the 16 genes of Table 1 and the 4 invariant genes (Table 3) that can be used in the Taqman® method.**
AFP forward primer: GCCAGTGCTGCACTTCTTCA
   AFP reverse primer: TGTTTCATCCACCACCAAGCT
   AFP probe: ATGCCAACAGGAGGCCATGCTTCA
   (for each polynucleotide, the sequence is given from 5' to 3')
ALDH2 forward primer: TGCAGGATGGCATGACCAT
   ALDH2 reverse primer: TCTTGAACTTCAGGATCTGCATCA
   ALDH2 probe: CCAAGGAGGAGATCTTCGGGCCA
   APCS forward primer: AGCTGGGAGTCCTCATCAGGTA
   APCS reverse primer: CGCAGACCCTTTTTCACCAA
   APCS probe: TGCTGAATTTTGGATCAATGGGACACC
APOC4 forward primer: TGAAGGAGCTGCTGGAGACA
   APOC4 reverse primer: CGGGCTCCAGAACCATTG
   APOC4 probe: TGGTGAACAGGACCAGAGACGGGTG
AQP9 forward primer: GCCATCGGCCTCCTGATTA
   AQP9 reverse primer: GTTCATGGCACAGCCACTGT
   AQP9 probe: TGTCATTGCTTCCTCCCTGGGACTG
BUB1 forward primer: ACATCTGGTTTTCAGTGTGTTGAGA
   BUB1 reverse primer: GTTGCAGCAACCCCAAAGTAA
   BUB1 probe: TCAGCAACAAACCATGGAACTACCAGATCG
C1S forward primer: TCCCAATGACAAGACCAAATTCT
   C1S reverse primer: AGAGCCCATAGGTCCCACACT
   C1S probe: CGCAGCTGGCCTGGTGTCCTG
CYP2E1 forward primer: CATGAGATTCAGCGGTTCATCA
   CYP2E1 reverse primer: GGTGTCTCGGGTTGCTTCA
   CYP2E1 probe: CCTCGTGCCCTCCAACCTGCC
DLG7 forward primer: GCTGGAGAGGAGACATCAAGAAC
   DLG7 reverse primer: CCTGGTTGTAGAGGTGAAAAAGTAATC
   DLG7 probe: TGCCAGACACATTTCTTTTGGTGGTAACC
DUSP9 forward primer: GGCCTACCTCATGCAGAAGCT
   DUSP9 reverse primer: GGGAGATGTTAGACTTCTTCCTCTTG
   DUSP9 probe: CACCTCTCTCTCAACGATGCCTATGACCTG
E2F5 forward primer: CCTGTTCCCCCACCTGATG
   E2F5 reverse primer: TTTCTGTGGAGTCACTGGAGTCA
   E2F5 probe: CCTCACACAGCCTTCCTCCCAGTCC
GHR forward primer: CCCAGGTGAGCGACATTACA
   GHR reverse primer: CATCCCTGCCTTATTCTTTTGG
   GHR probe: CAGCAGGTAGTGTGGTCCTTTCCCCG
HPD forward primer: CCCACGCTCTTCCTGGAA
   HPD reverse primer: TTGCCGGCTCCAAAACC
   HPD probe: TCATCCAGCGCCACAACCACCA
IGSF1 forward primer: GACCATTGCCCTTGAAGAGTGT
   IGSF1 reverse primer: GAGAGGTTGATGAAGGAGAATTGG
   IGSF1 probe: ACCAAGAAGGAGAACCAGGCACCCC
NLE1 forward primer: TGCCTCCTTTGACAAGTCCAT
   NLE1 reverse primer: CGCGTAGGGAAGCCAGGTA
   NLE1 probe: TGGGATGGCAGGACGGGCA
RPL10A forward primer TCGGCCCAGGTTTAAATAAGG
   RPL10A reverse primer CCACTTTGGCCACCATGTTT
   RPL10A Taqman probe AGTTCCCTTCCCTGCTCACACACAACG
ACTG1 forward primer: GGCGCCCAGCACCAT
   ACTG1 reverse primer: CCGATCCACACCGAGTACTTG
   ACTG1 probe: ATCAAGATCATCGCACCCCCAGAGC
EEF1A1 forward primer: GCGGTGGGTGTCATCAAAG
   EEF1A11 reverse primer: TGGGCAGACTTGGTGACCTT
   EEF1A11 probe: AGTGGACAAGAAGGCTGCTGGAGCTG
PNN forward primer: GAATTCCCGGTCCGACAGA
   PNN reverse primer: TTTCGGTCTCTTTCACTTCTTGAA
   PNN probe: AGAGGTCTATATCAGAGAGTAGTCGATCAGGCAAAAGA
RHOT2 forward primer: CCCAGCACCACCATCTTCAC
   RHOT2 reverse primer: CCAGAAGGAAGAGGGATGCA
   RHOT2 Taqman probe: CAGCTCGCCACCATGGCCG

### RESULTS

**Identification of two HB subclasses by gene expression profiling**

For robust unsupervised classification, we generated and screened a series of 24 dendrograms to identify samples that co-clustered whatever the method and the gene list. We obtained two robust subgroups of tumors named robust Cluster 1 (rC1, n=8) and robust Cluster 2 (rC2, n=5) (Figure 1A). Comparison of rC1 and rC2 expression profiles identified 824 genes (p < 0.001, false discovery rate (FDR) = 0.02) (Figure 1B). KEGG pathway analysis pinpointed a strong enrichment of cell cycle related genes (p < 10⁻¹¹), most being up-regulated in rC2 tumors. These genes were mainly assigned to GO categories including mitosis regulation, spindle checkpoint, nucleotide biosynthesis, RNA helicase activity, ribosome biogenesis, and translational regulation. Evidence that rC2 tumors were faster proliferating than rC1 tumors was further confirmed by Ki-67 immunostaining (see Figure 2A).

The remaining tumors were classified into C1 (rC1-related) and C2 (rC2-related) subclasses by applying a predictive approach based on the rC1/rC2 gene signature and using robust samples as training set (Figure 1C). Both groups exhibited similar, high rates of β-catenin mutations, and accordingly, immunohistochemistry (IHC) of β-catenin showed cytoplasmic and nuclear staining of the protein in the majority of HBs. However, β-catenin localization was predominantly membranous and cytoplasmic in C1 tumors, whereas it showed frequent loss of membrane anchoring and intense nuclear accumulation in C2 tumors (Figure 1C).

We observed differential expression of a number of Wnt members and targets between subclasses. C2 tumors showed increased expression of *MYCN, BIRGS* that encodes the anti-apoptotic factor Survivin, *NPM1* (encoding nucleophosmin) and *HDAC2*. By contrast, most C1 tumors prominently expressed the Wnt antagonist *DKK3, BMP4*, and genes previously found to be activated in liver tumors carrying mutant β-catenin (Boyault et al., 2007; Renard et al., 2007; Stahl et al., 2005). Remarkably, most genes related to liver functions are expressed in the perivenous area of adult livers, such as *GLUL, RHBG*, and two members of the cytochrome p450 family: *CYP2E1* and *GYP1A* (Benhamouche et al., 2006; Braeuning et al., 2006) (Figure 1D).

Further evidence that the rC1 subclass was enriched in genes assigned to the hepatic perivenous program was provided by Gene Set Enrichment Analysis (GSEA), a computational method for assessing enrichment of a predefined gene list in one class as compared with another (Subramanian et al., 2005). Thus, Wnt/β-catenin signaling appears to activate different transcriptional programs in HB subtypes, likely reflecting different cellular contexts.

**HB subclasses evoke distinct phases of liver development**

Next, we sought to determine whether HB subclasses were associated with specific histological phenotypes. Mixed epithelial-mesenchymal tumors that represented 20% of cases were not significantly associated with C1 and C2 subclasses. By contrast, a tight association was found with the main epithelial component, which defines the cell type occupying more than 50% of tumor cross-sectional areas. Sixteen out of 18 C1 tumors displayed a predominant fetal phenotype, including 4 'pure fetal' cases, whereas all C2 tumors showed a more immature pattern, with prevailing embryonal or crowded-fetal histotypes associated with high proliferation (Finegold, 1994) (p < 0.0001) (Figure 2A). Further relationship between molecular subclasses and hepatic developmental stages was provided by the finding that a number of mature hepatocyte markers were markedly downregulated in C2 compared to C1 tumors (Tables 1 and 2). Conversely, C2 tumors showed strong overexpression (35-fold) of the oncofetal *AFP* gene associated to high protein levels in tumor cells by IHC (Figure 2A) and in patients' sera (r = 0.79, p < 0.0001). C2 tumors also abundantly expressed hepatic progenitor markers such as KRT19 (encoding cytokeratin 19) and TACSTD1, also known as Ep-CAM (Figure 2B).

To better define the relationships between HB subclasses and phases of hepatic differentiation, we first generated a liver development-related gene signature by making use of publicly available mouse fetal and adult liver data sets (Otu et al., 2007). When applied to HB samples, this signature was able to distinguish by hierarchical clustering two HB groups closely matching the C1/C2 classification. Next, we integrated HB gene expression data with the orthologous genes expressed in mouse livers at embryonic days (E) 11.5 to 18.5, and at 8 days of birth. In unsupervised clustering, most C2 tumors co-clustered with mouse livers at early stages of embryonic development (E11.5 and E12.5), whereas C1 tumors gathered with mouse livers at late fetal and postnatal stages. Together, these data comfort the notion that tumor cells in C2 and C1 subtypes are arrested at different points of the hepatic differentiation program.

**Identification of a 16-gene signature as HB classifier**

To investigate the relevance of molecular HB classification in an independent set of tumors, we defined a HB classifier signature derived from the top list of genes differentially expressed between rC1 and rC2 clusters. After qPCR assessment, a list of 16 top genes at p ≤ 10⁻⁷ was selected to form a class predictor (Table 1). Most of these genes show drastic variations in expression level during liver development, and among them, BUB1 and DLG7 have been repeatedly identified as hESC markers (Assou et al., 2007). The 16-gene expression profile was first investigated in rC1 and rC2 samples used as training set, and it predicted classification with 100% of accuracy in these samples, using either microarray or qPCR data. The robustness of this signature was confirmed by correct classification into C1 and C2 subclasses of all 13 remaining tumors analyzed by microarray (Figure 1E). Expression profiles of fetal livers and normal liver for these 16-gene signature were also assayed (Figure 1F). This signature was therefore employed to classify a new, independent set of 41 HB samples by qPCR (Figures 4 and 5 and Table 8), resulting in 21 tumors categorized as C1 and 20 tumors as C2 subtype (Figure 3).

Extending our previous observation, C1/C2 classification in this new set of tumors was unrelated to *CTNNB1* mutation rate. Using qPCR, we also confirmed enhanced expression in C2 tumors of liver progenitor markers such as AFP, Ep-CAM, and KRT19, as well as MYCN (Figure 3). Moreover, while a similar percentage of C1 and C2 tumors displayed mesenchymal components, a predominant fetal histotype was found in 95% of tumors of the C1 subtype, whereas in 82% of C2 tumors, the major component displayed less differentiated patterns such as embryonal, crowded-fetal, macrotrabecular and SCUD types (p < 0.0001) (Figure 3). To further assess the association of HB subclasses with liver development, 5 human fetal livers at different weeks of gestation were included in the qPCR studies. In unsupervised clustering, fetal livers at late (> 35 weeks) and earlier (17 to 26 weeks) developmental stages were classified as C1 and C2 respectively, further supporting that HB subclasses reflect maturation arrest at different developmental phases.

**Table 8. Gene expression of the prognostic signature for liver cancer by quantitative RT-PCR. NL, non-tumor liver; C1, good prognosis hepatoblastomas; C2, bad prognosis hepatoblastomas. Shown are the median values of 46 hepatoblastomas from 41 patients, the minimal and maximal values in each class, and the fold changes between classes. Data are presented in arbitrary units after normalization of the raw quantitative PCR values with genes (ACTG1, EFF1A1, PNN and RHOT2) that presents highly similar values in all samples. Gene expression of the 16 genes are presented on Figures 4 and 5.**

| | **C1** | | | **C2** | | | **NL** | | | **Fold-change** | | | |
|---|---|---|---|---|---|---|---|---|---|---|---|---|---|
| | **median** | **min** | **max** | **median** | **min** | **max** | **median** | **min** | **max** | **C1/NL** | **C2/NL** | **C2/C1** | **C1/C2** |
| **AFP** | 0,4 | 0,0 | 33,3 | 30,7 | 0,0 | 456,1 | 0,2 | 0,0 | 8,8 | 2,3 | 38,1 | 16,5 | 0,1 |
| **ALDH2** | 87,1 | 13,2 | 356,7 | 15,0 | 2,2 | 74,4 | 240,4 | 151,6 | 387,6 | 0,3 | 0,1 | 0,2 | 5,2 |
| **APCS** | 61,6 | 1,1 | 338,9 | 1,9 | 0,0 | 276,2 | 158,6 | 92,7 | 509,5 | 0,2 | 0,0 | 0,1 | 19,8 |
| **APOC4** | 21,3 | 4,3 | 122,8 | 1,6 | 0,1 | 24,2 | 47,0 | 22,3 | 112,4 | 0,5 | 0,0 | 0,1 | 16,1 |
| **AQP9** | 60,6 | 8,0 | 540,6 | 2,5 | 0,1 | 90,1 | 46,6 | 38,0 | 72,7 | 1,3 | 0,1 | 0,1 | 18,9 |
| **BUB1** | 0,0 | 0,0 | 0,4 | 0,9 | 0,1 | 3,9 | 0,0 | 0,0 | 0,1 | 1,2 | 16,1 | 13,4 | 0,1 |
| **C1S** | 51,1 | 14,9 | 277,2 | 7,5 | 1,3 | 96,0 | 223,4 | 129,3 | 565,3 | 0,2 | 0,0 | 0,2 | 5,7 |
| **CYP2E1** | 583,2 | 97,7 | 3463,0 | 19,7 | 0,4 | 1504,0 | 1128,6 | 527,6 | 1697,0 | 0,7 | 0,0 | 0,0 | 51,6 |
| **DLG7** | 0,0 | 0,0 | 0,0 | 0,1 | 0,0 | 0,5 | 0,0 | 0,0 | 0,0 | 1,7 | 12,4 | 7,3 | 0,1 |
| **DUSP9** | 1,5 | 0,4 | 45,7 | 19,1 | 0,0 | 179,0 | 0,6 | 0,2 | 1,3 | 4,0 | 18,3 | 4,6 | 0,2 |
| **E2F5** | 0,2 | 0,0 | 2,0 | 1,1 | 0,1 | 11,7 | 0,1 | 0,0 | 0,5 | 1,8 | 6,5 | 3,5 | 0,3 |
| **GHR** | 5,2 | 0,0 | 54,0 | 0,5 | 0,0 | 2,4 | 35,2 | 20,8 | 54,5 | 0,1 | 0,0 | 0,1 | 8,6 |
| **HPD** | 22,9 | 0,9 | 182,0 | 1,2 | 0,1 | 23,8 | 111,5 | 62,6 | 165,7 | 0,2 | 0,0 | 0,1 | 14,0 |
| **IGSF1** | 0,1 | 0,0 | 1,7 | 1,7 | 0,0 | 19,8 | 0,1 | 0,0 | 0,1 | 2,2 | 22,4 | 10,2 | 0,1 |
| **NLE** | 0,4 | 0,1 | 4,8 | 0,8 | 0,3 | 5,1 | 0,4 | 0,2 | 0,8 | 1,2 | 2,2 | 1,8 | 0,5 |
| **RPL10A** | 73,3 | 12,0 | 230,4 | 98,2 | 11,9 | 432,8 | 86,9 | 54,1 | 159,9 | 0,8 | 1,1 | 1,5 | 0,7 |

**The 16-gene signature as a strong independent prognostic factor**

**In a first set of 61 patients**

The clinical impact of HB molecular classification was addressed in a first set of 61 patients (Figures 7 and 8), comprising 37 (61 %) C1 and 24 (39%) C2 cases. Besides strong association with predominant immature histotypes, HBs of the C2 subclass were tightly associated with features of advanced tumor stage, such as vascular invasion and extrahepatic metastasis (Figure 6A). Accordingly, overall survival of these patients was markedly impaired. Kaplan-Meier estimates of overall survival probability at 2-years were 50% for patients with C2 tumors and 90% for patients with C1 tumors (p = 0.0001, log rank test), and similar trends were seen for disease-free survival probabilities (data not shown). Next, we examined whether pre-operative chemotherapy treatment given to 48 patients could affect tumor classification. These cases were evenly distributed among HB subclasses, with no significant association with molecular classification. Of note, available pretreatment biopsies were assigned to the same subclass as matched resected tumors in 3 out of 4 cases (see Figure 3; HB112 and HB112b have been both classified as C1 grade, and HB114 and HB114b have been both classified as C2 grade). We examined the performance of the 16-gene signature on the 48 tumors resected after chemotherapy, and found significant difference in outcome between patients with C1 and C2 type HBs (p = 0.0021, log rank test) (Figure 6B). Remarkably, Kaplan-Meier analysis confirmed C2 subclass as a poor prognostic group in 17 cases for which pre-treatment biopsies or primary surgery specimens were available (p = 0.0318, log rank test) (Figure 6C).

We further assessed the prognostic validity of the 16-gene signature for all patients in multivariate analysis, using a Cox proportional hazards model with pathological and clinical variables associated to patients' survival. This analysis identified the signature as an independent prognostic factor, with better performance than tumor stage defined by PRETEXT stage, vascular invasion and extrahepatic metastases (Figure 6D). Thus, this signature demonstrated strong prognostic relevance when compared to current clinical criteria.

**In a second set of 86 patients**

The clinical impact of HB molecular classification was addressed in a second set of patients (comprising the sample of the first set), comprising 53 (61%) C1 and 33 (39%) C2 cases. Besides strong association with predominant immature histotypes, HBs of the C2 subclass were tightly associated with features of advanced tumor stage, such as vascular invasion and extrahepatic metastasis (Figure 9A). Accordingly, overall survival of these patients was markedly impaired. Kaplan-Meier estimates of overall survival probability at 2-years were 60% for patients with C2 tumors and 94% for patients with C1 tumors (p = 0.00001, log rank test), and similar trends were seen for disease-free survival probabilities (Table 9).

**Table 9: Survival analysis (Kaplan Meier, log rank test); DFS: disease-free survival; Others: dead or alive with recurrent disease.**

| **N. of patients** | **61 C1+25 C2 = 86** | **P value** |
|---|---|---|
| Survival (all patients) | Alive/Dead | |
| C1 | 50/3 | <0.00001 |
| C2 | 20/13 | |
| DFS (all patients) | DFS/others | |
| C1 | 48/5 | <0.00001 |
| C2 | 18/15 | |
| Survival (non-treated patients) | Alive/Dead | |
| C1 | 12/0 | 0.0164 |
| C2 | 11/6 | |
| DFS (non-treated patients) | DFS/others | |
| C1 | 12/0 | 0.0213 |
| C2 | 12/6 | |

Next, we examined whether pre-operative chemotherapy treatment given to 73 patients could affect tumor classification. These cases were evenly distributed among HB subclasses, with no significant association with molecular classification. We examined the performance of the 16-gene signature on the 73 tumors resected after chemotherapy, and found significant difference in outcome between patients with C1 and C2 type HBs (p = 0.0002, log rank test) (Figure 9B). Remarkably, Kaplan-Meier analysis confirmed C2 subclass as a poor prognostic group in 29 cases for which pre-treatment biopsies or primary surgery specimens were available (p = 0.0164, log rank test) (Figure 9C).

We further assessed the prognostic validity of the 16-gene signature for all patients in multivariate analysis, using a Cox proportional hazards model with pathological and clinical variables associated to patients' survival. This analysis identified the signature as an independent prognostic factor, with better performance than tumor stage defined by PRETEXT stage, vascular invasion and extrahepatic metastases (Figure 9D).

Finally, various clinical elements of 103 HB samples from 86 patients were compared with respect to their classification as C1 or C2 grade using the 16-gene signature (Table 10).

**Table 10: Clinical correlations. STD : standard risks (cisplatine) - High : high risk (cisplatine/doxorubicine, intensified treatment); Tumor stage (defined as Vasc. Inv and/or metastasis and/or PRETEXT stage IV); metastasis: extrahepatic metastasis (mainly lung); vascular invasion is determined by imagery; Pretext IV (involved an intrahepatic extent of the tumor to all hepatic sections); multifocality (more than 2 tumor nodules); Ep: pure epithelial form - Mixed: mesenchymatous and epithelial mixed form; Fetal: well differentiated; non fetal: embryonic, atypic, SCUD and/or macrotrabecular cells.**

| **N. of patients** | **61+25 = 86** | **p-value (chi-square)** |
|---|---|---|
| Gender | ns | |
| Chemotherapy treatment | Yes/No | |
| C1 | 47/6 | ns |
| C2 | 26/7 | |
| Chemotherapy protocol | STD/High | |
| C1 | 30/13 | 0.007 |
| C2 | 9/16 | |
| TUMOR STAGE | Early/Advanced | |
| C1 | 32/20 | 0.005 |
| C2 | 10/23 | |
| Metastasis | No/Yes | |
| C1 | 43/10 | 0.004 |
| C2 | 17/16 | |
| Vascular Invasion | No/Yes | |
| C1 | 36/15 | 0.005 |
| C2 | 13/20 | |
| Advanced Pretext stage (IV) | No/Yes | |
| C1 | 42/9 | ns |
| C2 | 24/7 | |
| Multifocality | | |
| C1 | No/Yes 36/17 | ns |
| C2 | 18/14 | |
| Histology | | |
| C1 | Ep/Mixed 31/21 | ns |
| C2 | 20/13 | |
| Main Epith Comp | Fetal/NonFetal | |
| C1 | 48/4 | <0.0001 |
| C2 | 6/22 | |

The above results carried out on a first set of 61 patients, and on a second completed set of 86 patients, demonstrate that the 16-gene signature, identified in the present application, is a strong prognostic relevance when compared to current clinical criteria.

### DISCUSSION

The present application demonstrates that, using integrated molecular and genetic studies, hepatoblastoma encompass two major molecular subclasses of tumors that evoke early and late phases of prenatal liver development. Aberrant activation of the canonical Wnt pathway represented a seminal event in both tumor types, with cumulated mutation rates of β-catenin, APC and AXIN over 80%. However, depending on tumor differentiation stage, Wnt signaling activated distinct transcriptional programs involved in tumor growth and invasiveness or in liver metabolism. Further comparisons of immature, embryonal-type HBs with the bulk of more differentiated, fetal-type tumors revealed a tight correlation between stage of hepatic maturation arrest and clinical behavior, notably vascular invasion and metastatic spread, and patients' survival.

**Molecular HB subclasses are determined by liver differentiation stages**

In this study, expression-based classification of HB was achieved through a highly reliable statistical method combining different unsupervised hierarchical clustering approaches. This method led to the selection of two robust tumor subgroups, and this robustness was confirmed using a new, independent set of samples and 16 relevant genes discriminating these tumor subgroups. These results demonstrated that the most significant differences between HB subclasses can be ascribed to distinct hepatic differentiation stages, as defined by comparison with expression profiles of mouse livers at early (E11.5- E12.5) and late (E14.5-E18.5) embryonic stages. These studies also provide biological relevance to early histologic classification that distinguished fetal and embryonal cells as major HB components (Weinberg and Finegold, 1983). The C1 subclass recapitulates liver features at the latest stage of intrauterine life, both by expression profile and by mostly fetal morphologic patterns, while in the C2 subclass, transcriptional program and predominant embryonal histotype resemble earlier stages of liver development. Thus, despite frequent morphological heterogeneity in HB, these expression-based subclasses closely matched the histologic types found to be prevailing after microscopic examination of the entire tumor mass.

These results, showing that childhood liver tumors recapitulate programs of their developing counterpart, are in line with recent studies using cross-species comparisons. It has been demonstrated that clinically distinct medulloblastoma subtypes can be identified by their similarity with precise stages of murine cerebellar development (Kho et al., 2004). Evidence for conserved mechanisms between development and tumorigenesis was also obtained in Wilms' tumor, the embryonic kidney malignancy, which shares expression of stemness and imprinted genes with murine metanephric blastema (Dekel et al., 2006). It was noticed that HBs, like Wilms' tumors, exhibit robust overexpression of a number of paternally expressed genes like *DLK1, IGF2, PEG3*, and *PEG10* that are involved in growth induction processes and downregulated with differentiation during development.

Previous studies using stem cell markers and markers of hepatocytic and biliary lineages have described differential patterns among HB components that reflect sequential stages of liver development (Schnater et al., 2003). The present data extent these observations, and indicate that immature C2-type tumor cells evoke hepatic cancer progenitor cells, with distinctive overexpression of highly relevant markers such as cytokeratin 19 and Ep-CAM (Roskams, 2006). Recently, embryonic stem/progenitor cells have been isolated from human fetal livers, either by enrichment of blast-like cells in primary hepatoblast cultures or by immunoselection of Ep-CAM-positive epithelial cells (Dan et al., 2006; Schmelzer et al., 2007). These cell lines have self-renewal capacity and can differentiate into mature hepatocytes and cholangiocytes, and one of them also gives rise to various mesenchymal lineages (Dan et al., 2006). Whether HBs arise from transformation of these cell types is presently unknown. As malignant mesenchymal derivatives are frequently admixed with epithelial tissues in HB, it is tempting to speculate that this tumor occurs from a multipotent progenitor harboring characteristics of mesenchymal-epithelial transitional cells. Moreover, since no significant differences in gene expression profiles was noted here between pure epithelial and mixed epithelial-mesenchymal HBs, tumor cells likely kept intrinsic capacities to undergo epithelial-mesenchymal transition.

A salient feature of immature HBs is the characteristic interplay of stemness and proliferation found in aggressive tumors (Glinsky et al., 2005). The C2-type expression profile was significantly enriched in hESC markers, including the mitotic cell cycle and spindle assembly checkpoint regulators cyclin B1, BUB1, BUB1 B, and Aurora kinases. These mitotic kinases are centrosomal proteins that ensure proper spindle assembly and faithful chromosome segregation in mitosis. Overexpression of these kinases or other components of the spindle checkpoint induces centrosome amplification and defects in chromosome segregation leading to chromosome number instability and aneuploidy (Marumoto et al., 2005; Zhou et al., 1998). Non-disjunctional events are involved in developmental syndromes (Hassold and Hunt, 2001), and might be responsible for increased rate of chromosomal imbalances evidenced here in C2-type HBs.

**Context-dependent transcriptional programs driven by Wnt signalling**

Mutational activation of β-catenin is a hallmark of HB, and accordingly, we found intracellular accumulation and nuclear localization of the protein in virtually all tumors, albeit with variable frequencies and intensities. Both immature and differentiated tumors overexpressed AXIN2 and DKK1, reflecting an attempt to activate a negative feedback loop aimed at limiting the Wnt signal. However, the two HB subtypes showed significant differences in β-catenin immunoexpression, illustrated by concomitant nuclear accumulation and decreased membranous localization of the protein in poorly differentiated, highly proliferative HBs. Heterogeneous distribution of nuclear β-catenin within colorectal tumors has been linked to different levels of Wnt signaling activity, resulting from differential combinations of autocrine and paracrine factors (Fodde and Brabletz, 2007). Similarly, nuclear β-catenin might be related to the absence of membranous E-cadherin in immature HBs, as we reported previously (Wei et al., 2000), and to cross-talks with growth-stimulating pathways in less differentiated cells. In this context, increased dosage of Wnt signaling might induce migratory and invasive phenotype.

Major differences between the two HB subtypes were found here in expression levels of Wnt targets involved in liver functions. Recent studies have demonstrated that Wnt/β-catenin signaling governs liver metabolic zonation by controlling positively the perivenous gene expression program and negatively the periportal program (Benhamouche et al., 2006). In our study, overexpression of hepatic perivenous markers such as GLUL was prominent in differentiated HBs, while genes encoding periportal functions like GLS2 were downregulated. This profile is highly similar to those of human and murine HCCs expressing mutant β-catenin (Boyault et al., 2007; Stahl et al., 2005), and corresponds to an hepatic signature of Wnt target genes. Accordingly, the zonation-related profile was lessened in poorly differentiated HBs, and mutant β-catenin was found to activate a different, muscle-related expression program in the pediatric Wilms' tumor (Zirn et al., 2006).

**Clinical implications**

The clinical behavior of many human solid tumors has been related to their differentiation status and proliferative rate. We show that HB does not depart from this rule, with strong correlation of molecular subclasses linked to hepatic differentiation with clinical tumor stage and patient's outcome. This correlation was mainly determined by differences in invasive and metastatic phenotypes between the two subclasses, but not by differences in tumor localization or tumor extension across liver sections, which defines the preoperative staging (PRETEXT) utilized to evaluate tumor resectability (Perilongo et al., 2000). Major differences in expression profiles of the two molecular HB subtypes led us to elucidate a 16-gene signature that proved highly efficient in stratification of HBs as well as normal livers according to hepatic developmental stage. Most importantly, this classifier also discriminated aggressive tumors, exhibited powerful survival predictor capacities in pre-treatment biopsies and surgical specimens, and demonstrated strong prognostic relevance when confronted to current clinical criteria in multivariate analysis. Although immature HBs have been associated to worse clinical outcome as opposed to differentiated HBs (Weinberg and Finegold, 1983), frequent cellular heterogeneity has hampered the use of histopathologic criteria for defining risk groups, excepted for a minority of cases showing 'pure fetal' or SCUD types. The expression signature afforded here enables direct appraisal of the global degree of tumor cell maturation, allowing to bypass these difficulties. Thus, it can improve the outcome prediction and clinical management of hepatoblastoma, by identifying cases with increased risk of developing metastasis, or conversely, by avoiding unnecessary over-treatment.

In conclusion, the present application identifies a 16-gene signature that distinguishes two HB subclasses and that is able to discriminate invasive and metastatic hepatoblastomas, and predicts prognosis with high accuracy. The identification of this expression signature with dual capacities may be used in recognizing liver developmental stage and in predicting disease outcome. This signature can be applied to improve clinical management of pediatric liver cancer and develop novel therapeutic strategies, and is therefore relevant for therapeutic targeting of tumor progenitor populations in liver cancer.

### REFERENCES

Assou, S., Le Carrour, T., Tondeur, S., Strom, S., Gabelle, A., Marty, S., Nadal, L., Pantesco, V., Reme, T., Hugnot, J. P., et al. (2007). A meta-analysis of human embryonic stem cells transcriptome integrated into a web-based expression atlas. Stem Cells 25, 961-973.
Boyault, S., Rickman, D. S., de Reynies, A., Balabaud, C., Rebouissou, S., Jeannot, E., Herault, A., Saric, J., Belghiti, J., Franco, D., et al. (2007). Transcriptome classification of HCC is related to gene alterations and to new therapeutic targets. Hepatology 45, 42-52.
Finegold, M. J., Lopez-Terrada, D. H., Bowen, J., Washington, M. K., and Qualman, S. J. (2007). Protocol for the examination of specimens from pediatric patients with hepatoblastoma. Arch Pathol Lab Med 131, 520-529.
Fodde, R., and Brabletz, T. (2007). Wnt/beta-catenin signaling in cancer stemness and malignant behavior. Curr Opin Cell Biol 19, 150-158.
Glinsky, G. V., Berezovska, O., and Glinskii, A. B. (2005). Microarray analysis identifies a death-from-cancer signature predicting therapy failure in patients with multiple types of cancer. J Clin Invest 115, 1503-1521.
Hirschman, B. A., Pollock, B. H., and Tomlinson, G. E. (2005). The spectrum of APC mutations in children with hepatoblastoma from familial adenomatous polyposis kindreds. J Pediatr 147, 263-266.
Irizarry, R. A., Hobbs, B., Collin, F., Beazer-Barclay, Y. D., Antonellis, K. J., Scherf, U., and Speed, T. P. (2003). Exploration, normalization, and summaries of high density oligonucleotide array probe level data. Biostatistics 4, 249-264.
Lee, J. S., Heo, J., Libbrecht, L., Chu, I. S., Kaposi-Novak, P., Calvisi, D. F., Mikaelyan, A., Roberts, L. R., Demetris, A. J., Sun, Z., et al. (2006). A novel prognostic subtype of human hepatocellular carcinoma derived from hepatic progenitor cells. Nat Med 12, 410-416.
McLin, V. A., Rankin, S. A., and Zorn, A. M. (2007). Repression of Wnt/β-catenin signaling in the anterior endoderm is essential for liver and pancreas development. Development 134, 2207-2217.
Perilongo, G., Shafford, E., and Plaschkes, J. (2000). SIOPEL trials using preoperative chemotherapy in hepatoblastoma. Lancet Oncol 1, 94-100.
Rowland, J. M. (2002). Hepatoblastoma: assessment of criteria for histologic classification. Med Pediatr Oncol 39, 478-483.
Schnater, J. M., Kohler, S. E., Lamers, W. H., von Schweinitz, D., and Aronson, D. C. (2003). Where do we stand with hepatoblastoma? A review. Cancer 98, 668-678.
Taniguchi, K., Roberts, L. R., Aderca, I. N., Dong, X., Qian, C., Murphy, L. M., Nagorney, D. M., Burgart, L. J., Roche, P. C., Smith, D. I., et al. (2002). Mutational spectrum of beta-catenin, AXIN1, and AXIN2 in hepatocellular carcinomas and hepatoblastomas. Oncogene 21, 4863-4871.
Wei, Y., Fabre, M., Branchereau, S., Gauthier, F., Perilongo, G., and Buendia, M. A. (2000). Activation of beta-catenin in epithelial and mesenchymal hepatoblastomas. Oncogene 19, 498-504.

## Claims

1. Method to determine the gene expression profile on a biological sample, comprising:
a. assaying the expression of a set of genes in a sample previously obtained from a patient diagnosed for a liver tumor, wherein said set comprises from 2 to 16 genes or consists of 2 to 16 genes, said 2 to 16 genes being chosen in the group consisting in the alpha-fetoprotein (AFP), aldehyde dehydrogenase 2 (ALDH2), amyloid P component serum (APCS), apolipoprotein C-IV (APOC4), aquaporin 9 (AQP9), budding uninhibited by benzimidazoles 1 (BUB1), complement componant 1 (C1S), cytochrome p450 2E1 (CYP2E1), discs large homolog 7 (DLG7), dual specificity phosphatase 9 (DUSP9), E2F5 transcription factor (E2F5), growth hormone receptor (GHR), 4-hydroxyphenylpyruvase dioxygenase (DHP), immunoglogulin superfamily member 1 (IGSF1), Notchless homolog 1 (NLE1) and the ribosomal protein L10a (RPL10A) genes; and
b. determining the gene expression profile of said sample.

2. Method according to claim 1, which further comprises determining the grade of the liver tumor providing the sample, for example by comparing the obtained gene expression profile of said sample to the gene expression profile of a reference sample or to the gene expression profiles of a collection of reference samples

3. Method according to claim 1 or 2, wherein the assay of the expression of said set of genes comprises a step of detecting nucleotide targets, wherein each nucleotide target is a product resulting from the expression of one of the genes in said set.

4. Method according to claim 2, wherein said nucleotide targets are mRNA.

5. Method according to any one of claims 1 to 4, wherein the assay of the expression of said set of genes comprises an amplification step, such as performed by qualitative polymerase chain reaction prior to a step of detecting the mRNA of each gene of said set.

6. Method according to any one of claims 1 to 5, wherein the assay of the expression of said set of genes comprises a hybridization step, such as one performed by hybridization on a solid or liquid support, especially on an array, prior to a step of detecting the mRNA of each gene of said set.

7. Method according to any one of claims 2 to 5, wherein said detected nucleotide targets are quantified with respect to at least one nucleotide target, expression product of an invariant gene, such as ACTG1, EFF1A1, PNN and RHOT2 genes.

8. Method according to any one of claims 1 to 7, wherein said liver tumor is a hepatoblastoma (HB) or a hepatocellular carcinoma (HCC).

9. Method according to any one of claims 2 to 8, wherein said method comprises, before step a., the preparation of said nucleotide targets from the sample.

10. Method according to any one of claims 1 to 9, wherein said set of genes comprises or consists in a set chosen in the group consisting of:
(a) E2F5 and HPD genes;
(b) APCS, BUB1, E2F5, GHR and HPD genes;
(c) ALDH2, APCS, APOC4, BUB1, C1 S, CYP2E1, E2F5, GHR and HPD genes;
(d) ALDH2, APCS, APOC4, AQP9, BUB1, C1S, DUSP9, E2F5 and RPL10A genes;
(e) ALDH2, APCS, APOC4, AQP9, C1S, CYP2E1, E2F5, GHR, IGSF1 and RPL10A genes; and
(f) AFP, ALDH2, APCS, APOC4, AQP9, BUB1, C1S, CYP2E1, DLG7, DUSP9, E2F5, GHR, HPD, IGSF1, NLE1 and RPL10A genes.

11. Kit, suitable to carry out the method as defined in any one of claims 1 to 10, comprising
a. a plurality of pairs of primers specific for a set of genes to be assayed, said set comprising 2 to 16 genes or consisting of 2 to 16 genes, said 2 to 16 genes being chosen in the group consisting of AFP, ALDH2, APCS, APOC4, AQP9, BUB1, C1S, CYP2E1, DLG7, DUSP9, E2F5, GHR, HPD, IGSF1, NLE1 and RPL10A genes; and
b. optionally reagents necessary for the amplification of the nucleotide targets of these genes by said primers, and optionally reagents for detecting the amplification products.

12. Kit according to claim 11, wherein each primer is 10 to 30 bp in length and has at least 80% similarity with its complementary sequence in the nucleotide target, preferably 100%.

13. Kit according to claim 11 or 12, wherein said pairs of primers are chosen in the group consisting of:
| **gene** | **Forward primer (5'-3')** | **Reverse primer (5'-3')** |
|---|---|---|
| AFP | AACTATTGGCCTGTGGCGAG | TCATCCACCACCAAGCTGC |
| ALDH2 | GTTTGGAGCCCAGTCACCCT | GGGAGGAAGCTTGCATGATTC |
| APCS | GGCCAGGAATATGAACAAGCC | CTTCTCCAGCGGTGTGATCA |
| APOC4 | GGAGCTGCTGGAGACAGTGG | TTTGGATTCGAGGAACCAGG |
| AQP9 | GCTTCCTCCCTGGGACTGA | CAACCAAAGGGCCCACTACA |
| BUB1 | ACCCCTGAAAAAGTGATGCCT | TCATCCTGTTCCAAAAATCCG |
| C1S | TTGTTTGGTTCTGTCATCCGC | TGGAACACATTTCGGCAGC |
| CYP2E1 | CAACCAAGAATTTCCTGATCCAG | AAGAAACAACTCCATGCGAGC |
| DLG7 | GCAGGAAGAATGTGCTGAAACA | TCCAAGTCTTTGAGAAGGGCC |
| DUSP9 | CGGAGGCCATTGAGTTCATT | ACCAGGTCATAGGCATCGTTG |
| E2F5 | CCATTCAGGCACCTTCTGGT | ACGGGCTTAGATGAACTCGACT |
| GHR | CTTGGCACTGGCAGGATCA | AGGTGAACGGCACTTGGTG |
| HPD | ATCTTCACCAAACCGGTGCA | CCATGTTGGTGAGGTTACCCC |
| IGSF1 | CACTCACACTGAAAAACGCCC | GGGTGGAGCAATTGAAAGTCA |
| NLE1 | ATGTGAAGGCCCAGAAGCTG | GAGAACTTCGGGCCGTCTC |
| RPL10A | TATCCCCCACATGGACATCG | TGCCTTATTTAAACCTGGGCC |
and,
a modified group of primers with respect to the above, wherein one or more primer(s) is modified, provided said primer(s) has at least 80% similarity with its non-modified version above.

14. A set of probes, suitable to carry out the method as defined in any one of claims 1 to 10, comprising a plurality of probes specific for a set of genes to assay, said set comprising or having from 2 to 16 genes, said 2 to 16 genes being chosen in the group consisting of AFP, ALDH2, APCS, APOC4, AQP9, BUB1, C1S, CYP2E1, DLG7, DUSP9, E2F5, GHR, HPD, IGSF1, NLE1 and RPL10A genes.

15. A set of probes according to claim 14, wherein said probes are 50 to 200 bp in length and have at least 80% similarity to the complementary sequence of the nucleotide target of the gene, preferably 100%.

16. **A** solid support, especially an array comprising a set of probes as defined in claims 14 or 15 linked to a support.

17. A composition comprising a set of probes as defined in claim 14 or 15, in solution.

18. A kit comprising a set of probes as defined in claim 14 or 15, a solid support as defined in claim 16 or a composition as defined in claim 17, and optionally reagents necessary for the hybridization of said nucleotide targets to said probes.

19. Set of probes, solid support, arrays, compositions or kits according to any one of claims 11 to 18, suitable for assaying a set of genes which comprises or consists in a set chosen in the group consisting of:
(a) E2F5 and HPD genes;
(b) APCS, BUB1, E2F5, GHR and HPD genes;
(c) ALDH2, APCS, APOC4, BUB1, C1 S, CYP2E1, E2F5, GHR and HPD genes;
(d) ALDH2, APCS, APOC4, AQP9, BUB1, C1S, DUSP9, E2F5 and RPL10A genes;
(e) ALDH2, APCS, APOC4, AQP9, C1S, CYP2E1, E2F5, GHR, IGSF1 and RPL10A genes; and
(f) AFP, ALDH2, APCS, APOC4, AQP9, BUB1, C1S, CYP2E1, DLG7, DUSP9, E2F5, GHR, HPD, IGSF1, NLE1 and RPL10A genes.

20. **Use** of a set of probes, solid support, arrays, compositions or kits according to any one of claims 11 to 18, to determine the grade of a liver tumor in a sample obtained from a patient.

21. Use of a set of probes, arrays, compositions or kits according to any one of claims 11 to 18, to determine, in a patient, the risk of developing metastasis.

22. Use of a set of probes, arrays, compositions or kits according to any one of claims 11 to 18, to define the therapeutic regimen to apply to said patient.
